(19) Europäisches Patentamt
European Patent Office
Office européen des brevets

(11) EP 3 808 766 A1

# (12) EUROPEAN PATENT APPLICATION

(43) Date of publication:
**21.04.2021 Bulletin 2021/16**

(21) Application number: **19306341.9**

(22) Date of filing: **15.10.2019**

(51) Int Cl.:
***C07K 14/725*** (2006.01) ***C07K 14/705*** (2006.01)
***C07K 16/28*** (2006.01) ***A61P 1/00*** (2006.01)
***A61P 17/06*** (2006.01) ***A61P 19/02*** (2006.01)
***A61P 37/02*** (2006.01) *C12N 5/0783* (2010.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Sangamo Therapeutics France**
**06560 Valbonne Sophia Antipolis (FR)**

(72) Inventors:
- **ABEL, Tobias**
  **06600 Antibes (FR)**
- **FENARD, David**
  **06600 Antibes (FR)**
- **GERTNER-DARDENNE, Julie**
  **06130 Grasse (FR)**

(74) Representative: **Icosa**
**83 avenue Denfert-Rochereau**
**75014 Paris (FR)**

## (54) CHIMERIC ANTIGEN RECEPTOR SPECIFIC FOR INTERLEUKIN-23 RECEPTOR

(57) The present invention relates to a chimeric antigen receptor (CAR) specific for an IL-23 receptor, and to a nucleic acid encoding the same. The present invention further relates to a T cell expressing said CAR, and to the use thereof for treating an autoimmune and/or inflammatory disease or disorder. The preferred CAR has an scFv fragment specific for IL23R, the CD8 extracellular hinge domain, the 4-1BB transmembrane domain and the intracellular signalling domains of 4-1BB and CD3zeta.

EP 3 808 766 A1

## Description

### FIELD OF INVENTION

The present invention relates to the field of immunotherapy. In particular, the present invention relates to a chimeric antigen receptor (CAR) specific for interleukin-23 receptor and comprising a transmembrane domain derived from 4-1BB, to T cells expressing said CAR and to the use thereof for treating an autoimmune and/or inflammatory disease or disorder.

### BACKGROUND OF THE INVENTION

The interleukin-23 (IL-23), a member of IL-12 cytokine family, is composed of two subunits, p19 and p40. The receptor for IL-23 (IL-23R) consists of an IL-23Rα subunit in complex with an IL-12Rβ1 subunit, which is a common subunit for the IL-12 receptor and interacts with Tyrosine kinase 2 (Tyk2). The IL-23R is associated constitutively with Janus Kinase 2 (JAK2) and associates with STAT3 and STAT4 upon its activation by IL-23.
The IL-23R is mainly expressed on immune cells, in particular T cells (*e.g.*, Th17 and γδ T cells), macrophages, dendritic cells and NK cells (Duvallet et al., 2011). It has been recently shown that non-activated neutrophils express a basal amount of IL-23R and that IL-23R expression is increased upon cell activation (Chen et al., 2016).
IL-23R activation by IL-23 induces IL-23R phosphorylation as well as the recruitment of STAT3 and STAT4 forming homodimers that, once phosphorylated, translocate to nucleus and consequently induce the expression of the transcription factor RORγt. RORγt, in turn, activates the transcription of downstream pro-inflammatory cytokines such as IL-17A, IL-17F, IL-22, IL-6 and IFN-γ that are involved in the activation of the immune response (Razawy et al., 2018, Sivanesan et al., 2016 and Duvallet et al., 2011). In particular, the IL-23/IL-23R signaling pathway has been described as critical for promoting the proliferation and the differentiation of IL-17-secreting immune cells, in particular $CD4^+$ Th17 cells and γδ T cells.
In the art, the expression of IL23R has been described as a common feature of pathogenic inflammatory cells involved in the appearance and maintenance of autoimmune diseases and chronic inflammation. Expression of IL23R at the surface is induced by IL23 exposure, and depends on inflammation levels. In human cells, whereas expression of IL23R at the RNA level is well documented, little was shown regarding the presence of the protein on the cell surface, and thus regarding its availability for a targeting agent.
The inhibition of IL-23/IL-23R signaling pathway has been investigated as a possible therapeutic approach for autoimmune and inflammatory diseases or disorders. In particular, Ustekinumab, an antibody directed to IL-12p40 subunit, has been tested with successful results on non- responsive patients with Crohn's disease and on patients with psoriasis (Fegan et al., 2016, Mease et al., 2015). Ustekinumab has been approved as a treatment for psoriasis in the United States. However, a case report revealed that psoriatic arthritis had been worsen in 4 patients following the treatment with Ustekinumab (Simon et al., 2016).
In addition, for a long-term treatment of autoimmune and/or inflammatory diseases or disorders, induction of tolerance is required, which may not be obtained when targeting the cytokine.
There is consequently still a need for a method for inhibiting the IL-23/IL-23R signaling pathway, thereby treating autoimmune and/or inflammatory diseases or disorders, that does not present the drawbacks of the method of the prior art.
The applicant herein provides a chimeric antigen receptor (CAR) directed to IL-23R and comprising a transmembrane domain derived from 4-1BB. The present invention further relates to a Treg cell population expressing said IL-23R CAR. Said Treg cell population may in particular be used for treating diseases or disorders related to IL-23R expressing cells, in particular autoimmune and/or inflammatory diseases or disorders.

### SUMMARY

The present invention relates to a chimeric antigen receptor (CAR) specific for at least one IL-23 receptor (IL-23R), wherein said CAR comprises:

(i) an extracellular binding domain, wherein said binding domain binds to said IL-23R,
(ii) optionally an extracellular hinge domain,
(iii) a transmembrane domain derived from 4-1BB,
(iv) an intracellular signaling domain, and,
(v) optionally a tag and/or a leader sequence.

In one embodiment, the extracellular binding domain comprises a scFv fragment directed against said IL-23R.
In one embodiment, the extracellular binding domain comprises a scFv fragment directed against said IL-23R, wherein said scFv comprises :

a heavy chain variable domain (VH) having the sequence of SEQ ID NO: 1 or a sequence having at least about 70% identity to SEQ ID NO: 1,

a light chain variable domain (VL) having a sequence selected from the group consisting of SEQ ID NOs: 2, 3 and 4 and sequences having at least about 70% identity to SEQ ID NO: 2, 3 or 4, and

optionally a linker between the VH and the VL,

preferably wherein said scFv has the sequence SEQ ID NO: 11 or a sequence having at least about 70% identity to SEQ ID NO: 11.

In one embodiment, the hinge domain is a hinge region of human CD8, preferably having a sequence of SEQ ID NO: 20 or SEQ ID NO: 60, or a sequence having at least about 70% identity to SEQ ID NO: 20 or SEQ ID NO: 60.

In one embodiment, the transmembrane domain is a transmembrane domain derived from the human 4-1BB, preferably having the sequence of SEQ ID NO: 28 or a sequence having at least about 70% identity to SEQ ID NO: 28.

In one embodiment, the intracellular signaling domain comprises a costimulatory signaling domain of human 4-1BB, preferably having the sequence of SEQ ID NO: 36 or a sequence having at least about 70% identity to SEQ ID NO: 36 and a T cell primary signaling human CD3 zeta, preferably having the sequence of SEQ ID NO: 31 or a sequence having at least 70% identity to SEQ ID NO: 31.

In one embodiment, the CAR comprises a transmembrane domain derived from the human 4-1BB contiguous to a costimulatory signaling domain of human 4-1BB, the transmembrane and costimulatory signaling domains of 4-1BB having the sequence SEQ ID NO: 53.

In one embodiment, the CAR comprises

(i) an anti-IL-23R scFv, preferably comprising a VH having the sequence of SEQ ID NO: 1 and a VL having the sequence of SEQ ID NO: 2, linked by a $(G_4S)_3$ linker (SEQ ID NO: 9),
(ii) a hinge domain derived from CD8$\alpha$, preferably SEQ ID NO: 20 or SEQ ID NO: 60,
(iii) a human 4-1BB transmembrane domain, preferably SEQ ID NO: 28,
(iv) an intracellular signaling domain comprising a human 4-1BB signaling domain, preferably SEQ ID NO: 36 and a human CD3 zeta domain, preferably SEQ ID NO: 31, and
(v) optionally a tag and/or a leader sequence.

The present invention further relates to a nucleic acid sequence encoding a CAR as described hereinabove.

The present invention further relates to a vector comprising the nucleic acid sequence as described hereinabove.

Another object of the present invention is a T cell population, engineered to express on the cell surface a CAR as described hereinabove.

In one embodiment, said T cell population is a regulatory T cell population, preferably said regulatory T cell population is selected from the group consisting of $CD4^+CD25^+Foxp3^+$ Treg, Tr1 cells, TGF-$\beta$ secreting Th3 cells, regulatory NKT cells, regulatory $\gamma\delta$ T cells, regulatory $CD8^+$ T cells, and double negative regulatory T cells.

The present invention further relates to a composition comprising at least one T cell population engineered to express on the cell surface a CAR as described hereinabove, said composition being preferably a pharmaceutical composition further comprising at least one pharmaceutically acceptable excipient or carrier.

Another object of the present invention is an *ex vivo* method for obtaining a T cell population engineered to express on the cell surface a CAR as described hereinabove, wherein said *ex vivo* method comprises the genetic modification, preferably the transduction, of at least one T cell with a nucleic acid encoding an IL-23R-CAR, and optionally an expansion step of the transduced cells.

The present invention further relates to a T cell population as described hereinabove, or to a composition as described hereinabove, for use in treating an IL-23R expressing cell-mediated disease or disorder in a subject in need thereof, wherein said IL-23R expressing cell-mediated disease or disorder is preferably an autoimmune and/or inflammatory disease and/or disorder.

In one embodiment, said autoimmune and/or inflammatory disease and/or disorder is selected from the group consisting of inflammatory bowel diseases (such as, for example, Crohn's disease and ulcerative colitis), systemic lupus erythematosus, rheumatoid arthritis, juvenile idiopathic arthritis, Sjogren syndrome, systemic sclerosis, ankylosing spondylitis, Type 1 diabetes, autoimmune thyroid disorders, multiple sclerosis, Myasthenia Gravis, psoriasis, psoriatic arthritis and uveitis, preferably wherein said autoimmune and/or inflammatory disease and/or disorder is Crohn's disease.

## DEFINITIONS

In the present invention, the following terms have the following meanings:

- The terms "a" and "an" refer to one or to more than one (*i.e.*, to at least one) of the grammatical object of the article. By way of example, "an element" means one element or more than one element.

- The term "about" when referring to a measurable value such as an amount, a temporal duration, and the like, is meant to encompass variations of ±20% or in some instances ±10%, or in some instances ±5%, or in some instances ±1%, or in some instances ±0.1% from the specified value, as such variations are appropriate to perform the disclosed methods.

- The term "activation" as used herein, refers to the state of a T cell (*e.g.,* a regulatory T cell) that has been sufficiently stimulated to induce a detectable cellular response. Activation can also be associated with detectable effector function(s) such as cytokine production or suppressive activity. The term "activated" regulatory T cells refers to, among other things, regulatory T cells that are capable of suppressing an immune response.

- The term "antibody", as used herein, refers to a protein, or polypeptide sequence derived from an immunoglobulin molecule which specifically binds with an antigen. Antibodies can be polyclonal or monoclonal, multiple or single chain, or intact immunoglobulins, and may be derived from natural sources or from recombinant sources. The term "antibody" also includes multispecific antibodies (e.g., bispecific antibodies), and antibody fragments, so long as they exhibit the desired biological activity. Antibodies can be multimers of immunoglobulin molecules, such as tetramers of immunoglobulin molecules. The basic four-chain antibody unit is a heterotetrameric glycoprotein composed of two identical light (L) chains and two identical heavy (H) chains. The L chain from any vertebrate species can be assigned to one of two clearly distinct types, called kappa ([kappa]) and lambda ([lambda]), based on the amino acid sequences of their constant domains (CL). Depending on the amino acid sequence of the constant domain of their heavy chains (CH), immunoglobulins can be assigned to different classes or isotypes. There are five classes of immunoglobulins: IgA, IgD, IgE, IgG, and IgM, having heavy chains designated alpha ([alpha]), delta ([delta]), epsilon ([epsilon]), gamma ([gamma]) and mu ([mu]), respectively. The [gamma] and [alpha] classes are further divided into subclasses on the basis of relatively minor differences in CH sequence and function, e.g., humans express the following subclasses: IgG1, IgG2, IgG3, IgG4, IgA1, and IgA2. Each L chain is linked to an H chain by one covalent disulfide bond, while the two H chains are linked to each other by one or more disulfide bonds depending on the H chain isotype. Each H and L chain also has regularly spaced intrachain disulfide bridges. Each H chain has at the N-terminus, a variable domain (VH) followed by three constant domains (CH) for each of the [alpha] and [gamma] chains and four CH domains for [mu] and [epsilon] isotypes. Each L chain has at the N-terminus, a variable domain (VL) followed by a constant domain (CL) at its other end. The VL is aligned with the VH and the CL is aligned with the first constant domain of the heavy chain (CH1). Particular amino acid residues are believed to form an interface between the light chain and heavy chain variable domains. The pairing of a VH and a VL together forms a single antigen-binding site. An IgM antibody consists of five of the basic heterotetramer units along with an additional polypeptide called a J chain, and therefore, contains ten antigen-binding sites, while secreted IgA antibodies can polymerize to form polyvalent assemblages comprising 2-5 of the basic 4-chain units along with J chain. In the case of IgGs, the 4-chain unit is generally about 150,000 Daltons. For the structure and properties of the different classes of antibodies, see, *e.g.,* Basic and Clinical Immunology, 8th edition, Daniel P. Stites, Abba I. Terr and Tristram G. Parslow (eds.), Appleton & Lange, Norwalk, Conn., 1994, page 71, and Chapter 6.

- The term "monoclonal antibody" as used herein refers to an antibody obtained from a population of substantially homogeneous antibodies, *i.e.*, the individual antibodies comprised in the population are identical except for possible naturally occurring mutations that may be present in minor amounts. Monoclonal antibodies are highly specific, being directed against a single antigenic site. Furthermore, in contrast to polyclonal antibody preparations that include different antibodies directed against different determinants (epitopes), each monoclonal antibody is directed against a single determinant on the antigen. In addition to their specificity, the monoclonal antibodies are advantageous in that they may be synthesized uncontaminated by other antibodies. The modifier "monoclonal" is not to be construed as requiring production of the antibody by any particular method. For example, a monoclonal antibody may be prepared by the hybridoma methodology first described by Kohler et al., Nature, 256:495 (1975), or may be made using recombinant DNA methods in bacterial, eukaryotic animal or plant cells (see, *e.g.*, U.S. Pat. No. 4,816,567). A "monoclonal antibody" may also be isolated from phage antibody libraries using the techniques described in Clackson et al., Nature, 352:624-628 (1991) and Marks et al., J. Mol. Biol., 222:581-597 (1991), for example. The monoclonal antibodies herein include "chimeric" antibodies.

- The term "antibody fragment" refers to at least one portion of an intact antibody, preferably the antigen binding region or variable region of the intact antibody, that retains the ability to specifically interact with (*e.g.*, by binding, steric hindrance, stabilizing/destabilizing, spatial distribution) an epitope of an antigen. Examples of antibody fragments include, but are not limited to, Fab, Fab', $F(ab')_2$, Fv fragments, scFv antibody fragments, disulfide-linked Fvs (sdFv), a Fd fragment consisting of the VH and CHI domains, linear antibodies, single domain antibodies such as sdAb (either VL or VH), camelid VHH domains, multi-specific antibodies formed from antibody fragments such as a bivalent fragment comprising two Fab fragments linked by a disulfide bridge at the hinge region, and an isolated CDR or other epitope binding fragments of an antibody. An antigen binding fragment can also be incorporated into single domain antibodies, maxibodies, minibodies, nanobodies, intrabodies, diabodies, triabodies, tetrabodies, a v-NAR and a bis-scFv (see, *e.g.,* Hollinger and Hudson, Nature Biotechnology 23:1126-1136, 2005). Antigen binding fragments can also be grafted into scaffolds based on polypeptides such as a fibronectin type III (see U.S. Patent No. 6,703,199, which describes fibronectin polypeptide minibodies). Papain digestion of antibodies produces two identical antigen-binding fragments, called "Fab" fragments, and a residual "Fc" fragment, a designation reflecting the ability to crystallize readily. The Fab fragment consists of an entire L chain along with the variable region domain of the H chain (VH), and the first constant domain of one heavy chain (CH1). Each Fab fragment is monovalent with respect to antigen binding, *i.e.*, it has a single antigen-binding site. Pepsin treatment of an antibody yields a single large $F(ab')_2$ fragment that roughly corresponds to two disulfide linked Fab fragments having divalent antigen-binding activity and is still capable of crosslinking antigen. Fab' fragments differ from Fab fragments by having additional few residues at the carboxy terminus of the CH1 domain including one or more cysteines from the antibody hinge region. Fab'-SH is the designation herein for Fab' in which the cysteine residue(s) of the constant domains bear a free thiol group. $F(ab')_2$ antibody fragments originally were produced as pairs of Fab' fragments that have hinge cysteines between them. Other chemical couplings of antibody fragments are also known.

- The "Fc" fragment of an antibody comprises the carboxy-terminal portions of both H chains held together by disulfides. The effector functions of antibodies are determined by sequences in the Fc region, which region is also the part recognized by Fc receptors (FcR) found on certain types of cells.

- "Fv" is the minimum antibody fragment that contains a complete antigen-recognition and -binding site. This fragment consists of a dimer of one heavy- and one light-chain variable region domain in tight, non-covalent association. From the folding of these two domains emanate six hypervariable loops (three loops each from the H and L chain) that contribute the amino acid residues for antigen binding and confer antigen binding specificity to the antibody. However, even a single variable domain (or half of an Fv comprising only three CDRs specific for an antigen) has the ability to recognize and bind antigen, although at a lower affinity than the entire binding site.

- The term "scFv" refers to a fusion protein comprising at least one antibody fragment comprising a variable region of a light chain and at least one antibody fragment comprising a variable region of a heavy chain, wherein the light and heavy chain variable regions are contiguously linked, *e.g.*, via a synthetic linker, *e.g.*, a short flexible polypeptide linker, and capable of being expressed as a single chain polypeptide, and wherein the scFv retains the specificity of the intact antibody from which it is derived. Unless specified, as used herein an scFv may have the VL and VH variable regions in either order, e.g., with respect to the N-terminal and C-terminal ends of the polypeptide, the scFv may comprise VL-linker-VH or may comprise VH-linker-VL.

- An "intact" antibody is one which comprises an antigen-binding site as well as a CL and at least heavy chain constant domains, CH1, CH2 and CH3. The constant domains may be native sequence constant domains (e.g., human native sequence constant domains) or amino acid sequence variants thereof. A "native sequence" polynucleotide is one that has the same nucleotide sequence as a polynucleotide derived from nature. A "native sequence" polypeptide is one that has the same amino acid sequence as a polypeptide (*e.g.*, antibody) derived from nature (*e.g.*, from any species). Such native sequence polynucleotides and polypeptides can be isolated from nature or can be produced by recombinant or synthetic means.

- The term "antibody heavy chain", refers to the larger of the two types of polypeptide chains present in antibody molecules in their naturally occurring conformations, and which normally determines the class to which the antibody belongs.

- The term "antibody light chain", refers to the smaller of the two types of polypeptide chains present in antibody molecules in their naturally occurring conformations. Kappa (K) and lambda ($\lambda$) light chains refer to the two major antibody light chain isotypes.

- The term "antigen" or "Ag" refers to a molecule that provokes an immune response. This immune response may involve either antibody production, and/or the activation of specific immunologically-competent cells, or both. The skilled artisan will understand that any macromolecule, including virtually all proteins or peptides, can serve as an antigen. Furthermore, antigens can be derived from recombinant or genomic DNA. A skilled artisan will understand that any DNA, which comprises a nucleotide sequence or a partial nucleotide sequence encoding a protein that elicits an immune response therefore encodes an "antigen" as that term is used herein. Furthermore, one skilled in the art will understand that an antigen does not necessarily need to be encoded solely by a full-length nucleotide sequence of a gene. It is readily apparent that the present invention includes, but is not limited to, the use of partial nucleotide sequences of more than one gene and that these nucleotide sequences are arranged in various combinations to encode polypeptides that elicit the desired immune response. Moreover, a skilled artisan will understand that an antigen does not necessarily need to be encoded by a "gene" at all. It is readily apparent that an antigen can be synthesized or can be derived from a biological sample, or might be macromolecule besides a polypeptide. Such a biological sample can include, but is not limited to a tissue sample, a cell or a fluid with other biological components.

- The term "adnectin", also known as monobody, is well known in the art and refers to proteins designed to bind with high affinity and specificity to antigens. They belong to the class of molecules collectively called "antibody mimetics".

- The term "alphabody", as used herein, that may also be referred to as Cell-Penetrating Alphabodies, refers to a type of antibody mimetics consisting of small 10 kDa proteins engineered to bind to a variety of antigens. Alphabodies are able to reach and bind to intracellular protein targets.

- The term "affibody" is well known in the art and refers to affinity proteins based on a 58 amino acid residue protein domain, derived from one of the IgG binding domain of staphylococcal protein A.

- The term "affilin" is well known in the art and refers to artificial proteins designed to selectively bind antigens. They resemble antibodies in their affinity and specificity to antigens but not in structure which makes them a type of antibody mimetic.

- The term "anticalin" is well known in the art and refers to an antibody mimetic technology, wherein the binding specificity is derived from lipocalins. Anticalins may also be formatted as dual targeting protein, called Duocalins.

- The term "armadillo repeat protein-based scaffold", as used herein, refers to a type of antibody mimetics corresponding to artificial peptide binding scaffolds based on armadillo repeat proteins. Armadillo repeat proteins are characterized by an armadillo domain, composed of tandem armadillo repeats of approximately 42 amino acids, which mediates interactions with peptides or proteins.

- The term "atrimer" is well known in the art and refers to binding molecules for target protein that trimerize as a perquisite for their biological activity. They are relatively large compared to other antibody mimetic scaffolds.

- The term "avimer" is well known in the art and refers to an antibody mimetic technology

- The term "DARPins" (Designed Ankyrin Repeat Proteins) is well known in the art and refers to an antibody mimetic DRP (designed repeat protein) technology developed to exploit the binding abilities of non-antibody polypeptides.

- The term "diabody" refers to small antibody fragments prepared by constructing scFv fragments with short linkers (about 5-10 residues) between the VH and VL domains such that inter-chain but not intra-chain pairing of the V domains is achieved, resulting in a bivalent fragment, *i.e.*, fragment having two antigen binding sites. Bispecific diabodies are heterodimers of two "crossover" scFv fragments in which the VH and VL domains of the two antibodies are present on different polypeptide chains. Diabodies are described more fully in, for example, EP 0404097; WO 93/11161; and Holliger et al., Proc. Natl. Acad. Sci. USA, 90:6444-6448 (1993).

- The term "evasins" is well known in the art and refers to a class of chemokine-binding proteins.

- The term "fynomers" is well known in the art and refers to proteins that belong to the class of antibody mimetic. They are attractive binding molecules due to their high thermal stability and reduced immunogenicity.

- The term "knottin" (that may also be referred to as inhibitor cystine not), as used herein, refers to an antibody mimetic comprising a protein structural motif containing three disulfide bridges.

- The term "domain kunitz peptide" refers to a type of antibody mimetics, and is based on the active domains of proteins inhibiting the function of proteases.

- The term "nanobody" is well known in the art and refers to an antibody-derived therapeutic protein that contains the unique structural and functional properties of naturally-occurring heavy chain antibodies. These heavy chain antibodies contain a single variable domain (VHH) and two constant domains (CH2 and CH3).

- The term "unibody" is well known in the art and refers to an antibody fragment lacking the hinge region of IgG4 antibodies. The deletion of the hinge region results in a molecule that is essentially half the size of traditional IgG4 antibodies and has a univalent binding region rather than the bivalent biding region of IgG4 antibodies.

- The term "versabodies" is well known in the art and refers to another antibody mimetic technology. They are small proteins of 3-5 kDa with >15% cysteines, which form a high disulfide density scaffold, replacing the hydrophobic core the typical proteins have.

- The term "antigen presenting cell" or "APC" refers to an immune system cell such as an accessory cell (*e.g.*, a B-cell, a dendritic cell, and the like) that displays a foreign antigen complexed with major histocompatibility complexes (MHC's) on its surface. T-cells may recognize these complexes using their T-cell receptors (TCRs). APCs process antigens and present them to T-cells.

- The term "allogeneic" refers to any material derived from a different individual of the same specie as the individual to whom the material is introduced. Two or more individuals are said to be allogeneic to one another when the genes at one or more loci are not identical. In some aspects, allogeneic material from individuals of the same species may be sufficiently unlike genetically to interact antigenically.

- The term "autologous" refers to any material derived from the same individual to whom it is later to be re-introduced.

- The term "chimeric receptor or chimeric antigen receptor or CR or CAR" refers to one polypeptide or to a set of polypeptides, typically two in the simplest embodiments, which when in an immune cell, provides the cell with specificity for a target ligand and with intracellular signal generation. In some embodiments, the set of polypeptides are contiguous with each other. In some embodiments, the chimeric receptor is a chimeric fusion protein comprising the set of polypeptides. In some embodiments, the set of polypeptides include a dimerization switch that, upon the presence of a dimerization molecule, can couple the polypeptides to one another, e.g., can couple a ligand binding domain to an intracellular signaling domain. In one embodiment, the chimeric receptor comprises an optional leader sequence at the amino-terminus (N-ter) of the chimeric receptor fusion protein. In one embodiment, the chimeric receptor further comprises a leader sequence at the N-terminus of the extracellular ligand binding domain, wherein the leader sequence is optionally cleaved from the ligand binding domain during cellular processing and localization of the chimeric receptor to the cellular membrane.

- The term "conservative sequence modifications" refers to amino acid modifications that do not significantly affect or alter the biologic function of the protein containing the amino acid sequence. Such conservative modifications include amino acid substitutions, additions and deletions. Modifications can be introduced into a protein by standard techniques known in the art, such as site-directed mutagenesis and PCR-mediated mutagenesis. Conservative amino acid substitutions are ones in which the amino acid residue is replaced with an amino acid residue that has similar properties, such that one skilled in the art of peptide chemistry would expect the secondary structure and hydropathic nature of the polypeptide to be substantially unchanged. Amino acid substitutions are generally therefore based on the relative similarity of the amino acid side-chain substituents, for example, their hydrophobicity, hydrophilicity, charge, size, and the like. Exemplary substitutions that take various of the foregoing characteristics into consideration are well known to those of skill in the art and include: arginine and lysine; glutamate and aspartate; serine and threonine; glutamine and asparagine; and valine, leucine and isoleucine. Amino acid substitutions may further be made on the basis of similarity in polarity, charge, solubility, hydrophobicity, hydrophilicity and/or the amphipathic nature of the residues. For example, negatively charged amino acids include aspartic acid and glutamic acid; positively charged amino acids include lysine and arginine; and amino acids with uncharged polar head groups having similar hydrophilicity values include leucine, isoleucine and valine; glycine and alanine; asparagine and glutamine; and serine, threonine, phenylalanine and tyrosine. Other groups of amino acids that may represent conservative changes include: (1) ala, pro, gly, glu, asp, gln, asn, ser, thr; (2) cys, ser, tyr, thr; (3) val, ile, leu, met, ala, phe; (4) lys, arg, his; and (5) phe, tyr, trp, his. Other families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine, tryptophan), nonpolar side chains (e.g., alanine, valine, leucine, isoleucine, proline, phenylalanine, methionine), beta-branched side chains (e.g., threonine, valine, isoleucine) and aromatic side chains (e.g., tyrosine, phenylalanine, tryptophan, histidine). Thus, one or more amino acid residues within a chimeric receptor of the invention can be replaced with other amino acid residues from the same side chain family and the altered chimeric receptor can be tested using the functional assays described herein.

- The term "constitutive" promoter refers to a nucleotide sequence which, when operably linked with a polynucleotide which encodes or specifies a gene product, causes the gene product to be produced in a cell under most or all physiological conditions of the cell.

- The term a "costimulatory molecule" refers to a cognate binding partner on a T cell that specifically binds with a costimulatory ligand, thereby mediating a costimulatory response by the T cell, such as, but not limited to, proliferation. Costimulatory molecules are cell surface molecules other than antigen receptors or their ligands that contribute to an efficient immune response. A costimulatory signaling domain can be the intracellular portion of a costimulatory molecule. A costimulatory molecule can be represented in the following protein families: TNF receptor proteins, Immunoglobulin-like proteins, cytokine receptors, integrins, signaling lymphocytic activation molecules (SLAM proteins), and activating NK cell receptors.

- The term "derived from" as used herein, indicates a relationship between a first and a second molecule. It generally refers to structural similarity between the first molecule and a second molecule and does not connote or include a process or source limitation on a first molecule that is derived from a second molecule. For example, in the case of an intracellular signaling domain that is derived from a CD3 zeta molecule, the intracellular signaling domain retains sufficient CD3 zeta structure such that is has the required function, namely, the ability to generate a signal under the appropriate conditions. It does not connote or include a limitation to a particular process of producing the intracellular signaling domain, *e.g.*, it does not mean that, to provide the intracellular signaling domain, one must start with a CD3 zeta sequence and delete unwanted sequence, or impose mutations, to arrive at the intracellular signaling domain.

- The term "encoding" refers to the inherent property of specific sequences of nucleotides in a polynucleotide, such as a gene, a cDNA, or an mRNA, to serve as templates for synthesis of other polymers and macromolecules in biological processes having either a defined sequence of nucleotides (e.g., rRNA, tRNA and mRNA) or a defined sequence of amino acids and the biological properties resulting therefrom. Thus, a gene, cDNA, or RNA, encodes a protein if transcription and translation of mRNA corresponding to that gene produces the protein in a cell or other biological system. Both the coding strand, the nucleotide sequence of which is identical to the mRNA sequence and is usually provided in sequence listings, and the non-coding strand, used as the template for transcription of a gene or cDNA, can be referred to as encoding the protein or other product of that gene or cDNA. Unless otherwise specified, a "nucleotide sequence encoding an amino acid sequence" includes all nucleotide sequences that are degenerate versions of each other and that encode the same amino acid sequence. The phrase "nucleotide sequence that encodes a protein or a RNA" may also include introns to the extent that the nucleotide sequence encoding the protein may in some version contain an intron(s).

- The term "endogenous" refers to any material from or produced inside an organism, cell, tissue or system.

- The term "engineered" or "modified" refers to a cell that has been transfected, transformed or transduced.

- The term "exogenous" refers to any material introduced from or produced outside an organism, cell, tissue or system.

- The term "expression" refers to the transcription and/or translation of a particular nucleotide sequence driven by a promoter.

- The term "expression vector" refers to a vector comprising a recombinant polynucleotide comprising expression control sequences operatively linked to a nucleotide sequence to be expressed. An expression vector comprises sufficient cis-acting elements for expression; other elements for expression can be supplied by the host cell or in an *in vitro* expression system. Expression vectors include all those known in the art, including cosmids, plasmids (*e.g.*, naked or contained in liposomes) and viruses (*e.g.*, lentiviruses, retroviruses, adenoviruses, and adeno-associated viruses) that incorporate the recombinant polynucleotide.

- The term "homology" or "identity" refers to the subunit sequence identity between two polymeric molecules, *e.g.*, between two nucleic acid molecules, such as, two DNA molecules or two RNA molecules, or between two polypeptide molecules. When a subunit position in both of the two molecules is occupied by the same monomeric subunit; e.g., if a position in each of two DNA molecules is occupied by adenine, then they are homologous or identical at that position. The homology between two sequences is a direct function of the number of matching or homologous positions; *e.g.,* if half *(e.g.,* five positions in a polymer of ten subunits in length) of the positions in two sequences are homologous, the two sequences are 50% homologous; if 90% of the positions (*e.g*., 9 of 10), are matched or homologous, the two sequences are 90% homologous. Thus, the term "homologous" or "identical", when used in a relationship between the sequences of two or more polypeptides or of two or more nucleic acid molecules, refers to the degree of sequence relatedness between polypeptides or nucleic acid molecules, as determined by the number of matches between strings of two or more amino acid or nucleotide residues. "Identity" measures the percent of identical matches between the smaller of two or more sequences with gap alignments (if any) addressed by a particular mathematical model or computer program (*i.e*., "algorithms"). Identity of related polypeptides can be readily calculated by known methods. Such methods include, but are not limited to, those described in Computational Molecular Biology, Lesk, A. M., ed., Oxford University Press, New York, 1988; Biocomputing: Informatics and Genome Projects, Smith, D. W., ed., Academic Press, New York, 1993; Computer Analysis of Sequence Data, Part 1, Griffin, A. M., and Griffin, H. G., eds., Humana Press, New Jersey, 1994; Sequence Analysis in Molecular Biology, von Heinje, G., Academic Press, 1987; Sequence Analysis Primer, Gribskov, M. and Devereux, J., eds., M. Stockton Press, New York, 1991; and Carillo et al., SIAM J. Applied Math. 48, 1073 (1988). Preferred methods for determining identity are designed to give the largest match between the sequences tested. Methods of determining identity are described in publicly available computer programs. Preferred computer program methods for determining identity between two sequences include the GCG program package, including GAP (Devereux et al., Nucl. Acid. Res. \2, 387 (1984); Genetics Computer Group, University of Wisconsin, Madison, Wis.), BLASTP, BLASTN, and FASTA (Altschul et al., J. Mol. Biol. 215, 403-410 (1990)). The BLASTX program is publicly available from the National Center for Biotechnology Information (NCBI) and other sources (BLAST Manual, Altschul et al. NCB/NLM/NIH Bethesda, Md. 20894; Altschul et al., supra). The well-known Smith Waterman algorithm may also be used to determine identity.

- The terms "humanized" forms of non-human (e.g., murine) antibodies refer to chimeric immunoglobulins, immunoglobulin chains or fragments thereof (such as Fv, Fab, Fab', $F(ab')_2$ or other antigen-binding subsequences of antibodies) which contain minimal sequence derived from non-human immunoglobulin. For the most part, humanized antibodies and antibody fragments thereof are human immunoglobulins (recipient antibody or antibody fragment) in which residues from a complementary-determining region (CDR) of the recipient are replaced by residues from a CDR of a non-human species (donor antibody) such as mouse, rat or rabbit having the desired specificity, affinity, and capacity. In some instances, Fv framework region (FR) residues of the human immunoglobulin are replaced by corresponding non-human residues. Furthermore, a humanized antibody/antibody fragment can comprise residues which are found neither in the recipient antibody nor in the imported CDR or framework sequences. These modifications can further refine and optimize antibody or antibody fragment performance. In general, the humanized antibody or antibody fragment thereof will comprise substantially all of at least one, and typically two, variable domains, in which all or substantially all of the CDR regions correspond to those of a non-human immunoglobulin and all or a significant portion of the FR regions are those of a human immunoglobulin sequence. The humanized antibody or antibody fragment can also comprise at least a portion of an immunoglobulin constant region (Fc), typically that of a human immunoglobulin. For further details, see Jones et al., Nature, 321: 522-525, 1986; Reichmann et al., Nature, 332: 323-329, 1988; Presta, Curr. Op. Struct. Biol., 2: 593-596, 1992.

- As used herein, *"in vitro* transcribed RNA" refers to RNA, preferably mRNA, that has been synthesized *in vitro.* Generally, the *in vitro* transcribed RNA is generated from an *in vitro* transcription vector. The *in vitro* transcription vector comprises a template that is used to generate the *in vitro* transcribed RNA.

- The term "inducible" promoter refers to a nucleotide sequence which, when operably linked with a polynucleotide which encodes or specifies a gene product, causes the gene product to be produced in a cell substantially only when an inducer which corresponds to the promoter is present in the cell.

- As used herein, a "5' cap" (also termed an RNA cap, an RNA 7-methylguanosine cap or an RNA m7G cap) is a modified guanine nucleotide that has been added to the "front" or 5' end of a eukaryotic messenger RNA shortly after the start of transcription. The 5' cap thus consists of a terminal group which is linked to the first transcribed nucleotide. Its presence is critical for recognition by the ribosome and protection from RNases. Cap addition is coupled to transcription, and occurs co-transcriptionally, such that each influences the other. Shortly after the start of transcription, the 5' end of the mRNA being synthesized is bound by a cap-synthesizing complex associated with RNA polymerase. This enzymatic complex catalyzes the chemical reactions that are required for mRNA capping. Synthesis proceeds as a multi-step biochemical reaction. The capping moiety can be modified to modulate functionality of mRNA such as its stability or efficiency of translation.

- In the context of the present invention, the following abbreviations for the commonly occurring nucleic acid bases are used. "A" refers to adenine, "C" refers to cytosine, "G" refers to guanine, "T" refers to thymine, and "U" refers to uracil.

- The term "instructional material" includes a publication, a recording, a diagram, or any other medium of expression which can be used to communicate the usefulness of the compositions and methods of the invention. The instructional material of the kit of the invention may, for example, be affixed to a container which contains the cell of the invention or be shipped together with a container which contains the cell of the invention. Alternatively, the instructional material may be shipped separately from the container with the intention that the instructional material cell be used cooperatively by the recipient.

- The term "intracellular signaling domain" as used herein, refers to an intracellular portion of a molecule. The intracellular signaling domain generates a signal that promotes an immune effector function of the chimeric receptor containing cell. Examples of immune effector function in a chimeric receptor-T cell may include cytolytic activity, suppressive activity, regulatory activity and helper activity, including the secretion of cytokines.

- The term "isolated" means altered or removed from the natural state. For example, a nucleic acid or a peptide naturally present in a living animal is not "isolated", but the same nucleic acid or peptide partially or completely separated from the coexisting materials of its natural state is "isolated". An isolated nucleic acid or peptide can exist in substantially purified form, or can exist in a non-native environment such as, for example, a host cell. Typically, a preparation of isolated nucleic acid or peptide contains the nucleic acid or peptide at least about 80% pure, at least about 85% pure, at least about 90% pure, at least about 95% pure, greater than 95% pure, greater than about 96% pure, greater than about 97% pure, greater than about 98% pure, or greater than about 99% pure. An "isolated nucleic acid" is a nucleic acid that is substantially separated from other genome DNA sequences as well as proteins or complexes such as ribosomes and polymerases, which naturally accompany a native sequence. The term embraces a nucleic acid sequence that has been removed from its naturally occurring environment, and includes recombinant or cloned DNA isolates and chemically synthesized analogues or analogues biologically synthesized by heterologous systems. An "isolated polypeptide" is one that has been identified and separated and/or recovered from a component of its natural environment.

- The term "lentivirus" refers to a genus of the Retroviridae family. Lentiviruses are unique among the retroviruses in being able to infect non-dividing cells; they can deliver a significant amount of genetic information into the DNA of the host cell, so they are one of the most efficient methods of a gene delivery vector. HIV, SIV, and FIV are all examples of lentiviruses.

- The term "lentiviral vector" refers to a vector derived from at least a portion of a lentivirus genome, including especially a self-inactivating lentiviral vector as provided in Milone et al., Mol. Ther. 17(8): 1453-1464 (2009). Other examples of lentivirus vectors that may be used in the clinic, include but are not limited to, the LENTIVECTOR® gene delivery technology from Oxford BioMedica, the LENTIMAX™ vector system from Lentigen and the like. Nonclinical types of lentiviral vectors are also available and would be known to one skilled in the art.

- The term "ligand" refers to a member of a pair ligand/receptor, and binds to the other member of the pair.

- The term "nucleic acid" or "polynucleotide" refers to a polymer of nucleotides covalently linked by phosphodiester bonds, such as deoxyribonucleic acids (DNA) or ribonucleic acids (RNA), in either single- or double-stranded form. Unless specifically limited, the term encompasses nucleic acids containing known analogues of natural nucleotides that have similar binding properties as the reference nucleic acid and are metabolized in a manner similar to naturally occurring nucleotides. Unless otherwise indicated, a particular nucleic acid sequence also implicitly encompasses conservatively modified variants thereof (e.g., degenerate codon substitutions), alleles, orthologs, SNPs, and complementary sequences as well as the sequence explicitly indicated. Specifically, degenerate codon substitutions may be achieved by generating sequences in which the third position of one or more selected (or all) codons is substituted with mixed-base and/or deoxyinosine residues (Batzer et al., Nucleic Acid Res. 19:5081 (1991); Ohtsuka et al., J. Biol. Chem. 260:2605-2608 (1985); and Rossolini et al., Mol. Cell. Probes 8:91-98 (1994)).

- The term "operatively linked" or "transcriptional control" refers to functional linkage between a regulatory sequence and a heterologous nucleic acid sequence resulting in expression of the latter. For example, a first nucleic acid sequence is operably linked with a second nucleic acid sequence when the first nucleic acid sequence is placed in a functional relationship with the second nucleic acid sequence. For instance, a promoter is operably linked to a coding sequence if the promoter affects the transcription or expression of the coding sequence. Operably linked DNA sequences can be contiguous with each other and, e.g., where necessary to join two protein coding regions, are in the same reading frame.

- The terms "peptide", "polypeptide", and "protein" are used interchangeably, and refer to a compound comprised of amino acid residues covalently linked by peptide bonds. A protein or peptide must contain at least two amino acids, and no limitation is placed on the maximum number of amino acids that can comprise a protein's or peptide's sequence. Polypeptides include any peptide or protein comprising two or more amino acids joined to each other by peptide bonds. As used herein, the term refers to both short chains, which also commonly are referred to in the art as peptides, oligopeptides and oligomers, for example, and to longer chains, which generally are referred to in the art as proteins, of which there are many types. "Polypeptides" include, for example, biologically active fragments, substantially homologous polypeptides, oligopeptides, homodimers, heterodimers, variants of polypeptides, modified polypeptides, derivatives, analogs, fusion proteins, among others. A polypeptide includes a natural peptide, a recombinant peptide, or a combination thereof.

- The term "pharmaceutically acceptable excipient" or "pharmaceutically acceptable carrier" refers to an excipient that does not produce an adverse, allergic or other untoward reaction when administered to an animal, preferably a human. It includes any and all solvents, dispersion media, coatings, antibacterial and antifungal agents, isotonic and absorption delaying agents and the like. For human administration, preparations should meet sterility, pyrogenicity, general safety and purity standards as required by regulatory offices, such as, for example, FDA Office or EMA.

- The term "poly(A)" refers to a series of adenosines monophosphate attached to the mRNA. In one embodiment of a construct for transient expression, the polyA is between 50 and 5000 adenosines monophosphate, preferably greater than or equal to 64, more preferably greater than or equal to 100, most preferably greater than or equal to 300 or 400 adenosines monophosphate. Poly(A) sequences can be modified chemically or enzymatically to modulate mRNA functionality such as localization, stability or efficiency of translation.

- The term "polyadenylation" refers to the covalent linkage of a polyadenylyl moiety, or its modified variant, to a messenger RNA molecule. In eukaryotic organisms, most messenger RNA (mRNA) molecules are polyadenylated at the 3' end. The 3' poly (A) tail is a long sequence of adenine nucleotides (often several hundred) added to the pre-mRNA through the action of an enzyme, polyadenylate polymerase. In higher eukaryotes, the poly(A) tail is added onto transcripts that contain a specific sequence, the polyadenylation signal. The poly(A) tail and the protein bound to it aid in protecting mRNA from degradation by exonucleases. Polyadenylation is also important for transcription termination, export of the mRNA from the nucleus, and translation. Polyadenylation occurs in the nucleus immediately after transcription of DNA into RNA, but additionally can also occur later in the cytoplasm. After transcription has been terminated, the mRNA chain is cleaved through the action of an endonuclease complex associated with RNA polymerase. The cleavage site is usually characterized by the presence of the base sequence AAUAAA near the cleavage site. After the mRNA has been cleaved, adenosine residues are added to the free 3' end at the cleavage site.

- The term "promoter" refers to a DNA sequence recognized by the synthetic machinery of the cell, or introduced synthetic machinery, required to initiate the specific transcription of a polynucleotide sequence.

- The term "promoter/regulatory sequence" refers to a nucleic acid sequence which is required for expression of a gene product operably linked to the promoter/regulatory sequence. In some instances, this sequence may be the core promoter sequence and in other instances, this sequence may also include an enhancer sequence and other regulatory elements which are required for expression of the gene product. The promoter/regulatory sequence may, for example, be one which expresses the gene product in a tissue specific manner.

- The term "recombinant protein or peptide" refers to a protein or peptide (*e.g.*, an antibody) which is generated using recombinant DNA technology, such as, for example, a protein or peptide (*e.g.*, an antibody) expressed by a bacteriophage or yeast expression system. The term should also be construed to mean a protein or peptide (*e.g.*, an antibody) which has been generated by the synthesis of a DNA molecule encoding the protein or peptide (*e.g.*, the antibody) and which DNA molecule expresses a protein or peptide (*e.g.*, an antibody), or an amino acid sequence specifying the protein or peptide (*e.g.*, the antibody), wherein the DNA or amino acid sequence has been obtained using recombinant DNA or amino acid sequence technology which is available and well known in the art.

- The term "signaling domain" refers to the functional portion of a protein which acts by transmitting information within the cell to regulate cellular activity via defined signaling pathways by generating second messengers or functioning as effectors by responding to such messengers.

- The term "signal transduction pathway" refers to the biochemical relationship between a variety of signal transduction molecules that play a role in the transmission of a signal from one portion of a cell to another portion of a cell.

- The term "specifically binds" refers to an antibody, or a ligand, which recognizes and binds with a binding partner present in a sample, but which antibody or ligand does not substantially recognize or bind other molecules in the sample.

- The term "stimulation" refers to a primary response induced by binding of a stimulatory molecule (*e.g.*, a TCR/CD3 complex or chimeric receptor) with its cognate ligand thereby mediating a signal transduction event, such as, but not limited to, signal transduction via the TCR/CD3 complex or signal transduction via signaling domains of the chimeric receptor. Stimulation can mediate altered expression of certain molecules.

- The term "stimulatory molecule" refers to a molecule expressed by an immune cell (*e.g.*, T cell, NK cell, B cell) that provides the cytoplasmic signaling sequence(s) that regulate activation of the immune cell in a stimulatory way for at least some aspect of the immune cell signaling pathway. In one aspect, the signal is a primary signal that is initiated by, for instance, binding of a TCR/CD3 complex with an MHC molecule loaded with peptide, and which leads to mediation of a T cell response, including, but not limited to, proliferation, activation, differentiation, and the like. A primary cytoplasmic signaling sequence (also referred to as a "primary signaling domain") that acts in a stimulatory manner may contain a signaling motif which is known as immunoreceptor tyrosine-based activation motif or ITAM.

- The term "subject" is intended to include living organisms in which an immune response can be elicited (e.g., mammals, human). In one embodiment, a subject may be a "patient", *i.e.*, a warm-blooded animal, more preferably a human, who/which is awaiting the receipt of, or is receiving medical care or was/is/will be the object of a medical procedure, or is monitored for the development of the targeted disease or condition, such as, for example, an inflammatory or autoimmune condition. In one embodiment, the subject is an adult (for example a subject above the age of 18). In another embodiment, the subj ect is a child (for example a subj ect below the age of 18). In one embodiment, the subject is a male. In another embodiment, the subject is a female. In one embodiment, the subject is affected, preferably is diagnosed, with an autoimmune and/or inflammatory disease or disorder, such as, for example an autoantibody-mediated autoimmune disease. In one embodiment, the subject is at risk of developing an autoimmune and/or inflammatory disease or disorder, such as, for example an autoantibody-mediated autoimmune disease. Examples of risks factor include, but are not limited to, genetic predisposition, or familial history of an autoimmune and/or inflammatory disease or disorder.

- The term "substantially purified cell" refers to a cell that is essentially free of other cell types. A substantially purified cell also refers to a cell which has been separated from other cell types with which it is normally associated in its naturally occurring state. In one embodiment, a substantially purified cell refers to a cell which is at least about 75% free, 80% free, or 85% free, and preferably about 90%, 95%, 96%, 97%, 98%, or 99% free, from other cell types with which it is normally associated in its naturally occurring state. In some instances, a population of substantially purified cells refers to a homogenous population of cells. In one embodiment, a population of substantially purified cells refers to a population of cells at least about 75% homogenous, 80% homogenous, or 85% homogenous, and preferably about 90%, 95%, 96%, 97%, 98%, or 99% homogenous. In other instances, this term refers simply to cells that have been separated from the cells with which they are naturally associated in their natural state. In some aspects, the cells are cultured *in vitro.* In other aspects, the cells are not cultured *in vitro.*

- The terms "therapeutically effective amount" refer to an amount of cells expressing a CAR as described herein, effective to achieve a particular biological result. Thus, the terms "therapeutically effective amount" mean a level or amount of agent that is aimed at, without causing significant negative or adverse side effects to the target, (1) delaying or preventing the onset of the targeted disease or condition; (2) slowing down or stopping the progression, aggravation, or deterioration of one or more symptoms of the targeted disease or condition; (3) bringing about ameliorations of the symptoms of the targeted disease or condition; (4) reducing the severity or incidence of the targeted disease or condition; or (5) curing the targeted disease or condition. A therapeutically effective amount may be administered prior to the onset of the targeted disease or condition, for a prophylactic or preventive action. Alternatively, or additionally, the therapeutically effective amount may be administered after initiation of the targeted disease or condition, for a therapeutic action.

- The term "transfected" or "transformed" or "transduced" refers to a process by which exogenous nucleic acid is transferred or introduced into the host cell. A "transfected" or "transformed" or "transduced" cell is one which has been transfected, transformed or transduced with exogenous nucleic acid. The cell includes the primary subject cell and its progeny.

- The term "transfer vector" refers to a composition of matter which comprises an isolated nucleic acid and which can be used to deliver the isolated nucleic acid to the interior of a cell. Numerous vectors are known in the art including, but not limited to, linear polynucleotides, polynucleotides associated with ionic or amphiphilic compounds, plasmids, and viruses. Thus, the term "transfer vector" includes an autonomously replicating plasmid or a virus. The term should also be construed to further include non-plasmid and non-viral compounds which facilitate transfer of nucleic acid into cells, such as, for example, a poly lysine compound, liposome, and the like. Examples of viral transfer vectors include, but are not limited to, adenoviral vectors, adeno-associated virus vectors, retroviral vectors, lentiviral vectors, and the like.

- As used herein, "transient" refers to expression of a non-integrated transgene for a period of hours, days or weeks, wherein the period of time of expression is less than the period of time for expression of the gene if integrated into the genome or contained within a stable plasmid replicon in the host cell.

- As used herein, the terms "treat", "treatment" and "treating" refer to the reduction or amelioration of the progression, severity and/or duration of a targeted disease or condition, *e.g.*, an autoimmune condition, or the amelioration of one or more symptoms (preferably, one or more discernible symptoms) of a targeted disease or condition, *e.g.*, an autoimmune condition, wherein said amelioration results from the administration of one or more therapies (*e.g.*, one or more therapeutic agents such as a Treg cell of the invention). In specific embodiments, the terms "treat", "treatment" and "treating" refer to the amelioration of at least one measurable physical parameter of a targeted disease or condition, *e.g.*, an autoimmune and/or inflammatory disease or disorder. In other embodiments, the terms "treat", "treatment" and "treating" refer to the inhibition of the progression of a targeted disease or condition, *e.g.*, an autoimmune and/or inflammatory disease or disorder, either physically by, *e.g.*, stabilization of a discernible symptom, physiologically by, *e.g.*, stabilization of a physical parameter, or both. In other embodiments, the terms "treat", "treatment" and "treating" refer to the reduction or amelioration of the progression, severity and/or duration of a targeted disease or condition, *e.g.*, an autoimmune and/or inflammatory disease or disorder, or the amelioration of one or more symptoms of a targeted disease or condition, *e.g.*, an autoimmune and/or inflammatory disease or disorder. "Treating" or "treatment" refers to both therapeutic treatment and prophylactic or preventative measures; wherein the object is to prevent or slow down (lessen) the targeted disease or condition. Those in need of treatment include those already with the condition as well as those prone to have the condition or those in whom the condition is to be prevented. A subject is successfully "treated" for a disease or condition if, after receiving a therapeutic amount of a population of cells comprising a chimeric receptor according to the present invention, the subject shows observable and/or measurable improvement in one or more of the following: reduction in the number of pathogenic cells; reduction in the percent of total cells that are pathogenic; relief to some extent of one or more of the symptoms associated with the specific condition; reduced morbidity and mortality, and/or improvement in quality of life issues. The above parameters for assessing successful treatment and improvement in the condition are readily measurable by routine procedures familiar to a physician.

- The term "Treg cell" refers to a cell capable of suppressing, inhibiting or preventing excessive or unwanted inflammatory responses, such as, for example, autoimmunity or allergic reactions. In one embodiment, the Treg cell population of the invention is capable of suppressive activity. In one embodiment, said suppressive activity is contact independent. In another embodiment, said suppressive activity is contact dependent. In one embodiment, the Treg cell population of the invention presents a suppressive action on effector T cells, preferably said suppressive action is dependent on TCR expression and/or activation.

- The term polynucleotide "variant" as used herein, is a polynucleotide that typically differs from a polynucleotide specifically disclosed herein in one or more substitutions, deletions, additions and/or insertions. Such variants may be naturally occurring or may be synthetically generated, for example, by modifying one or more of the polynucleotide sequences of the invention and evaluating one or more biological activities of the encoded polypeptide as described herein and/or using any of a number of techniques well known in the art. Accordingly, the term "polypeptide variant" as used herein, is a polypeptide that typically differs from a polypeptide specifically disclosed herein in one or more substitutions, deletions, additions and/or insertions. Such variants may be naturally occurring or may be synthetically generated, for example, by modifying one or more of the above polypeptide sequences and evaluating one or more biological activities of the polypeptide as described herein and/or using any of a number of techniques well known in the art. Modifications may be made in the structure of the polynucleotides and polypeptides of the present invention and still obtain a functional molecule that encodes a variant or derivative polypeptide with desirable characteristics. When it is desired to alter the amino acid sequence of a polypeptide to create an equivalent, or even an improved, variant or portion of a polypeptide of the invention, one skilled in the art will typically change one or more of the codons of the encoding DNA sequence. For example, certain amino acids may be substituted for other amino acids in a protein structure without appreciable loss of its ability to bind other polypeptides (*e.g.*, antigens) or cells. Since it is the binding capacity and nature of a protein that defines that protein's biological functional activity, certain amino acid sequence substitutions can be made in a protein sequence, and, of course, its underlying DNA coding sequence, and nevertheless obtain a protein with similar properties. It is thus contemplated that various changes may be made in the peptide sequences of the present invention, or corresponding DNA sequences that encode said peptides without appreciable loss of their biological utility or activity. In many instances, a polypeptide variant will contain one or more conservative substitutions. A variant may also, or alternatively, contain non-conservative changes. In a preferred embodiment, variant polypeptides differ from a native sequence by substitution, deletion or addition of five amino acids or fewer. Variants may also (or alternatively) be modified by, for example, the deletion or addition of amino acids that have minimal influence on the immunogenicity, secondary structure and hydropathic nature of the polypeptide.

- The term "zeta" or alternatively "zeta chain", "CD3-zeta" or "TCR-zeta" is defined as the protein provided as GenBank Acc. No. BAG36664.1, or the equivalent residues from a non-human species, *e.g.*, mouse, rodent, monkey, ape and the like, and a "zeta stimulatory domain" or alternatively a "CD3-zeta stimulatory domain" or a "TCR-zeta stimulatory domain" is defined as the amino acid residues from the cytoplasmic domain of the zeta chain, or functional derivatives thereof, that are sufficient to functionally transmit an initial signal necessary for T cell activation. In one embodiment, the cytoplasmic domain of zeta comprises residues 52 through 164 of GenBank Acc. No. BAG36664.1 or the equivalent residues from a non-human species, *e.g.*, mouse, rodent, monkey, ape and the like, that are functional orthologs thereof.

## DETAILED DESCRIPTION

A first object of the present invention is a chimeric antigen receptor (CAR) specific for at least one IL-23 receptor (IL-23R), wherein said CAR comprises (i) an extracellular binding domain, wherein said binding domain binds to said IL-23R (that may be referred as an extracellular IL-23R binding domain), (ii) optionally an extracellular hinge domain, (iii) a transmembrane domain derived from 4-1BB (preferably from human 4-1BB), (iv) an intracellular signaling domain, and (v) optionally a tag and/or a leader sequence.

In one embodiment, the CAR comprises one or more polypeptides.

In one embodiment, the CAR of the invention recognizes and is capable to bind to an IL-23R expressed on the cell surface.

In another embodiment, the CAR of the invention recognizes and is capable to bind to a soluble IL-23R (*i.e.,* not membrane bound).

In one embodiment, the CAR of the invention recognizes and binds to a human IL-23R. Human IL-23R is a protein encoded by a 2.8 kb long mRNA comprising 11 exons (Genbank accession number: NM_144701).

In one embodiment, the CAR of the invention recognizes and is capable to bind to an IL-23R variant, preferably a variant of a human IL-23R.

In one embodiment, a variant peptide of IL-23R refer to a modified IL-23R peptide wherein 1, 2, 3, 4, 5, 6, 7, 8, 9 or 10 amino acids are deleted, added or substituted as compared to the original peptide.

Splice variants of the human IL-23R have been previously identified (Kan et al, 2008). In particular, 24 different isoforms of IL-23R were described: isoform_v1 (encoded by a mRNA having the Genbank accession number AM990313), isoform_v2 (encoded by a mRNA having the Genbank accession number AM990314), isoform_v3 (encoded by a mRNA having the Genbank accession number AM990315), isoform_v4 (encoded by a mRNA having the Genbank accession number AM990316), isoform_v5 (encoded by a mRNA having the Genbank accession number AM990317), isoform_v6 (encoded by a mRNA having the Genbank accession number AM990318), isoform_v7 (encoded by a mRNA having the Genbank accession number AM990319), isoform_v8 (encoded by a mRNA having the Genbank accession number AM990320), isoform_v9 (encoded by a mRNA having the Genbank accession number AM990321), isoform_v10 (encoded by a mRNA having the Genbank accession number AM990322), isoform_v11 (encoded by a mRNA having the Genbank accession number AM990323), isoform_v12 (encoded by a mRNA having the Genbank accession number AM990324), isoform_v13 (encoded by a mRNA having the Genbank accession number AM990325), isoform_v14 (encoded by a mRNA having the Genbank accession number AM990326), isoform_v15 (encoded by a mRNA having the Genbank accession number AM990327), isoform_v16 (encoded by a mRNA having the Genbank accession number AM990328), isoform_v17 (encoded by a mRNA having the Genbank accession number AM990329), isoform_v18 (encoded by a mRNA having the Genbank accession number AM990330), isoform_v19 (encoded by a mRNA having the Genbank accession number AM990331), isoform_v20 (encoded by a mRNA having the Genbank accession number AM990332), isoform_v21 (encoded by a mRNA having the Genbank accession number AM990333), isoform_v22 (encoded by a mRNA having the Genbank accession number AM990334), isoform_v23 (encoded by a mRNA having the Genbank accession number AM990335) and isoform_v24 (encoded by a mRNA having the Genbank accession number AM990336).

Therefore, in one embodiment, the CAR of the invention recognizes and is capable to bind to a splice variant of the human IL-23R selected from the group comprising isoform_v1, isoform_v2, isoform_v3, isoform_v4, isoform_v5, isoform_v6, isoform_v7, isoform_v8, isoform_v9, isoform_v10, isoform_v11, isoform_v12, isoform_v13, isoform_v14, isoform_v15, isoform_v16, isoform_v17, isoform_v18, isoform_v19, isoform_v20, isoform_v21, isoform_v22, isoform_v23 and isoform_v24.

Moreover, single nucleotide polymorphisms in the alpha subunit of human IL-23R have been previously described (Kan et al., 2008 and Sivanesan et al. 2016).

In one embodiment, the CAR of the invention recognizes and is capable to bind to an IL-23R variant comprising a single nucleotide polymorphism (SNP) in the alpha subunit, wherein said SNP is selected from the group comprising R381Q, G149R, V362I and combinations thereof.

In one embodiment, the CAR of the invention recognizes and is capable to bind to a murine IL-23R.

In one embodiment, the extracellular IL-23R binding domain is an antibody directed to an IL-23R or an antigen binding fragment thereof.

The portion of the CAR of the invention comprising an antibody or antigen binding fragment thereof may exist in a variety of forms where the antigen binding domain is expressed as part of a contiguous polypeptide chain including, for example, a single chain antibody (scFv), a single domain antibody fragment (sdAb), a humanized antibody or bispecific antibody (Harlow et al., 1999, In: Using Antibodies: A Laboratory Manual, Cold Spring Harbor Laboratory Press, NY; Harlow et al., 1989, In: Antibodies: A Laboratory Manual, Cold Spring Harbor, New York; Houston et al., 1988, Proc. Natl. Acad. Sci. USA 85:5879-5883; Bird et al., 1988, Science 242:423-426).

In one embodiment, the CAR of the invention comprises a whole antibody, a single chain antibody, a dimeric single chain antibody, a Fv, a scFv, a Fab, a $F(ab)'_2$, a defucosylated antibody, a bi-specific antibody, a diabody, a triabody, a tetrabody, a unibody, a domain antibody, a nanobody, or an antigen-binding fragment thereof.

In one embodiment, the extracellular IL-23R binding domain is an antibody mimetic. Examples of antibody mimetics include, but are not limited to, an affibody, an alphabody, an armadillo repeat protein-based scaffold, a knottin, a kunitz domain peptide, an affilin, an affitin, an adnectin, an atrimer, an evasin, a DARPin, an anticalin, an avimer, a fynomer, a versabody or a duocalin.

In one embodiment, the extracellular binding domain of the CAR of the invention comprises or consists in an antibody fragment, such as, for example, a scFv.

The precise amino acid sequence boundaries of a given CDR can be determined using any of a number of well-known schemes, including those described by Kabat et al. (1991), "Sequences of Proteins of Immunological Interest" 5th Ed. Public Health Service, National Institutes of Health, Bethesda, MD ("Kabat" numbering scheme), Al-Lazikani et al., (1997) JMB 273,927-948 ("Chothia" numbering scheme), or a combination thereof.

In one embodiment, the antibody comprised in the CAR of the invention is a multispecific antibody molecule, *e.g.*, it comprises a plurality of immunoglobulin variable domain sequences, wherein a first immunoglobulin variable domain sequence of the plurality has binding specificity for a first epitope and a second immunoglobulin variable domain sequence of the plurality has binding specificity for a second epitope. In one embodiment, a multispecific antibody molecule is a bispecific antibody molecule. A bispecific antibody has specificity for no more than two antigens. A bispecific antibody molecule is characterized by a first immunoglobulin variable domain sequence which has binding specificity for a first epitope and a second immunoglobulin variable domain sequence that has binding specificity for a second epitope.

In one embodiment, the CAR comprises a scFv comprising a heavy chain $V_H$ having a sequence SEQ ID NO: 1, or a sequence having at least about 70%, preferably at least about 75%, 80%, 85%, 90%, 95% or more identity with SEQ ID NO: 1.

(SEQ ID NO: 1)

EVQLVESGGGLVQPGGSLRLSCAASGIDFNSNYYMCWVRQAPGKGLEWIGCIY
VGSHVNTYYANWAKGRFTISRDNSKNTVYLQMNSLRAEDTAVYYCATSGSSV
LYFKFWGQGTLVTVSS

In one embodiment, the nucleic acid encoding the CAR of the invention comprises a sequence encoding a heavy chain $V_H$ of a scFv, said nucleic acid sequence comprising or consisting of SEQ ID NO: 56, or a sequence having at least about 70%, preferably at least about 75%, 80%, 85%, 90%, 95% or more identity with SEQ ID NO: 56.

(SEQ ID NO: 56)

GAGGTGCAGCTAGTTGAATCTGGTGGAGGCCTGGTTCAGCCTGGTGGCTCTC
TGAGACTGTCTTGTGCCGCCAGCGGCATCGACTTCAACAGCAACTACTACAT
GTGCTGGGTCCGACAGGCCcccGGCAAAGGACTTGAATGGATTGGCTGCATC
TACGTGGGCAGCCATGTGAACACCTACTACGCAAACTGGGCCAAGGGCAGA
TTCACCATCAGCCGGGACAACAGCAAGAACACCGTGTACCTGCAGATGAAC
AGCCTGAGAGCAGAAGATACTGCCGTGTACTACTGTGCCACATCTGGCAGC
AGCGTGCTGTACTTCAAGTTCTGGGGCCAGGGCACCCTGGTCACAGTTTCTT
CA

In one embodiment, the CAR comprises or consists of a scFv comprising a light chain $V_L$ selected from the group comprising SEQ ID NO: 2, 3 and 4 or sequences having at least about 70%, preferably at least about 75%, 80%, 85%, 90%, 95% or more identity with said SEQ ID NO: 2, 3 and 4, preferably comprises a light chain $V_L$ having the sequence SEQ ID NO: 2 or a sequence having at least about 70%, preferably at least about 75%, 80%, 85%, 90%, 95% or more identity with said SEQ ID NO: 2.

(SEQ ID NO: 2)

DIQMTQSPSSLSASVGDRVTITCQASENIYSFLAWYQQKPGKAPKLLIYSASKLA
AGVPSRFSGSGSGTDFTLTISSLQPEDFATYYCQQTNRYSNPDIYNVFGQGTKLT
VLG

(SEQ ID NO: 3)

DIQMTQSPSSLSASVGDRVTITCQASENIYSFLAWYQQKPGKAPKLLIYSASKLA
AGVPSRFSGSGSGTDFTLTISSLQPEDFATYYCQQTNRYSNPDIYNVFGGGTKLT
VLG

(SEQ ID NO: 4)

DIQMTQSPSSLSASVGDRVTITCQASENIYSFLAWYQQKPGKAPKLLIYSASKLA
AGVPSRFSGSGSGTDFTLTISSLQPEDFATYYCQQTNRYSNPDIYNVFGTGTKVT
VLG

In one embodiment, the nucleic acid encoding the CAR of the invention comprises a sequence encoding a light chain $V_L$ of a scFv, said nucleic acid sequence comprising or consisting of SEQ ID NO: 57, or a sequence having at least about 70%, preferably at least about 75%, 80%, 85%, 90%, 95% or more identity with SEQ ID NO: 57.

(SEQ ID NO: 57)

GACATCCAGATGACACAGAGCCCCAGCAGCCTGTCTGCCTCTGTGGGAGAC
AGAGTGACCATCACTTGCCAGGCCAGCGAGAACATCTACAGCTTCCTGGCC
TGGTACCAGCAGAAGCCAGGCAAGGCCCCTAAGCTGCTGATCTACAGCGCC
TCTAAACTAGCTGCCGGGGTGCCAAGCAGATTTTCTGGCTCTGGCAGCGGC
ACCGACTTCACCCTGACCATATCAAGCCTGCAGCCTGAGGACTTCGCCACCT
ACTACTGCCAGCAGACCAACAGGTACAGCAACCCCGACATCTACAACGTGT
TTGGACAGGGCACCAAGCTGACAGTGCTTGGA

In one embodiment, the scFv comprises a heavy chain $V_H$ having a SEQ ID NO: 1 (or a sequence having at least about 70%, preferably at least about 75%, 80%, 85%, 90%, 95% or more identity with said SEQ ID NO: 1) and a light chain $V_L$ selected from the group comprising SEQ ID NO: 2, 3, 4 and sequences having at least about 70%, preferably at least about 75%, 80%, 85%, 90%, 95% or more identity with said SEQ ID NO: 2, 3 and 4, preferably a $V_L$ having a SEQ ID NO: 2 (or a sequence having at least about 70%, preferably at least about 75%, 80%, 85%, 90%, 95% or more identity with said SEQ ID NO: 2).

In one embodiment, the scFv comprises a heavy chain $V_H$ having a SEQ ID NO: 1 (or a sequence having at least about 70%, preferably at least about 75%, 80%, 85%, 90%, 95% or more identity with said SEQ ID NO: 1) and a light chain $V_L$ having a SEQ ID NO: 2 (or a sequence having at least about 70%, preferably at least about 75%, 80%, 85%, 90%, 95% or more identity with said SEQ ID NO: 2).

In one embodiment, the scFv comprises a heavy chain $V_H$ having a SEQ ID NO: 1 (or a sequence having at least about 70%, preferably at least about 75%, 80%, 85%, 90%, 95% or more identity with said SEQ ID NO: 1) and a light chain $V_L$ having a SEQ ID NO: 3 (or a sequence having at least about 70%, preferably at least about 75%, 80%, 85%, 90%, 95% or more identity with said SEQ ID NO: 3).

In one embodiment, the scFv comprises a heavy chain $V_H$ having a SEQ ID NO: 1 (or a sequence having at least about 70%, preferably at least about 75%, 80%, 85%, 90%, 95% or more identity with said SEQ ID NO: 1) and a light chain $V_L$ having a SEQ ID NO: 4 (or a sequence having at least about 70%, preferably at least about 75%, 80%, 85%, 90%, 95% or more identity with said SEQ ID NO: 4).

In one embodiment, the scFv comprises a linker that links the $V_H$ and the $V_L$ chains.

In one embodiment, the linker is a short oligo- or polypeptide, preferably having a length ranging from 2 to 10 amino acids.

For example, a glycine-serine doublet provides a particularly suitable hinge domain (GS linker). In one embodiment, the hinge domain is a Gly/Ser linker. Examples of Gly/Ser linkers include, but are not limited to, GS linkers, $G_2S$ linkers, $G_3S$ linkers, $G_4S$ linkers.

A non-limiting example of $G_2S$ linker is GGS.

$G_3S$ linkers comprise the amino acid sequence $(Gly\text{-}Gly\text{-}Gly\text{-}Ser)_n$ also referred to as $(GGGS)_n$ or (SEQ ID NO: $5)_n$, where n is a positive integer equal to or greater than 1 (such as, example, n=1, n=2, n=3. n=4, n=5, n=6, n=7, n=8, n=9 or n=10). Examples of $G_3S$ linkers include, but are not limited to, GGGSGGGSGGGSGGGS (SEQ ID NO: 6).

Examples of $G_4S$ linkers include, but are not limited to, ($Gly_4$ Ser) corresponding to GGGGS (SEQ ID NO:7);

(Gly$_4$ Ser)$_2$ corresponding to GGGGSGGGGS (SEQ ID NO: 8); (Gly$_4$Ser)$_3$ corresponding to GGGGSGGGGSGGGGS (SEQ ID NO: 9); and (Gly$_4$ Ser)$_4$ corresponding to GGGGSGGGGSGGGGSGGGGS (SEQ ID NO: 10).

In one embodiment, the linker is a (G$_4$S)$_3$ linker (SEQ ID NO: 9), that may be encoded by a sequence SEQ ID NO: 55.

(SEQ ID NO: 55)
GGAGGTGGAGGCTCTGGCGGTGGAGGAAGTGGTGGGGGAGGCTCT

In one embodiment, the scFv comprises or consists in a sequence SEQ ID NO: 11, SEQ ID NO: 12 or SEQ ID NO: 13 or a sequence having at least about 70%, preferably at least about 75%, 80%, 85%, 90%, 95% or more identity with said SEQ ID NO: 11, SEQ ID NO: 12 or SEQ ID NO: 13. In one embodiment, the scFv comprises or consists in a sequence SEQ ID NO: 11 or a sequence having at least about 70%, preferably at least about 75%, 80%, 85%, 90%, 95% or more identity with said SEQ ID NO: 11.

(SEQ ID NO: 11)

DIQMTQSPSSLSASVGDRVTITCQASENIYSFLAWYQQKPGKAPKLLIYSASKLA
AGVPSRFSGSGSGTDFTLTISSLQPEDFATYYCQQTNRYSNPDIYNVFGQGTKLT
VLGGGGGSGGGGSGGGGSEVQLVESGGGLVQPGGSLRLSCAASGIDFNSNYY
MCWVRQAPGKGLEWIGCIYVGSHVNTYYANWAKGRFTISRDNSKNTVYLQM
NSLRAEDTAVYYCATSGSSVLYFKFWGQGTLVTVSS

(SEQ ID NO: 12)

DIQMTQSPSSLSASVGDRVTITCQASENIYSFLAWYQQKPGKAPKLLIYSASKLA
AGVPSRFSGSGSGTDFTLTISSLQPEDFATYYCQQTNRYSNPDIYNVFGGGTKLT
VLGGGGGSGGGGSGGGGSEVQLVESGGGLVQPGGSLRLSCAASGIDFNSNYY
MCWVRQAPGKGLEWIGCIYVGSHVNTYYANWAKGRFTISRDNSKNTVYLQM
NSLRAEDTAVYYCATSGSSVLYFKFWGQGTLVTVSS

(SEQ ID NO: 13)

DIQMTQSPSSLSASVGDRVTITCQASENIYSFLAWYQQKPGKAPKLLIYSASKLA
AGVPSRFSGSGSGTDFTLTISSLQPEDFATYYCQQTNRYSNPDIYNVFGTGTKVT
VLGGGGGSGGGGSGGGGSEVQLVESGGGLVQPGGSLRLSCAASGIDFNSNYY
MCWVRQAPGKGLEWIGCIYVGSHVNTYYANWAKGRFTISRDNSKNTVYLQM
NSLRAEDTAVYYCATSGSSVLYFKFWGQGTLVTVSS

In one embodiment, the CAR of the invention comprises an extracellular IL-23R binding domain and at least one other extracellular antigen binding domain. Therefore, according to this embodiment, the CAR of the invention is capable of binding IL-23R and at least one other antigen.

In one embodiment, the CAR of the invention is capable of binding an IL-23R, and a distinct antigen or ligand. In another embodiment, the CAR of the invention is capable of binding a first epitope on an IL-23R, and a distinct epitope on the same IL-23R. In another embodiment, the CAR of the invention is capable of binding an IL-23R, and a distinct IL-23R (such as, for example, a variant of IL-23R).

In one embodiment, said at least one other extracellular antigen binding domain is an antibody directed to a specific antigen or an antigen binding fragment thereof.

In one embodiment, said at least one other extracellular antigen binding domain comprises or consists in an antibody fragment, such as, for example, a scFv.

In one embodiment, said at least one other extracellular antigen binding domain (preferably scFv) is capable of binding to a food antigen from the common human diet.

The term "food antigen from common human diet" refers to an immunogenic peptide, which comes from foodstuffs common for humans, such as food antigens of the following non-limiting list: bovine antigens such as lipocalin, Ca-binding S100, alpha-lactalbumin, lactoglobulins such as beta-lactoglobulin, bovine serum albumin, caseins. Food-antigens may also be atlantic salmon antigens such as parvalbumin; chicken antigens such as ovomucoid, ovalbumin, Ag22, conalbumin, lysozyme or chicken serum albumin; peanut antigens; shrimp antigens such as tropomyosin; wheat antigens such as agglutinin or gliadin; celery antigens such as celery profilin; carrot antigens such as carrot profilin; apple antigens such as thaumatin, apple lipid transfer protein, or apple profilin; pear antigens such as pear profilin, or isoflavone reductase; avocado antigens such as endochitinase; apricot antigens such as apricot lipid transfer protein; peach antigens such as peach lipid transfer protein or peach profilin; soybean antigens such as HPS, soybean profilin or (SAM22) PR-I0 prot; fragments, variants and mixtures thereof.

In another embodiment, said at least one other extracellular antigen binding domain (preferably scFv) is capable of binding to an autoantigen, such as, for example, a multiple sclerosis-associated antigen, a joint-associated antigen, an eye-associated antigen, a human HSP antigen, a skin-associated antigen or an antigen involved in graft rejection or GVHD

Examples of "multiple sclerosis-associated antigen" include, but are not limited to myelin basic protein (MBP). myelin associated glycoprotein (MAG), myelin oligodendrocyte glycoprotein (MOG), proteolipid protein (PLP), oligodendrocyte myelin oligoprotein (OMGP), myelin associated oligodendrocyte basic protein (MOBP), oligodendrocyte specific protein (OSP/Claudinl 1), heat shock proteins, oligodendrocyte specific proteins (OSP), NOGO A, glycoprotein Po, peripheral myelin protein 22 (PMP22), 2'3'-cyclic nucleotide 3'-phosphodiesterase (CNPase), fragments, variants and mixtures thereof.

Examples of "joint-associated antigen" include, but are not limited to citrulline-substituted cyclic and linear filaggrin peptides, type II collagen peptides, human cartilage glycoprotein 39 (HCgp39) peptides, HSP, heterogeneous nuclear ribonucleoprotein (hnRNP) A2 peptides, hnRNP B1, hnRNP D, Ro60/52, HSP60, HSP65, HSP70 and HSP90, BiP, keratin, vimentin, fibrinogen, type I, III, IV and V collagen peptides, annexin V, Glucose 6 phosphate isomerase (GPI), acetyl-calpastatin, pyruvate dehydrogenase (PDH), aldolase, topoisomerase I, snRNP, PARP, Scl-70, Scl-100, phospholipid antigens including anionic cardiolipin and phosphatidylserine, neutrally charged phosphatidylethanolamine and phosphatidylcholine, matrix metalloproteinase, fibrillin, aggreccan, fragments, variants and mixtures thereof. Other examples of joint-associated antigens include, but are not limited to, citrullinated vimentin, citrullinated type II collagen, citrullinated fibrinogen.

Examples of "eye-associated antigen" include, but are not limited to type II collagen, retinal arrestin, S-arrestin, interphotoreceptor retinoid-binding proteins (IRBP1), beta-crystallin B1, retinal proteins, choroid proteins and fragments, variants and mixtures thereof. Other examples of eye-associated antigens include, but are not limited to, citrullinated vimentin, citrullinated type II collagen, citrullinated fibrinogen.

Examples of "human HSP antigen" include, but are not limited to human HSP60, HSP70, HSP90, fragments, variants and mixtures thereof.

In one embodiment, the antigen is an inflammatory nervous system condition-associated antigen, preferably a multiple sclerosis-associated antigen. Examples of inflammatory nervous system condition-associated antigens, preferably of multiple sclerosis-associated antigens include, but are not limited to myelin basic protein (MBP), myelin associated glycoprotein (MAG), myelin oligodendrocyte protein (MOG), proteolipid protein (PLP), oligodendrocyte myelin oligoprotein (OMGP), myelin associated oligodendrocyte basic protein (MOBP), oligodendrocyte specific protein (OSP/Claudinl 1), heat shock proteins, oligodendrocyte specific proteins (OSP), NOGO A, glycoprotein Po, peripheral myelin protein 22 (PMP22), 2'3'-cyclic nucleotide 3'-phosphodiesterase (CNPase), fragments, variants and mixtures thereof.

In one embodiment, the antigen is a skin-associated antigen. Examples of skin-associated antigens include, but are not limited to, keratinocytes antigens, an antigen present in the dermis or epidermis, a melanocyte antigen (such as, for example, melanin or tyrosinase), desmoglein (*e.g.*, desmoglein 1 or 3, that may also be referred to as Dsg1/3), BP180, BP230, plectin, integrins (*e.g.*, integrin $\alpha4\beta6$), collagens (*e.g.*, collagen type VII), laminins (*e.g.*, laminin 332 or laminin $\gamma1$), plakins (*e.g.*, envoplakin, periplakin, or desmoplakins), keratins (*e.g.*, KRT5, KRT8, KRT15, KRT17 and KRT31), keratin filament-associated proteins, filaggrin, corneodesmosin, and elastin.

In one embodiment, the antigen is an antigen involved in graft rejection or GVHD Examples of such antigens include, but are not limited to, the MHC specific to the transplanted tissue or to the host, $\beta2$-microglobulin, antigens from ABO system, antigens from rhesus system (in particular antigens from the C, c, E et e and D system) and isohaemagglutinins. Other examples of antigens that may be involved in graft rejection or GVHD include, but are not limited to HLA-DR (in particular during the first six months following grafting), HLA-B (in particular during the first two years following grafting), HLA-A, minor histocompatibility antigens (miHA, e.g., HLA-E, HLA-F and HLA-G), HLAs corresponding to MHC class I (A, B, and C), HLAs corresponding to MHC class II (DP, DM, DOA, DOB, DQ, and DR) and HLAs corresponding to MHC class III (*e.g.*, components of the complement system).

In one embodiment, the antigen is a HLA-A2 cell surface protein. In one embodiment, the extracellular binding domain comprises an antibody directed to HLA-A2 or an antigen binding fragment thereof.

The term "HLA-A2" as used herein refers to human leukocyte antigen (HLA) proteins including cell surface proteins, encoded by the HLA-A*02 allele family at the HLA-A locus of the HLA gene complex. HLA proteins encompassed by the term "HLA-A2" include HLA proteins identified as belonging to the HLA-A*02 antigen type by serological testing or genotyping. Additional names for the HLA-A*02 antigen type include "HLA-A2", HLA-A02" and "HLA-A*2". Different naming systems have been developed which identify HLA proteins encoded by this family of alleles including the HLA naming system developed in 2010 by the WHO Committee for Factors of the HLA System. The term "HLA-A2" refer to HLA proteins encoded by alleles having designations according to this naming system which begin with "HLA-A*02", including but not limited to designations which begin with "HLA-A*02:01", "HLA-A*02:02", "HLA-A*02:03", "HLA-A*02:04", "HLA-A*02:05", "HLA-A*02:06", "HLA-A*02:07", "HLA-A*02:08", "HLA-A*02:09", "HLA-A*02:10", and "HLA-A*02:11". The allele designations may be italicized. The allele designations which begin with "HLA-A*02:" followed by 2 or 3 additional digits may constitute the complete designation or a beginning portion of the designation. The term "HLA-A2" also refer to HLA proteins identified with designations which begin with "HLA-A*02" according to this naming system, including but not limited to the designations "HLA-A*02:01", "HLA-A*02:02", "HLA-A*02:03", "HLA-A*02:04", "HLA-A*02:05", "HLA-A*02:06", "HLA-A*02:07", "HLA-A*02:08", "HLA-A*02:09", "HLA-A*02:10", and "HLA-A*02:11".

Other examples of autoantigens include, without limitation, aquaporin water channels (such as, for example, aquaporin-4 water channel (AQP4)), Hu, Ma2, collapsin response-mediator protein 5 (CRMP5), and amphiphysin, voltage-gated potassium channel (VGKC), N-methyl-d-aspartate receptor (NMDAR), α-amino-3-hydroxy-5-methyl-4-isoxazoleproprionic acid (AMPAR), thyroid peroxidase, thyroglobulin, anti-N-methyl-D-aspartate receptor (NR1 subunit), Rh blood group antigens, I antigen, desmoglein 1 or 3 (Dsg1/3), BP180, BP230, Acetylcholine nicotinic postsynaptic receptors, thyrotropin receptors, platelet integrin, GpIIb:IIIa, Collagen (such as, for example, Collagen alpha-3(IV) chain), rheumatoid factor, calpastatin, citrullinated proteins, Myelin basic protein (MBP), Myelin oligodendrocyte glycoprotein (MOG) peptides, alpha-beta-crystallin, DNA, histone, ribosomes, RNP, tissue transglutaminase (TG2), intrinsic factor, 65-kDa antigen, phosphatidylserine, ribosomal phosphoproteins, anti-neutrophil cytoplasmic antibody, Scl-70, U1-RNP, ANA, SSA, anti-SSB, antinuclear antibodies (ANA), antineutrophil cytoplasm antibodies (ANCA), Jo-1, antimitochondrial antibodies, gp210, p62, sp100, antiphospholipid antibodies, U1-70 kd snRNP, GQlb ganglioside, GM1, asialo GM1, GD1b, anti-smooth muscle antibodies (ASMA), anti-liver-kidney microsome-1 antibodies (ALKM-1), anti-liver cytosol antibody-1 (ALC-1), IgA antiendomysial antibodies, neutrophil granule proteins, streptococcal cell wall antigen, intrinsic factor of gastric parietal cells, insulin (IAA), glutamic acid decarboxylase (GAA or GAD) and protein tyrosine phosphatase (such as, for example, IA2 or ICA512), PLA2R1 and THSD7A1.

In one embodiment, the antigen is a cancer antigen.

As used herein, the term "cancer antigen" refers to antigens which are differentially expressed by cancer cells and can thereby be exploited in order to target cancer cells. Cancer antigens are antigens which can potentially stimulate apparently tumor- specific immune responses. Some of these antigens are encoded, although not necessarily expressed, by normal cells. These antigens can be characterized as those which are normally silent (i.e., not expressed) in normal cells, those that are expressed only at certain stages of differentiation and those that are temporally expressed such as embryonic and fetal antigens. Other cancer antigens are encoded by mutant cellular genes, such as oncogenes (e.g., activated ras oncogene), suppressor genes (e.g., mutant p53), and fusion proteins resulting from internal deletions or chromosomal translocations. Still other cancer antigens can be encoded by viral genes such as those carried on RNA and DNA tumor viruses. Many tumor antigens have been defined in terms of multiple solid tumors: MAGE 1, 2, & 3, defined by immunity; MART-1/Melan-A, gp100, carcinoembryonic antigen (CEA), HER2, mucins (i.e., MUC-1), prostate-specific antigen (PSA), and prostatic acid phosphatase (PAP). In addition, viral proteins such as some encoded by hepatitis B (HBV), Epstein-Barr (EBV), and human papilloma (HPV) have been shown to be important in the development of hepatocellular carcinoma, lymphoma, and cervical cancer, respectively.

Other cancer antigens include, but are not limited to, 707-AP (707 alanine proline), AFP (alpha (a)-fetoprotein), ART-4 (adenocarcinoma antigen recognized by T4 cells), BAGE (B antigen; b-catenin/m, b-catenin/mutated), BCMA (B cell maturation antigen), Bcr-abl (breakpoint cluster region-Abelson), CAIX (carbonic anhydrase IX), CD19 (cluster of differentiation 19), CD20 (cluster of differentiation 20), CD22 (cluster of differentiation 22), CD30 (cluster of differentiation 30), CD33 (cluster of differentiation 33), CD44v7/8 (cluster of differentiation 44, exons 7/8), CAMEL (CTL-recognized antigen on melanoma), CAP-1 (carcinoembryonic antigen peptide - 1), CASP-8 (caspase-8), CDC27m (cell-division cycle 27 mutated), CDK4/m (cycline-dependent kinase 4 mutated), CEA (carcinoembryonic antigen), CT (cancer/testis (antigen)), Cyp-B (cyclophilin B), DAM (differentiation antigen melanoma), EGFR (epidermal growth factor receptor), EGFRv111 (epidermal growth factor receptor, variant III), EGP-2 (epithelial glycoprotein 2), EGP-40 (epithelial glycoprotein 40), Erbb2, 3, 4 (erythroblastic leukemia viral oncogene homolog-2, -3, 4), ELF2M (elongation factor 2 mutated), ETV6-AML1 (Ets variant gene 6/acute myeloid leukemia 1 gene ETS), FBP (folate binding protein), fAchR (Fetal acetylcholine receptor), G250 (glycoprotein 250), GAGE (G antigen), GD2 (disialoganglioside 2), GD3 (disialoganglioside 3), GnT-V (N-acetylglucosaminyltransferase V), Gp100 (glycoprotein 100kD), HAGE (helicose antigen), HER-2/neu (human epidermal receptor-2/neurological; also known as EGFR2), HLA-A (human leukocyte antigen-A) HPV (human papilloma virus), HSP70- 2M (heat shock protein 70 - 2 mutated), HST-2 (human signet ring tumor - 2), hTERT or hTRT (human telomerase reverse transcriptase), iCE (intestinal carboxyl esterase), IL-13R-a2 (Interleukin-13 receptor subunit alpha-2), KIAA0205, KDR (kinase insert domain receptor), κ-light chain, LAGE (L antigen), LDLR/FUT (low density lipid receptor/GDP-L-fucose: b-D-galactosidase 2-a-Lfucosyltransferase), LeY (Lewis-Y antibody), L1 CAM (L1 cell adhesion molecule), MAGE (melanoma antigen), MAGE-A1 (Melanoma-associated antigen 1), mesothelin, Murine CMV infected cells, MART-1/Melan-A (melanoma antigen recognized by T cells- I/Melanoma antigen A), MC1 R (melanocortin 1 receptor), Myosin/m (myosin mutated), MUC1 (mucin 1), MUM-1 , -2, -3 (melanoma ubiquitous mutated 1 , 2, 3), NA88-A (NA cDNA clone of patient M88), NKG2D (Natural killer group 2, member D) ligands, NY-BR-1 (New York breast differentiation antigen 1), NY-ESO-1 (New York esophageal squamous cell carcinoma-1), oncofetal antigen (h5T4), P15 (protein 15), p190 minor bcr-abl (protein of 190KD bcr-abl), Pml/RARa (promyelocytic leukaemia/retinoic acid receptor a), PRAME (preferentially expressed antigen of melanoma), PSA (prostate-specific antigen), PSCA (Prostate stem cell antigen), PSMA (prostate-specific membrane antigen), RAGE (renal antigen), RU1 or RU2 (renal ubiquitous 1 or 2), SAGE (sarcoma antigen), SART-1 or SART-3 (squamous antigen rejecting tumor 1 or 3), SSX1 , -2, -3, 4 (synovial sarcoma X1 , -2, - 3, -4), TAA (tumor-associated antigen), TAG-72 (Tumor-associated glycoprotein 72), TEL/AML1 (translocation Ets-family leukemia/acute myeloid leukemia 1), TPI/m (triosephosphate isomerase mutated), TRP-1 (tyrosinase related protein 1, or gp75), TRP-2 (tyrosinase related protein 2), TRP-2/INT2 (TRP-2/intron 2), VEGF-R2 (vascular endothelial growth factor receptor 2), or WT1 (Wilms' tumor gene).

In one embodiment, said at least one other extracellular antigen binding domain (preferably scFv) is capable of binding to an antigen associated with infected cells.

As used herein, the term "infected cells" refers to cells contaminated with something that affects their quality, character, or condition unfavorably.

In one embodiment, the antigen is associated with virally infected cells. In another embodiment, the antigen is associated with bacterially infected cells. In another embodiment, the antigen is associated with fungally infected cells. In another embodiment, the antigen is associated with parasitic infected cells.

In another embodiment, said at least one other extracellular antigen binding domain (preferably scFv) is capable of binding to an inhaled allergen, an ingested allergen or a contact allergen.

Examples of inhaled allergens include, but are not limited to, allergens from Astigmata (*e.g.*, Acarus siro (Storage mite, Aca s 13), Blomia tropicalis (Mite, Blo t), Dermatophagoides farinae (American house dust mite, Der f), Dermatophagoides microceras (House dust mite, Der m), Dermatophagoides pteronyssinus (European house dust mite, Der p), Euroglyphus maynei (House dust mite, Eur m), Glycyphagus domesticus (Storage mite, Gly d 2), Lepidoglyphus destructor (Storage mite, Lep d), Tyrophagus putrescentiae (Storage mite, Tyr p)); Blattaria (e.g., Blattella germanica (German cockroach, Bla g), Periplaneta americana (American cockroach, Per a)); Coleoptera (*e.g.*, Harmonia axyridis (Asian ladybeetle, Har a)), Diptera (*e.g.*, Aedes aegypti (Yellow fever mosquito, Aed a), Chironomus kiiensis (Midge, Chi k), Chironomus thummi thummi (Midge, Chi t), Forcipomyia taiwana (Biting midge, For t), Glossina morsitans (Savannah Tsetse fly, Glo m), Hemidiptera: Triatoma protracta (California kissing bug, Tria p)), Hymenoptera (*e.g.*, Apis cerana (Eastern hive bee, Api c), Apis dorsata (Giant honeybee, Api d), Apis mellifera (Honey bee, Api m), Bombus pennsylvanicus (Bumble bee, Bom p), Bombus terrestris (Bumble bee, Bom t), Dolichovespula arenaria (Yellow hornet, Dol a), Dolichovespula maculata (White face hornet, Dol m), Myrmecia pilosula (Australian jumper ant, Myr p), Polistes annularis (Wasp, Pol a), Polistes dominulus (Mediterranean paper wasp, Pol d), Polistes exclamans (Wasp, Pol e), Polistes fuscatus (Wasp, Pol f), Polistes gallicus (Wasp, Pol g), Polistes metricus (Wasp, Pol m), Polybia paulista (Wasp, Pol p), Polybia scutellaris (Wasp, Pol s), Solenopsis geminata (Tropical fire ant, Sol g), Solenopsis invicta (Red imported fire ant, Sol i), Solenopsis richteri (Black fire ant, Sol r), Solenopsis saevissima (Brazilian fire ant, Sol s), Vespa crabro (European hornet, Vesp c), Vespa mandarinia (Giant asian hornet, Vesp m), Vespula fiavopilosa (Yellow jacket, Vesp f), Vespula germanica (Yellow jacket, Vesp g), Vespula maculifrons (Yellow jacket, Vesp m), Vespula pensylvanica (Yellow jacket, Vesp p), Vespula squamosa (Yellow jacket, Vesp s), Vespula vidua (Wasp, Vesp vi), Vespula vulgaris (Yellow jacket, Vesp v)), Ixodida (*e.g.*, Argas reflexus (Pigeon tick, Arg r)), Lepidoptera (*e.g.*, Bombyx niori (Silk moth, Bomb n), Plodia interpunctella (Indianmeal moth, Plo i), Thaumetopoea pityocampa (Pine processionary moth, Tha p)), Thysanura (*e.g.*, Lepisma saccharina (Silverfish, Lep s)), Siphonaptera (*e.g.,* Ctenocephalides felis felis (Cat flea, Cte f)), Carnivora (*e.g.*, Canis familiaris (dog, Can f), Felis domesticus (cat, Fel d)); Lagomorpha (*e.g.*, Oryctolagus cuniculus (rabbit, Ory c), Perissodactlyla: Equus caballus (domestic horse, Equ c)), Pleuronectiformes (*e.g.*, Lepidorhombus whiffiagonis (Megrim, Whiff, Gallo, Lep w)), Rodentia (*e.g.*, Cavia porcellus (guinea pig, Cav p), Mus musculus (mouse, Mus m), Rattus norvegius (rat, Rat n)); Coniferales: Chamaecyparis obtusa (Japanese cypress, Cha o), Cupressus arizonica (Cypress, Cup a), Cryptomeria japonica (Sugi, Cry j), Cupressus sempervirens (Common cypress, Cup s), Juniperus ashei (Mountain cedar, Jun a), Juniperus oxycedrus (Prickly juniper, Jun o), Juniperus sabinoides (Mountain cedar, Jun s), Juniperus virginiana (Eastern red cedar, Jun v)); Gentianales (e.g., Catharanthus roseus (Rosy periwinkle, Cat r)); Poales (*e.g.*, Anthoxanthum odoratum (Sweet vernal grass, Ant o 1), Cynodon dactylon (Bermuda grass, Cyn d 1, Cyn d 7, Cyn d 12, Cyn d 15, Cyn d 22w, Cyn d 23, Cyn d 24), Dactylis glomerata (Orchard grass, Dae g 1, Dae g 2, Dae g 3, Dae g 4, Dae g 5), Festuca pratensis (Meadow fescue, Fes p 4)), Holcus lanatus (Velvet grass, Hol I 1, Hol I 5), Hordeum vulgare (Barley, Hor v 1, Hor v 5, Hor v 12, Hor v 15, Hor v 16, Hor v 17, Hor v 21), Lolium perenne (Rye grass, Lol p 1, Lol p 2, Lol p 3, Lol p 4, Lol p 5, Lol p 11), Oryza sativa (Rice, Ory s 1, Ory s 12), Paspalum notarum (Bahia grass, Pas n 1), Phalaris aquatica (Canary grass, Pha a 1, Pha a 5), Phleum pratense (Timothy, Phl p 1, Phl p 2, Phl p 4, Phl p 5, Phl p 6, Phl p 7, Phl p 11, Phl p 12, Phl p 13), Poa pratensis (Kentucky blue grass, Poa p 1, Poa p 5), Secale cereale (Rye, Sec c 1, Sec c 20), Sorghum halepense (Johnson grass, Sor h 1), Triticum aestivum (Wheat, Tri a 12, Tri a 14, Tri a 185, Tri a 19, Tri a 25, Tri a 26, Tri a 27, Tri a 28, Tri a 29, Tri a 30), Zea mays (Maize, Zea m 1, Zea m 12, Zea m 14, Zea m 25), Fagales: Alnus glutinosa (Alder, Aln g 1, Aln g 4), Betula verrucosa (Birch, Bet v 1, Bet v 2, Bet v 3, Bet v 4, Bet v 5, Bet v 6, Bet v 7), Carpinus betuhxs (Hornbeam, Car b 1)); Lamiales (*e.g.*, Fraxinus excelsior (Ash, Fra e 1), Ligustrum vulgare (Privet, Lig v), Syringa vulgaris (Lilac, Syr v)); Malpighiales (*e.g.*, Hevea brasiliensis (para rubber tree (latex), Hev b 1, Hev b 2, Hev b 3, Hev b 4, Hev b 5, Hev b 6, Hev b 7, Hev b 8, Hev b 9, Hev b 10, Hev b 11, Hev b 12, Hev b 13)); Proteales (*e.g.*, Platanus acerifolia (London plane tree, Pla a 1, Pla a 2, Pla a 3), Platanus orientalis (Oriental plane, Pla or 1, Pla or 2, Pla or 3)).

Examples of ingested allergens include, but are not limited to, allergens from Fungi Ascomycota, such as, for example, Dothideales (*e.g.*, Alternaria alternata (Alternaria rot fungus, Alt a), Cladosporium cladosporioides (Cla c), Cladosporium herbarum (Cla h), Curvularia lunata (Cur 1), - Eurotiales: Aspergillus flavus (Asp fl), Aspergillus fumigatus (Asp f), Aspergillus niger (Asp n), Aspergillus oryzae (Asp o), Penicillium brevicompactum (Pen b), Penicillium chrysogenum (Pen ch), Penicillium citrinum (Pen c), Penicillium oxalicum (Pen o)), Hypocreales (*e.g.*, Fusarium culmorum (Fus c)); Onygenales (*e.g.*, Trichophyton rubrum (Tri r), Trichophyton tonsurans (Tri t), Saccharomycetales: Candida albicans (Yeast, Cand a), Candida boidinii (Yeast, Cand b)); Tuberculariales (*e.g.*, Epicoccum purpurascens (Epi p)), allergens from Fungi Basidiomycota, such as, for example, Hymenomycetes (*e.g.*, Coprinus comatus (Shaggy mane, Cop c), Psilocybe cubensis (Magic mushroom, Psi c), Urediniomycetes (*e.g.*, Rhodotorula mucilaginosa (Yeast, Rho m)); Ustilaginomycetes (*e.g.*, Malassezia furfur (Pityriasis versicolor infect. Agent, Mala f), Malassezia sympodialis (Mala s)); antibiotics (such as, for example, Penicillins, Cephalosporins, Aminosides, Quinolones, Macrolides, Tetracycline, Sulfamids); drugs (such as, for example, acetylsalicylic acid, vaccines, morphines and derivatives); vitamins such as, for example, vitamin K1; and food allergens (such as, for example, allergen from milk, egg, peanut, tree nut (walnut, cashew, etc.), fish, shellfish, soy, wheat, and carrot, apple, pear, avocado, apricot, peach).

Examples of contact allergens include, but are not limited to, heavy metals (such as, for example, nickel, chrome, gold), latex, haptens such as, for example halothane, hydralazine.

In one embodiment, said at least one other extracellular antigen binding domain (preferably scFv) is capable of binding to an antigen selected from the group comprising ovalbumin, MOG, type II collagen fragments, variants and mixtures thereof. In one embodiment, said at least one other extracellular antigen binding domain (preferably scFv) is capable of binding to an antigen selected from the group comprising citrullinated vimentin, citrullinated type II collagen, citrullinated fibrinogen, variants and mixtures thereof.

In one embodiment, said at least one other extracellular antigen binding domain (preferably scFv) is capable of binding to ovalbumin, fragments, variants and mixtures thereof.

In another embodiment, said at least one other extracellular antigen binding domain (preferably scFv) is capable of binding to MOG, fragments, variants and mixtures thereof.

In another embodiment, said at least one other extracellular antigen binding domain (preferably scFv) is capable of binding to type II collagen, fragments, variants and mixtures thereof.

In another embodiment, said at least one other extracellular antigen binding domain (preferably scFv) is capable of binding to citrullinated vimentin, citrullinated type II collagen, citrullinated fibrinogen, fragments, variants and mixtures thereof.

In one embodiment, the extracellular IL-23R binding domain is connected to a transmembrane domain by a hinge domain.

In one embodiment, the hinge domain is a short oligo- or polypeptide linker, preferably having a length ranging from 2 to 10 amino acids, as described hereinabove.

Another example of hinge domain that may be used in the present invention is described in WO2012/138475, incorporated herein by reference.

In one embodiment, the hinge domain comprises an amino acid sequence selected from the group comprising the amino acid sequence AGSSSSGGSTTGGSTT (SEQ ID NO: 14), the amino acid sequence GTTAASGSSGGSSSGA (SEQ ID NO: 15), the amino acid sequence SSATATAGTGSSTGST (SEQ ID NO: 16), and the amino acid sequence TSGSTGTAASSTSTST (SEQ ID NO: 17).

In one embodiment, the hinge domain is encoded by a nucleotide sequence of GGTGGCGGAGGTTCTGGAGGTGGAGGTTCC (SEQ ID NO: 18).

In another embodiment, the hinge domain is a $KIR_2DS_2$ hinge corresponding to KIRRDSS (SEQ ID NO: 19).

In one embodiment, the hinge domain is derived from CD8 (preferably from human CD8), and corresponds to a portion of the extracellular domain of CD8, preferably to the juxtamembrane part of CD8. In one embodiment, the hinge domain comprises or consists of the 45, 50, 55, 60, 65, 70 or more juxtamembrane extracellular amino acids of CD8.

In one embodiment, the hinge domain comprises or consists in the amino acid sequence of a CD8 hinge having the sequence SEQ ID NO: 20 or an amino acid sequence with at least about 95, preferably about 96%, 97%, 98% or 99% identity to SEQ ID NO: 20. In one embodiment, the hinge domain is a CD8 hinge encoded by the nucleic acid sequence SEQ ID NO: 21 or SEQ ID NO: 81 or SEQ ID NO: 83 or a nucleic acid sequence with at least about 95, preferably about 96%, 97%, 98% or 99% identity to SEQ ID NO: 21 or 81 or 83.
(SEQ ID NO: 20)
TTTPAPRPPTPAPTIASQPLSLRPEACRPAAGGAVHTRGLDFACD

(SEQ ID NO: 21)

ACAACAACACCTGCTCCTCGGCCTCCTACACCAGCTCCTACAATTGCCAGCC
AGCCACTGTCTCTGAGGCCCGAAGCTTGCAGGCCTGCTGCTGGCGGAGCCG
TGCATACAAGAGGACTGGATTTCGCCTGCGAC

(SEQ ID NO: 81)

ACCACGACGCCAGCGCCGCGACCACCAACACCGGCGCCCACCATCGCGTCG
CAGCCCCTGTCCCTGCGCCCAGAGGCGTGCCGGCCAGCGGCGGGGGGCGCA
GTGCACACGAGGGGGCTGGACTTCGCCTGTGAT

(SEQ ID NO: 83)

ACAACAACCCCTGCTCCTCGGCCTCCTACACCAGCTCCTACAATTGCCAGCC
AGCCACTGTCTCTGAGGCCCGAAGCTTGTAGACCTGCTGCTGGCGGAGCCG
TGCATACAAGAGGACTGGATTTCGCCTGCGAC

In one embodiment, the hinge domain comprises or consists in the amino acid sequence of a CD8 hinge having the sequence SEQ ID NO: 60 or an amino acid sequence with at least about 95, preferably about 96%, 97%, 98% or 99% identity to SEQ ID NO: 60. In one embodiment, the hinge domain is a CD8 hinge encoded by the nucleic acid sequence SEQ ID NO: 61 or a nucleic acid sequence with at least about 95, preferably about 96%, 97%, 98% or 99% identity to SEQ ID NO: 61.

(SEQ ID NO: 60)

SALSNSIMYFSHFVPVFLPAKPTTTPAPRPPTPAPTIASQPLSLRPEACRPAAGGA
VHTRGLDFACD

(SEQ ID NO: 61)

AGCGCCCTGAGCAACAGCATCATGTACTTCAGCCACTTCGTGCCCGTGTTTC
TGCCCGCCAAACCTACAACAACaCCTGCTCCTCGGCCTCCTACACCAGCTCC
TACAATTGCCAGCCAGCCACTGTCTCTGAGGCCCGAAGCTtgcaggCCTGCTGC
TGGCGGAGCCGTGCATACAAGAGGACTGGATTTCGCCTGCGAC

In another embodiment, the hinge domain comprises or consists in the amino acid sequence of a IgG4 hinge (SEQ ID NO: 22), or an amino acid sequence with at least about 95, preferably about 96%, 97%, 98% or 99% identity to SEQ ID NO: 22. In one embodiment, the hinge domain is an IgG4 hinge encoded by the nucleic acid sequence SEQ ID NO: 23 or a nucleic acid sequence with at least about 95, preferably about 96%, 97%, 98% or 99% identity to SEQ ID NO: 23.

(SEQ ID NO: 22)

ESKYGPPCPPCPAPEFLGGPSVFLFPPKPKDTLMISRTPEVTCVVVDVSQEDPEV
QFNWYVDGVEVHNAKTKPREEQFNSTYRVVSVLTVLHQDWLNGKEYKCKVS
NKGLPSSIEKTISKAKGQPREPQVYTLPPSQEEMTKNQVSLTCLVKGFYPSDIAV
EWESNGQPENNYKTTPPVLDSDGSFFLYSRLTVDKSRWQEGNVFSCSVMHEAL
HNHYTQKSLSLSLGKM

(SEQ ID NO: 23)

GAGAGCAAGTACGGCCCTCCCTGCCCCCCTTGCCCTGCCCCCGAGTTCCTGG
GCGGACCCAGCGTGTTCCTGTTCCCCCCCAAGCCCAAGGACACCCTGATGAT
CAGCCGGACCCCCGAGGTGACCTGTGTGGTGGTGGACGTGTCCCAGGAGGA
CCCCGAGGTCCAGTTCAACTGGTACGTGGACGGCGTGGAGGTGCACAACGC
CAAGACCAAGCCCCGGGAGGAGCAGTTCAATAGCACCTACCGGGTGGTGTC
CGTGCTGACCGTGCTGCACCAGGACTGGCTGAACGGCAAGGAATACAAGTG
TAAGGTGTCCAACAAGGGCCTGCCCAGCAGCATCGAGAAAACCATCAGCAA
GGCCAAGGGCCAGCCTCGGGAGCCCCAGGTGTACACCCTGCCCCCTAGCCA
AGAGGAGATGACCAAGAACCAGGTGTCCCTGACCTGCCTGGTGAAGGGCTT
CTACCCCAGCGACATCGCCGTGGAGTGGGAGAGCAACGGCCAGCCCGAGA

ACAACTACAAGACCACCCCCCCTGTGCTGGACAGCGACGGCAGCTTCTTCCT
GTACAGCCGGCTGACCGTGGACAAGAGCCGGTGGCAGGAGGGCAACGTCTT
TAGCTGCTCCGTGATGCACGAGGCCCTGCACAACCACTACACCCAGAAGAG
CCTGAGCCTGTCCCTGGGCAAGATG

In another embodiment, the hinge domain comprises or consists in the amino acid sequence of a IgD hinge (SEQ ID NO: 24) or an amino acid sequence with at least about 95, preferably about 96%, 97%, 98% or 99% identity to SEQ ID NO: 24. In one embodiment, the hinge domain is an IgD hinge encoded by the nucleic acid sequence SEQ ID NO: 25 or a nucleic acid sequence with at least about 95, preferably about 96%, 97%, 98% or 99% identity to SEQ ID NO: 25.

(SEQ ID NO: 24)

RWPESPKAQASSVPTAQPQAEGSLAKATTAPATTRNTGRGGEEKKKEKEKEEQ
EERETKTPECPSHTQPLGVYLLTPAVQDLWLRDKATFTCFVVGSDLKDAHLTW
EVAGKVPTGGVEEGLLERHSNGSQSQHSRLTLPRSLWNAGTSVTCTLNHPSLPP
QRLMALREPAAQAPVKLSLNLLASSDPPEAASWLLCEVSGFSPPNILLMWLEDQ
REVNTSGFAPARPPPQPGSTTFWAWSVLRVPAPPSPQPATYTCVVSHEDSRTLL
NASRSLEVSYVTDH

(SEQ ID NO: 25)

AGGTGGCCCGAAAGTCCCAAGGCCCAGGCATCTAGTGTTCCTACTGCACAG
CCCCAGGCAGAAGGCAGCCTAGCCAAAGCTACTACTGCACCTGCCACTACG
CGCAATACTGGCCGTGGCGGGGAGGAGAAGAAAAAGGAGAAAGAGAAAG
AAGAACAGGAAGAGAGGGAGACCAAGACCCCTGAATGTCCATCCCATACC
CAGCCGCTGGGCGTCTATCTCTTGACTCCCGCAGTACAGGACTTGTGGCTTA
GAGATAAGGCCACCTTTACATGTTTCGTCGTGGGCTCTGACCTGAAGGATGC
CCATTTGACTTGGGAGGTTGCCGGAAAGGTACCCACAGGGGGGGTTGAGGA
AGGGTTGCTGGAGCGCCATTCCAATGGCTCTCAGAGCCAGCACTCAAGACT
CACCCTTCCGAGATCCCTGTGGAACGCCGGGACCTCTGTCACATGTACTCTA
AATCATCCTAGCCTGCCCCCACAGCGTCTGATGGCCCTTAGAGAGCCAGCC
GCCCAGGCACCAGTTAAGCTTAGCCTGAATCTGCTCGCCAGTAGTGATCCCC

CAGAGGCCGCCAGCTGGCTCTTATGCGAAGTGTCCGGCTTTAGCCCGCCCA
ACATCTTGCTCATGTGGCTGGAGGACCAGCGAGAAGTGAACACCAGCGGCT
TCGCTCCAGCCCGGCCCCCACCCCAGCCGGGTTCTACCACATTCTGGGCCTG
GAGTGTCTTAAGGGTCCCAGCACCACCTAGCCCCCAGCCAGCCACATACAC
CTGTGTTGTGTCCCATGAAGATAGCAGGACCCTGCTAAATGCTTCTAGGAGT
CTGGAGGTTTCCTACGTGACTGACCATT

In another embodiment, the hinge region comprises or consists in the amino acid sequence of a CD28 hinge (SEQ ID NO: 26) or an amino acid sequence with at least about 95, preferably about 96%, 97%, 98% or 99% identity to SEQ ID NO: 26. In one embodiment, the hinge domain is a CD28 hinge encoded by the nucleic acid SEQ ID NO: 27 or a nucleic acid sequence with at least about 95, preferably about 96%, 97%, 98% or 99% identity to SEQ ID NO: 27.
(SEQ ID NO: 26)
IEVMYPPPYLDNEKSNGTIIHVKGKHLCPSPLFPGPSKP

(SEQ ID NO: 27)

ATTGAAGTTATGTATCCTCCTCCTTACCTAGACAATGAGAAGAGCAATGGA
ACCATTATCCATGTGAAAGGGAAACACCTTTGTCCAAGTCCCCTATTTCCCG
GACCTTCTAAGCCC

According to the present invention, the CAR of the invention comprises a transmembrane domain derived from 4-1BB (CD137). In one embodiment, the transmembrane domain is derived from human 4-1BB.

In one embodiment, the transmembrane domain comprises or consists in the amino acid sequence of a human 4-1BB transmembrane domain (SEQ ID NO: 28) or an amino acid sequence with at least about 95, preferably about 96%, 97%, 98% or 99% identity to SEQ ID NO: 28. In one embodiment, the transmembrane domain is a human 4-1BB transmembrane domain encoded by the nucleic acid sequence SEQ ID NO: 29 or a nucleic acid sequence with at least about 95, preferably about 96%, 97%, 98% or 99% identity to SEQ ID NO: 29.
(SEQ ID NO: 28)
IISFFLALTSTALLFLLFFLTLRFSVV

(SEQ ID NO: 29)

ATCATCTCCTTCTTTCTTGCGCTGACcTCGACTGCGTTGCTCTTCCTGCTGTTC
TTCCTCACGCTCCGTTTCTCTGTTGTT

In one embodiment, the intracellular signaling domain may comprise the entire intracellular portion, or the entire native intracellular signaling domain, of the molecule from which it is derived, or a functional fragment or derivative thereof.

In one embodiment, the intracellular signaling domain comprises a T cell primary signaling domain (or a sequence derived therefrom) and optionally one or more intracellular domain(s) of a T cell costimulatory molecule (or sequence(s) derived therefrom).

In one embodiment, the intracellular signaling domain of the CAR of the invention consists in a primary signaling domain.

In one embodiment, the intracellular signaling domain comprises one or more intracellular domain(s) of a T cell costimulatory molecule. In one embodiment, the intracellular signaling domain consists in one or more intracellular domain(s) of a T cell costimulatory molecule.

In another embodiment, the intracellular signaling domain of the CAR of the invention comprises at least one costimulatory domain and a primary signaling domain.

In another embodiment, the intracellular signaling domain of the CAR of the invention comprises at least two costimulatory domains and a primary signaling domain.

In one embodiment of the invention, the T cell primary signaling domain comprises a signaling domain of a protein selected in the group of CD3 zeta, CD3 gamma, CD3 delta, CD3 epsilon, common FcR gamma (FCER1G), FcR beta (Fc Epsilon Rib), CD79a, CD79b, Fcgamma RIIa, DAP10, and DAP 12 and sequences derived therefrom.

In one embodiment, the T cell primary signaling domain comprises or consists in a functional signaling domain of CD3 zeta.

In one embodiment, the T cell primary signaling domain comprises or consists in the amino acid sequence of the CD3-zeta domain of SEQ ID NO: 30, 31, 32 or 33, or an amino acid sequence with at least about 95, preferably about 96%, 97%, 98% or 99% identity to SEQ ID NO: 30, 31, 32 or 33.

(SEQ ID NO: 30)

RVKFSRSADAPAYQQGQNQLYNELNLGRREEYDVLDKRRGRDPEMGGKPRRK
NPQEGLYNELQKDKMAEAYSEIGMKGERRRGKGHDGLYQGLSTATKDTYDAL
HMQALPPR

(SEQ ID NO: 31)

RVKFSRSADAPAYKQGQNQLYNELNLGRREEYDVLDKRRGRDPEMGGKPRRK
NPQEGLYNELQKDKMAEAYSEIGMKGERRRGKGHDGLYQGLSTATKDTYDAL
HMQALPPR

(SEQ ID NO: 32)

RVKFSRSADAPAYKQGQNQLYNELNLGRREEYDVLDKRRGRDPEMGGKPQRR
KNPQEGLYNELQKDKMAEAYSEIGMKGERRRGKGHDGLYQGLSTATKDTYD
ALHMQALPPR

(SEQ ID NO: 33)

RVKFSRSADAPAYQQGQNQLYNELNLGRREEYDVLDKRRGRDPEMGGKPQRR
KNPQEGLYNELQKDKMAEAYSEIGMKGERRRGKGHDGLYQGLSTATKDTYD
ALHMQALPPR

In another embodiment, the CD3 zeta primary signaling domain comprises or consists in an amino acid sequence having at least one, two or three modifications but not more than 20, 10 or 5 modifications of an amino acid sequence of SEQ ID NO: 30, 31, 32 or 33, or an amino acid sequence with at least about 95, preferably about 96%, 97%, 98% or 99% identity to SEQ ID NO: 30, 31, 32 or 33.

Thus, in one embodiment, the nucleic acid sequence encoding the T cell primary signaling domain comprises or consists in the nucleic acid sequence of the CD3-zeta domain of SEQ ID NO: 34, SEQ ID NO: 35, SEQ ID NO: 78, SEQ ID NO: 85 or SEQ ID NO: 86, or a nucleotide sequence with at least about 95, preferably about 96%, 97%, 98% or 99% identity to SEQ ID NO: 34, 35, 78, 85 or 86.

(SEQ ID NO: 34)

AGAGTGAAGTTCAGCAGATCCGCCGACGCTCCTGCCTATCAGCAGGGCCAA
AACCAGCTGTACAACGAGCTGAACCTGGGGAGAAGAGAAGAGTACGACGT
GCTGGACAAGCGGAGAGGCAGAGATCCTGAAATGGGCGGCAAGCCCAGAC
GGAAGAATCCTCAAGAGGGCCTGTATAATGAGCTGCAGAAAGACAAGATG
GCCGAGGCCTACAGCGAGATCGGAATGAAGGGCGAGCGCAGAAGAGGCAA
GGGACACGATGGACTGTACCAGGGCCTGAGCACCGCCACCAAGGATACCTA
TGATGCCCTGCACATGCAGGCCCTGCCTCCAAGA

(SEQ ID NO: 35)

AGAGTGAAGTTCAGCAGGAGCGCAGACGCCCCCGCGTACAAGCAGGGCCA
GAACCAGCTCTATAACGAGCTCAATCTAGGACGAAGAGAGGAGTACGATGT
TTTGGACAAGAGACGTGGCCGGGACCCTGAGATGGGGGGAAAGCCGAGAA
GGAAGAACCCTCAGGAAGGCCTGTACAATGAACTGCAGAAAGATAAGATG
GCGGAGGCCTACAGTGAGATTGGGATGAAAGGCGAGCGCCGGAGGGGCAA
GGGGCACGATGGCCTTTACCAGGGTCTCAGTACAGCCACCAAGGACACCTA
CGACGCCCTTCACATGCAGGCCCTGCCCCCTCGC

(SEQ ID NO: 78)

AGAGTGAAGTTCAGCAGGAGCGCAGACGCCCCCGCGTACCAGCAGGGCCA
GAACCAGCTCTATAACGAGCTCAATCTAGGACGAAGAGAGGAGTACGATGT
TTTGGACAAGAGACGTGGCCGGGACCCTGAGATGGGGGGAAAGCCGAGAA
GGAAGAACCCTCAGGAAGGCCTGTACAATGAACTGCAGAAAGATAAGATG

GCGGAGGCCTACAGTGAGATTGGGATGAAAGGCGAGCGCCGGAGGGGCAA
GGGGCACGATGGCCTTTACCAGGGTCTCAGTACAGCCACCAAGGACACCTA
CGACGCCCTTCACATGCAGGCCCTGCCCCCTCGC

(SEQ ID NO: 85)

AGAGTGAAGTTCAGCAGATCCGCCGACGCTCCTGCCTATCAGCAGGGCCAA
AACCAGCTcTACAACGAGCTGAACCTGGGGAGAAGAGAAGAGTACGACGTG
CTGGACAAGCGGAGAGGCAGAGATCCTGAAATGGGCGGCAAGCCCAGACG
GAAGAATCCTCAAGAGGGCCTGTATAATGAGCTaCAGAAAGACAAGATGGC
aGAGGCCTACAGCGAGATCGGAATGAAGGGCGAGCGCAGAAGAGGCAAGG
GACACGATGGACTGTACCAGGGCCTGAGCACCGCCACCAAGGATACCTATG
ATGCCCTGCACATGCAGGCCCTGCCTCCAAGA

(SEQ ID NO: 86)

AGAGTGAAGTTCAGCAGATCCGCCGACGCTCCTGCCTATCAGCAGGGCCAA
AACCAGCTcTACAACGAGCTGAACCTGGGGAGAAGAGAAGAGTACGACGTG
CTGGACAAGCGGAGAGGCAGAGATCCTGAAATGGGCGGCAAGCCCAGACG
GAAGAATCCTCAAGAGGGCCTGTATAATGAGCTaCAGAAAGACAAGATGGC
aGAGGCCTACAGCGAGATCGGAATGAAGGGCGAGCGCAGAAGAGGCAAGG
GACACGATGGACTGTACCAGGGCCTGAGCACCGCCACCAAGGATACCTATG
ATGCCCTGCACATGCAGGCCCTGCCTCCAAGATAG

T cell primary signaling domains that act in a stimulatory manner may comprise signaling motifs known as immunoreceptor tyrosine-based activation motifs (ITAMS).

Examples of ITAM containing T cell primary intracellular signaling domains that are of particular use in the invention include, but are not limited to, those of (or derived from) CD3 zeta, common FcR gamma (FCER1G), Fc gamma RIIa, FcR beta (Fc Epsilon R1b), CD3 gamma, CD3 delta, CD3 epsilon, CD5, CD22, CD66b, CD79a, CD79b, DAP10, and DAP12.

In one embodiment, the T cell primary signaling domain comprises a modified ITAM domain, *e.g.,* a mutated ITAM domain which has altered (*e.g.,* increased or decreased) activity as compared to the native ITAM domain. In one embodiment, a primary signaling domain comprises a modified ITAM-containing primary intracellular signaling domain, *e.g.,* an optimized and/or truncated ITAM-containing primary intracellular signaling domain. In an embodiment, a primary signaling domain comprises one, two, three, four or more ITAM motifs.

In one embodiment, the intracellular signaling domain of the CAR of the invention comprises a T cell primary signaling domain (such as, for example, a CD3-zeta signaling domain), combined with one or more costimulatory signaling domains.

Examples of intracellular domains of a T cell costimulatory molecule include, but are not limited to, the signaling domains of proteins selected in the group of 4-1BB (CD137), CD27, CD28, an MHC class I molecule, BTLA, a Toll ligand receptor, OX40, CD30, CD40, PD-1, ICOS (CD278), lymphocyte function-associated antigen-1 (LFA-1), CD2, CD7, LIGHT, NKG2C, B7-H3, a ligand that specifically binds with CD83, CDS, ICAM-1, GITR, ARHR, BAFFR, HVEM (LIGHTR), SLAMF7, NKp80 (KLRF1), NKp44, NKp30, NKp46, CD160 (BY55), CD19, CD19a, CD4, CD8alpha, CD8beta, IL2ra, IL6Ra, IL2R beta, IL2R gamma, IL7R alpha, IL-13RA1/RA2, IL-33R(IL1RL1), IL-10RA/RB, IL-4R, IL-5R (CSF2RB), IL-21R, ITGA4, VLA1, CD49a, ITGA4, IA4, CD49D, ITGA6, VLA-6, CD49f, ITGAD, CD11d, ITGAE, CD103, ITGAL, CD11a/CD18, ITGAM, CD11b, ITGAX, CD11c, ITGB1, CD29, ITGB2, CD18, ITGB7, NKG2D, NKG2C, CTLA-4 (CD152), CD95, TNFR1 (CD120a/TNFRSF1A), TNFR2 (CD120b/TNFRSF1B), TGFbR1/2/3, TRANCE/RANKL, DNAM1 (CD226), SLAMF4 (CD244, 2B4), CD84, CD96 (Tactile), CEACAM1, CRTAM, Ly9 (CD229), PSGL1, CD100 (SEMA4D), CD69, SLAMF6 (NTB-A, Ly108), SLAM (SLAMF1, CD150, IPO-3), BLAME (SLAMF8), SELPLG (CD162), LTBR, LAT, GADS, SLP-76, PAG/Cbp, common gamma chain, a ligand that specifically binds with CD83, NKp44, NKp30, NKp46, or NKG2D, and any combination thereof.

In one embodiment of the invention, the chimeric receptor comprises at least one intracellular domain of a T cell costimulatory molecule selected from the group comprising 4-1BB, ICOS, CD27, OX40, CD28, CTLA4 and PD-1.

In one embodiment of the invention, the chimeric receptor comprises at least one intracellular domain of 4-1BB, preferably of human 4-1BB.

In one embodiment, the T cell costimulatory signaling domain comprises or consists in the amino acid sequence of a 4-1BB intracellular domain (preferably SEQ ID NO: 36) or an amino acid sequence with at least about 95, preferably about 96%, 97%, 98% or 99% identity to SEQ ID NO: 36. In another embodiment, the T cell costimulatory signaling domain comprises or consists in an amino acid sequence having at least one, two or three modifications but not more than 20, 10 or 5 modifications of an amino acid sequence of SEQ ID NO: 36.

(SEQ ID NO: 36)

KRGRKKLLYIFKQPFMRPVQTTQEEDGCSCRFPEEEEGGCEL

In one embodiment, the chimeric receptor comprises a transmembrane domain of 4-1BB and an intracellular domain of 4-1BB. In one embodiment, the transmembrane domain of 4-1BB and the intracellular domain of 4-1BB are contiguous. In one embodiment, the chimeric receptor comprises a transmembrane domain of human 4-1BB contiguous to an intracellular domain of human 4-1BB, preferably a sequence SEQ ID NO: 53.

(SEQ ID NO: 53)

IISFFLALTSTALLFLLFFLTLRFSVVKRGRKKLLYIFKQPFMRPVQTTQEEDGCS
CRFPEEEEGGCEL

Thus, in one embodiment, the nucleic acid sequence encoding the T cell costimulatory signaling domain comprises or consists of the nucleic acid sequence of a 4-1BB intracellular domain (preferably SEQ ID NO: 39 or 79) or a nucleic acid sequence with at least about 95, preferably about 96%, 97%, 98% or 99% identity to SEQ ID NO: 39 or 79.

(SEQ ID NO: 39)

AAGCGGGGCAGAAAGAAACTGCTCTACATCTTCAAGCAGCCCTTCATGCGG
CCCGTGCAGACCACACAAGAGGAAGATGGCTGCTCCTGCAGATTCCCCGAG
GAAGAAGAAGGCGGCTGCGAGCTG

(SEQ ID NO: 79)

AAACGGGGCAGAAAGAAACTCCTGTATATATTCAAACAACCATTTATGAGA
CCAGTACAAACTACTCAAGAGGAAGATGGCTGTAGCTGCCGATTTCCAGAA
GAAGAAGAAGGAGGATGTGAACTG

In one embodiment, the T cell costimulatory signaling domain comprises or consists in the amino acid sequence of a CD27 intracellular domain (SEQ ID NO: 37) or an amino acid sequence with at least about 95, preferably about 96%, 97%, 98% or 99% identity to SEQ ID NO: 37. In another embodiment, the T cell costimulatory signaling domain comprises or consists in an amino acid sequence having at least one, two or three modifications but not more than 20, 10 or 5 modifications of an amino acid sequence of SEQ ID NO: 37.

(SEQ ID NO: 37)

QRRKYRSNKGESPVEPAEPCRYSCPREEEGSTIPIQEDYRKPEPACSP

Thus, in one embodiment, the nucleic acid sequence encoding the T cell costimulatory signaling domain comprises or consists of the nucleic acid sequence of a CD27 intracellular domain (preferably SEQ ID NO: 40) or a nucleic acid sequence with at least about 95, preferably about 96%, 97%, 98% or 99% identity to SEQ ID NO: 40.

(SEQ ID NO: 40)

CAACGAAGGAAATATAGATCAAACAAAGGAGAAAGTCCTGTGGAGCCTGC
AGAGCCTTGTCGTTACAGCTGCCCCAGGGAGGAGGAGGGCAGCACCATCCC
CATCCAGGAGGATTACCGAAAACCGGAGCCTGCCTGCTCCCCC

In one embodiment, the T cell costimulatory signaling domain comprises or consists in the amino acid sequence of a CD28 intracellular domain (SEQ ID NO: 38) or an amino acid sequence with at least about 95, preferably about 96%, 97%, 98% or 99% identity to SEQ ID NO: 38. In another embodiment, the T cell costimulatory signaling domain comprises or consists in an amino acid sequence having at least one, two or three modifications but not more than 20, 10 or 5 modifications of an amino acid sequence of SEQ ID NO: 38.
(SEQ ID NO: 38)
RSKRSRLLHSDYMNMTPRRPGPTRKHYQPYAPPRDFAAYRS
Thus, in one embodiment, the nucleic acid sequence encoding the T cell costimulatory signaling domain comprises or consists of the nucleic acid sequence of a CD28 intracellular domain (preferably SEQ ID NO: 41 or SEQ ID NO: 84) or a nucleic acid sequence with at least about 95, preferably about 96%, 97%, 98% or 99% identity to SEQ ID NO: 41 or 84.

(SEQ ID NO: 41)

AGGAGTAAGAGGAGCAGGCTCCTGCACAGTGACTACATGAACATGACTCCC
CGCCGCCCCGGGCCCACCCGCAAGCATTACCAGCCCTATGCCCCACCACGC
GACTTCGCAGCCTATCGCTCC

(SEQ ID NO: 84)

CGGAGCAAGAGAAGCAGACTGCTGCACAGCGACTACATGAACATGACCCCT
AGACGGCCCGGACCTACCAGAAAGCACTACCAGCCTTACGCTCCTCCTAGA
GACTTCGCCGCCTACAGATCC

In one embodiment of the invention, the chimeric receptor comprises a combination of at least two intracellular domains of a T cell costimulatory molecule, preferably selected from an intracellular domain of 4-1BB, an intracellular domain of CD28, and an intracellular domain of CD27.
In one embodiment, the chimeric receptor comprises the amino acid sequence of a 4-1BB intracellular domain (SEQ ID NO: 36) or an amino acid sequence with at least about 95, preferably about 96%, 97%, 98% or 99% identity to SEQ ID NO: 36 and the amino acid sequence of a CD27 intracellular domain (SEQ ID NO: 37) or an amino acid sequence with at least about 95, preferably about 96%, 97%, 98% or 99% identity to SEQ ID NO: 37.
In another embodiment, the chimeric receptor comprises the amino acid sequence of a 4-1BB intracellular domain (SEQ ID NO: 36) or an amino acid sequence with at least about 95, preferably about 96%, 97%, 98% or 99% identity to SEQ ID NO: 36 and the amino acid sequence of a CD28 intracellular domain (SEQ ID NO: 38) or an amino acid sequence with at least about 95, preferably about 96%, 97%, 98% or 99% identity to SEQ ID NO: 38.
In yet another embodiment, the chimeric receptor comprises the amino acid sequence of a CD27 intracellular domain (SEQ ID NO: 37) or an amino acid sequence with at least about 95, preferably about 96%, 97%, 98% or 99% identity to SEQ ID NO: 37 and the amino acid sequence of a CD28 intracellular domain (SEQ ID NO: 38) or an amino acid sequence with at least about 95, preferably about 96%, 97%, 98% or 99% identity to SEQ ID NO: 38.
In one embodiment, the chimeric receptor comprises the amino acid sequence of a 4-1BB intracellular domain (SEQ ID NO: 36) or an amino acid sequence with at least about 95, preferably about 96%, 97%, 98% or 99% identity to SEQ ID NO: 36 and the amino acid sequence of a CD27 intracellular domain (SEQ ID NO: 37) or an amino acid sequence with at least about 95, preferably about 96%, 97%, 98% or 99% identity to SEQ ID NO: 37 and the amino acid sequence of a CD28 intracellular domain (SEQ ID NO: 38) or an amino acid sequence with at least about 95, preferably about 96%, 97%, 98% or 99% identity to SEQ ID NO: 38.

Thus, in one embodiment, the nucleic acid sequence encoding the T cell costimulatory signaling domain comprises the nucleic acid sequence of a 4-1BB intracellular domain (SEQ ID NO: 39 or 79) or a nucleic acid sequence with at least about 95, preferably about 96%, 97%, 98% or 99% identity to SEQ ID NO: 39 or 79, and/or the nucleic acid sequence of a CD27 intracellular domain (SEQ ID NO: 40) or a nucleic acid sequence with at least about 95, preferably about 96%, 97%, 98% or 99% identity to SEQ ID NO: 40, and/or the nucleic acid sequence of a CD28 intracellular domain (SEQ ID NO: 41 or 84), or a nucleic acid sequence with at least about 95, preferably about 96%, 97%, 98% or 99% identity to SEQ ID NO: 41 or 84.

In embodiment, the intracellular signaling domain of the CAR of the invention comprises:

- the amino acid sequence of a 4-1BB intracellular domain of SEQ ID NO: 36 or an amino acid sequence with at least about 95, preferably about 96%, 97%, 98% or 99% identity to SEQ ID NO: 36; and
- the amino acid sequence of a CD3-zeta intracellular domain of SEQ ID NO: 30, 31, 32 or 33, or an amino acid sequence with at least about 95, preferably about 96%, 97%, 98% or 99% identity to SEQ ID NO: 30, 31, 32 or 33;

wherein the sequences comprised in the intracellular domain are expressed in the same frame and as a single polypeptide chain.

In embodiment, the intracellular signaling domain of the CAR of the invention comprises:

- the amino acid sequence of a 4-1BB intracellular domain of SEQ ID NO: 36 or an amino acid sequence with at least about 95, preferably about 96%, 97%, 98% or 99% identity to SEQ ID NO: 36; and at least one of the amino acid sequence of a CD27 intracellular domain of SEQ ID NO: 37 or an amino acid sequence with at least about 95, preferably about 96%, 97%, 98% or 99% identity to SEQ ID NO: 37, and of the amino acid sequence of a CD28 intracellular domain of SEQ ID NO: 38 or an amino acid sequence with at least about 95, preferably about 96%, 97%, 98% or 99% identity to SEQ ID NO: 38; and
- the amino acid sequence of a CD3-zeta intracellular domain of SEQ ID NO: 30, 31, 32 or 33, or an amino acid sequence with at least about 95, preferably about 96%, 97%, 98% or 99% identity to SEQ ID NO: 30, 31, 32 or 33;

wherein the sequences comprised in the intracellular domain are expressed in the same frame and as a single polypeptide chain.

In embodiment, the intracellular signaling domain of the CAR of the invention comprises:

- the amino acid sequence of a 4-1BB intracellular domain of SEQ ID NO: 36 or an amino acid sequence with at least about 95, preferably about 96%, 97%, 98% or 99% identity to SEQ ID NO: 36, and/or the amino acid sequence of a CD27 intracellular domain of SEQ ID NO: 37 or an amino acid sequence with at least about 95, preferably about 96%, 97%, 98% or 99% identity to SEQ ID NO: 37, and/or the amino acid sequence of a CD28 intracellular domain of SEQ ID NO: 38 or an amino acid sequence with at least about 95, preferably about 96%, 97%, 98% or 99% identity to SEQ ID NO: 38; and
- the amino acid sequence of a CD3-zeta intracellular domain of SEQ ID NO: 30, 31, 32 or 33, or an amino acid sequence with at least about 95, preferably about 96%, 97%, 98% or 99% identity to SEQ ID NO: 30, 31, 32 or 33;

wherein the sequences comprised in the intracellular domain are expressed in the same frame and as a single polypeptide chain.

In one embodiment, the nucleic acid sequence encoding the intracellular signaling domain of the CAR of the invention comprises:

- the nucleic acid sequence of a 4-1BB intracellular domain of SEQ ID NO: 39 or 79 or a nucleic acid sequence with at least about 95, preferably about 96%, 97%, 98% or 99% identity to SEQ ID NO: 39 or 79; and
- the nucleic acid sequence of a CD3-zeta intracellular domain of SEQ ID NO: 34 or SEQ ID NO: 35 or SEQ ID NO: 78 or SEQ ID NO: 85 or SEQ ID NO: 86, or a sequence with at least about 95, preferably about 96%, 97%, 98% or 99% identity to SEQ ID NO: 34, 35, 78, 85 or 86.

In one embodiment, the nucleic acid sequence encoding the intracellular signaling domain of the CAR of the invention comprises:

- the nucleic acid sequence of a 4-1BB intracellular domain of SEQ ID NO: 39 or 79 or a nucleic acid sequence with at least about 95, preferably about 96%, 97%, 98% or 99% identity to SEQ ID NO: 39 or 79; and at least one of the nucleic acid sequence of a CD27 intracellular domain of SEQ ID NO: 40 or a nucleic acid sequence with at least about 95, preferably about 96%, 97%, 98% or 99% identity to SEQ ID NO: 40, and/or the nucleic acid sequence of a CD28 intracellular domain of SEQ ID NO: 41 or 84 or a nucleic acid sequence with at least about 95, preferably about 96%, 97%, 98% or 99% identity to SEQ ID NO: 41 or 84; and

- the nucleic acid sequence of a CD3-zeta intracellular domain of SEQ ID NO: 34 or SEQ ID NO: 35 or SEQ ID NO: 78 or SEQ ID NO: 85 or SEQ ID NO: 86, or a sequence with at least about 95, preferably about 96%, 97%, 98% or 99% identity to SEQ ID NO: 34, 35, 78, 85 or 86.

In one embodiment, the nucleic acid sequence encoding the intracellular signaling domain of the CAR of the invention comprises:

- the nucleic acid sequence of a 4-1BB intracellular domain of SEQ ID NO: 39 or 79 or a nucleic acid sequence with at least about 95, preferably about 96%, 97%, 98% or 99% identity to SEQ ID NO: 39 or 79, and/or the nucleic acid sequence of a CD27 intracellular domain of SEQ ID NO: 40 or a nucleic acid sequence with at least about 95, preferably about 96%, 97%, 98% or 99% identity to SEQ ID NO: 40, and/or the nucleic acid sequence of a CD28 intracellular domain of SEQ ID NO: 41 or 84 or a nucleic acid sequence with at least about 95, preferably about 96%, 97%, 98% or 99% identity to SEQ ID NO: 41 or 84; and
- the nucleic acid sequence of a CD3-zeta intracellular domain of SEQ ID NO: 34 or SEQ ID NO: 35 or SEQ ID NO: 78 or SEQ ID NO: 85 or SEQ ID NO: 86, or a sequence with at least about 95, preferably about 96%, 97%, 98% or 99% identity to SEQ ID NO: 34, 35, 78, 85 or 86.

In one embodiment, the intracellular signaling domain of the CAR of the invention comprises at least two different domains (*e.g.*, a primary signaling domain and at least one intracellular domain of a T cell costimulatory molecule) that may be linked to each other in a random or in a specified order.

Optionally, a short oligo- or polypeptide linker, for example, between 2 and 10 amino acids (*e.g.,* 2, 3, 4, 5, 6, 7, 8, 9, or 10 amino acids) in length may form the linkage between distinct signaling domains. In one embodiment, a glycine-serine doublet (GS) is used as a suitable linker. In one embodiment, a single amino acid, *e.g.,* an alanine (A), a glycine (G), is used as a suitable linker. Other examples of linker are described herein.

In another embodiment, the intracellular signaling domain of the CAR of the invention comprises two or more, *e.g.*, 2, 3, 4, 5, or more, costimulatory signaling domains. In another embodiment, the two or more, *e.g.*, 2, 3, 4, 5, or more, costimulatory signaling domains, are separated by a linker molecule, *e.g.,* a linker molecule as described hereinabove.

In one embodiment, the intracellular signaling domain of the chimeric receptor of the invention comprises the primary signaling domain of CD3-zeta (preferably SEQ ID NO: 30, 31, 32 or 33) and the co-stimulatory signaling domain of 4-1BB (preferably SEQ ID NO: 36).

In one embodiment, the intracellular signaling domain of the chimeric receptor of the invention comprises the primary signaling domain of CD3-zeta (preferably SEQ ID NO: 30, 31, 32 or 33) and the co-stimulatory signaling domain of CD28 (preferably SEQ ID NO: 38).

In another embodiment, the intracellular signaling domain of the chimeric receptor of the invention comprises the signaling domain of CD3-zeta (preferably SEQ ID NO: 30, 31, 32 or 33) and the signaling domain of CD27 (preferably SEQ ID NO: 37).

In one embodiment, the CAR of the invention further comprises a leader sequence located N-terminally from the IL-23R specific extracellular binding domain. A non-limiting example of leader sequence is a leader sequence of CD8 that may comprise or consists in the sequence SEQ ID NO: 42 (that may be encoded by a sequence SEQ ID NO: 54) or SEQ ID NO: 80.

(SEQ ID NO: 42)
MALPVTALLLPLALLLHAARPS

(SEQ ID NO: 54)

ATGGCTCTGCCTGTGACAGCTCTGCTGCTGCCTCTGGCTCTGCTTCTTCATGC
CGCCAGACCATCT

(SEQ ID NO: 80)
MALPVTALLLPLALLLHAARP

In one embodiment, the CAR further comprises a tag, such as, for example, a tag for quality control, enrichment, tracking in vivo and the like. Said tag may be localized N-terminally, C-terminally and/or internally. Examples of tags that may be used in the CAR of the invention are well known by the skilled artisan. For example, but without limitation, a tag used in the invention can be a tag selected from the group comprising or consisting of Hemagglutinin Tag, Poly Arginine Tag, Poly Histidine Tag, Myc Tag, Strep Tag, STag, HAT Tag, 3x Flag Tag, Calmodulin-binding peptide Tag, SBP Tag, Chitin binding domain Tag, GST Tag, Maltose-Binding protein Tag, Fluorescent Protein Tag, T7 Tag, V5 Tag and Xpress Tag. Other examples of tag include, without limitation, NWSHPQFEK (SEQ ID NO: 43) or SAWSHPQFEK (SEQ ID NO: 44).

In one embodiment, the CAR of the invention further comprises P2A (SEQ ID NO: 45, that may be encoded by SEQ ID NO: 58) and GFP (SEQ ID NO: 46, that may be encoded by SEQ ID NO: 59) sequences.

(SEQ ID NO: 45)
GSGATNF SLLKQAGDVEENPGP

(SEQ ID NO: 46)

MVSKGEELFTGVVPILVELDGDVNGHKFSVSGEGEGDATYGKLTLKFICTTGKL
PVPWPTLVTTLTYGVQCFSRYPDHMKQHDFFKSAMPEGYVQERTIFFKDDGNY
KTRAEVKFEGDTLVNRIELKGIDFKEDGNILGHKLEYNYNSHNVYIMADKQKN
GIKVNFKIRHNIEDGSVQLADHYQQNTPIGDGPVLLPDNHYLSTQSALSKDPNE
KRDHMVLLEFVTAAGITLGMDELYK

(SEQ ID NO: 58)

GGAAGCGGAGCTACTAACTTCAGCCTGCTGAAGCAGGCTGGAGACGTGGAG
GAGAACCCTGGACCT

(SEQ ID NO: 59)

ATGGTGAGCAAGGGCGAGGAGCTGTTCACCGGGGTGGTGCCCATCCTGGTC
GAGCTGGACGGCGACGTAAACGGCCACAAGTTCAGCGTGTCCGGCGAGGGC
GAGGGCGATGCCACCTACGGCAAGCTGACCCTGAAGTTCATCTGCACCACC
GGCAAGCTGCCCGTGCCCTGGCCCACCCTCGTGACCACCCTGACCTACGGC
GTGCAGTGCTTCAGCCGCTACCCCGACCACATGAAGCAGCACGACTTCTTCA
AGTCCGCCATGCCCGAAGGCTACGTCCAGGAGCGCACCATCTTCTTCAAGG
ACGACGGCAACTACAAGACCCGCGCCGAGGTGAAGTTCGAGGGCGACACC
CTGGTGAACCGCATCGAGCTGAAGGGCATCGACTTCAAGGAGGACGGCAAC
ATCCTGGGGCACAAGCTGGAGTACAACTACAACAGCCACAACGTCTATATC
ATGGCCGACAAGCAGAAGAACGGCATCAAGGTGAACTTCAAGATCCGCCAC
AACATCGAGGACGGCAGCGTGCAGCTCGCCGACCACTACCAGCAGAACACC
CCCATCGGCGACGGCCCCGTGCTGCTGCCCGACAACCACTACCTGAGCACC
CAGTCCGCCCTGAGCAAAGACCCCAACGAGAAGCGCGATCACATGGTCCTG
CTGGAGTTCGTGACCGCCGCCGGGATCACTCTCGGCATGGACGAGCTGTAC
AAGTAA

According to a first embodiment, the CAR of the invention comprises an extracellular IL-23R binding domain, optionally an extracellular hinge domain, a transmembrane domain, and an intracellular signaling domain.

In one embodiment, the CAR of the invention comprises an IL-23R binding domain; a transmembrane domain of 4-1BB (preferably SEQ ID NO: 28); and a CD3-zeta primary signaling domain (preferably SEQ ID NO: 30, 31, 32 or 33).

In another embodiment, the CAR of the invention comprises an IL-23R binding domain; a hinge domain of CD8 (preferably SEQ ID NO: 20 or SEQ ID NO: 60); a transmembrane domain of 4-1BB (preferably SEQ ID NO: 28); and a CD3-zeta primary signaling domain (preferably SEQ ID NO: 30, 31, 32 or 33).

In another embodiment, the CAR of the invention comprises an IL-23R binding domain; a hinge domain of IgG4 (preferably SEQ ID NO: 22); a transmembrane domain of 4-1BB (preferably SEQ ID NO: 28); and a CD3-zeta primary signaling domain (preferably SEQ ID NO: 30, 31, 32 or 33).

In another embodiment, the CAR of the invention comprises an IL-23R binding domain; a hinge domain of IgD (preferably SEQ ID NO: 24); a transmembrane domain of 4-1BB (preferably SEQ ID NO: 28); and a CD3-zeta primary signaling domain (preferably SEQ ID NO: 30, 31, 32 or 33).

In another embodiment, the CAR of the invention comprises an IL-23R binding domain; a hinge domain of CD28 (preferably SEQ ID NO: 26); a transmembrane domain of 4-1BB (preferably SEQ ID NO: 28); and a CD3-zeta primary signaling domain (preferably SEQ ID NO: 30, 31, 32 or 33).

According to a second embodiment, the CAR of the invention comprises an IL-23R binding domain, optionally an extracellular hinge domain, a transmembrane domain, a single intracellular domain of a T cell costimulatory molecule and a T cell primary signaling domain.

In one embodiment, the CAR of the invention comprises an IL-23R binding domain; a transmembrane domain of 4-1BB (preferably SEQ ID NO: 28); an intracellular domain of 4-1BB (preferably SEQ ID NO: 36); and a CD3-zeta primary signaling domain (preferably SEQ ID NO: 30, 31, 32 or 33).

In one embodiment, the CAR of the invention comprises an IL-23R binding domain; a hinge domain of CD8 (preferably SEQ ID NO: 20 or SEQ ID NO: 60); a transmembrane domain of 4-1BB (preferably SEQ ID NO: 28); an intracellular domain of 4-1BB (preferably SEQ ID NO: 36); and a CD3-zeta primary signaling domain (preferably SEQ ID NO: 30, 31, 32 or 33).

In one embodiment, the CAR of the invention comprises an IL-23R binding domain; a hinge domain of IgG4 (preferably SEQ ID NO: 22); a transmembrane domain of 4-1BB (preferably SEQ ID NO: 28); an intracellular domain of 4-1BB (preferably SEQ ID NO: 36); and a CD3-zeta primary signaling domain (preferably SEQ ID NO: 30, 31, 32 or 33).

In one embodiment, the CAR of the invention comprises an IL-23R binding domain; a hinge domain of IgD (preferably SEQ ID NO: 24); a transmembrane domain of 4-1BB (preferably SEQ ID NO: 28); an intracellular domain of 4-1BB (preferably SEQ ID NO: 36); and a CD3-zeta primary signaling domain (preferably SEQ ID NO: 30, 31, 32 or 33).

In one embodiment, the CAR of the invention comprises an IL-23R binding domain; a hinge domain of CD28 (preferably SEQ ID NO: 26); a transmembrane domain of 4-1BB (preferably SEQ ID NO: 28); an intracellular domain of 4-1BB (preferably SEQ ID NO: 36); and a CD3-zeta primary signaling domain (preferably SEQ ID NO: 30, 31, 32 or 33).

In one embodiment, the CAR of the invention comprises an IL-23R binding domain; a transmembrane domain of 4-1BB (preferably SEQ ID NO: 28); an intracellular domain of CD28 (preferably SEQ ID NO: 38); and a CD3-zeta primary signaling domain (preferably SEQ ID NO: 30, 31, 32 or 33).

In one embodiment, the CAR of the invention comprises an IL-23R binding domain; a hinge domain of CD8 (preferably SEQ ID NO: 20 or SEQ ID NO: 60); a transmembrane domain of 4-1BB (preferably SEQ ID NO: 28); an intracellular domain of CD28 (preferably SEQ ID NO: 38); and a CD3-zeta primary signaling domain (preferably SEQ ID NO: 30, 31, 32 or 33).

In one embodiment, the CAR of the invention comprises an IL-23R binding domain; a hinge domain of IgG4 (preferably SEQ ID NO: 22); a transmembrane domain of 4-1BB (preferably SEQ ID NO: 28); an intracellular domain of CD28 (preferably SEQ ID NO: 38); and a CD3-zeta primary signaling domain (preferably SEQ ID NO: 30, 31, 32 or 33).

In one embodiment, the CAR of the invention comprises an IL-23R binding domain; a hinge domain of IgD (preferably SEQ ID NO: 24); a transmembrane domain of 4-1BB (preferably SEQ ID NO: 28); an intracellular domain of CD28 (preferably SEQ ID NO: 38); and a CD3-zeta primary signaling domain (preferably SEQ ID NO: 30, 31, 32 or 33).

In one embodiment, the CAR of the invention comprises an IL-23R binding domain; a hinge domain of CD28 (preferably SEQ ID NO: 26); a transmembrane domain of 4-1BB (preferably SEQ ID NO: 28); an intracellular domain of CD28 (preferably SEQ ID NO: 38); and a CD3-zeta primary signaling domain (preferably SEQ ID NO: 30, 31, 32 or 33). In one embodiment, the CAR of the invention comprises an IL-23R binding domain; a transmembrane domain of 4-1BB (preferably SEQ ID NO: 28); an intracellular domain of CD27 (preferably SEQ ID NO: 37); and a CD3-zeta primary signaling domain (preferably SEQ ID NO: 30, 31, 32 or 33).

In one embodiment, the CAR of the invention comprises an IL-23R binding domain; a hinge domain of CD8 (preferably SEQ ID NO: 20 or SEQ ID NO: 60); a transmembrane domain of 4-1BB (preferably SEQ ID NO: 28); an intracellular domain of CD27 (preferably SEQ ID NO: 37); and a CD3-zeta primary signaling domain (preferably SEQ ID NO: 30, 31, 32 or 33).

In one embodiment, the CAR of the invention comprises an IL-23R binding domain; a hinge domain of IgG4 (preferably SEQ ID NO: 22); a transmembrane domain of 4-1BB (preferably SEQ ID NO: 28); an intracellular domain of CD27 (preferably SEQ ID NO: 37); and a CD3-zeta primary signaling domain (preferably SEQ ID NO: 30, 31, 32 or 33).

In one embodiment, the CAR of the invention comprises an IL-23R binding domain; a hinge domain of IgD (preferably SEQ ID NO: 24); a transmembrane domain of 4-1BB (preferably SEQ ID NO: 28); an intracellular domain of CD27 (preferably SEQ ID NO: 37); and a CD3-zeta primary signaling domain (preferably SEQ ID NO: 30, 31, 32 or 33).

In one embodiment, the CAR of the invention comprises an IL-23R binding domain; a hinge domain of CD28 (preferably SEQ ID NO: 26); a transmembrane domain of 4-1BB (preferably SEQ ID NO: 28); an intracellular domain of CD27 (preferably SEQ ID NO: 37); and a CD3-zeta primary signaling domain (preferably SEQ ID NO: 30, 31, 32 or 33).

According to a third embodiment, the CAR of the invention comprises an IL-23R binding domain, optionally an extracellular hinge domain, a transmembrane domain, two intracellular domains of a T cell costimulatory molecule and a T cell primary signaling domain.

In one embodiment, the CAR of the invention comprises an IL-23R binding domain; a transmembrane domain of 4-1BB (preferably SEQ ID NO: 28); an intracellular domain of 4-1BB (preferably SEQ ID NO: 36); an intracellular domain of CD28 (preferably SEQ ID NO: 38); and a CD3-zeta primary signaling domain (preferably SEQ ID NO: 30, 31, 32 or 33).

In one embodiment, the CAR of the invention comprises an IL-23R binding domain; a hinge domain of CD8 (preferably SEQ ID NO: 20 or SEQ ID NO: 60); a transmembrane domain of 4-1BB (preferably SEQ ID NO: 28); an intracellular domain of 4-1BB (preferably SEQ ID NO: 36); an intracellular domain of CD28 (preferably SEQ ID NO: 38); and a CD3-zeta primary signaling domain (preferably SEQ ID NO: 30, 31, 32 or 33).

In one embodiment, the CAR of the invention comprises an IL-23R binding domain; a hinge domain of IgG4 (preferably SEQ ID NO: 22); a transmembrane domain of 4-1BB (preferably SEQ ID NO: 28); an intracellular domain of 4-1BB (preferably SEQ ID NO: 36); an intracellular domain of CD28 (preferably SEQ ID NO: 38); and a CD3-zeta primary signaling domain (preferably SEQ ID NO: 30, 31, 32 or 33).

In one embodiment, the CAR of the invention comprises an IL-23R binding domain; a hinge domain of IgD (preferably SEQ ID NO: 24); a transmembrane domain of 4-1BB (preferably SEQ ID NO: 28); an intracellular domain of 4-1BB (preferably SEQ ID NO: 36); an intracellular domain of CD28 (preferably SEQ ID NO: 38); and a CD3-zeta primary signaling domain (preferably SEQ ID NO: 30, 31, 32 or 33).

In one embodiment, the CAR of the invention comprises an IL-23R binding domain; a hinge domain of CD28 (preferably SEQ ID NO: 26); a transmembrane domain of 4-1BB (preferably SEQ ID NO: 28); an intracellular domain of 4-1BB (preferably SEQ ID NO: 36); an intracellular domain of CD28 (preferably SEQ ID NO: 38); and a CD3-zeta primary signaling domain (preferably SEQ ID NO: 30, 31, 32 or 33).

In one embodiment, the CAR of the invention comprises an IL-23R binding domain; a transmembrane domain of 4-1BB (preferably SEQ ID NO: 28); an intracellular domain of 4-1BB (preferably SEQ ID NO: 36); an intracellular domain of CD27 (preferably SEQ ID NO: 37); and a CD3-zeta primary signaling domain (preferably SEQ ID NO: 30, 31, 32 or 33).

In one embodiment, the CAR of the invention comprises an IL-23R binding domain; a hinge domain of CD8 (preferably SEQ ID NO: 20 or SEQ ID NO: 60); a transmembrane domain of 4-1BB (preferably SEQ ID NO: 28); an intracellular domain of 4-1BB (preferably SEQ ID NO: 36); an intracellular domain of CD27 (preferably SEQ ID NO: 37); and a CD3-zeta primary signaling domain (preferably SEQ ID NO: 30, 31, 32 or 33).

In one embodiment, the CAR of the invention comprises an IL-23R binding domain; a hinge domain of IgG4 (preferably SEQ ID NO: 22); a transmembrane domain of 4-1BB (preferably SEQ ID NO: 28); an intracellular domain of 4-1BB (preferably SEQ ID NO: 36); an intracellular domain of CD27 (preferably SEQ ID NO: 37); and a CD3-zeta primary signaling domain (preferably SEQ ID NO: 30, 31, 32 or 33).

In one embodiment, the CAR of the invention comprises an IL-23R binding domain; a hinge domain of IgD (preferably SEQ ID NO: 24); a transmembrane domain of 4-1BB (preferably SEQ ID NO: 28); an intracellular domain of 4-1BB (preferably SEQ ID NO: 36); an intracellular domain of CD27 (preferably SEQ ID NO: 37); and a CD3-zeta primary signaling domain (preferably SEQ ID NO: 30, 31, 32 or 33).

In one embodiment, the CAR of the invention comprises an IL-23R binding domain; a hinge domain of CD28 (preferably SEQ ID NO: 26); a transmembrane domain of 4-1BB (preferably SEQ ID NO: 28); an intracellular domain of 4-1BB (preferably SEQ ID NO: 36); an intracellular domain of CD27 (preferably SEQ ID NO: 37); and a CD3-zeta primary signaling domain (preferably SEQ ID NO: 30, 31, 32 or 33).

In one embodiment, the CAR of the invention comprises an IL-23R binding domain; a transmembrane domain of 4-1BB (preferably SEQ ID NO: 28); an intracellular domain of CD27 (preferably SEQ ID NO: 37); an intracellular domain of CD28 (preferably SEQ ID NO: 38); and a CD3-zeta primary signaling domain (preferably SEQ ID NO: 30, 31, 32 or 33).

In one embodiment, the CAR of the invention comprises an IL-23R binding domain; a hinge domain of CD8 (preferably SEQ ID NO: 20 or SEQ ID NO: 60); a transmembrane domain of 4-1BB (preferably SEQ ID NO: 28); an intracellular domain of CD27 (preferably SEQ ID NO: 37); an intracellular domain of CD28 (preferably SEQ ID NO: 38); and a CD3-zeta primary signaling domain (preferably SEQ ID NO: 30, 31, 32 or 33).

In one embodiment, the CAR of the invention comprises an IL-23R binding domain; a hinge domain of IgG4 (preferably SEQ ID NO: 22); a transmembrane domain of 4-1BB (preferably SEQ ID NO: 28); an intracellular domain of CD27 (preferably SEQ ID NO: 37); an intracellular domain of CD28 (preferably SEQ ID NO: 38); and a CD3-zeta primary signaling domain (preferably SEQ ID NO: 30, 31, 32 or 33).

In one embodiment, the CAR of the invention comprises an IL-23R binding domain; a hinge domain of IgD (preferably SEQ ID NO: 24); a transmembrane domain of 4-1BB (preferably SEQ ID NO: 28); an intracellular domain of CD27 (preferably SEQ ID NO: 37); an intracellular domain of CD28 (preferably SEQ ID NO: 38); and a CD3-zeta primary signaling domain (preferably SEQ ID NO: 30, 31, 32 or 33).

In one embodiment, the CAR of the invention comprises an IL-23R binding domain; a hinge domain of CD28 (preferably SEQ ID NO: 26); a transmembrane domain of 4-1BB (preferably SEQ ID NO: 28); an intracellular domain of CD27 (preferably SEQ ID NO: 37); an intracellular domain of CD28 (preferably SEQ ID NO: 38); and a CD3-zeta primary signaling domain (preferably SEQ ID NO: 30, 31, 32 or 33).

In one embodiment, the CAR of the invention comprises (i) an IL-23R binding domain, (ii) a hinge region of human CD8, (iii) a transmembrane domain of 4-1BB (preferably human 4-1BB), (iv) an intracellular domain of human 4-1BB (preferably human 4-1BB)and (v) an intracellular domain of human CD3ζ chain.

In one embodiment, the part of the CAR comprising a hinge region of human CD8, a transmembrane domain of human 4-1BB, an intracellular domain of human 4-1BB and an intracellular domain of human CD3ζ chain corresponds to the amino acid sequence of SEQ ID NO: 47 or an amino acid sequence with at least about 95, preferably about 96%, 97%, 98% or 99% identity to SEQ ID NO: 47. In one embodiment, the part of the CAR comprising a hinge region of human CD8, a transmembrane domain of human 4-1BB, an intracellular domain of human 4-1BB and an intracellular domain of human CD3ζ chain may be encoded by a nucleic acid sequence of SEQ ID NO: 48 or an amino acid sequence with at least about 95, preferably about 96%, 97%, 98% or 99% identity to SEQ ID NO: 48.

(SEQ ID NO: 47)

TTTPAPRPPTPAPTIASQPLSLRPEACRPAAGGAVHTRGLDFACDIISFFLALTST
ALLFLLFFLTLRFSVVKRGRKKLLYIFKQPFMRPVQTTQEEDGCSCRFPEEEEGG
CELTRRVKFSRSADAPAYQQGQNQLYNELNLGRREEYDVLDKRRGRDPEMGG
KPRRKNPQEGLYNELQKDKMAEAYSEIGMKGERRRGKGHDGLYQGLSTATKD
TYDALHMQALPPR

(SEQ ID NO: 48)

ACAACAACACCTGCTCCTCGGCCTCCTACACCAGCTCCTACAATTGCCAGCC
AGCCACTGTCTCTGAGGCCCGAAGCTTGCAGGCCTGCTGCTGGCGGAGCCG
TGCATACAAGAGGACTGGATTTCGCCTGCGACATCATCTCCTTCTTTCTTGC
GCTGACCTCGACTGCGTTGCTCTTCCTGCTGTTCTTCCTCACGCTCCGTTTCT
CTGTTGTTAAGCGGGGCAGAAAGAAACTGCTCTACATCTTCAAGCAGCCCTT
CATGCGGCCCGTGCAGACCACACAAGAGGAAGATGGCTGCTCCTGCAGATT
CCCCGAGGAAGAAGAAGGCGGCTGCGAGCTGACGCGTAGAGTGAAGTTCA
GCAGATCCGCCGACGCTCCTGCCTATCAGCAGGGCCAAAACCAGCTGTACA
ACGAGCTGAACCTGGGGAGAAGAGAAGAGTACGACGTGCTGGACAAGCGG
AGAGGCAGAGATCCTGAAATGGGCGGCAAGCCCAGACGGAAGAATCCTCA
AGAGGGCCTGTATAATGAGCTGCAGAAAGACAAGATGGCCGAGGCCTACA
GCGAGATCGGAATGAAGGGCGAGCGCAGAAGAGGCAAGGGACACGATGGA
CTGTACCAGGGCCTGAGCACCGCCACCAAGGATACCTATGATGCCCTGCAC
ATGCAGGCCCTGCCTCCAAGA

In one embodiment, the CAR of the invention comprises an IL-23R binding domain, linked to an amino acid sequence of SEQ ID NO: 47 or a sequence or an amino acid sequence with at least about 95, preferably about 96%, 97%, 98% or 99% identity to SEQ ID NO: 47.

In one embodiment, the part of the CAR comprising a hinge region of human CD8, a transmembrane domain of human 4-1BB, an intracellular domain of human 4-1BB and an intracellular domain of human CD3ζ chain corresponds to the amino acid sequence of SEQ ID NO: 66 or an amino acid sequence with at least about 95, preferably about 96%, 97%, 98% or 99% identity to SEQ ID NO: 66. In one embodiment, the part of the CAR comprising a hinge region of human CD8, a transmembrane domain of human 4-1BB, an intracellular domain of human 4-1BB and an intracellular domain of human CD3ζ chain may be encoded by a nucleic acid sequence of SEQ ID NO: 67 or an amino acid sequence with at least about 95, preferably about 96%, 97%, 98% or 99% identity to SEQ ID NO: 67.

(SEQ ID NO: 66)

TTTPAPRPPTPAPTIASQPLSLRPEACRPAAGGAVHTRGLDFACDIISFFLALTST
ALLFLLFFLTLRFSVVKRGRKKLLYIFKQPFMRPVQTTQEEDGCSCRFPEEEEGG
CELRVKFSRSADAPAYQQGQNQLYNELNLGRREEYDVLDKRRGRDPEMGGKP
RRKNPQEGLYNELQKDKMAEAYSEIGMKGERRRGKGHDGLYQGLSTATKDTY
DALHMQALPPR

(SEQ ID NO: 67)

ACAACAACACCTGCTCCTCGGCCTCCTACACCAGCTCCTACAATTGCCAGCC
AGCCACTGTCTCTGAGGCCCGAAGCTTGCAGGCCTGCTGCTGGCGGAGCCG
TGCATACAAGAGGACTGGATTTCGCCTGCGACATCATCTCCTTCTTTCTTGC
GCTGACCTCGACTGCGTTGCTCTTCCTGCTGTTCTTCCTCACGCTCCGTTTCT
CTGTTGTTAAGCGGGGCAGAAAGAAACTGCTCTACATCTTCAAGCAGCCCTT
CATGCGGCCCGTGCAGACCACACAAGAGGAAGATGGCTGCTCCTGCAGATT
CCCCGAGGAAGAAGAAGGCGGCTGCGAGCTGAGAGTGAAGTTCAGCAGAT
CCGCCGACGCTCCTGCCTATCAGCAGGGCCAAAACCAGCTCTACAACGAGC
TGAACCTGGGGAGAAGAGAAGAGTACGACGTGCTGGACAAGCGGAGAGGC
AGAGATCCTGAAATGGGCGGCAAGCCCAGACGGAAGAATCCTCAAGAGGG
CCTGTATAATGAGCTACAGAAAGACAAGATGGCAGAGGCCTACAGCGAGAT
CGGAATGAAGGGCGAGCGCAGAAGAGGCAAGGGACACGATGGACTGTACC

AGGGCCTGAGCACCGCCACCAAGGATACCTATGATGCCCTGCACATGCAGG
CCCTGCCTCCAAGATAG

In one embodiment, the CAR of the invention comprises an IL-23R binding domain, linked to an amino acid sequence of SEQ ID NO: 66 or a sequence or an amino acid sequence with at least about 95, preferably about 96%, 97%, 98% or 99% identity to SEQ ID NO: 66.

In one embodiment, the part of the CAR comprising a hinge region of human CD8, a transmembrane domain of human 4-1BB, an intracellular domain of human 4-1BB and an intracellular domain of human CD3ζ chain corresponds to the amino acid sequence of SEQ ID NO: 74 or an amino acid sequence with at least about 95, preferably about 96%, 97%, 98% or 99% identity to SEQ ID NO: 74. In one embodiment, the part of the CAR comprising a hinge region of human CD8, a transmembrane domain of human 4-1BB, an intracellular domain of human 4-1BB and an intracellular domain of human CD3ζ chain may be encoded by a nucleic acid sequence of SEQ ID NO: 75 or an amino acid sequence with at least about 95, preferably about 96%, 97%, 98% or 99% identity to SEQ ID NO: 75.

(SEQ ID NO: 74)

SALSNSIMYFSHFVPVFLPAKPTTTPAPRPPTPAPTIASQPLSLRPEACRPAAGGA
VHTRGLDFACDIISFFLALTSTALLFLLFFLTLRFSVVKRGRKKLLYIFKQPFMRP
VQTTQEEDGCSCRFPEEEEGGCELTRRVKFSRSADAPAYQQGQNQLYNELNLG
RREEYDVLDKRRGRDPEMGGKPRRKNPQEGLYNELQKDKMAEAYSEIGMKGE
RRRGKGHDGLYQGLSTATKDTYDALHMQALPPR

(SEQ ID NO: 75)

AGCGCCCTGAGCAACAGCATCATGTACTTCAGCCACTTCGTGCCCGTGTTTC
TGCCCGCCAAACCTACAACAACACCTGCTCCTCGGCCTCCTACACCAGCTCC
TACAATTGCCAGCCAGCCACTGTCTCTGAGGCCCGAAGCTTGCAGGCCTGCT
GCTGGCGGAGCCGTGCATACAAGAGGACTGGATTTCGCCTGCGACATCATC
TCCTTCTTTCTTGCGCTGACCTCGACTGCGTTGCTCTTCCTGCTGTTCTTCCTC

ACGCTCCGTTTCTCTGTTGTTAAGCGGGGCAGAAAGAAACTGCTCTACATCT
TCAAGCAGCCCTTCATGCGGCCCGTGCAGACCACACAAGAGGAAGATGGCT
GCTCCTGCAGATTCCCCGAGGAAGAAGAAGGCGGCTGCGAGCTGACGCGTA
GAGTGAAGTTCAGCAGATCCGCCGACGCTCCTGCCTATCAGCAGGGCCAAA
ACCAGCTGTACAACGAGCTGAACCTGGGGAGAAGAGAAGAGTACGACGTG
CTGGACAAGCGGAGAGGCAGAGATCCTGAAATGGGCGGCAAGCCCAGACG
GAAGAATCCTCAAGAGGGCCTGTATAATGAGCTGCAGAAAGACAAGATGG
CCGAGGCCTACAGCGAGATCGGAATGAAGGGCGAGCGCAGAAGAGGCAAG
GGACACGATGGACTGTACCAGGGCCTGAGCACCGCCACCAAGGATACCTAT
GATGCCCTGCACATGCAGGCCCTGCCTCCAAGA

In one embodiment, the CAR of the invention comprises an IL-23R binding domain, linked to an amino acid sequence of SEQ ID NO: 74 or a sequence or an amino acid sequence with at least about 95, preferably about 96%, 97%, 98% or 99% identity to SEQ ID NO: 74.

In one embodiment, the part of the CAR comprising a hinge region of human CD8, a transmembrane domain of human 4-1BB, an intracellular domain of human 4-1BB and an intracellular domain of human CD3ζ chain corresponds to the amino acid sequence of SEQ ID NO: 76 or an amino acid sequence with at least about 95, preferably about 96%, 97%, 98% or 99% identity to SEQ ID NO: 76. In one embodiment, the part of the CAR comprising a hinge region of human CD8, a transmembrane domain of human 4-1BB, an intracellular domain of human 4-1BB and an intracellular domain of human CD3ζ chain may be encoded by a nucleic acid sequence of SEQ ID NO: 77 or an amino acid sequence with at least about 95, preferably about 96%, 97%, 98% or 99% identity to SEQ ID NO: 77.

(SEQ ID NO: 76)

SALSNSIMYFSHFVPVFLPAKPTTTPAPRPPTPAPTIASQPLSLRPEACRPAAGGA
VHTRGLDFACDIISFFLALTSTALLFLLFFLTLRFSVVKRGRKKLLYIFKQPFMRP
VQTTQEEDGCSCRFPEEEEGGCELRVKFSRSADAPAYQQGQNQLYNELNLGRR
EEYDVLDKRRGRDPEMGGKPRRKNPQEGLYNELQKDKMAEAYSEIGMKGERR
RGKGHDGLYQGLSTATKDTYDALHMQALPPR

(SEQ ID NO: 77)

AGCGCCCTGAGCAACAGCATCATGTACTTCAGCCACTTCGTGCCCGTGTTTC
TGCCCGCCAAACCTACAACAACaCCTGCTCCTCGGCCTCCTACACCAGCTCC
TACAATTGCCAGCCAGCCACTGTCTCTGAGGCCCGAAGCTtgcaggCCTGCTGC
TGGCGGAGCCGTGCATACAAGAGGACTGGATTTCGCCTGCGACATCATCTC
CTTCTTTCTTGCGCTGACcTCGACTGCGTTGCTCTTCCTGCTGTTCTTCCTCAC
GCTCCGTTTCTCTGTTGTTAAGCGGGGCAGAAAGAAACTGCTCTACATCTTC
AAGCAGCCCTTCATGCGGCCCGTGCAGACCACACAAGAGGAAGATGGCTGC
TCCTGCAGATTCCCCGAGGAAGAAGAAGGCGGCTGCGAGCTGAGAGTGAA
GTTCAGCAGATCCGCCGACGCTCCTGCCTATCAGCAGGGCCAAAACCAGCTc
TACAACGAGCTGAACCTGGGGAGAAGAGAAGAGTACGACGTGCTGGACAA
GCGGAGAGGCAGAGATCCTGAAATGGGCGGCAAGCCCAGACGGAAGAATC
CTCAAGAGGGCCTGTATAATGAGCTaCAGAAAGACAAGATGGCaGAGGCCT
ACAGCGAGATCGGAATGAAGGGCGAGCGCAGAAGAGGCAAGGGACACGAT
GGACTGTACCAGGGCCTGAGCACCGCCACCAAGGATACCTATGATGCCCTG
CACATGCAGGCCCTGCCTCCAAGATAG

In one embodiment, the CAR of the invention comprises an IL-23R binding domain, linked to an amino acid sequence of SEQ ID NO: 76 or a sequence or an amino acid sequence with at least about 95, preferably about 96%, 97%, 98% or 99% identity to SEQ ID NO: 76.

In one embodiment, the CAR of the invention comprises an anti-IL-23R scFv (*e.g.,* comprising or consisting of a sequence SEQ ID NO: 11, 12 or 13, preferably SEQ ID NO: 11), a hinge region of CD8, a transmembrane domain of human 4-1BB, an intracellular domain of human 4-1BB and an intracellular domain of human CD3ζ.

In one embodiment, the CAR of the invention comprises a CD8 leader sequence having the SEQ ID NO: 42, an anti-human IL-23R scFv, comprising a $V_H$ having the sequence SEQ ID NO: 1 and a $V_L$ having SEQ ID NO: 2, linked by a $(G_4S)_3$ linker (SEQ ID NO: 9), a hinge domain derived from CD8α having the sequence of SEQ ID NO: 20, a human 4-1BB transmembrane domain having the SEQ ID NO: 28, and an intracellular signaling domain comprising a human 4-1BB signaling domain having SEQ ID NO: 36 and a human CD3 zeta domain having SEQ ID NO: 31.

In one embodiment, the CAR of the invention has a sequence SEQ ID NO: 50 or a sequence with at least about 95, preferably about 96%, 97%, 98% or 99% identity to SEQ ID NO: 50.

(SEQ ID NO: 50)

MALPVTALLLPLALLLHAARPSEVQLVESGGGLVQPGGSLRLSCAASGIDFNSN
YYMCWVRQAPGKGLEWIGCIYVGSHVNTYYANWAKGRFTISRDNSKNTVYL
QMNSLRAEDTAVYYCATSGSSVLYFKFWGQGTLVTVSSGGGGSGGGGSGGGG
SDIQMTQSPSSLSASVGDRVTITCQASENIYSFLAWYQQKPGKAPKLLIYSASKL
AAGVPSRFSGSGSGTDFTLTISSLQPEDFATYYCQQTNRYSNPDIYNVFGQGTKL
TVLGTTTPAPRPPTPAPTIASQPLSLRPEACRPAAGGAVHTRGLDFACDIISFFLA
LTSTALLFLLFFLTLRFSVVKRGRKKLLYIFKQPFMRPVQTTQEEDGCSCRFPEE
EEGGCELTRRVKFSRSADAPAYQQGQNQLYNELNLGRREEYDVLDKRRGRDP
EMGGKPRRKNPQEGLYNELQKDKMAEAYSEIGMKGERRRGKGHDGLYQGLS
TATKDTYDALHMQALPPR

In one embodiment, the CAR of the invention is encoded by a sequence SEQ ID NO: 49 or a sequence with at least about 95, preferably about 96%, 97%, 98% or 99% identity to SEQ ID NO: 49.

(SEQ ID NO: 49)

ATGGCTCTGCCTGTGACAGCTCTGCTGCTGCCTCTGGCTCTGCTTCTTCATGC
CGCCAGACCATCTGAGGTGCAGCTAGTTGAATCTGGTGGAGGCCTGGTTCA
GCCTGGTGGCTCTCTGAGACTGTCTTGTGCCGCCAGCGGCATCGACTTCAAC

AGCAACTACTACATGTGCTGGGTCCGACAGGCCCCCGGCAAAGGACTTGAATGGATTGGCTGCATCTACGTGGGCAGCCATGTGAACACCTACTACGCAAACTGGGCCAAGGGCAGATTCACCATCAGCCGGGACAACAGCAAGAACACCGTGTACCTGCAGATGAACAGCCTGAGAGCAGAAGATACTGCCGTGTACTACTGTGCCACATCTGGCAGCAGCGTGCTGTACTTCAAGTTCTGGGGCCAGGGCACCCTGGTCACAGTTTCTTCAGGAGGTGGAGGCTCTGGCGGTGGAGGAAGTGGTGGGGGAGGCTCTGACATCCAGATGACACAGAGCCCCAGCAGCCTGTCTGCCTCTGTGGGAGACAGAGTGACCATCACTTGCCAGGCCAGCGAGAACATCTACAGCTTCCTGGCCTGGTACCAGCAGAAGCCAGGCAAGGCCCCTAAGCTGCTGATCTACAGCGCCTCTAAACTAGCTGCCGGGGTGCCAAGCAGATTTTCTGGCTCTGGCAGCGGCACCGACTTCACCCTGACCATATCAAGCCTGCAGCCTGAGGACTTCGCCACCTACTACTGCCAGCAGACCAACAGGTACAGCAACCCCGACATCTACAACGTGTTTGGACAGGGCACCAAGCTGACAGTGCTTGGAACAACAACACCTGCTCCTCGGCCTCCTACACCAGCTCCTACAATTGCCAGCCAGCCACTGTCTCTGAGGCCCGAAGCTTGCAGGCCTGCTGCTGGCGGAGCCGTGCATACAAGAGGACTGGATTTCGCCTGCGACATCATCTCCTTCTTTCTTGCGCTGACCTCGACTGCGTTGCTCTTCCTGCTGTTCTTCCTCACGCTCCGTTTCTCTGTTGTTAAGCGGGGCAGAAAGAAACTGCTCTACATCTTCAAGCAGCCCTTCATGCGGCCCGTGCAGACCACACAAGAGGAAGATGGCTGCTCCTGCAGATTCCCCGAGGAAGAAGAAGGCGGCTGCGAGCTGACGCGTAGAGTGAAGTTCAGCAGATCCGCCGACGCTCCTGCCTATCAGCAGGGCCAAAACCAGCTGTACAACGAGCTGAACCTGGGGAGAAGAGAAGAGTACGACGTGCTGGACAAGCGGAGAGGCAGAGATCCTGAAATGGGCGGCAAGCCCAGACGGAAGAATCCTCAAGAGGGCCTGTATAATGAGCTGCAGAAAGACAAGATGGCCGAGGCCTACAGCGAGATCGGAATGAAGGGCGAGCGCAGAAGAGGCAAGGGACACGATGGACTGTACCAGGGCCTGAGCACCGCCACCAAGGATACCTATGATGCCCTGCACATGCAGGCCCTGCCTCCAAGA

In one embodiment, the CAR of the invention has a sequence SEQ ID NO: 65 or a sequence with at least about 95, preferably about 96%, 97%, 98% or 99% identity to SEQ ID NO: 65.

(SEQ ID NO: 65)

MALPVTALLLPLALLLHAARPSEVQLVESGGGLVQPGGSLRLSCAASGIDFNSN
YYMCWVRQAPGKGLEWIGCIYVGSHVNTYYANWAKGRFTISRDNSKNTVYL
QMNSLRAEDTAVYYCATSGSSVLYFKFWGQGTLVTVSSGGGGSGGGGSGGGG
SDIQMTQSPSSLSASVGDRVTITCQASENIYSFLAWYQQKPGKAPKLLIYSASKL
AAGVPSRFSGSGSGTDFTLTISSLQPEDFATYYCQQTNRYSNPDIYNVFGQGTKL
TVLGTTTPAPRPPTPAPTIASQPLSLRPEACRPAAGGAVHTRGLDFACDIISFFLA
LTSTALLFLLFFLTLRFSVVKRGRKKLLYIFKQPFMRPVQTTQEEDGCSCRFPEE
EEGGCELRVKFSRSADAPAYQQGQNQLYNELNLGRREEYDVLDKRRGRDPEM
GGKPRRKNPQEGLYNELQKDKMAEAYSEIGMKGERRRGKGHDGLYQGLSTAT
KDTYDALHMQALPPR

In one embodiment, the CAR of the invention is encoded by a sequence SEQ ID NO: 64 or a sequence with at least about 95, preferably about 96%, 97%, 98% or 99% identity to SEQ ID NO: 64.

(SEQ ID NO: 64)

ATGGCTCTGCCTGTGACAGCTCTGCTGCTGCCTCTGGCTCTGCTTCTTCATGC
CGCCAGACCATCTGAGGTGCAGCTAGTTGAATCTGGTGGAGGCCTGGTTCA
GCCTGGTGGCTCTCTGAGACTGTCTTGTGCCGCCAGCGGCATCGACTTCAAC
AGCAACTACTACATGTGCTGGGTCCGACAGGCCcccGGCAAAGGACTTGAAT
GGATTGGCTGCATCTACGTGGGCAGCCATGTGAACACCTACTACGCAAACT
GGGCCAAGGGCAGATTCACCATCAGCCGGGACAACAGCAAGAACACCGTG
TACCTGCAGATGAACAGCCTGAGAGCAGAAGATACTGCCGTGTACTACTGT
GCCACATCTGGCAGCAGCGTGCTGTACTTCAAGTTCTGGGGCCAGGGCACC
CTGGTCACAGTTTCTTCAGGAGGTGGAGGCTCTGGCGGTGGAGGAAGTGGT
GGGGGAGGCTCTGACATCCAGATGACACAGAGCCCCAGCAGCCTGTCTGCC
TCTGTGGGAGACAGAGTGACCATCACTTGCCAGGCCAGCGAGAACATCTAC
AGCTTCCTGGCCTGGTACCAGCAGAAGCCAGGCAAGGCCCCTAAGCTGCTG
ATCTACAGCGCCTCTAAACTAGCTGCCGGGGTGCCAAGCAGATTTTCTGGCT
CTGGCAGCGGCACCGACTTCACCCTGACCATATCAAGCCTGCAGCCTGAGG

ACTTCGCCACCTACTACTGCCAGCAGACCAACAGGTACAGCAACCCCGACA
TCTACAACGTGTTTGGACAGGGCACCAAGCTGACAGTGCTTGGAACAACAA
CaCCTGCTCCTCGGCCTCCTACACCAGCTCCTACAATTGCCAGCCAGCCACT
GTCTCTGAGGCCCGAAGCTtgcaggCCTGCTGCTGGCGGAGCCGTGCATACAA
GAGGACTGGATTTCGCCTGCGACATCATCTCCTTCTTTCTTGCGCTGACcTCG
ACTGCGTTGCTCTTCCTGCTGTTCTTCCTCACGCTCCGTTTCTCTGTTGTTAA
GCGGGGCAGAAAGAAACTGCTCTACATCTTCAAGCAGCCCTTCATGCGGCC
CGTGCAGACCACACAAGAGGAAGATGGCTGCTCCTGCAGATTCCCCGAGGA
AGAAGAAGGCGGCTGCGAGCTGAGAGTGAAGTTCAGCAGATCCGCCGACG
CTCCTGCCTATCAGCAGGGCCAAAACCAGCTcTACAACGAGCTGAACCTGG
GGAGAAGAGAAGAGTACGACGTGCTGGACAAGCGGAGAGGCAGAGATCCT
GAAATGGGCGGCAAGCCCAGACGGAAGAATCCTCAAGAGGGCCTGTATAA
TGAGCTaCAGAAAGACAAGATGGCaGAGGCCTACAGCGAGATCGGAATGAA
GGGCGAGCGCAGAAGAGGCAAGGGACACGATGGACTGTACCAGGGCCTGA
GCACCGCCACCAAGGATACCTATGATGCCCTGCACATGCAGGCCCTGCCTC
CAAGATAG

In one embodiment, the CAR of the invention comprises P2A and GFP sequences, and has a sequence SEQ ID NO: 63 or a sequence with at least about 95, preferably about 96%, 97%, 98% or 99% identity to SEQ ID NO: 63.

(SEQ ID NO: 63)

MALPVTALLLPLALLLHAARPSEVQLVESGGGLVQPGGSLRLSCAASGIDFNSN
YYMCWVRQAPGKGLEWIGCIYVGSHVNTYYANWAKGRFTISRDNSKNTVYL
QMNSLRAEDTAVYYCATSGSSVLYFKFWGQGTLVTVSSGGGGSGGGGSGGGG
SDIQMTQSPSSLSASVGDRVTITCQASENIYSFLAWYQQKPGKAPKLLIYSASKL
AAGVPSRFSGSGSGTDFTLTISSLQPEDFATYYCQQTNRYSNPDIYNVFGQGTKL
TVLGTTTPAPRPPTPAPTIASQPLSLRPEACRPAAGGAVHTRGLDFACDIISFFLA
LTSTALLFLLFFLTLRFSVVKRGRKKLLYIFKQPFMRPVQTTQEEDGCSCRFPEE
EEGGCELTRRVKFSRSADAPAYQQGQNQLYNELNLGRREEYDVLDKRRGRDP
EMGGKPRRKNPQEGLYNELQKDKMAEAYSEIGMKGERRRGKGHDGLYQGLS
TATKDTYDALHMQALPPRASGSGATNFSLLKQAGDVEENPGPMVSKGEELFTG

VVPILVELDGDVNGHKFSVSGEGEGDATYGKLTLKFICTTGKLPVPWPTLVTTL
TYGVQCFSRYPDHMKQHDFFKSAMPEGYVQERTIFFKDDGNYKTRAEVKFEG
DTLVNRIELKGIDFKEDGNILGHKLEYNYNSHNVYIMADKQKNGIKVNFKIRHN
IEDGSVQLADHYQQNTPIGDGPVLLPDNHYLSTQSALSKDPNEKRDHMVLLEF
VTAAGITLGMDELYK

In one embodiment, the CAR of the invention comprises P2A and GFP sequences, and is encoded by a sequence SEQ ID NO: 62 or a sequence with at least about 95, preferably about 96%, 97%, 98% or 99% identity to SEQ ID NO: 62.

(SEQ ID NO: 62)

ATGGCTCTGCCTGTGACAGCTCTGCTGCTGCCTCTGGCTCTGCTTCTTCATGC
CGCCAGACCATCTGAGGTGCAGCTAGTTGAATCTGGTGGAGGCCTGGTTCA
GCCTGGTGGCTCTCTGAGACTGTCTTGTGCCGCCAGCGGCATCGACTTCAAC
AGCAACTACTACATGTGCTGGGTCCGACAGGCCCCCGGCAAAGGACTTGAA
TGGATTGGCTGCATCTACGTGGGCAGCCATGTGAACACCTACTACGCAAAC
TGGGCCAAGGGCAGATTCACCATCAGCCGGGACAACAGCAAGAACACCGT
GTACCTGCAGATGAACAGCCTGAGAGCAGAAGATACTGCCGTGTACTACTG
TGCCACATCTGGCAGCAGCGTGCTGTACTTCAAGTTCTGGGGCCAGGGCAC
CCTGGTCACAGTTTCTTCAGGAGGTGGAGGCTCTGGCGGTGGAGGAAGTGG
TGGGGGAGGCTCTGACATCCAGATGACACAGAGCCCCAGCAGCCTGTCTGC
CTCTGTGGGAGACAGAGTGACCATCACTTGCCAGGCCAGCGAGAACATCTA
CAGCTTCCTGGCCTGGTACCAGCAGAAGCCAGGCAAGGCCCCTAAGCTGCT
GATCTACAGCGCCTCTAAACTAGCTGCCGGGGTGCCAAGCAGATTTTCTGGC
TCTGGCAGCGGCACCGACTTCACCCTGACCATATCAAGCCTGCAGCCTGAG
GACTTCGCCACCTACTACTGCCAGCAGACCAACAGGTACAGCAACCCCGAC
ATCTACAACGTGTTTGGACAGGGCACCAAGCTGACAGTGCTTGGAACAACA
ACACCTGCTCCTCGGCCTCCTACACCAGCTCCTACAATTGCCAGCCAGCCAC
TGTCTCTGAGGCCCGAAGCTTGCAGGCCTGCTGCTGGCGGAGCCGTGCATA
CAAGAGGACTGGATTTCGCCTGCGACATCATCTCCTTCTTTCTTGCGCTGAC
CTCGACTGCGTTGCTCTTCCTGCTGTTCTTCCTCACGCTCCGTTTCTCTGTTGT
TAAGCGGGGCAGAAAGAAACTGCTCTACATCTTCAAGCAGCCCTTCATGCG

GCCCGTGCAGACCACACAAGAGGAAGATGGCTGCTCCTGCAGATTCCCCGA
GGAAGAAGAAGGCGGCTGCGAGCTGACGCGTAGAGTGAAGTTCAGCAGAT
CCGCCGACGCTCCTGCCTATCAGCAGGGCCAAAACCAGCTGTACAACGAGC
TGAACCTGGGGAGAAGAGAAGAGTACGACGTGCTGGACAAGCGGAGAGGC
AGAGATCCTGAAATGGGCGGCAAGCCCAGACGGAAGAATCCTCAAGAGGG
CCTGTATAATGAGCTGCAGAAAGACAAGATGGCCGAGGCCTACAGCGAGAT
CGGAATGAAGGGCGAGCGCAGAAGAGGCAAGGGACACGATGGACTGTACC
AGGGCCTGAGCACCGCCACCAAGGATACCTATGATGCCCTGCACATGCAGG
CCCTGCCTCCAAGAGCTAGCGGAAGCGGAGCTACTAACTTCAGCCTGCTGA
AGCAGGCTGGAGACGTGGAGGAGAACCCTGGACCTATGGTGAGCAAGGGC
GAGGAGCTGTTCACCGGGGTGGTGCCCATCCTGGTCGAGCTGGACGGCGAC
GTAAACGGCCACAAGTTCAGCGTGTCCGGCGAGGGCGAGGGCGATGCCACC
TACGGCAAGCTGACCCTGAAGTTCATCTGCACCACCGGCAAGCTGCCCGTG
CCCTGGCCCACCCTCGTGACCACCCTGACCTACGGCGTGCAGTGCTTCAGCC
GCTACCCCGACCACATGAAGCAGCACGACTTCTTCAAGTCCGCCATGCCCG
AAGGCTACGTCCAGGAGCGCACCATCTTCTTCAAGGACGACGGCAACTACA
AGACCCGCGCCGAGGTGAAGTTCGAGGGCGACACCCTGGTGAACCGCATCG
AGCTGAAGGGCATCGACTTCAAGGAGGACGGCAACATCCTGGGGCACAAG
CTGGAGTACAACTACAACAGCCACAACGTCTATATCATGGCCGACAAGCAG
AAGAACGGCATCAAGGTGAACTTCAAGATCCGCCACAACATCGAGGACGGC
AGCGTGCAGCTCGCCGACCACTACCAGCAGAACACCCCCATCGGCGACGGC
CCCGTGCTGCTGCCCGACAACCACTACCTGAGCACCCAGTCCGCCCTGAGC
AAAGACCCCAACGAGAAGCGCGATCACATGGTCCTGCTGGAGTTCGTGACC
GCCGCCGGGATCACTCTCGGCATGGACGAGCTGTACAAGTAA

In one embodiment, the CAR of the invention comprises a CD8 leader sequence having the SEQ ID NO: 42, an anti-human IL-23R scFv, comprising a $V_H$ having the sequence SEQ ID NO: 1 and a $V_L$ having SEQ ID NO: 2, linked by a $(G_4S)_3$ linker (SEQ ID NO: 9), a hinge domain derived from CD8$\alpha$ having the sequence of SEQ ID NO: 60, a human 4-1BB transmembrane domain having the SEQ ID NO: 28, and an intracellular signaling domain comprising a human 4-1BB signaling domain having SEQ ID NO: 36 and a human CD3 zeta domain having SEQ ID NO: 31.

In one embodiment, the CAR of the invention has a sequence SEQ ID NO: 69 or a sequence with at least about 95, preferably about 96%, 97%, 98% or 99% identity to SEQ ID NO: 69.

(SEQ ID NO: 69)

MALPVTALLLPLALLLHAARPSEVQLVESGGGLVQPGGSLRLSCAASGIDFNSN
YYMCWVRQAPGKGLEWIGCIYVGSHVNTYYANWAKGRFTISRDNSKNTVYL
QMNSLRAEDTAVYYCATSGSSVLYFKFWGQGTLVTVSSGGGGSGGGGSGGGG
SDIQMTQSPSSLSASVGDRVTITCQASENIYSFLAWYQQKPGKAPKLLIYSASKL
AAGVPSRFSGSGSGTDFTLTISSLQPEDFATYYCQQTNRYSNPDIYNVFGQGTKL
TVLGSALSNSIMYFSHFVPVFLPAKPTTTPAPRPPTPAPTIASQPLSLRPEACRPA
AGGAVHTRGLDFACDIISFFLALTSTALLFLLFFLTLRFSVVKRGRKKLLYIFKQP
FMRPVQTTQEEDGCSCRFPEEEEGGCELTRRVKFSRSADAPAYQQGQNQLYNE
LNLGRREEYDVLDKRRGRDPEMGGKPRRKNPQEGLYNELQKDKMAEAYSEIG
MKGERRRGKGHDGLYQGLSTATKDTYDALHMQALPPR

In one embodiment, the CAR of the invention is encoded by a sequence SEQ ID NO: 68 or a sequence with at least about 95, preferably about 96%, 97%, 98% or 99% identity to SEQ ID NO: 68.

(SEQ ID NO: 68)

ATGGCTCTGCCTGTGACAGCTCTGCTGCTGCCTCTGGCTCTGCTTCTTCATGC
CGCCAGACCATCTGAGGTGCAGCTAGTTGAATCTGGTGGAGGCCTGGTTCA
GCCTGGTGGCTCTCTGAGACTGTCTTGTGCCGCCAGCGGCATCGACTTCAAC
AGCAACTACTACATGTGCTGGGTCCGACAGGCCCCCGGCAAAGGACTTGAA
TGGATTGGCTGCATCTACGTGGGCAGCCATGTGAACACCTACTACGCAAAC
TGGGCCAAGGGCAGATTCACCATCAGCCGGGACAACAGCAAGAACACCGT
GTACCTGCAGATGAACAGCCTGAGAGCAGAAGATACTGCCGTGTACTACTG
TGCCACATCTGGCAGCAGCGTGCTGTACTTCAAGTTCTGGGGCCAGGGCAC
CCTGGTCACAGTTTCTTCAGGAGGTGGAGGCTCTGGCGGTGGAGGAAGTGG
TGGGGGAGGCTCTGACATCCAGATGACACAGAGCCCCAGCAGCCTGTCTGC
CTCTGTGGGAGACAGAGTGACCATCACTTGCCAGGCCAGCGAGAACATCTA

CAGCTTCCTGGCCTGGTACCAGCAGAAGCCAGGCAAGGCCCCTAAGCTGCT GATCTACAGCGCCTCTAAACTAGCTGCCGGGGTGCCAAGCAGATTTTCTGGC TCTGGCAGCGGCACCGACTTCACCCTGACCATATCAAGCCTGCAGCCTGAG GACTTCGCCACCTACTACTGCCAGCAGACCAACAGGTACAGCAACCCCGAC ATCTACAACGTGTTTGGACAGGGCACCAAGCTGACAGTGCTTGGAAGCGCC CTGAGCAACAGCATCATGTACTTCAGCCACTTCGTGCCCGTGTTTCTGCCCG CCAAACCTACAACAACACCTGCTCCTCGGCCTCCTACACCAGCTCCTACAAT TGCCAGCCAGCCACTGTCTCTGAGGCCCGAAGCTTGCAGGCCTGCTGCTGGC GGAGCCGTGCATACAAGAGGACTGGATTTCGCCTGCGACATCATCTCCTTCT TTCTTGCGCTGACCTCGACTGCGTTGCTCTTCCTGCTGTTCTTCCTCACGCTC CGTTTCTCTGTTGTTAAGCGGGGCAGAAAGAAACTGCTCTACATCTTCAAGC AGCCCTTCATGCGGCCCGTGCAGACCACACAAGAGGAAGATGGCTGCTCCT GCAGATTCCCCGAGGAAGAAGAAGGCGGCTGCGAGCTGACGCGTAGAGTG AAGTTCAGCAGATCCGCCGACGCTCCTGCCTATCAGCAGGGCCAAAACCAG CTGTACAACGAGCTGAACCTGGGGAGAAGAGAAGAGTACGACGTGCTGGA CAAGCGGAGAGGCAGAGATCCTGAAATGGGCGGCAAGCCCAGACGGAAGA ATCCTCAAGAGGGCCTGTATAATGAGCTGCAGAAAGACAAGATGGCCGAGG CCTACAGCGAGATCGGAATGAAGGGCGAGCGCAGAAGAGGCAAGGGACAC GATGGACTGTACCAGGGCCTGAGCACCGCCACCAAGGATACCTATGATGCC CTGCACATGCAGGCCCTGCCTCCAAGA

In one embodiment, the CAR of the invention has a sequence SEQ ID NO: 73 or a sequence with at least about 95, preferably about 96%, 97%, 98% or 99% identity to SEQ ID NO: 73.

(SEQ ID NO: 73)

MALPVTALLLPLALLLHAARPSEVQLVESGGGLVQPGGSLRLSCAASGIDFNSN YYMCWVRQAPGKGLEWIGCIYVGSHVNTYYANWAKGRFTISRDNSKNTVYL QMNSLRAEDTAVYYCATSGSSVLYFKFWGQGTLVTVSSGGGGSGGGGSGGGG SDIQMTQSPSSLSASVGDRVTITCQASENIYSFLAWYQQKPGKAPKLLIYSASKL AAGVPSRFSGSGSGTDFTLTISSLQPEDFATYYCQQTNRYSNPDIYNVFGQGTKL TVLGSALSNSIMYFSHFVPVFLPAKPTTTPAPRPPTPAPTIASQPLSLRPEACRPA

AGGAVHTRGLDFACDIISFFLALTSTALLFLLFFLTLRFSVVKRGRKKLLYIFKQP
FMRPVQTTQEEDGCSCRFPEEEEGGCELRVKFSRSADAPAYQQGQNQLYNELN
LGRREEYDVLDKRRGRDPEMGGKPRRKNPQEGLYNELQKDKMAEAYSEIGMK
GERRRGKGHDGLYQGLSTATKDTYDALHMQALPPR

In one embodiment, the CAR of the invention is encoded by a sequence SEQ ID NO: 72 or a sequence with at least about 95, preferably about 96%, 97%, 98% or 99% identity to SEQ ID NO: 72.

(SEQ ID NO: 71)

ATGGCTCTGCCTGTGACAGCTCTGCTGCTGCCTCTGGCTCTGCTTCTTCATGC
CGCCAGACCATCTGAGGTGCAGCTAGTTGAATCTGGTGGAGGCCTGGTTCA
GCCTGGTGGCTCTCTGAGACTGTCTTGTGCCGCCAGCGGCATCGACTTCAAC
AGCAACTACTACATGTGCTGGGTCCGACAGGCCCCCGGCAAAGGACTTGAA
TGGATTGGCTGCATCTACGTGGGCAGCCATGTGAACACCTACTACGCAAAC
TGGGCCAAGGGCAGATTCACCATCAGCCGGGACAACAGCAAGAACACCGT
GTACCTGCAGATGAACAGCCTGAGAGCAGAAGATACTGCCGTGTACTACTG
TGCCACATCTGGCAGCAGCGTGCTGTACTTCAAGTTCTGGGGCCAGGGCAC
CCTGGTCACAGTTTCTTCAGGAGGTGGAGGCTCTGGCGGTGGAGGAAGTGG
TGGGGGAGGCTCTGACATCCAGATGACACAGAGCCCCAGCAGCCTGTCTGC
CTCTGTGGGAGACAGAGTGACCATCACTTGCCAGGCCAGCGAGAACATCTA
CAGCTTCCTGGCCTGGTACCAGCAGAAGCCAGGCAAGGCCCCTAAGCTGCT
GATCTACAGCGCCTCTAAACTAGCTGCCGGGGTGCCAAGCAGATTTTCTGGC
TCTGGCAGCGGCACCGACTTCACCCTGACCATATCAAGCCTGCAGCCTGAG
GACTTCGCCACCTACTACTGCCAGCAGACCAACAGGTACAGCAACCCCGAC
ATCTACAACGTGTTTGGACAGGGCACCAAGCTGACAGTGCTTGGAAGCGCC
CTGAGCAACAGCATCATGTACTTCAGCCACTTCGTGCCCGTGTTTCTGCCCG
CCAAACCTACAACAACACCTGCTCCTCGGCCTCCTACACCAGCTCCTACAAT
TGCCAGCCAGCCACTGTCTCTGAGGCCCGAAGCTTGCAGGCCTGCTGCTGGC
GGAGCCGTGCATACAAGAGGACTGGATTTCGCCTGCGACATCATCTCCTTCT
TTCTTGCGCTGACCTCGACTGCGTTGCTCTTCCTGCTGTTCTTCCTCACGCTC
CGTTTCTCTGTTGTTAAGCGGGGCAGAAAGAAACTGCTCTACATCTTCAAGC

AGCCCTTCATGCGGCCCGTGCAGACCACACAAGAGGAAGATGGCTGCTCCT
GCAGATTCCCCGAGGAAGAAGAAGGCGGCTGCGAGCTGAGAGTGAAGTTC
AGCAGATCCGCCGACGCTCCTGCCTATCAGCAGGGCCAAAACCAGCTCTAC
AACGAGCTGAACCTGGGGAGAAGAGAAGAGTACGACGTGCTGGACAAGCG
GAGAGGCAGAGATCCTGAAATGGGCGGCAAGCCCAGACGGAAGAATCCTC
AAGAGGGCCTGTATAATGAGCTACAGAAAGACAAGATGGCAGAGGCCTAC
AGCGAGATCGGAATGAAGGGCGAGCGCAGAAGAGGCAAGGGACACGATGG
ACTGTACCAGGGCCTGAGCACCGCCACCAAGGATACCTATGATGCCCTGCA
CATGCAGGCCCTGCCTCCAAGATAG

In one embodiment, the CAR of the invention comprises P2A and GFP sequences, and has a sequence SEQ ID NO: 71 or a sequence with at least about 95, preferably about 96%, 97%, 98% or 99% identity to SEQ ID NO: 71.

(SEQ ID NO: 71)

MALPVTALLLPLALLLHAARPSEVQLVESGGGLVQPGGSLRLSCAASGIDFNSN
YYMCWVRQAPGKGLEWIGCIYVGSHVNTYYANWAKGRFTISRDNSKNTVYL
QMNSLRAEDTAVYYCATSGSSVLYFKFWGQGTLVTVSSGGGGSGGGGSGGGG
SDIQMTQSPSSLSASVGDRVTITCQASENIYSFLAWYQQKPGKAPKLLIYSASKL
AAGVPSRFSGSGSGTDFTLTISSLQPEDFATYYCQQTNRYSNPDIYNVFGQGTKL
TVLGSALSNSIMYFSHFVPVFLPAKPTTTPAPRPPTPAPTIASQPLSLRPEACRPA
AGGAVHTRGLDFACDIISFFLALTSTALLFLLFFLTLRFSVVKRGRKKLLYIFKQP
FMRPVQTTQEEDGCSCRFPEEEEGGCELTRRVKFSRSADAPAYQQGQNQLYNE
LNLGRREEYDVLDKRRGRDPEMGGKPRRKNPQEGLYNELQKDKMAEAYSEIG
MKGERRRGKGHDGLYQGLSTATKDTYDALHMQALPPRASGSGATNFSLLKQA
GDVEENPGPMVSKGEELFTGVVPILVELDGDVNGHKFSVSGEGEGDATYGKLT
LKFICTTGKLPVPWPTLVTTLTYGVQCFSRYPDHMKQHDFFKSAMPEGYVQER
TIFFKDDGNYKTRAEVKFEGDTLVNRIELKGIDFKEDGNILGHKLEYNYNSHNV
YIMADKQKNGIKVNFKIRHNIEDGSVQLADHYQQNTPIGDGPVLLPDNHYLSTQ
SALSKDPNEKRDHMVLLEFVTAAGITLGMDELYK

In one embodiment, the CAR of the invention comprises P2A and GFP sequences, and is encoded by a sequence SEQ ID NO: 70 or a sequence with at least about 95, preferably about 96%, 97%, 98% or 99% identity to SEQ ID NO: 70.

(SEQ ID NO: 70)

ATGGCTCTGCCTGTGACAGCTCTGCTGCTGCCTCTGGCTCTGCTTCTTCATGC
CGCCAGACCATCTGAGGTGCAGCTAGTTGAATCTGGTGGAGGCCTGGTTCA
GCCTGGTGGCTCTCTGAGACTGTCTTGTGCCGCCAGCGGCATCGACTTCAAC
AGCAACTACTACATGTGCTGGGTCCGACAGGCCCCCGGCAAAGGACTTGAA
TGGATTGGCTGCATCTACGTGGGCAGCCATGTGAACACCTACTACGCAAAC
TGGGCCAAGGGCAGATTCACCATCAGCCGGGACAACAGCAAGAACACCGT
GTACCTGCAGATGAACAGCCTGAGAGCAGAAGATACTGCCGTGTACTACTG
TGCCACATCTGGCAGCAGCGTGCTGTACTTCAAGTTCTGGGGCCAGGGCAC
CCTGGTCACAGTTTCTTCAGGAGGTGGAGGCTCTGGCGGTGGAGGAAGTGG
TGGGGGAGGCTCTGACATCCAGATGACACAGAGCCCCAGCAGCCTGTCTGC
CTCTGTGGGAGACAGAGTGACCATCACTTGCCAGGCCAGCGAGAACATCTA
CAGCTTCCTGGCCTGGTACCAGCAGAAGCCAGGCAAGGCCCCTAAGCTGCT
GATCTACAGCGCCTCTAAACTAGCTGCCGGGGTGCCAAGCAGATTTTCTGGC
TCTGGCAGCGGCACCGACTTCACCCTGACCATATCAAGCCTGCAGCCTGAG
GACTTCGCCACCTACTACTGCCAGCAGACCAACAGGTACAGCAACCCCGAC
ATCTACAACGTGTTTGGACAGGGCACCAAGCTGACAGTGCTTGGAAGCGCC
CTGAGCAACAGCATCATGTACTTCAGCCACTTCGTGCCCGTGTTTCTGCCCG
CCAAACCTACAACAACACCTGCTCCTCGGCCTCCTACACCAGCTCCTACAAT
TGCCAGCCAGCCACTGTCTCTGAGGCCCGAAGCTTGCAGGCCTGCTGCTGGC
GGAGCCGTGCATACAAGAGGACTGGATTTCGCCTGCGACATCATCTCCTTCT
TTCTTGCGCTGACCTCGACTGCGTTGCTCTTCCTGCTGTTCTTCCTCACGCTC
CGTTTCTCTGTTGTTAAGCGGGGCAGAAAGAAACTGCTCTACATCTTCAAGC
AGCCCTTCATGCGGCCCGTGCAGACCACACAAGAGGAAGATGGCTGCTCCT
GCAGATTCCCCGAGGAAGAAGAAGGCGGCTGCGAGCTGACGCGTAGAGTG
AAGTTCAGCAGATCCGCCGACGCTCCTGCCTATCAGCAGGGCCAAAACCAG
CTGTACAACGAGCTGAACCTGGGGAGAAGAGAAGAGTACGACGTGCTGGA

CAAGCGGAGAGGCAGAGATCCTGAAATGGGCGGCAAGCCCAGACGGAAGA
ATCCTCAAGAGGGCCTGTATAATGAGCTGCAGAAAGACAAGATGGCCGAGG
CCTACAGCGAGATCGGAATGAAGGGCGAGCGCAGAAGAGGCAAGGGACAC
GATGGACTGTACCAGGGCCTGAGCACCGCCACCAAGGATACCTATGATGCC
CTGCACATGCAGGCCCTGCCTCCAAGAGCTAGCGGAAGCGGAGCTACTAAC
TTCAGCCTGCTGAAGCAGGCTGGAGACGTGGAGGAGAACCCTGGACCTATG
GTGAGCAAGGGCGAGGAGCTGTTCACCGGGGTGGTGCCCATCCTGGTCGAG
CTGGACGGCGACGTAAACGGCCACAAGTTCAGCGTGTCCGGCGAGGGCGAG
GGCGATGCCACCTACGGCAAGCTGACCCTGAAGTTCATCTGCACCACCGGC
AAGCTGCCCGTGCCCTGGCCCACCCTCGTGACCACCCTGACCTACGGCGTGC
AGTGCTTCAGCCGCTACCCCGACCACATGAAGCAGCACGACTTCTTCAAGTC
CGCCATGCCCGAAGGCTACGTCCAGGAGCGCACCATCTTCTTCAAGGACGA
CGGCAACTACAAGACCCGCGCCGAGGTGAAGTTCGAGGGCGACACCCTGGT
GAACCGCATCGAGCTGAAGGGCATCGACTTCAAGGAGGACGGCAACATCCT
GGGGCACAAGCTGGAGTACAACTACAACAGCCACAACGTCTATATCATGGC
CGACAAGCAGAAGAACGGCATCAAGGTGAACTTCAAGATCCGCCACAACAT
CGAGGACGGCAGCGTGCAGCTCGCCGACCACTACCAGCAGAACACCCCCAT
CGGCGACGGCCCCGTGCTGCTGCCCGACAACCACTACCTGAGCACCCAGTC
CGCCCTGAGCAAAGACCCCAACGAGAAGCGCGATCACATGGTCCTGCTGGA
GTTCGTGACCGCCGCCGGGATCACTCTCGGCATGGACGAGCTGTACAAGTA
A

The present invention further relates to a T cell, preferably an isolated T cell, engineered to express on the cell surface a CAR as described hereinabove.

The present invention also relates to an isolated and/or substantially purified T cell population comprising cells engineered to express on the cell surface a CAR as described hereinabove.

In one embodiment, the T cells of the invention are suppressive for cells expressing at their surface the IL-23R recognized by the CAR.

In one embodiment, the T cells of the invention are cytotoxic for cells expressing at their surface the IL-23R recognized by the CAR.

In one embodiment, the T cell population of the invention comprises or consists in regulatory T cells (Treg), $CD8^+$ T cells, $CD4^+$ T cells and NK T cells.

In one embodiment, the T cells of the invention are Treg cells.

In one embodiment, the T cell is a regulatory immune cell, such as, for example, any regulatory immune cell suitable for use in cellular therapy.

In one embodiment, the Treg cells of the population of the invention all express a chimeric receptor (CAR) as defined herein and may thus be defined as CAR-monospecific (*i.e.*, all the Treg cells recognize the same antigen (IL-23R) with the CAR they express). In one embodiment, the Treg cell population is TCR-monospecific (*i.e.,* all the Treg cells recognize the same antigen with their TCR). In another embodiment, the Treg cell population is TCR-polyspecific (*i.e.,* the Treg cells may recognize different antigens with their TCR).

In one embodiment, the Treg cell population is TCR-monospecific, and the TCR recognizes an antigen, a fragment of an antigen, a variant of an antigen or a mixture thereof.

In one embodiment, the Treg cell population is TCR-monospecific, and the TCR is specific of a food antigen from the common human diet.

In another embodiment, the Treg cell population is TCR-monospecific, and the TCR is specific of an autoantigen, such as, for example, a multiple sclerosis-associated antigen, a joint-associated antigen, an eye-associated antigen, a human HSP antigen, a skin-associated antigen or an antigen involved in graft rejection or GVHD. Examples of autoantigens, in particular of multiple sclerosis-associated antigens, joint-associated antigens, eye-associated antigens, human HSP antigens, skin-associated antigens and antigens involved in graft rejection or GVHD are given herein.

In another embodiment, the Treg cell population is TCR-monospecific, and the TCR is specific of an inhaled allergen, an ingested allergen or a contact allergen.

In one embodiment, the Treg cell population is TCR-monospecific, and the TCR is specific of an antigen selected from the group comprising ovalbumin, MOG, type II collagen fragments, variants and mixtures thereof.

In one embodiment, the Treg cell population is TCR-monospecific, and the TCR is specific of ovalbumin, fragments, variants and mixtures thereof.

In another embodiment, the Treg cell population is TCR-monospecific, and the TCR is specific of MOG, fragments, variants and mixtures thereof.

In another embodiment, the Treg cell population is TCR-monospecific, and the TCR is specific of type II collagen, fragments, variants and mixtures thereof.

In one embodiment, Treg cells expressing the CAR of the invention are suppressive against cells expressing IL-23R recognized by the CAR.

In one embodiment, the CAR of the invention when expressed by a Treg cell, confers to the Treg cell the ability to bind to cells expressing IL-23R on their cell surface and be activated by the IL-23R, differently from the antigen that the Treg cells are or would have been specific or activated by.

The Treg cell population of the invention may thus be defined as a redirected Treg cell population. As used herein, the term "redirected" refers to a Treg cell carrying a chimeric receptor as described herein, which confers to the Treg cell the ability to bind to and be activated by a ligand that is different from the one the Treg cell is or would have been specific or be activated by.

In one embodiment, Treg cells of the invention are not cytotoxic. In another embodiment, Treg cells of the invention are cytotoxic.

Examples of cells expressing IL-23R include, but are not limited to, Th17, αβ T cells, neutrophils, gamma delta T cells, NK, NKT, dendritic cells and macrophages.

In one embodiment, Treg cells of the invention may be selected form the group comprising $CD4^+$ $CD25^+$ $Foxp3^+$ Treg, Tr1 cells, TGF-β secreting Th3 cells, regulatory NK T cells, regulatory γδ T cells, regulatory $CD8^+$ T cells, and double negative regulatory T cells.

In one embodiment, the regulatory cell is a $CD4^+$ regulatory T cell (Treg). In one embodiment, the Treg is a thymus derived Treg or an adaptive or induced Treg. In one embodiment, the Treg is a $CD4^+FOXP3^+$ regulatory T cell or a $CD4^+FOXP3^-$ regulatory T cell (Tr1 cell), preferably a $CD4^+FOXP3^+$ regulatory T cell.

In one embodiment, the regulatory cell is a $CD8^+$ regulatory T cell (Treg). In one embodiment, the $CD8^+$ regulatory T cell is selected from the group consisting of a $CD8^+CD28^-$ regulatory T cell, a $CD8^+CD103^+$ regulatory T cell, a $CD8^+FoxP3^+$ regulatory T cell, a $CD8^+CD122^+$ regulatory T cell, and any combination thereof. In one embodiment, the regulatory cell is an $INF\gamma^+IL10^+IL34^+$ $CD8^+CD45RC^{low}$ regulatory T cell.

In one embodiment, the Treg cells of the invention are mammal cells, preferably human Treg cells.

In one embodiment, the Treg is derived from stem cells, such as, for example, induced-stem cells, including, without limitation, induced pluripotent stem cells (iPS or iPSC).

As used herein, the term "induced pluripotent stem cells", "iPS" or "iPSC" refers to artificial pluripotent stem cells, derived from non-pluripotent cells, in particular from adult somatic cells, by dedifferentiation or reprogramming. In particular, iPSC may be obtained by introducing a specific set of pluripotency-associated genes into a cell, such as, for example, the transcription factors Oct4 (Pou5f1), Sox2, cMyc, and Klf4. In addition to their morphology, self-renewal property and pluripotency similar to those of embryonic stem cells, iPSCs also exhibit epigenetic reprogramming with an overall profile of histone methylation and gene expression very close to that of embryonic stem cells. IPSCs in particular express pluripotency markers, such as for example, Nanog, Sox2, Oct4 and Ssea3 / 4 proteins.

In one embodiment, the regulatory cell has the following phenotype: $CD4^+CD25^+$, such as, for example, $CD4^+CD25^+CD127^-$, such as, for example, $CD4^+CD25^+CD127^-$ $CD45RA^+$. Preferably, the regulatory immune cell has the following phenotype: $FoxP3^+CD4^+CD25^+$, such as, for example, $FoxP3^+CD4^+CD25^+CD127^-$, such as, for example, $FoxP3^+CD4^+CD25^+CD127^-CD45RA^+$.

In one embodiment, the regulatory cell presents at least one of the following phenotypes: $CD4^+CD25^+$, $FoxP3^+$, $CD127^{lo/-}$, CTLA-$4^+$, $CD39^+$, $Helios^+$, $CD62L^{+/hi}$, $VLA4^+$, $LFA1^+$, $CD49b^{int}$, $ITGb7^{int}$, PSGL-$1^+$, $ICOS^+$, $GITR^+$, PD-$1^{int}$, $Perf^{lo/-}$, $CCR7^+$. In one embodiment, the immune regulatory cell does not express Granzyme A and/or Granzyme B.

In one embodiment, the determination of the expression level of molecules is conducted by flow cytometry, immunofluorescence or image analysis, for example high content analysis. Preferably, the determination of the expression level of molecules is conducted by flow cytometry. In one embodiment, before conducting flow cytometry analysis, cells are fixed and permeabilized, thereby allowing detecting intracellular proteins.

In one embodiment, the determination of the expression level of a molecule in a cell population comprises determining the percentage of cells of the cell population expressing the molecule (*i.e.* cells "+" for the molecule). Preferably, said percentage of cells expressing the molecule is measured by FACS.

The terms "expressing (or +)" and "not expressing (or -)" are well known in the art and refer to the expression level of the cell marker of interest, in that the expression level of the cell marker corresponding to "+" is high or intermediate, also referred as "+/-", and the expression level of the cell marker corresponding to "-" is null.

The term "low" or "lo" or "lo/-" is well known in the art and refers to the expression level of the cell marker of interest, in that the expression level of the cell marker is low by comparison with the expression level of that cell marker in the population of cells being analyzed as a whole. More particularly, the term "lo" refers to a distinct population of cells that express the cell marker at a lower level than one or more other distinct population of cells.

The term "high" or "hi" or "bright" is well known in the art and refers to the expression level of the cell marker of interest, in that the expression level of the cell marker is high by comparison with the expression level of that cell marker in the population of cells being analyzed as a whole.

Generally, cells in the top 2, 3, 4, or 5% of staining intensity are designated "hi", with those falling in the top half of the population categorized as being "+". Those cells falling below 50%, of fluorescence intensity are designated as "lo" cells and below 5% as "-" cells.

The expression level of the cell marker of interest is determined by comparing the Median Fluorescence Intensity (MFI) of the cells from the cell population stained with fluorescently labeled antibody specific for this marker to the fluorescence intensity (FI) of the cells from the same cell population stained with fluorescently labeled antibody with an irrelevant specificity but with the same isotype, the same fluorescent probe and originated from the same specie (referred as Isotype control). The cells from the population stained with fluorescently labeled antibody specific for this marker and that show equivalent MFI or a lower MFI than the cells stained with the isotype controls are not expressing this marker and then are designated (-) or negative. The cells from the population stained with fluorescently labeled antibody specific for this marker and that show a MFI value superior to the cells stained with the isotype controls are expressing this marker and then are designated (+) or positive.

In one embodiment, the cells of the Treg cell population of the invention express at their cell surface a CAR of the invention, and another receptor (herein referred to as "second receptor"), that binds to another ligand than the IL-23R recognized by the CAR of the invention. According to the invention, this other receptor comprises an extracellular ligand binding domain, optionally a hinge, optionally a transmembrane domain, and an intracellular signaling domain, as previously described.

In one embodiment, the second receptor is endogenous (such as, for example, the endogenous TCR). In another embodiment, the second receptor is exogenous, and its expression is induced in the cells of the Treg cell population of the invention by transformation or transduction of a nucleic acid encoding it. Said exogenous receptor may be an exogenous TCR or a CAR. Therefore, in one embodiment, the Treg cells of the invention express two CARs, wherein the first one recognizes a IL-23R, and the second one recognizes a distinct ligand. In another embodiment, the Treg cells of the invention express two CARs, wherein the first one recognizes a first epitope on an IL-23R, and the second one recognizes a distinct epitope on the same IL-23R. In another embodiment, the Treg cells of the invention express two CARs, wherein the first one recognizes an IL-23R, and the second one recognizes a distinct IL-23R (such as, for example, a variant of IL-23R).

In one embodiment, at least one of the CAR of the invention and the second receptor (preferably the second CAR) is inducible, *i.e.,* its expression on the cell surface may be induced.

In one embodiment, the expression of one of the CAR of the invention and the second receptor (preferably the second CAR) is induced by the activation of the other receptor. In a first embodiment, the expression of the CAR of the invention is induced by the activation of the second receptor. In a second embodiment, the expression of the second receptor is induced by the activation of the CAR of the invention. Inducible CARs were previously described in the art, such as, for example, by Roybal et al (Cell, 2006).

In one embodiment, the second receptor, preferably the second CAR, is specific of an antigen, a fragment of an antigen, a variant of an antigen or a mixture thereof.

In one embodiment, the second receptor, preferably the second CAR, is specific of a food antigen from the common human diet. Examples of food antigen from the common human diet are listed hereinabove.

In another embodiment, the second receptor, preferably the second CAR, is specific of an autoantigen, such as, for example, a multiple sclerosis-associated antigen, a joint-associated antigen, an eye-associated antigen, a human HSP antigen, a skin-associated antigen or an antigen involved in graft rejection or GVHD

Examples of multiple sclerosis-associated antigens are listed hereinabove.

Examples of joint-associated antigens are listed hereinabove.

Examples of eye-associated antigens are listed hereinabove.

Examples of human HSP antigens are listed hereinabove.

In one embodiment, the antigen is an inflammatory nervous system condition -associated antigen, preferably a multiple sclerosis-associated antigen. Examples of inflammatory nervous system condition-associated antigens, preferably of multiple sclerosis-associated antigens are listed hereinabove.

In one embodiment, the antigen is a skin-associated antigen. Examples of skin-associated antigens are listed hereinabove.

In one embodiment, the antigen is an antigen involved in graft rejection or GVHD Examples of such antigens are listed hereinabove.

In one embodiment, the antigen is a HLA-A2 cell surface protein. In one embodiment, the extracellular binding domain comprises an antibody directed to HLA-A2 or an antigen binding fragment thereof.

Other examples of autoantigens are listed hereinabove.

In one embodiment, the antigen is a cancer antigen. Examples of cancer antigens are listed hereinabove.

In one embodiment, the antigen is associated with infected cells. In one embodiment, the antigen is associated with virally infected cells. In another embodiment, the antigen is associated with bacterially infected cells. In another embodiment, the antigen is associated with fungally infected cells. In another embodiment, the antigen is associated with parasitic infected cells.

In another embodiment, the second receptor, preferably the second CAR, is specific of an inhaled allergen, an ingested allergen or a contact allergen.

In one embodiment, the second receptor, preferably the second CAR, is specific of an antigen selected from the group comprising ovalbumin, MOG, type II collagen fragments, variants and mixtures thereof.

In one embodiment, the second receptor, preferably the second CAR, is specific of ovalbumin, fragments, variants and mixtures thereof.

In another embodiment, the second receptor, preferably the second CAR, is specific of MOG, fragments, variants and mixtures thereof.

In another embodiment, the second receptor, preferably the second CAR, is specific of type II collagen, fragments, variants and mixtures thereof.

In one embodiment, the CAR of the invention comprises a first intracellular signaling domain, and the second receptor comprises a distinct second intracellular signaling domain. In a first embodiment, the CAR of the invention comprises a T cell primary signaling domain (such as, for example, CD3 zeta), and the second receptor comprises a costimulatory signaling domain (such as, for example, of 4-1BB, CD28 or a combination of costimulatory signaling domain of 4-1BB and CD28). In a second embodiment, the CAR of the invention comprises a costimulatory signaling domain (such as, for example, of 4-1BB, CD28 or a combination of costimulatory signaling domain of 4-1BB and CD28), and the second receptor comprises a T cell primary signaling domain (such as, for example, CD3 zeta).

Consequently, according to these embodiments, the complete activation of the Treg cell population of the invention requires both the binding of the CAR of the invention to IL-23R, and the binding of the second receptor to the ligand to which it is directed.

In one embodiment, the ligand recognized by the second receptor is expressed or present at the diseased tissue or organ, or at the site of the autoimmune response. Consequently, suppressive activity for cells expressing IL-23R will be induced only at the diseased tissue or organ or at the site of the autoimmune response, when said ligand will be present and recognized by the second receptor on the cells of Treg cell population.

In one embodiment, the chimeric receptor of the invention further comprises an extracellular ligand binding domain recognizing a ligand distinct from the IL-23R recognized by the chimeric receptor. In one embodiment, said ligand binding domain is an antibody or an antigen binding fragment thereof.

In one embodiment, the chimeric receptor of the invention comprises an extracellular ligand binding domain comprising an IL-23R binding domain and another ligand binding domain recognizing a ligand distinct from said IL-23R. In one embodiment, said ligand binding domain is a bifunctional antibody recognizing both the IL-23R and the distinct ligand.

In one embodiment, the Treg cells population is obtained by *in vitro* differentiation of naive T cells.

The present invention also relates to a nucleic acid sequence encoding a CAR as described hereinabove, wherein said nucleic acid sequence comprises (i) a nucleic acid sequence of an extracellular IL-23R binding domain, (ii) optionally a nucleic acid sequence of an extracellular hinge domain, (iii) a nucleic acid sequence of a transmembrane domain derived from 4-1BB, preferably from human 4-1BB, (iv) one or more nucleic acid sequence(s) of n intracellular signaling domain and (v) optionally a nucleic acid sequence of a Tag and or a leader sequence.

Another object of the invention is a vector comprising the nucleic acid sequence encoding a CAR as described hereinabove.

Examples of vectors that may be used in the present invention include, but are not limited to, a DNA vector, a RNA vector, a plasmid, a phagemid, a phage derivative, an animal virus and a cosmid.

Viral vector technology is well known in the art and is described, for example, in Sambrook et al. (2001, Molecular Cloning: A Laboratory Manual, Cold Spring Harbor Laboratory, New York), and in other virology and molecular biology manuals. Viruses, which are useful as vectors include, but are not limited to, retroviruses, adenoviruses, adeno- associated viruses, herpes viruses, and lentiviruses.

In general, a suitable vector contains an origin of replication functional in at least one organism, a promoter sequence, convenient restriction endonuclease sites, and one or more selectable markers, (e.g., WO01/96584; WO01/29058; and US6,326,193 incorporated herein by reference).

A number of viral based systems have been developed for gene transfer into mammalian cells. For example, retroviruses provide a convenient platform for gene delivery systems. A selected gene can be inserted into a vector and packaged in retroviral particles using techniques known in the art. The recombinant virus can then be isolated and delivered to cells of the subject either *in vivo* or *ex vivo.* A number of retroviral systems are known in the art. In some embodiments, adenovirus vectors are used. A number of adenovirus vectors are known in the art. In one embodiment, lentivirus vectors are used.

Additional transcriptionally active elements, e.g., promoters and enhancers, may regulate the frequency of transcriptional initiation. Typically, regarding core promote, these are located in the region 30-110 bp upstream of the start site, although a number of promoters have recently been shown to contain functional elements downstream of the start site as well, and enhancers elements are generally located 500-2000bp upstream of the start site. The spacing between promoter elements frequently is flexible, so that promoter function is preserved when elements are inverted or moved relative to one another. In the thymidine kinase (tk) promoter, the spacing between promoter elements can be increased to 50 bp apart before activity begins to decline. Depending on the promoter, it appears that individual elements can function either cooperatively or independently to activate transcription.

One example of a suitable promoter is the immediate early cytomegalovirus (CMV) promoter sequence. This promoter sequence is a strong constitutive promoter sequence capable of driving high levels of expression of any polynucleotide sequence operatively linked thereto. Another example of a suitable promoter is Elongation Growth Factor - la (EF-la). Another example of a suitable promoter is phosphoglycerate kinase (PGK) promoter. However, other constitutive promoter sequences may also be used, including, but not limited to the simian virus 40 (SV40) early promoter, mouse mammary tumor virus (MMTV), human immunodeficiency virus (HIV) long terminal repeat (LTR) promoter, MoMuLV promoter, an avian leukemia virus promoter, an Epstein-Barr virus immediate early promoter, a Rous sarcoma virus promoter, as well as human gene promoters such as, but not limited to, the actin promoter, the myosin promoter, the hemoglobin promoter, and the creatine kinase promoter. Further, the invention should not be limited to the use of constitutive promoters. Inducible promoters are also contemplated as part of the invention. The use of an inducible promoter provides a molecular switch capable of turning on expression of the polynucleotide sequence which it is operatively linked to when such expression is desired, or turning off the expression when expression is not desired. Examples of inducible promoters include, but are not limited to a metallothionine promoter, a glucocorticoid promoter, a progesterone promoter, and a tetracycline promoter. In addition, bi-directional promoters allowing efficient and coordinate expression of two or more genes may also be of interest in the present invention. Examples of bi-directional promoters include but are not limited to the promoters described by Luigi Naldini in US2006200869, incorporated herein by reference, disclosing a bi-directional promoter comprising i) a first minimal promoter sequence derived from cytomegalovirus (CMV) or mouse mammary tumor virus (MMTV) genomes and ii) a full efficient promoter sequence derived from an animal gene.

In order to assess the expression of a CAR polypeptide or portions thereof, the expression vector to be introduced into a T cell can also contain either a selectable marker gene such as CD34 or a reporter gene or both to facilitate identification and selection of expressing cells from the population of cells sought to be transfected or infected through viral vectors. In other aspects, the selectable marker may be carried on a separate piece of DNA and used in a co-transfection procedure. Both selectable markers and reporter genes may be flanked with appropriate regulatory sequences to enable expression in the host cells. Useful selectable markers include, for example, antibiotic-resistance genes, such as neo and the like.

In some embodiments of the invention, suicide gene technology may be used. Different suicide gene technologies are described in the art depending on their mechanism of action (Jones et al. Frontiers in Pharmacology, 2014 (5): 254). Examples of gene-directed enzyme prodrug therapy (GDEPT) converting a nontoxic drug to a toxic drug include herpes simplex virus thymidine kinase (HSV-TK) and cytosine deaminase (CD). Other examples are chimeric proteins composed of a drug binding domain linked to apoptotic components such as for example the inducible Fas (iFas) or the inducible Caspase 9 (iCasp9) systems. Other examples include systems mediated by therapeutic antibodies such as inducing overexpression of c-myc at the surface of the engineered cell to induce their deletion by administration of an anti-c-myc antibody. The use of EGFR is described as a similar system compared to the c-myc system. In one embodiment, the suicide gene technology used is the technology described in WO2013153391 or WO2016120216 (incorporated herein by reference). WO2013153391 describes a polypeptide comprising a marker moiety (such as, for example, a minimal epitope of CD34) and a suicide moiety, wherein the suicide moiety comprises a minimal epitope based on an epitope from CD20, such that cells expressing said polypeptide can be selectively killed using a lytic antibody such as, for example, Rituximab. More particularly, said peptide may have the formula St-R1-S1-Q-S2-R2 wherein St is a stalk sequence which, when the polypeptide is expressed at the surface of a target cell, causes the R and Q epitopes to be projected from the cell surface; R1 and R2 are a Rituximab-binding epitope; S1 and S2 are optional spacer sequences, which may be the same or different; and Q is a QBEND-10-binding epitope. An example of such a peptide is SEQ ID NO: 51: CPYSNPSLCSGGGGSELPTQGTFSNVSTNVSPAKPTTTACPYSNPSLCSGGGGSP APRPPTPAPTIASQPLSLRPEACRPAAGGAVHTRGLDFACDIYIWAPLAGTCGVL LLSLVITLYCNHRNRRRVCKCPRPW. WO2016120216 describes a CAR comprising an extracellular binding domain (scFv) modified to allow cell sorting and cell depletion, wherein said modification consists of inserting one or more selected epitopes within the scFv, said epitopes having a specificity to be recognized by one or more specific antibody(ies). In particular, said selected epitope may be an epitope from CD20, such that cells expressing said CAR can be selectively killed using a lytic antibody such as, for example, Rituximab.

Reporter genes are used for identifying potentially transfected cells and for evaluating the functionality of regulatory sequences. In general, a reporter gene is a gene that is not present in or expressed by the recipient organism or tissue and that encodes a polypeptide whose expression is manifested by some easily detectable property, e.g., enzymatic activity. Expression of the reporter gene is assayed at a suitable time after the DNA has been introduced into the recipient cells. Suitable reporter genes may include genes encoding luciferase, beta-galactosidase, chloramphenicol acetyl transferase, secreted alkaline phosphatase, or the green fluorescent protein gene (*e.g.,* Ui-Tei et al., 2000 FEBS Letters 479: 79-82). Suitable expression systems are well known and may be prepared using known techniques or obtained commercially. In general, the construct with the minimal 5' flanking region showing the highest level of expression of reporter gene is identified as the promoter. Such promoter regions may be linked to a reporter gene and used to evaluate agents for the ability to modulate promoter-driven transcription.

Methods of introducing and expressing genes into a cell are known in the art. In the context of an expression vector, the vector can be readily introduced into a host cell, *e.g.*, mammalian, bacterial, yeast, or insect cell by any method in the art. For example, the expression vector can be transferred into a host cell by physical, chemical, or biological means.

Physical methods for introducing a polynucleotide into a host cell include calcium phosphate precipitation, lipofection, particle bombardment, microinjection, electroporation, and the like. Methods for producing cells comprising vectors and/or exogenous nucleic acids are well-known in the art. See, for example, Sambrook et al. (2001, Molecular Cloning: A Laboratory Manual, Cold Spring Harbor Laboratory, New York). A preferred method for the introduction of a polynucleotide into a host cell is calcium phosphate transfection.

Biological methods for introducing a polynucleotide of interest into a host cell include the use of DNA and RNA vectors. Viral vectors, and especially retroviral vectors, have become the most widely used method for inserting genes into mammalian, e.g., human cells. Other viral vectors can be derived from lentivirus, poxviruses, herpes simplex virus I, adenoviruses and adeno-associated viruses, and the like. See, for example, US5,350,674 and 5,585,362.

Chemical means for introducing a polynucleotide into a host cell include colloidal dispersion systems, such as macromolecule complexes, nanocapsules, microspheres, beads, and lipid-based systems including oil-in-water emulsions, micelles, mixed micelles, and liposomes. An exemplary colloidal system for use as a delivery vehicle *in vitro* and *in vivo* is a liposome (*e.g.,* an artificial membrane vesicle).

In the case where a non-viral delivery system is utilized, an exemplary delivery vehicle is a liposome. The use of lipid formulations is contemplated for the introduction of the nucleic acids into a host cell (*in vitro, ex vivo* or *in vivo*). In another aspect, the nucleic acid may be associated with a lipid. The nucleic acid associated with a lipid may be encapsulated in the aqueous interior of a liposome, interspersed within the lipid bilayer of a liposome, attached to a liposome via a linking molecule that is associated with both the liposome and the oligonucleotide, entrapped in a liposome, complexed with a liposome, dispersed in a solution containing a lipid, mixed with a lipid, combined with a lipid, contained as a suspension in a lipid, contained or complexed with a micelle, or otherwise associated with a lipid. Lipid, lipid/DNA or lipid/expression vector associated compositions are not limited to any particular structure in solution. For example, they may be present in a bilayer structure, as micelles, or with a "collapsed" structure. They may also simply be interspersed in a solution, possibly forming aggregates that are not uniform in size or shape. Lipids are fatty substances which may be naturally occurring or synthetic lipids. For example, lipids include the fatty droplets that naturally occur in the cytoplasm as well as the class of compounds which contain long-chain aliphatic hydrocarbons and their derivatives, such as fatty acids, alcohols, amines, amino alcohols, and aldehydes.

Lipids suitable for use can be obtained from commercial sources. For example, dimyristyl phosphatidylcholine ("DMPC") can be obtained from Sigma, St. Louis, MO; dicetyl phosphate ("DCP") can be obtained from K & K Laboratories (Plainview, NY); cholesterol ("Choi") can be obtained from Calbiochem-Behring; dimyristyl phosphatidylglycerol ("DMPG") and other lipids may be obtained from Avanti Polar Lipids, Inc. (Birmingham, AL). Stock solutions of lipids in chloroform or chloroform/methanol can be stored at about -20°C. Chloroform is used as the only solvent since it is more readily evaporated than methanol. "Liposome" is a generic term encompassing a variety of single and multilamellar lipid vehicles formed by the generation of enclosed lipid bilayers or aggregates. Liposomes can be characterized as having vesicular structures with a phospholipid bilayer membrane and an inner aqueous medium. Multilamellar liposomes have multiple lipid layers separated by aqueous medium. They form spontaneously when phospholipids are suspended in an excess of aqueous solution. The lipid components undergo self-rearrangement before the formation of closed structures and entrap water and dissolved solutes between the lipid bilayers (Ghosh et al., 1991 Glycobiology 5: 505-10). However, compositions that have different structures in solution than the normal vesicular structure are also encompassed. For example, the lipids may assume a micellar structure or merely exist as nonuniform aggregates of lipid molecules. Also contemplated are lipofectamine-nucleic acid complexes.

Regardless of the method used to introduce exogenous nucleic acids into a host cell, in order to confirm the presence of the recombinant DNA sequence in the host cell, a variety of assays may be performed. Such assays include, for example, "molecular biological" assays well known to those of skill in the art, such as Southern and Northern blotting, RT-PCR and PCR; "biochemical" assays, such as detecting the presence or absence of a particular peptide, *e.g.,* by immunological means (ELISAs and Western blots) or by assays described herein to identify agents falling within the scope of the invention.

In one embodiment, the T cells of the invention are modified through the introduction of RNA. In one embodiment, an *in vitro* transcribed RNA CAR can be introduced to a cell as a form of transient transfection. The RNA is produced by *in vitro* transcription using a polymerase chain reaction (PCR)-generated template. DNA of interest from any source can be directly converted by PCR into a template for *in vitro* mRNA synthesis using appropriate primers and RNA polymerase. The source of the DNA can be, for example, genomic DNA, plasmid DNA, phage DNA, cDNA, synthetic DNA sequence or any other appropriate source of DNA. The desired template for *in vitro* transcription is the CAR of the present invention.

In one embodiment, the DNA to be used for PCR contains an open reading frame. The DNA can be from a naturally occurring DNA sequence from the genome of an organism. In one embodiment, the DNA is a full-length gene of interest or a portion of a gene. The gene can include some or all of the 5' and/or 3' untranslated regions (UTRs). The gene can include exons and introns. In one embodiment, the DNA to be used for PCR is a human gene. In another embodiment, the DNA to be used for PCR is a human gene including the 5' and 3' UTRs. The DNA can alternatively be an artificial DNA sequence that is not normally expressed in a naturally occurring organism. An exemplary artificial DNA sequence is one that contains portions of genes that are ligated together to form an open reading frame that encodes a fusion protein. The portions of DNA that are ligated together can be from a single organism or from more than one organism.

PCR is used to generate a template for *in vitro* transcription of mRNA which is used for transfection. Methods for performing PCR are well known in the art. Primers for use in PCR are designed to have regions that are substantially complementary to regions of the DNA to be used as a template for the PCR. "Substantially complementary", as used herein, refers to sequences of nucleotides where a majority or all of the bases in the primer sequence are complementary, or one or more bases are non-complementary, or mismatched. Substantially complementary sequences are able to anneal or hybridize with the intended DNA target under annealing conditions used for PCR. The primers can be designed to be substantially complementary to any portion of the DNA template. For example, the primers can be designed to amplify the portion of a gene that is normally transcribed in cells (the open reading frame), including 5' and 3' UTRs. The primers can also be designed to amplify a portion of a gene that encodes a particular domain of interest. In one embodiment, the primers are designed to amplify the coding region of a human cDNA, including all or portions of the 5' and 3' UTRs. Primers useful for PCR are generated by synthetic methods that are well known in the art.

"Forward primers" are primers that contain a region of nucleotides that are substantially complementary to nucleotides on the DNA template that are upstream of the DNA sequence that is to be amplified. "Upstream" is used herein to refer to a location 5', to the DNA sequence to be amplified relative to the coding strand. "Reverse primers" are primers that contain a region of nucleotides that are substantially complementary to a double-stranded DNA template that are downstream of the DNA sequence that is to be amplified. "Downstream" is used herein to refer to a location 3' to the DNA sequence to be amplified relative to the coding strand.

Any DNA polymerase useful for PCR can be used in the methods disclosed herein. The reagents and polymerase are commercially available from a number of sources.

Chemical structures with the ability to promote stability and/or translation efficiency may also be used. The RNA preferably has 5' and 3' UTRs. In one embodiment, the 5' UTR is between zero and 3000 nucleotides in length. The length of 5' and 3' UTR sequences to be added to the coding region can be altered by different methods, including, but not limited to, designing primers for PCR that anneal to different regions of the UTRs. Using this approach, one of ordinary skill in the art can modify the 5' and 3' UTR lengths required to achieve optimal translation efficiency following transfection of the transcribed RNA. The 5' and 3' UTRs can be the naturally occurring, endogenous 5' and 3' UTRs for the gene of interest. Alternatively, UTR sequences that are not endogenous to the gene of interest can be added by incorporating the UTR sequences into the forward and reverse primers or by any other modifications of the template. The use of UTR sequences that are not endogenous to the gene of interest can be useful for modifying the stability and/or translation efficiency of the RNA. For example, it is known that AU-rich elements in 3' UTR sequences can decrease the stability of mRNA. Therefore, 3' UTRs can be selected or designed to increase the stability of the transcribed RNA based on properties of UTRs that are well known in the art.

In one embodiment, the 5' UTR can contain the Kozak sequence of the endogenous gene. Alternatively, when a 5' UTR that is not endogenous to the gene of interest is being added by PCR as described above, a consensus Kozak sequence can be redesigned by adding the 5' UTR sequence. Kozak sequences can increase the efficiency of translation of some RNA transcripts, but does not appear to be required for all RNAs to enable efficient translation. The requirement for Kozak sequences for many mRNAs is known in the art. In other embodiments, the 5' UTR can be derived from an RNA virus whose RNA genome is stable in cells. In other embodiments, various nucleotide analogues can be used in the 3' or 5' UTR to impede exonuclease degradation of the mRNA.

To enable synthesis of RNA from a DNA template without the need for gene cloning, a promoter of transcription should be attached to the DNA template upstream of the sequence to be transcribed. When a sequence that functions as a promoter for an RNA polymerase is added to the 5' end of the forward primer, the RNA polymerase promoter becomes incorporated into the PCR product upstream of the open reading frame that is to be transcribed. In one preferred embodiment, the promoter is a T7 polymerase promoter, as described elsewhere herein. Other useful promoters include, but are not limited to, T3 and SP6 RNA polymerase promoters. Consensus nucleotide sequences for T7, T3 and SP6 promoters are known in the art.

In one embodiment, the mRNA has both a cap on the 5' end and a 3' poly(A) tail which determine ribosome binding, initiation of translation and stability mRNA in the cell. On a circular DNA template, for instance, plasmid DNA, RNA polymerase produces a long concatemeric product which is not suitable for expression in eukaryotic cells. The transcription of plasmid DNA linearized at the end of the 3' UTR results in normal sized mRNA which is not effective in eukaryotic transfection even if it is polyadenylated after transcription.

On a linear DNA template, phage T7 RNA polymerase can extend the 3' end of the transcript beyond the last base of the template (Schenborn and Mierendorf, Nuc Acids Res., 13:6223-36 (1985); Nacheva and Berzal-Herranz, Eur. J. Biochem., 270: 1485-65 (2003).

The conventional method of integration of polyA/T stretches into a DNA template is molecular cloning. However, polyA/T sequence integrated into plasmid DNA can cause plasmid instability, which is why plasmid DNA templates obtained from bacterial cells are often highly contaminated with deletions and other aberrations. This makes cloning procedures not only laborious and time consuming but often not reliable. That is why a method which allows construction of DNA templates with polyA/T 3' stretch without cloning highly desirable.

The polyA/T segment of the transcriptional DNA template can be produced during PCR by using a reverse primer containing a polyT tail, such as 100T tail (size can be 50-5000 T), or after PCR by any other method, including, but not limited to, DNA ligation or *in vitro* recombination. Poly(A) tails also provide stability to RNAs and reduce their degradation. Generally, the length of a poly(A) tail positively correlates with the stability of the transcribed RNA. In one embodiment, the poly(A) tail is between 100 and 5000 adenosines.

Poly(A) tails of RNAs can be further extended following *in vitro* transcription with the use of a poly(A) polymerase, such as E. coli polyA polymerase (E-PAP). In one embodiment, increasing the length of a poly(A) tail from 100 nucleotides to between 300 and 400 nucleotides results in about a two-fold increase in the translation efficiency of the RNA. Additionally, the attachment of different chemical groups to the 3' end can increase mRNA stability. Such attachment can contain modified/artificial nucleotides, aptamers and other compounds. For example, ATP analogs can be incorporated into the poly(A) tail using poly(A) polymerase. ATP analogs can further increase the stability of the RNA.

5' caps on RNAs also provide stability to RNA molecules. In a preferred embodiment, RNAs produced by the methods disclosed herein include a 5' cap. The 5' cap is provided using techniques known in the art and described herein (Cougot, et al., Trends in Biochem. Sci., 29:436-444 (2001); Stepinski, et al., RNA, 7: 1468-95 (2001); Elango, et al., Biochim. Biophys. Res. Commun., 330:958-966 (2005)).

The RNAs produced by the methods disclosed herein can also contain an internal ribosome entry site (IRES) sequence. The IRES sequence may be any viral, chromosomal or artificially designed sequence which initiates cap-independent ribosome binding to mRNA and facilitates the initiation of translation. Any solutes suitable for cell electroporation, which can contain factors facilitating cellular permeability and viability such as sugars, peptides, lipids, proteins, antioxidants, and surfactants can be included.

RNA can be introduced into target cells using any of a number of different methods, for instance, commercially available methods which include, but are not limited to, electroporation (Amaxa Nucleofector-II (Amaxa Biosystems, Cologne, Germany)), (ECM 830 (BTX) (Harvard Instruments, Boston, Mass.) or the Gene Pulser II (BioRad, Denver, Colo.), Multiporator (Eppendort, Hamburg Germany), cationic liposome mediated transfection using lipofection, polymer encapsulation, peptide mediated transfection, or biolistic particle delivery systems such as "gene guns" (see, for example, Nishikawa, et al. Hum Gene Ther., 12(8):861-70 (2001).

In one embodiment, the CAR sequences are delivered into cells using a retroviral or lentiviral vector. CAR-expressing retroviral and lentiviral vectors can be delivered into different types of eukaryotic cells as well as into tissues and whole organisms using transduced cells as carriers or cell-free local or systemic delivery of encapsulated, bound or naked vectors. The method used can be for any purpose where stable expression is required or sufficient.

In another embodiment, the CAR sequences are delivered into cells using *in vitro* transcribed mRNA. *In vitro* transcribed mRNA CAR can be delivered into different types of eukaryotic cells as well as into tissues and whole organisms using transfected cells as carriers or cell-free local or systemic delivery of encapsulated, bound or naked mRNA. The method used can be for any purpose where transient expression is required or sufficient.

In another embodiment, the desired CAR can be expressed in the cells by way of transposons.

Prior to expansion and genetic modification of the Treg cells of the invention, a source of T cells is obtained from a subject. T cells can be obtained from a number of sources, including peripheral blood mononuclear cells, bone marrow, lymph node tissue, cord blood, thymus tissue, tissue from a site of infection, ascites, pleural effusion, spleen tissue, and tumors. In certain embodiments of the present invention, any number of T cell lines available in the art, may be used. In certain embodiments of the present invention, T cells can be obtained from a unit of blood collected from a subject using any number of techniques known to the skilled artisan, such as Ficoll™ separation. In one embodiment, cells from the circulating blood of an individual are obtained by apheresis. The apheresis product typically contains lymphocytes, including T cells, monocytes, granulocytes, B cells, other nucleated white blood cells, red blood cells, and platelets. In one embodiment, cells from the circulating blood of an individual are obtained by leukapheresis.

In one embodiment, the cells collected by leukapheresis may be washed to remove the plasma fraction and to place the cells in an appropriate buffer or media for subsequent processing steps. In one embodiment of the invention, the cells are washed with phosphate buffered saline (PBS). In an alternative embodiment, the wash solution lacks calcium and may lack magnesium or may lack many if not all divalent cations. After washing, the cells may be resuspended in a variety of biocompatible buffers, such as, for example, $Ca^{2+}$-free, $Mg^{2+}$-free PBS, PlasmaLyte A, or other saline solution with or without buffer. Alternatively, the undesirable components of the leukapheresis sample may be removed and the cells directly resuspended in culture media.

In another embodiment, T cells are isolated from peripheral blood lymphocytes by lysing the red blood cells and depleting the monocytes, for example, by centrifugation through a PERCOLL™ gradient or by counterflow centrifugal elutriation. A specific subpopulation of T cells can be further isolated by positive or negative selection techniques. For example, in one embodiment, T cells are isolated by incubation with anti-CD3/anti-CD28 (*i.e.,* 3x28)-conjugated beads, such as DYNABEADS® M-450 CD3/CD28 T, for a time period sufficient for positive selection of the desired T cells. In one embodiment, the time period is about 30 minutes. In a further embodiment, the time period ranges from 30 minutes to 36 hours or longer and all integer values there between. In a further embodiment, the time period is at least 1, 2, 3, 4, 5, or 6 hours. In yet another preferred embodiment, the time period is 10 to 24 hours. In one preferred embodiment, the incubation time period is 24 hours. Longer incubation times may be used to isolate T cells in any situation where there are few T cells as compared to other cell types. Thus, by simply shortening or lengthening the time T cells are allowed to bind to the CD3/CD28 beads and/or by increasing or decreasing the ratio of beads to T cells (as described further herein), subpopulations of T cells can be preferentially selected for or against at culture initiation or at other time points during the process. Additionally, by increasing or decreasing the ratio of anti-CD3 and/or anti-CD28 antibodies on the beads or other surface, subpopulations of T cells can be preferentially selected for or against at culture initiation or at other desired time points. The skilled artisan would recognize that multiple rounds of selection can also be used in the context of this invention.

In another embodiment, it may be desirable to perform the selection procedure and use the "unselected" cells in the activation and expansion process. "Unselected" cells can also be subjected to further rounds of selection. Enrichment of a T cell population by negative selection can be accomplished with a combination of antibodies directed to surface markers unique to the negatively selected cells. One method is cell sorting and/or selection via negative magnetic immuno-adherence or flow cytometry that uses a cocktail of monoclonal antibodies directed to cell surface markers present on the cells negatively selected. For example, to enrich for $CD4^+$ cells by negative selection, a monoclonal antibody cocktail typically includes antibodies to CD14, CD20, CD11b, CD16, HLA-DR, and CD8. In certain embodiments, T regulatory cells are depleted by anti-CD25 conjugated beads or other similar method of selection.

For isolation of a desired population of cells by positive or negative selection, the concentration of cells and surface (e.g., particles such as beads) can be varied. In certain embodiments, it may be desirable to significantly decrease the volume in which beads and cells are mixed together (*i.e.*, increase the concentration of cells), to ensure maximum contact of cells and beads. For example, in one embodiment, a concentration of 2 billion cells/ml is used. In one embodiment, a concentration of 1 billion cells/ml is used. In a further embodiment, greater than 100 million cells/ml is used. In a further embodiment, a concentration of cells of 10, 15, 20, 25, 30, 35, 40, 45, or 50 million cells/ml is used. In yet another embodiment, a concentration of cells from 75, 80, 85, 90, 95, or 100 million cells/ml is used. In further embodiments, concentrations of 125 or 150 million cells/ml can be used. Using high concentrations can result in increased cell yield, cell activation, and cell expansion. Further, use of high cell concentrations allows more efficient capture of cells that may weakly express target antigens of interest, such as CD28-negative T cells, or from samples where there are many tumor cells present (*i.e.*, leukemic blood, tumor tissue, etc.). Such populations of cells may have therapeutic value and would be desirable to obtain.

T cells for stimulation can also be frozen after a washing step. Wishing not to be bound by theory, the freeze and subsequent thaw step provides a more uniform product by removing granulocytes and to some extent monocytes in the cell population. After the washing step that removes plasma and platelets, the cells may be suspended in a freezing solution. While many freezing solutions and parameters are known in the art and will be useful in this context, one method involves using PBS containing 20% DMSO and 8% human serum albumin, or culture media containing 10% Dextran 40 and 5% Dextrose, 20% Human Serum Albumin and 7.5% DMSO, or 31.25% Plasmalyte-A, 31.25% Dextrose 5%, 0.45% NaCl, 10% Dextran 40 and 5% Dextrose, 20% Human Serum Albumin, and 7.5% DMSO or other suitable cell freezing media containing for example, Hespan and PlasmaLyte A, the cells then are frozen to -80°C at a rate of 1° per minute and stored in the vapor phase of a liquid nitrogen storage tank. Other methods of controlled freezing may be used as well as uncontrolled freezing immediately at -20°C or in liquid nitrogen.

In certain embodiments, cryopreserved cells are thawed and washed as described herein and allowed to rest for one hour at room temperature prior to activation.

Also contemplated in the context of the invention is the collection of blood samples or leukapheresis product from a subject at a time period prior to when the expanded cells as described herein might be needed. As such, the source of the cells to be expanded can be collected at any time point necessary, and desired cells, such as T cells, isolated and frozen for later use in T cell therapy for any number of diseases or conditions that would benefit from T cell therapy, such as those described herein. In one embodiment, a blood sample or a leukapheresis is taken from a generally healthy subject. In certain embodiments, a blood sample or a leukapheresis is taken from a generally healthy subject who is at risk of developing a disease, but who has not yet developed a disease, and the cells of interest are isolated and frozen for later use. In certain embodiments, the T cells may be expanded, frozen, and used at a later time. Whether prior to or after genetic modification of the Treg cells to express a desirable CAR, the T cells can be activated and expanded generally using methods as described, for example, in US6,352,694; 6,534,055; 6,905,680; 6,692,964; 5,858,358; 6,887,466; 6,905,681; 7,144,575; 7,067,318; 7,172,869; 7,232,566; 7,175,843; 5,883,223; 6,905,874; 6,797,514; 6,867,041; and US20060121005, incorporated herein by reference.

Generally, the Treg cells of the invention are expanded by contact with a surface having attached thereto an agent that stimulates a CD3/TCR complex associated signal and a ligand that stimulates a co-stimulatory molecule on the surface of the cells of the Treg cells. In particular, the Treg cells may be stimulated as described herein, such as by contact with an anti-CD3 antibody, or antigen-binding fragment thereof, or an anti-CD2 antibody immobilized on a surface, or by contact with a protein kinase C activator (e.g., bryostatin) in conjunction with a calcium ionophore. For co-stimulation of an accessory molecule on the surface of the T cells, a ligand that binds the accessory molecule is used. For example, a population of T cells can be contacted with an anti-CD3 antibody and an anti-CD28 antibody, under conditions appropriate for stimulating proliferation of the T cells. To stimulate proliferation of either $CD4^+$ T cells, an anti-CD3 antibody and an anti-CD28 antibody may be used. Examples of an anti-CD28 antibody include, without being limited to, 9.3, B-T3, XR-CD28 (Diaclone, Besancon, France). Other expansion methods commonly known in the art can be used (Berg et al., Transplant Proc. 30(8):3975-3977, 1998; Haanen et al., J. Exp. Med. 190(9): 13191328, 1999; Garland et al., J. Immunol Meth. 227(1-2):53-63, 1999).

In certain embodiments, the primary stimulatory signal and the co-stimulatory signal for the Treg cells of the invention may be provided by different protocols. For example, the agents providing each signal may be in solution or coupled to a surface. When coupled to a surface, the agents may be coupled to the same surface (*i.e.,* in "cis" formation) or to separate surfaces (*i.e.,* in "trans" formation). Alternatively, one agent may be coupled to a surface and the other agent in solution. In one embodiment, the agent providing the co-stimulatory signal is bound to a cell surface and the agent providing the primary activation signal is in solution or coupled to a surface. In certain embodiments, both agents can be in solution. In another embodiment, the agents may be in soluble form, and then crosslinked to a surface, such as a cell expressing Fc receptors or an antibody or other binding agent which will bind to the agents. In this regard, see for example, US20040101519 and 20060034810, incorporated herein by reference, for artificial antigen presenting cells (aAPCs) that are contemplated for use in activating and expanding T cells in the present invention.

In one embodiment, the two agents are immobilized on beads, either on the same bead, *i.e.,* "cis" or to separate beads, *i.e.,* "trans". By way of example, the agent providing the primary activation signal is an anti-CD3 antibody or an antigen-binding fragment thereof and the agent providing the co-stimulatory signal is an anti-CD28 antibody or antigen-binding fragment thereof; and both agents are co-immobilized to the same bead in equivalent molecular amounts. In one embodiment, a 1:1 ratio of each antibody bound to the beads for $CD4^+$ T cell expansion and T cell growth is used. In certain aspects of the present invention, a ratio of anti CD3:CD28 antibodies bound to the beads is used such that an increase in T cell expansion is observed as compared to the expansion observed using a ratio of 1:1. In one particular embodiment an increase of from about 1 to about 3 fold is observed as compared to the expansion observed using a ratio of 1:1. In one embodiment, the ratio of CD3:CD28 antibody bound to the beads ranges from 100:1 to 1:100 and all integer values there between. In one aspect of the present invention, more anti-CD28 antibody is bound to the particles than anti-CD3 antibody, i.e., the ratio of CD3:CD28 is less than one. In certain embodiments of the invention, the ratio of anti-CD28 antibody to anti CD3 antibody bound to the beads is greater than 2:1. In one particular embodiment, a 1:100 CD3:CD28 ratio of antibody bound to beads is used. In another embodiment, a 1:75 CD3:CD28 ratio of antibody bound to beads is used. In a further embodiment, a 1:50 CD3:CD28 ratio of antibody bound to beads is used. In another embodiment, a 1:30 CD3:CD28 ratio of antibody bound to beads is used. In one preferred embodiment, a 1:10 CD3:CD28 ratio of antibody bound to beads is used. In another embodiment, a 1:3 CD3:CD28 ratio of antibody bound to the beads is used. In yet another embodiment, a 3:1 CD3:CD28 ratio of antibody bound to the beads is used.

Ratios of particles to cells from 1:500 to 500:1 and any integer values in between may be used to stimulate T cells or other target cells. As those of ordinary skill in the art can readily appreciate, the ratio of particles to cells may depend on particle size relative to the target cell. For example, small sized beads could only bind a few cells, while larger beads could bind many. In certain embodiments, the ratio of cells to particles ranges from 1:100 to 100:1 and any integer values in-between and in further embodiments the ratio comprises 1:9 to 9:1 and any integer values in between, can also be used to stimulate T cells. The ratio of anti-CD3- and anti-CD28-coupled particles to T cells that results in T cell stimulation can vary as noted above, however certain preferred values include 1:100, 1:50, 1:40, 1:30, 1:20, 1:10, 1:9, 1:8, 1:7, 1:6, 1:5, 1:4, 1:3, 1:2, 1:1, 2:1, 3:1, 4:1, 5:1, 6:1, 7:1, 8:1, 9:1, 10:1, and 15:1 with one preferred ratio being at least 1:1 particles per T cell. In one embodiment, a ratio of particles to cells of 1:1 or less is used. In one particular embodiment, a preferred particle: cell ratio is 1:5. In further embodiments, the ratio of particles to cells can be varied depending on the day of stimulation. For example, in one embodiment, the ratio of particles to cells is from 1:1 to 10: 1 on the first day and additional particles are added to the cells every day or every other day thereafter for up to 10 days, at final ratios of from 1:1 to 1:10 (based on cell counts on the day of addition). In one particular embodiment, the ratio of particles to cells is 1:1 on the first day of stimulation and adjusted to 1:5 on the third and fifth days of stimulation. In another embodiment, particles are added on a daily or every other day basis to a final ratio of 1:1 on the first day, and 1:5 on the third and fifth days of stimulation. In another embodiment, the ratio of particles to cells is 2:1 on the first day of stimulation and adjusted to 1:10 on the third and fifth days of stimulation. In another embodiment, particles are added on a daily or every other day basis to a final ratio of 1:1 on the first day, and 1:10 on the third and fifth days of stimulation. One of skill in the art will appreciate that a variety of other ratios may be suitable for use in the present invention. In particular, ratios will vary depending on particle size and on cell size and type.

In further embodiments of the present invention, the Treg cells are combined with agent-coated beads, the beads and the cells are subsequently separated, and then the cells are cultured. In an alternative embodiment, prior to culture, the agent-coated beads and cells are not separated but are cultured together. In a further embodiment, the beads and cells are first concentrated by application of a force, such as a magnetic force, resulting in increased ligation of cell surface markers, thereby inducing cell stimulation.

By way of example, cell surface proteins may be ligated by allowing paramagnetic beads to which anti-CD3 and anti-CD28 are attached (3x28 beads) to contact the Treg cells of the invention. In one embodiment, the cells (for example, $10^4$ to $10^9$ T cells) and beads (for example, DYNABEADS® M-450 CD3/CD28 T paramagnetic beads at a ratio of 1: 1) are combined in a buffer, preferably PBS (without divalent cations such as, calcium and magnesium). Again, those of ordinary skill in the art can readily appreciate that any cell concentration may be used. For example, the target cell may be very rare in the sample and comprise only 0.01% of the sample or the entire sample (*i.e.,* 100%) may comprise the target cell of interest. Accordingly, any cell number is within the context of the present invention. In certain embodiments, it may be desirable to significantly decrease the volume in which particles and cells are mixed together (*i.e.,* increase the concentration of cells), to ensure maximum contact of cells and particles. For example, in one embodiment, a concentration of about 2 billion cells/ml is used. In another embodiment, greater than 100 million cells/ml is used. In a further embodiment, a concentration of cells of 10, 15, 20, 25, 30, 35, 40, 45, or 50 million cells/ml is used. In yet another embodiment, a concentration of cells from 75, 80, 85, 90, 95, or 100 million cells/ml is used. In further embodiments, concentrations of 125 or 150 million cells/ml can be used. Using high concentrations can result in increased cell yield, cell activation, and cell expansion. Further, use of high cell concentrations allows more efficient capture of cells that may weakly express target antigens of interest, such as CD28-negative T cells. Such populations of cells may have therapeutic value and would be desirable to obtain in certain embodiments.

In one embodiment of the present invention, the mixture may be cultured for several hours (about 3 hours) to about 14 days or any hourly integer value in between. In another embodiment, the mixture may be cultured for 21 days. In one embodiment of the invention the beads and the T cells are cultured together for about 8 days. In another embodiment, the beads and T cells are cultured together for 2-3 days. Several cycles of stimulation may also be desired such that culture time of T cells can be 60 days or more. Conditions appropriate for T cell culture include an appropriate media (e.g., Minimal Essential Media or RPMI Media 1640 or, X-vivo 15, (Lonza)) that may contain factors necessary for proliferation and viability, including serum (e.g., fetal bovine or human serum), interleukin-2 (IL-2), insulin, IFN-$\gamma$, IL-4, IL-7, GM-CSF, IL-10, IL-12, IL-15, TGF$\beta$, and TNF-$\alpha$ or any other additives for the growth of cells known to the skilled artisan. Other additives for the growth of cells include, but are not limited to, surfactant, plasmanate, and reducing agents such as N-acetyl-cysteine and 2-mercaptoethanol. Media can include RPMI 1640, AIM-V, DMEM, MEM, $\alpha$-MEM, F- 12, X-Vivo 15, and X-Vivo 20, Optimizer, with added amino acids, sodium pyruvate, and vitamins, either serum-free or supplemented with an appropriate amount of serum (or plasma) or a defined set of hormones, and/or an amount of cytokine(s) sufficient for the growth and expansion of T cells. Antibiotics, *e.g.*, penicillin and streptomycin, are included only in experimental cultures, not in cultures of cells that are to be infused into a subject. The target cells are maintained under conditions necessary to support growth, for example, an appropriate temperature (*e.g.,* 37°C) and atmosphere (*e.g.,* air plus 5% $CO_2$).

T cells that have been exposed to varied stimulation times may exhibit different characteristics. For example, typical blood or apheresed peripheral blood mononuclear cell products have a helper T cell population (Th, $CD4^+$) that is greater than the cytotoxic or suppressor T cell population (Tc, $CD8^+$). Ex vivo expansion of T cells by stimulating CD3 and CD28 receptors produces a population of T cells that prior to about days 8-9 consists predominately of Th cells, while after about days 8-9, the population of T cells comprises an increasingly greater population of Tc cells. Accordingly, depending on the purpose of treatment, infusing a subject with a T cell population comprising predominately of Th cells may be advantageous. Similarly, if an antigen- specific subset of Tc cells has been isolated it may be beneficial to expand this subset to a greater degree. Further, in addition to CD4 and CD8 markers, other phenotypic markers vary significantly, but in large part, reproducibly during the course of the cell expansion process. Thus, such reproducibility enables the ability to tailor an activated T cell product for specific purposes.

In one embodiment of the invention, the T cells may be cultured in the presence of rapamycin in order to obtain regulatory T cells, as described for example in WO2007110785 incorporated herein by reference. Another method to generate regulatory T cells is described in US2016024470 incorporated herein by reference, where T cells are cultured with a T cell receptor (TCR)/CD3 activator such as for example TCR/CD3 antibodies, a TCR co-stimulator activator such as for example CD28, CD137 (4-1 BB), GITR, B7-1/2, CD5, ICOS, OX40, CD40 or CD137 antibodies, and rapamycin.

In one embodiment of the invention, the T cells genetically modified by expression of the CAR may also have been genetically modified by expression of at least one intracellular factor such as ROR-C, Foxp3, Foxo1, T-bet, or Gata 3, c-Maf, AhR. In one embodiment, the genetically modified immune cell of the invention expresses Foxp3. In one embodiment, the genetically modified immune cell of the invention expresses Foxo1.

In one embodiment, the genetically modified Treg cell of the invention can be an allogeneic Treg cell. For example, the allogeneic Treg cell can be a T cell lacking expression of a functional human leukocyte antigen (HLA), *e.g.,* HLA class I and/or HLA class II, and/or a T cell lacking expression of an endogenous HLA.

In one embodiment, the Treg cells as described herein can be engineered such that they do not express a functional HLA on its surface. For example, a Treg cell as described herein can be engineered such that cell surface expression HLA, *e.g.,* HLA class 1 (in particular an HLA-A, HLA-B or HLA-C) and/or HLA class II or non-classical HLA molecules is downregulated.

In another embodiment, the Treg cell can lack a functional TCR and a functional HLA such as HLA class I (in particular an HLA-A, HLA-B or HLA-C) and/or HLA class II.

In another embodiment, a Treg cell described herein can be engineered such that it does not express an endogenous HLA on its surface.

In one embodiment, the Treg cell comprises a null mutation in a gene selected from a T cell receptor alpha or beta chain gene, a HLA Class I or II gene, a HLA Class II regulator gene (*e.g.*, RFXANK (regulatory factor X associated Ankyrin containing protein), RFX5 (regulatory factor X5), RFXAP (regulatory factor X associated protein; RFX subunits), and CIITA (Class II major histocompatibility complex transactivator)), a transporter associated with antigen processing, a minor histocompatibility antigen gene, and a $\beta 2$ microglobulin (B2M) gene. In one embodiment, the Treg cells of the present disclosure are human cells.

Modified Treg cells that lack expression of a functional TCR and/or HLA can be obtained by any suitable means, including a knock out or knock down of one or more subunit of TCR or HLA. For example, the Treg cell can include a knock down of TCR and/or HLA using siRNA, shRNA, clustered regularly interspaced short palindromic repeats (CRISPR) transcription-activator like effector nuclease (TALEN), zinc finger endonuclease (ZFN), meganuclease (mn, also known as homing endonuclease), or megaTAL (combining a TAL effector with a mn cleavage domain).

In one embodiment, the nucleic acid encoding a CAR as described herein is inserted at a specific locus in the genome of a Treg, such as, for example, at the locus of a gene to be deleted. In one embodiment, the nucleic acid encoding a CAR as described herein is inserted within a HLA locus, thereby resulting in the inhibition of HLA expression.

In one embodiment, TCR expression and/or HLA expression can be inhibited using siRNA or shRNA that targets a nucleic acid encoding a TCR and/or HLA in a T cell. Expression of siRNA and shRNAs in T cells can be achieved using any conventional expression system, e.g., such as a lentiviral expression system. Exemplary shRNAs that downregulate expression of components of the TCR are described, *e.g.*, in US2012/0321667. Exemplary siRNA and shRNA that downregulate expression of HLA class I and/or HLA class II genes are described, *e.g.*, in US2007/0036773.

"CRISPR" or "CRISPR to TCR and/or HLA" or "CRISPR to inhibit TCR and/or HLA" as used herein refers to a set of clustered regularly interspaced short palindromic repeats, or a system comprising such a set of repeats. "Cas", as used herein, refers to a CRISPR-associated protein. A "CRISPR/Cas" system refers to a system derived from CRISPR and Cas which can be used to silence or mutate a TCR and/or HLA gene.

Naturally-occurring CRISPR/Cas systems are found in approximately 40% of sequenced eubacteria genomes and 90% of sequenced archaea. Grissa et al. (2007) BMC Bioinformatics 8: 172. This system is a type of prokaryotic immune system that confers resistance to foreign genetic elements such as plasmids and phages and provides a form of acquired immunity. Barrangou et al. (2007) Science 315: 1709-1712; Marragini et al. (2008) Science 322: 1843-1845. The CRISPR/Cas system has been modified for use in gene editing (silencing, enhancing or changing specific genes) in eukaryotes such as mice or primates. Wiedenheft et al. (2012) Nature 482: 331-8. This is accomplished by introducing into the eukaryotic cell a plasmid containing a specifically designed CRISPR and one or more appropriate Cas. The CRISPR sequence, sometimes called a CRISPR locus, comprises alternating repeats and spacers. In a naturally-occurring CRISPR, the spacers usually comprise sequences foreign to the bacterium such as a plasmid or phage sequence; in the TCR and/or HLA CRISPR/Cas system, the spacers are derived from the TCR or HLA gene sequence. RNA from the CRISPR locus is constitutively expressed and processed by Cas proteins into small RNAs. These comprise a spacer flanked by a repeat sequence. The RNAs guide other Cas proteins to silence exogenous genetic elements at the RNA or DNA level. Horvath et al. (2010) Science 327: 167-170; Makarova et al. (2006) Biology Direct 1: 7. The spacers thus serve as templates for RNA molecules, analogously to siRNAs. Pennisi (2013) Science 341: 833-836. The CRISPR/Cas system can thus be used to edit a TCR and/or HLA gene (adding or deleting a basepair), or introducing a premature stop which thus decreases expression of a TCR and/or HLA. The CRISPR/Cas system can alternatively be used like RNA interference, turning off HLA gene in a reversible fashion. In a mammalian cell, for example, the RNA can guide the Cas protein to a TCR and/or HLA promoter, sterically blocking RNA polymerases.

Artificial CRISPR/Cas systems can be generated which inhibit TCR and/or HLA, using technology known in the art, *e.g.,* that described in US20140068797, and Cong (2013) Science 339: 819-823. Other artificial CRISPR/Cas systems that are known in the art may also be generated which inhibit TCR and/or HLA, *e.g.,* that described in Tsai (2014) Nature Biotechnol., 32:6 569-576, US8,871,445; 8,865,406; 8,795,965; 8,771,945; and 8,697,359.

"TALEN" or "TALEN to TCR and/or HLA" or "TALEN to inhibit TCR and/or HLA" refers to a transcription activator-like effector nuclease, an artificial nuclease which can be used to edit the TCR and/or HLA gene. TALENs are produced artificially by fusing a TAL effector DNA binding domain to a DNA cleavage domain. Transcription activator-like effectors (TALEs) can be engineered to bind any desired DNA sequence, including a portion of the TCR and/or HLA gene. By combining an engineered TALE with a DNA cleavage domain, a restriction enzyme can be produced which is specific to any desired DNA sequence, including a TCR and/or HLA sequence. These can then be introduced into a cell, wherein they can be used for genome editing. Boch (2011) Nature Biotech. 29: 135-6; and Boch et al. (2009) Science 326: 1509-12; Moscou et al. (2009) Science 326: 3501.

TALEs are proteins secreted by Xanthomonas bacteria. The DNA binding domain contains a repeated, highly conserved 33-34 amino acid sequence, with the exception of the 12th and 13th amino acids. These two positions are highly variable, showing a strong correlation with specific nucleotide recognition. They can thus be engineered to bind to a desired DNA sequence. To produce a TALEN, a TALE protein is fused to a nuclease (N), which is a wild-type or mutated Fokl endonuclease. Several mutations to Fokl have been made for its use in TALENs; these, for example, improve cleavage specificity or activity. Cermak et al. (2011) Nucl. Acids Res. 39: e82; Miller et al. (2011) Nature Biotech. 29: 143-8; Hockemeyer et al. (2011) Nature Biotech. 29: 731-734; Wood et al. (2011) Science 333: 307; Doyon et al. (2010) Nature Methods 8: 74-79; Szczepek et al. (2007) Nature Biotech. 25: 786-793; and Guo et al. (2010) /. Mol. Biol. 200: 96. The Fokl domain functions as a dimer, requiring two constructs with unique DNA binding domains for sites in the target genome with proper orientation and spacing. Both the number of amino acid residues between the TALE DNA binding domain and the Fokl cleavage domain and the number of bases between the two individual TALEN binding sites appear to be important parameters for achieving high levels of activity. Miller et al. (2011) Nature Biotech. 29: 143-8. A HLA TALEN can be used inside a cell to produce a double- stranded break (DSB). A mutation can be introduced at the break site if the repair mechanisms improperly repair the break via non-homologous end joining. For example, improper repair may introduce a frame shift mutation. Alternatively, foreign DNA can be introduced into the cell along with the TALEN; depending on the sequences of the foreign DNA and chromosomal sequence, this process can be used to correct a defect in the TCR and/or HLA gene or introduce such a defect into a wt TCR and/or HLA gene, thus decreasing expression of HLA. TALENs specific to sequences in TCR and/or HLA can be constructed using any method known in the art, including various schemes using modular components. Zhang et al. (2011) Nature Biotech. 29: 149-53; Geibler et al. (2011) PLoS ONE 6: el9509.

"ZFN" or "Zinc Finger Nuclease" or "ZFN to TCR and/or HLA" or "ZFN to inhibit TCR and/or HLA" refer to a zinc finger nuclease, an artificial nuclease which can be used to edit the TCR and/or HLA gene. Like a TALEN, a ZFN comprises a Fokl nuclease domain (or derivative thereof) fused to a DNA-binding domain. In the case of a ZFN, the DNA-binding domain comprises one or more zinc fingers. Carroll et al. (2011) Genetics Society of America 188: 773-782; and Kim et al. (1996) Proc. Natl. Acad. Sci. USA 93: 1156-1160. A zinc finger is a small protein structural motif stabilized by one or more zinc ions. A zinc finger can comprise, for example, $Cys_2His_2$ (SEQ ID NO: 82), and can recognize an approximately 3-bp sequence. Various zinc fingers of known specificity can be combined to produce multi-finger polypeptides which recognize about 6, 9, 12, 15 or 18-bp sequences. Various selection and modular assembly techniques are available to generate zinc fingers (and combinations thereof) recognizing specific sequences, including phage display, yeast one -hybrid systems, bacterial one -hybrid and two-hybrid systems, and mammalian cells.

Like a TALEN, a ZFN must dimerize to cleave DNA. Thus, a pair of ZFNs are required to target non-palindromic DNA sites. The two individual ZFNs must bind opposite strands of the DNA with their nucleases properly spaced apart. Bitinaite et al. (1998) Proc. Natl. Acad. Sci. USA 95: 10570-5. Also like a TALEN, a ZFN can create a double-stranded break in the DNA, which can create a frame-shift mutation if improperly repaired, leading to a decrease in the expression and amount of TCR and/or HLA in a cell. ZFNs can also be used with homologous recombination to mutate in the TCR and/or HLA gene. ZFNs specific to sequences in TCR and/or HLA can be constructed using any method known in the art. See, *e.g.*, Provasi (2011) Nature Med. 18: 807-815; Torikai (2013) Blood 122: 1341-1349; Cathomen et al. (2008) Mol. Ther. 16: 1200-7; Quo et al. (2010) /. Mol. Biol. 400: 96; US2011/0158957; and US2012/0060230.

"Meganuclease" or "meganuclease to TCR and/or HLA" or "meganuclease to inhibit TCR and/or HLA" refers to a monomeric endonuclease with large (>14 base pairs) recognition sites, which can be used to edit the TCR and/or HLA gene. Meganucleases (mn) are monomeric proteins with innate nuclease activity that are derived from bacterial homing endonucleases and engineered for a unique target site. Homing endonucleases are DNA-cleaving enzymes that can generate double strand breaks at individual loci in their host genomes, and thereby drive site-specific gene conversion events. (Stoddard, Structure. 2011 Jan 12;19(1):7-15). Despite their small size, homing endonucleases recognize long DNA sequences (typically 20 to 30 base pairs). Homing endonucleases are extremely widespread and are found in microbes, as well as in phages and viruses. The LAGLIDADG and His-Cys box enzymes (which are the most sequence-specific of these enzymes) rely upon antiparallel β-sheets that dock into the major grooves of their DNA target sites (Flick et al., 1998; Jurica et al., 1998). There they establish a collection of sequence-specific and non-specific contacts that are distributed nonuniformly across multiple consecutive basepairs (Chevalier et al., 2003; Scalley-Kim et al., 2007).

The LAGLIDADG homing endonuclease (LHE) family is the primary source of the engineered enzymes used for gene targeting applications. The LHE family is primarily encoded within archaea and in the chloroplast and mitochondrial genomes of algae and fungi (Chevalier et al., 2005; Dalgaard et al., 1997; Sethuraman et al., 2009). Meganucleases that possess a single conserved LAGLIDADG motif (SEQ ID NO: 52) per protein chain form homodimeric proteins that cleave palindromic and nearly palindromic DNA target sequences, while those that contain two such motifs per protein chain form larger, pseudo-symmetric monomers that can target completely asymmetric DNA sequences.

Meganucleases can be engineered to target TCR and/or HLA and thus create a double-stranded break in the DNA, which can create a frame-shift mutation if improperly repaired, leading to a decrease in the expression and amount of TCR and/or HLA in a cell.

"MegaTAL" or "megaTAL to TCR and/or HLA" or "megaTAL to inhibit TCR and/or HLA" refers to an artificial nuclease, which can be used to edit the TCR and/or HLA gene. MegaTALs are hybrid monomeric nucleases obtained through the fusion of minimal TAL (transcription activator-like) effector domains to the N-terminus of meganucleases derived from the LAGLIDADG homing endonuclease family (Nucleic Acids Res. 2014 Feb;42(4):2591-601; Takeuchi et al, Methods Mol Biol. 2015;1239:105-32. doi: 10.1007/978-1-4939-1862-1_6). MegaTALs thus consist of a site-specific meganuclease cleavage head with additional affinity and specificity provided by a TAL effector DNA binding domain.

MegaTALs can be engineered to target TCR and/or HLA and thus create a double-stranded break in the DNA, which can create a frame-shift mutation if improperly repaired, leading to a decrease in the expression and amount of TCR and/or HLA in a cell.

While not wishing to be bound by any particular theory, in some embodiments, a therapeutic T cell has short term persistence in a patient, due to shortened telomeres in the T cell; accordingly, transfection with a telomerase gene can lengthen the telomeres of the T cell and improve persistence of the T cell in the patient. See Carl June, "Adoptive T cell therapy for cancer in the clinic", Journal of Clinical Investigation, 117: 1466-1476 (2007). Thus, in an embodiment, the genetically modified Treg cell, ectopically expresses a telomerase subunit, *e.g.,* the catalytic subunit of telomerase, *e.g.,* TERT, *e.g.,* hTERT. In some aspects, this disclosure provides a method of producing a CR-expressing cell, comprising contacting a cell with a nucleic acid encoding a telomerase subunit, *e.g.*, the catalytic subunit of telomerase, *e.g.,* TERT, *e.g.,* hTERT. The cell may be contacted with the nucleic acid before, simultaneous with, or after being contacted with a construct encoding a CR.

The present invention further relates to a method for obtaining a Treg cell of the invention, wherein said method comprises transducing at least one Treg cell with a nucleic acid encoding a CAR as described hereinabove, and optionally expanding the transduced cells.

In one embodiment, the method is an ex vivo method.

In one embodiment, the method for obtaining Treg cells of the invention comprises:

- an isolation step of Treg cells from a PBMC population (*e.g.*, recovered by leukapheresis)
- one or more genetic modification step(s) wherein, in particular, a nucleic acid sequence encoding a CAR as described hereinabove is introduced or transferred within the Treg cells (other genetic modification steps may correspond, for example, to one or more gene disruption step(s), one or more gene correction step(s) or one or more gene addition step(s)),

- optionally an expansion step,
- optionally a washing step and,
- optionally a freezing step.

In one embodiment, the genetic modification step(s) correspond(s) to a gene disruption step, a gene correction step or a gene addition step, preferably a gene addition step. In one embodiment, the genetic modification step(s) is carried out by a method selected from the group comprising, but not limited to, transfection, transduction or gene editing.
Examples of methods of gene editing that may be used in the present invention include, but are not limited to, methods based on engineered nucleases, methods based on recombinant Adeno-Associated Virus (or AAV), methods based on Transposons (*e.g.*, Sleeping Beauty transposon system), methods based on homologous recombination, conditional targeting using site-specific recombinases (*e.g.*, Cre-LoxP and Flp-FRT systems), and Multiplex Automated Genomic Engineering (MAGE).
Non-limiting examples of engineered nucleases include, but are not limited to, clustered regularly interspaced short palindromic repeats (CRISPR) transcription-activator like effector nuclease (TALEN), zinc finger endonuclease (ZFN), meganuclease (mn, also known as homing endonuclease), or megaTAL (combining a TAL effector with a mn cleavage domain).
In one embodiment, the method for obtaining Treg cells of the invention comprises:

- an isolation step of Treg cells from a PBMC population (*e.g.*, recovered by leukapheresis)
- a transduction or transfection step with a vector comprising a nucleic acid sequence encoding a CAR as described hereinabove,
- optionally an expansion step,
- optionally a washing step and,
- optionally a freezing step.

Another object of the invention is a composition comprising, consisting or consisting essentially of at least one Treg cell population of the invention.
In one embodiment, said composition comprises, consists or consists essentially of at least one Treg cell population engineered to express on the cell surface a CAR specific for at least one IL-23R as described hereinabove, wherein said CAR comprises (i) an extracellular binding domain specific for IL-23R as described hereinabove, (ii) optionally an extracellular hinge domain as described hereinabove, (iii) a transmembrane domain as described hereinabove, (iv) an intracellular signaling domain as described hereinabove, and, (v) optionally a tag and/or a leader sequence as described hereinabove.
In one embodiment, said composition has been frozen and thawed.
Another object of the invention is a pharmaceutical composition comprising, consisting or consisting essentially of at least one Treg cell population as described hereinabove and at least one pharmaceutically acceptable excipient.
Another object of the invention is a medicament comprising, consisting or consisting essentially of at least one Treg cell population as described hereinabove.
In one embodiment, the pharmaceutical composition or medicament comprises at least one isolated and/or substantially purified Treg cell population of the invention.
In one embodiment, the pharmaceutical composition or medicament comprises a combination of Treg cell populations as described hereinabove (*i.e.*, at least two distinct Treg cell populations of the invention).
In one embodiment, a single dosing unit of the pharmaceutical composition comprises more than $10^4$ cells (*e.g.,* from about $10^5$ to about $10^6$ cells, from about $10^6$ to about $10^{10}$, from about $10^6$ to $10^7$, from about $10^6$ to $10^8$, from about $10^7$ to $10^8$, from about $10^7$ to $10^9$, or from about $10^8$ to $10^9$ cells). In one embodiment, a single dosing unit of the composition comprises about $10^6$, about $10^7$, about $10^8$, about $10^9$, or about $10^{10}$ or more cells.
In one embodiment, the composition, pharmaceutical composition or medicament of the invention further comprises at least one other Treg cell population, wherein cells of said other Treg cell population express on the cell surface a CAR specific of an antigen, a fragment of an antigen, a variant of an antigen or a mixture thereof.
In one embodiment, the cells of said other Treg cell population express on the cell surface a CAR specific of a food antigen from the common human diet.
In another embodiment, the cells of said other Treg cell population express on the cell surface a CAR specific of an autoantigen, such as, for example, a multiple sclerosis-associated antigen, a joint-associated antigen, an eye-associated antigen, a human HSP antigen, a skin-associated antigen or an antigen involved in graft rejection or GVHD
In another embodiment, the cells of said other Treg cell population express on the cell surface a CAR specific of an inhaled allergen, an ingested allergen or a contact allergen.

In one embodiment, the cells of said other Treg cell population express on the cell surface a CAR specific of an antigen selected from the group comprising ovalbumin, MOG, type II collagen fragments, variants and mixtures thereof.

In one embodiment, the cells of said other Treg cell population express on the cell surface a CAR specific of ovalbumin, fragments, variants and mixtures thereof.

In another embodiment, the cells of said other Treg cell population express on the cell surface a CAR specific of MOG, fragments, variants and mixtures thereof.

In another embodiment, the cells of said other Treg cell population express on the cell surface a CAR specific of type II collagen, fragments, variants and mixtures thereof.

In another embodiment, the cells of said other Treg cell population express on the cell surface a CAR specific of citrullinated vimentin, citrullinated type II collagen or citrullinated fibrinogen, fragments, variants and mixtures thereof.

In another embodiment, the cells of said other immune cell population express on the cell surface a CAR specific of HLA-A2, fragments, variants and mixtures thereof.

In one embodiment, the cells of said other Treg cell population express on the cell surface a CAR wherein the extracellular-binding domain of said CAR is a protein or fragments or variants thereof, such as for example, an autoantigen or fragments or variants thereof.

As used herein, the term "consisting essentially of", with reference to a pharmaceutical composition or medicament, means that the at least one Treg cell population of the invention is the only one therapeutic agent or agent with a biologic activity within said pharmaceutical composition or medicament.

Such compositions and medicaments may comprise buffers such as neutral buffered saline, phosphate buffered saline and the like; carbohydrates such as glucose, mannose, sucrose or dextrans, mannitol; proteins; polypeptides or amino acids such as glycine; antioxidants; chelating agents such as EDTA or glutathione; adjuvants (*e.g.*, aluminum hydroxide); and preservatives.

The administration of the compositions may be carried out in any convenient manner, including by aerosol inhalation, injection, ingestion, transfusion, implantation or transplantation. The compositions described herein may be administered to a patient trans arterially, subcutaneously, intradermally, intratumorally, intranodally, intramedullary, intramuscularly, by intravenous (i.v.) injection, or intraperitoneally. In one aspect, the T cell compositions of the present invention are administered to a patient by intradermal or subcutaneous injection.

In one embodiment, the Treg cell populations of the present invention are administered by i.v. injection.

The compositions of the present invention are in one embodiment formulated for intravenous administration.

In another embodiment, the Treg cell populations of the present invention may be injected directly into the site of the autoimmune and/or inflammatory disease or disorder.

The present invention further relates to a Treg cell expressing a CAR of the present invention, to a population of such cells, to a composition, to a pharmaceutical composition or to a medicament as described herein, for use in treating an IL-23R-expressing cell-mediated disease or disorder.

Another object of the invention is thus a method for treating in a subject in need thereof an IL-23R-expressing cell-mediated disease or disorder, wherein said method comprises administering to the subject at least one Treg cell or Treg cell population as described hereinabove.

In one embodiment, the method is a cell therapy method.

In one embodiment, the subject is administered (or is to be administered) with autologous cells. In other words, in one embodiment, the cell therapy is autologous.

In one embodiment, the cell therapy is heterologous.

In another embodiment, the subject is administered (or is to be administered) with allogenic cells. In other words, in one embodiment, the cell therapy is allogenic.

Another object of the present invention is a method for treating in a subject in need thereof an IL-23R-expressing cell-mediated disease or disorder, wherein said method comprises administering to the subject at least one CAR as described herein or nucleic acid encoding a CAR as described herein or vector comprising a CAR as described herein. In one embodiment, the method of the invention is a gene therapy method.

In one embodiment, the IL-23R-expressing cell-mediated disease or disorder is a proinflammatory cell mediated disease or disorder, a Th17-mediated disease or disorder or a $\gamma\delta$ T-mediated disease or disorder.

In one embodiment, the IL-23R-expressing cell-mediated disease is an autoimmune disease or disorder and/or an inflammatory disease or disorder.

Examples of IL-23R-expressing cell-mediated diseases or disorders include but are not limited to, Crohn's disease, ulcerative colitis (UC), bullous pemphigoid, lupus (including systemic lupus erythematosus (SLE), lupus nephritis (LN), discoid lupus, lupus erythematosus profundus, Chilbrain lupus erythematosus, tumidus lupus erythematosus, severe systemic lupus erythematosus, acute cutaneous lupus, chronic cutaneous lupus), multiple sclerosis, arthritis (such as, for example, rheumatoid arthritis, reactive arthritis (Reiter syndrome), juvenile idiopathic arthritis, ankylosing spondylitis and psoriatic arthritis), neuromyelitis optica (NMO), autoimmune limbic encephalitis (LE), Hashimoto's disease, N-methyl-D-aspartate receptor (NMDAR) encephalitis, autoimmune hemolytic anemia, pemphigus vulgaris, myasthenia gravis, Graves' disease, idiopathic thrombocytopenic purpura, Goodpasture's syndrome, celiac disease, pernicious anemia, vitiligo, scleroderma, psoriasis, Sjogren's syndrome, Wegener granulomatosis, polymyositis, dermatomyositis, primary biliary cirrhosis, antiphospholipid syndrome, mixed connective tissue disease, Miller Fisher syndrome, Guillain-Barre syndrome, acute motor axonal neuropathy, autoimmune hepatitis, dermatitis herpetiformis, Churg-Strauss disease, microscopic polyangiitis, IgA nephropathy, vasculitis caused by ANCA and other ANCA associated diseases, acute rheumatic fever, pernicious anemia, type 1 diabetes (T1D), membranous nephropathy, chronic inflammatory demyelinating polyneuropathy, thrombotic thrombocytopenic purpura, hyperviscosity in monoclonal gammopathies, hemolytic uremic syndrome (atypical, due to antibody to factor H), Wilson disease, fulminant, Lambert-Eaton myasthenic syndrome, RBC alloimmunization in pregnancy, mushroom poisoning, acute disseminated encephalomyelitis, hemolytic uremic syndrome (atypical, due to complement factor mutations), autoimmune hemolytic anemia (life-threatening cold agglutinin disease), myeloma cast nephropathy, post-transfusion purpura, autoimmune hemolytic anemia (warm autoimmune hemolytic anemia), hypertriglyceridemic pancreatitis, thyroid storm, stiff person syndrome, Hemolytic uremic syndrome (typical diarrhea-associated), immune thrombocytopenia, ABO-incompatible solid organ transplantation (SOT), cryoglobulinemia, heparin-induced thrombocytopenia, thyroid storm, chronic inflammatory demyelinating polyradiculoneuropathy, focal segmental glomerulosclerosis and fulminant hepatic failure.

In one embodiment, said IL-23R-expressing cell-mediated disease or disorder is selected from inflammatory bowel disease (such as, for example, Crohn's disease and ulcerative colitis), systemic lupus erythematosus, rheumatoid arthritis, juvenile idiopathic arthritis, Sjogren syndrome, systemic sclerosis, ankylosing spondylitis, Type 1 diabetes, autoimmune thyroid disorders, multiple sclerosis, Myasthenia Gravis, psoriasis, psoriatic arthritis or uveitis.

In one embodiment, IL-23R-expressing cell-mediated disease or disorder is Crohn's disease.

The terms "inflammatory disorder" or "inflammatory disease" are used interchangeably and as used herein refer to any abnormality associated with inflammation.

In one embodiment, the inflammatory condition comprises inflammatory diseases or disorder linked to a cancer.

In one embodiment, the inflammatory condition comprises inflammatory diseases or disorder linked to an autoimmune disease.

Examples of inflammatory diseases or disorders include, but are not limited to, arthritis, rheumatoid arthritis, ankylosing spondylitis, osteoarthritis, psoriatic arthritis, juvenile idiopathic arthritis, juvenile rheumatoid arthritis, arthritis uratica, gout, chronic polyarthritis, periarthritis humeroscapularis, cervical arthritis, lumbosacral arthritis, enteropathic arthritis and ankylosing spondylitis, asthma, dermatitis, psoriasis, scleroderma, polymyositis, dermatomyositis, juvenila dermatomyositis, primary biliary cirrhosis, fibrosis, cystic fibrosis, pulmonary fibrosis, cirrhosis, endomyocardial fibrosis, dediastinal fibrosis, myelofibrosis, retroperitoneal fibrosis, nephrogenic fibrosis, Keloids, scleroderma, arthrofibrosis, post transplantation late and chronic solid organ rejection, multiple sclerosis, systemic lupus erythematosus, lupus nephritis, pemphigus, Pemphigus vulgaris, Pemphigus herpetiformis, Pemphigus vegetans, IgA pemphigus, Pemphigus erythematosus, bullous pemphigoid, Pemphigoid gestationis, Mucous membrane dermatosis, Pemphigoid nodularis, Linear IgA bullous dermatosis, Bullous lichen planus, Epidermolysis bullosa acquisita, autoimmune diabetes, diabetic retinopathy, diabetic nephropathy, diabetic vasculopathy, ocular inflammation, uveitis, rhinitis, ischemia-reperfusion injury, post-angioplasty restenosis, chronic obstructive pulmonary disease (COPD), glomerulonephritis, Graves disease, gastrointestinal allergies, conjunctivitis, atherosclerosis, coronary artery disease, angina, small artery disease, acute disseminated encephalomyelitis, idiopathic thrombocytopenic purpura, multiple sclerosis, systemic sclerosis, antiphospholipid syndrome, Sjoegren's syndrome, autoimmune hemolytic anemia, colitis, Crohn's Disease, ulcerative colitis, Inflammatory Bowel Disease (IBD), embolism, pulmonary embolism, arterial embolism, venous embolism, allergic inflammation, cardiovascular disease, graft- related diseases, graft versus host disease (GVHD), disorders associated with graft transplantation rejection, chronic rejection, and tissue or cell allografts or xenografts, autoimmune diseases, degeneration after trauma, stroke, transplant rejection, allergic conditions and hypersensitivity, e.g., allergic rhinitis, allergic eczema and the like, skin diseases, dermal inflammatory disorders, or any combination thereof.

Examples of skin diseases include, but are not limited to, acne; actinic keratosis; atopic dermatitis; contact dermatitis; decubitus ulcers (bedsores); eczema; erythroderma; hemangioma, such as, for example, hemangioma of childhood; hypertrophic scarring; lichen planus; lichenoid disorders; lymphangiogenesis; psoriasis; pyogenic granulomas; molluscum contagious; neurofibromatosis; rosacea; recessive dystrophic epidermolysis bullosa; scars (keloids); scleroderma; seborrheic keratosis; skin cancers such as angiosarcoma, basal cell carcinoma, hemangioendothelioma, Karposi's sarcoma, malignant melanoma, melanoma, squamous cell carcinoma; skin ulcers; skin damages following skin grafts such as autotransplantation and allotransplantation; Steven-Johnson syndromes and toxic epidermal necrolysis; Sturge-Weber syndrome; tuberous sclerosis; venous ulcers; verruca vulgaris; warts, such as, for example, viral warts; wounds; and the like.

Examples of dermal inflammatory disorders include, but are not limited to, psoriasis, guttate psoriasis, inverse psoriasis, pustular psoriasis, erythroderma psoriasis, acute febrile neutrophilic dermatosis, eczema, asteatotic eczema, dyshidrotic eczema, vesicular palmoplanar eczema, acne vulgaris, atopic dermatitis, contact dermatitis, allergic contact dermatitis, dermatomyositis, exfoliative dermatitis, hand eczema, pompholyx, rosacea, rosacea caused by sarcoidosis, rosacea caused by scleroderma, rosacea caused by Sweet's syndrome, rosacea caused by systemic lupus erythematosus, rosacea caused by urticaria, rosacea caused by zoster-associated pain, Sweet's disease, neutrophilic hidradenitis, sterile pustulosis, drug eruptions, seborrheic dermatitis, pityriasis rosea, cutaneous kikuchi disease, pruritic urticarial papules and plaques of pregnancy, Stevens-Johnson syndrome and toxic epidermal necrolysis, tattoo reactions, Wells syndrome (eosinophilic cellulitis), reactive arthritis (Reiter's syndrome), bowel-associated dermatosis-arthritis syndrome, rheumatoid neutrophilic dermatosis, neutrophilic eccrine hidradenitis, neutrophilic dermatosis of the dorsal hands, balanitis circumscripta plasmacellularis, balanoposthitis, Behcet's disease, erythema annulare centrifugum, erythema dyschromicum perstans, erythema multiforme, granuloma annulare, hand dermatitis, lichen nitidus, lichen planus, lichen sclerosus et atrophicus, lichen simplex chronicus, lichen spinulosus, nummular dermatitis, pyoderma gangrenosum, sarcoidosis, subcorneal pustular dermatosis, urticaria, and transient acantholytic dermatosis.

In another embodiment, the CAR of the present invention may be used for treating in a subject in need thereof an autoimmune disease or disorder, wherein said method comprises administering to the subject at least one CAR as described herein or nucleic acid encoding a CAR as described herein or vector comprising a CAR as described herein.

Examples of autoimmune diseases include, but are not limited to, lupus (e.g., lupus erythematosus, lupus nephritis), Hashimoto's thyroiditis, primary myxedema, Graves' disease, pernicious anemia, autoimmune atrophic gastritis, Addison's disease, diabetes (e.g. insulin dependent diabetes mellitus, type I diabetes mellitus, type II diabetes mellitus), good pasture's syndrome, myasthenia gravis, pemphigus, Crohn's disease, sympathetic ophthalmia, autoimmune uveitis, multiple sclerosis, autoimmune hemolytic anemia, idiopathic thrombocytopenia, primary biliary cirrhosis, chronic action hepatitis, ulcerative colitis, Sjogren's syndrome, rheumatic diseases (e.g., rheumatoid arthritis), polymyositis, scleroderma, psoriasis, and mixed connective tissue disease.

In another embodiment, the CAR of the present invention may be used for treating in a subject in need thereof an allergic disease or disorder, wherein said method comprises administering to the subject at least one CAR as described herein or nucleic acid encoding a CAR as described herein or vector comprising a CAR as described herein.

Examples of allergic diseases include but are not limited to, allergic diseases against an inhaled allergen, an ingested allergen or a contact allergen. Other examples of allergic diseases include but are not limited to, allergic asthma, hypersensitivity lung diseases, food allergy, atopic dermatitis, allergic rhinitis, allergic rhinoconjunctivitis, chronic urticaria, delayed-type hypersensitivity disorders and systematic anaphylaxis.

In another embodiment, the CAR of the present invention may be used for treating in a subject in need thereof a cancer, wherein said method comprises administering to the subject at least one CAR as described herein or nucleic acid encoding a CAR as described herein or vector comprising a CAR as described herein.

As used herein, a "cancer" may be any cancer that is associated with a surface antigen or cancer marker.

Examples of cancers include but are not limited to, acute lymphoblastic leukemia (ALL), acute myeloid leukemia (AML), adenoid cystic carcinoma, adrenocortical, carcinoma, AIDS-related cancers, anal cancer, appendix cancer, astrocytomas, atypical teratoid/rhabdoid tumor, central nervous system, B- cell leukemia, lymphoma or other B cell malignancies, basal cell carcinoma, bile duct cancer, bladder cancer, bone cancer, osteosarcoma and malignant fibrous histiocytoma, brain stem glioma, brain tumors, breast cancer, bronchial tumors, burkitt lymphoma, carcinoid tumors, central nervous system cancers, cervical cancer, chordoma, chronic lymphocytic leukemia (CLL), chronic myelogenous leukemia (CML), chronic myeloproliferative disorders, colon cancer, colorectal cancer, craniopharyngioma, cutaneous t-cell lymphoma, embryonal tumors, central nervous system, endometrial cancer, ependymoblastoma, ependymoma, esophageal cancer, esthesioneuroblastoma, ewing sarcoma family of tumors extracranial germ cell tumor, extragonadal germ cell tumor extrahepatic bile duct cancer, eye cancer fibrous histiocytoma of bone, malignant, and osteosarcoma, gallbladder cancer, gastric (stomach) cancer, gastrointestinal carcinoid tumor, gastrointestinal stromal tumors (GIST), soft tissue sarcoma, germ cell tumor, gestational trophoblastic tumor, glioma, hairy cell leukemia, head and neck cancer, heart cancer, hepatocellular (liver) cancer, histiocytosis, hodgkin lymphoma, hypopharyngeal cancer, intraocular melanoma, islet cell tumors (endocrine pancreas), kaposi sarcoma, kidney cancer, langerhans cell histiocytosis, laryngeal cancer, leukemia, lip and oral cavity cancer, liver cancer (primary), lobular carcinoma in situ (LCIS), lung cancer, lymphoma, macroglobulinemia, male breast cancer, malignant fibrous histiocytoma of bone and osteosarcoma, medulloblastoma, medulloepithelioma, melanoma, merkel cell carcinoma, mesothelioma, metastatic squamous neck cancer with occult primary midline tract carcinoma involving NUT gene, mouth cancer, multiple endocrine neoplasia syndromes, multiple myeloma/plasma cell neoplasm, mycosis fungoides, myelodysplastic syndromes, myelodysplastic/myeloproliferative neoplasms, myelogenous leukemia, chronic (CML), Myeloid leukemia, acute (AML), myeloma, multiple, myeloproliferative disorders, nasal cavity and paranasal sinus cancer, nasopharyngeal cancer, neuroblastoma, non-hodgkin lymphoma, non-small cell lung cancer, oral cancer, oral cavity cancer, oropharyngeal cancer, osteosarcoma and malignant fibrous histiocytoma of bone, ovarian cancer, pancreatic cancer, papillomatosis, paraganglioma, paranasal sinus and nasal cavity cancer, parathyroid cancer, penile cancer, pharyngeal cancer, pheochromocytoma, pineal parenchymal tumors of intermediate differentiation, pineoblastoma and supratentorial primitive neuroectodermal tumors, pituitary tumor, plasma cell neoplasm/multiple myeloma, pleuropulmonary blastoma, pregnancy and breast cancer, primary central nervous system (CNS) lymphoma, prostate cancer, rectal cancer, renal cell (kidney) cancer, renal pelvis and ureter, transitional cell cancer, retinoblastoma, rhabdomyosarcoma, salivary gland cancer, sarcoma, sezary syndrome, small cell lung cancer, small intestine cancer, soft tissue sarcoma, squamous cell carcinoma, squamous neck cancer, stomach (gastric) cancer, supratentorial primitive neuroectodermal tumors, t- cell lymphoma, cutaneous, testicular cancer, throat cancer, thymoma and thymic carcinoma, thyroid cancer, transitional cell cancer of the renal pelvis and ureter, trophoblastic tumor, ureter and renal pelvis cancer, urethral cancer, uterine cancer, uterine sarcoma, vaginal cancer, vulvar cancer, Waldenstrom macroglobulinemia, Wilms Tumor.

In some aspects, the cancer is a B cell malignancy. Examples of B cell malignancies include, but are not limited to, Non-Hodgkin's Lymphomas (NHL), Diffuse Large B Cell Lymphoma (DLBCL), Small lymphocytic lymphoma (SLL/CLL), Mantle cell lymphoma (MCL), Follicular lymphoma (FL), Marginal zone lymphoma (MZL), Extranodal (MALT lymphoma), Nodal (Monocytoid B-cell lymphoma), Splenic, Diffuse large cell lymphoma, B cell chronic lymphocytic leukemia/lymphoma, Burkitt's lymphoma and Lymphoblastic lymphoma.

In one embodiment, the subject (*e.g.*, human) receives an initial administration of the Treg cell population of the invention, and optionally one or more subsequent administrations, wherein the one or more subsequent administrations are administered less than 15 days, e.g., 14, 13, 12, 11, 10, 9, 8, 7, 6, 5, 4, 3, or 2 days after the previous administration.

In one embodiment, a therapeutically effective amount of Treg cells is administered or is to be administered to the subject.

In one embodiment, the amount of Treg cells of the at least one immune cell population of the invention administered to the subject is at least of $10^2$, $10^3$, $10^4$, $10^5$, $10^6$, $10^7$, $10^8$ or $10^9$ cells.

In one embodiment, the amount of Treg cells of the at least one Treg cell population of the invention administered to the subject ranges from about $10^2$ to about $10^9$, from about $10^3$ to about $10^8$, from about $10^4$ to about $10^7$, or from about $10^5$ to about $10^6$ cells.

In another embodiment, the amount of Treg cells of the at least one Treg cell population of the invention administrated to the subject ranges from about $10^6$ to about $10^9$, from about $10^6$ to $10^7$, from about $10^6$ to $10^8$, from about $10^7$ to $10^9$, from about $10^7$ to $10^8$, from about $10^8$ to $10^9$. In another embodiment, the amount of Treg cells of the at least one Treg cell population of the invention administrated to the subject is about $10^6$, about $10^7$, about $10^8$, or is about $10^9$.

In one embodiment, the amount of Treg cells of the at least one Treg cell population of the invention administered to the subject is at least of $10^2$, $10^3$, $10^4$, $10^5$, $10^6$, $10^7$, $10^8$ or $10^9$ cells/kg body.

In one embodiment, the amount of Treg cells of the at least one Treg cell population of the invention administered to the subject ranges from about $10^4$ to $10^9$ cells/kg body weight or $10^5$ to $10^8$ cells/kg body weight, including all integer values within those ranges.

In one embodiment, the subject receives more than one administration of the at least one Treg cell population of the invention per week, *e.g.,* 2, 3, or 4 administrations of the at least one Treg cell population of the invention are administered per week to the subject. In one embodiment, the patient may be administered with the pharmaceutical composition once every two days, once every three days, once every four days, once a week, once every two weeks, once every three weeks, once a month, or at another frequency as necessary to establish a sufficient population of engineered Treg cells in the patient.

In one embodiment, the at least one Treg cell population is administered to the subject in need thereof in combination with an active agent. According to one embodiment, the at least one Treg cell population is administered before, at the same time or after the administration of an active agent.

Another object of the present invention is an article of manufacture containing materials useful for the treatment of an IL-23R-expressing cell-mediated disease or disorder.

The article of manufacture may comprise a container and a label or package insert on or associated with the container. Suitable containers include, for example, bottles, vials, syringes, pouch, etc. The containers may be formed from a variety of materials such as glass or plastic. The container holds a composition which is effective for treating the IL-23R-expressing cell-mediated disease or disorder, such as an autoimmune and/or inflammatory disease or disorder, and may have a sterile access port (for example the container may be an intravenous solution bag or a vial having a stopper pierceable by a hypodermic injection needle). At least one active agent in the composition is a Treg cell population of the invention.

The label or package insert may indicate that the composition is used for treating an IL-23R-expressing cell-mediated disease or disorder.

The article of manufacture, label or package insert may further comprise instructional material for administering the Treg cell population of the invention to the patient. Additionally, the article of manufacture may further comprise a second container comprising a pharmaceutically acceptable buffer, such as, for example, bacteriostatic water for injection (BWFI), phosphate-buffered saline, Ringer's solution and dextrose solution. It may further include other materials desirable from a commercial and user standpoint, including other buffers, diluents, filters, needles, and syringes.

Another object of the invention is a kit comprising at least one Treg cell population of the invention. By "kit" is intended any manufacture (*e.g.*, a package or a container) comprising at least one Treg cell population of the invention. The kit may be promoted, distributed, or sold as a unit for performing the methods of the present invention. Furthermore, any or all of the kit reagents may be provided within containers that protect them from the external environment, such as in sealed containers.

The kits may also contain a package insert describing the kit and methods for its use. Kits are also provided that are useful for various purposes (*e.g.*, for treating an IL-23R-expressing cell-mediated disease or disorder). Kits can be provided which contain the Treg cell population of the invention. As with the article of manufacture, the kit may comprise a container and a label or package insert on or associated with the container. The container holds a composition comprising at least one Treg cell population of the invention. Additional containers may be included that contain, *e.g.*, diluents and buffers. The label or package insert may provide a description of the composition as well as instructions for the intended use.

## BRIEF DESCRIPTION OF THE DRAWINGS

**Figure 1** represents a schematic view of an anti-IL-23R Chimeric Antigen Receptor (CAR) construct of the invention (such as, for example, the CAR having a sequence SEQ ID NO: 50) and two controls. The anti-IL23R CAR of the invention comprises a human CD8 leader sequence (CD8), a scFv directed against the human IL-23R (αIL-23R), a CD8 hinge (CD8 linker), a transmembrane domain (TM) derived from the human 4-1BB (4-1BB TM), an activation domain of human 4-1BB (4-IBB) and CD3 zeta (CD3Z). The CAR construct is in frame with a P2A-GFP coding sequence.

**Figure 2** is a graph monitoring the Treg cells phenotype transduced with an IL-23R-CAR of the present invention and controls at the end of the first cycle of expansion. Treg cells were labeled with antibodies directed against human CD4, CD25, CD127, and CTLA-4. For detection of FOXP3 and Helios transcription factors, an intra-nuclear labeling was performed. Error bars represent mean ± SEM.

**Figure 3** is a combination of dot plots of flow cytometry showing Treg cells activation (measured by CD69 expression, gated on GFP expression) following 24h stimulation through the CAR (via addition of IL-23R coated beads) or, through the TCR (via beads coated with anti-CD3 and anti-CD28).

**Figures 4A-C** are graphs showing that Treg cells expressing an IL23R-CAR exhibit efficient CAR-mediated suppressive activity. Contact-dependent suppression mediated by IL-23R-CAR Tregs wherein the CAR comprises intracellular and transmembrane domains of 4-1BB (Panel A) or of CD28 (Panel B) or of CD8 transmembrane domains and 4-1BB intracellular domains (Panel C) in the absence of any activation (empty circles) or after IL23R-induced CAR activation (full circles) was evaluated by measuring the proliferation of conventional T cells (Tconv) using flow cytometry. Error bars represent mean ± SEM.

## EXAMPLES

The present invention is further illustrated by the following examples.

### *Material and methods*

*Cells and reagents*

HEK293T cells (LentiX, Ozyme, France) were cultured in DMEM medium supplemented with 10% Fetal Bovine Serum (FBS).

*FoxP3 Treg isolation*

$CD4^+CD25^+CD127^{low}$ Treg cells were freshly isolated from buffy coats using the EasySep™ Human $CD4^+CD127^{low}CD25^+$ Regulatory T Cell Isolation Kit from StemCell. After isolation purity was assessed by FoxP3 staining. Isolated Treg cells were plated at $5 \times 10^5$ cells per well in a 24-well plate (Costar) into X-VIVO 15 media (Lonza) and activated with anti-CD3/anti-CD28 coated microbeads (Invitrogen, Carlsbad, CA) at a 1:1 bead-to-cell ratio. Tregs cells were incubated with rapamycin (100 ng/ml) and IL-2 (1000 units/ml, Miltenyi) concomitantly the activation. At day 2, 5, 7, and 9 IL-2 was added (1000 units/ml).

*CAR constructs*

The anti-IL23R CAR construct used in this experiment is presented in Figure 1. It comprises a human CD8 leader sequence (*e.g.*, having the sequence SEQ ID NO: 42), a scFv directed against the human IL23R (*e.g.*, having the sequence SEQ ID NO: 11), a CD8 hinge (*e.g.*, having the sequence SEQ ID NO: 20) and transmembrane domain derived from the human 4-1BB (*e.g.*, having the sequence SEQ ID NO: 28), an activation domain of human 4-1BB (*e.g.,* having the sequence SEQ ID NO: 36) and CD3 zeta (*e.g.,* having the sequence SEQ ID NO: 30). The CAR construct is in frame with a P2A-GFP coding sequence.

*Vector and titration*

The CAR constructs were produced using a classical 4-plasmid lentiviral system. Briefly, HEK293T cells were transfected with a third-generation lentiviral transfer vector expressing the CAR, the plasmid expressing HIV-1 gagpol (pMDLgpRRE), the plasmid expressing HIV-1 rev (pRSV.Rev) and the plasmid expressing VSV-G, the envelope glycoprotein of the vesicular stomatitis virus (pMD2.G). One-day post-transfection, viral supernatants were harvested, concentrated by centrifugation, aliquoted and frozen at - 80°C for long term storage. The infectious titers, expressed as the number of transducing units per milliliter (TU/ml), were obtained after transduction of Jurkat T cells with a serial dilution of viral supernatants and transduction efficiency evaluated after 3 days by monitoring the green fluorescent protein (GFP) expression using flow cytometry.

*Lentiviral transduction*

Tregs cells were transduced 2 days after activation with lentiviral vectors encoding the CARs described in **Table 1.**

Briefly transduction was carried out by loading $0.5x10^7$ Transduction unit (TU) per ml to each well. After 6 hours at 37°C, viral particles were removed by washout. Plates were then incubated at 37°C with 5% CO2. Five days after transduction, the transduction efficiency was analyzed: (i) the gene-transfer efficacy was measured by the analysis of the percentage of GFP positive cells using flow cytometry and (ii) the percentage of transduced cells expressing the CAR at cell surface was measured after a protein-L-APC labeling and flow cytometry analysis.

**Table 1**

| Name of the CAR | Anti-IL-23R scFv | TM | Co-signaling domain | CD3ζ |
|---|---|---|---|---|
| IL-23R-CAR (4-1BBTM/ 4-1BB) | 14-11-D07sc01 | 4-1BB | 4-1BB | YES |
| IL-23R-CAR (CD28TM/CD28) | 14-11-D07sc01 | CD28 | CD28 | YES |
| IL-23R-CAR (CD8TM/4-1BB) | 14-11-D07sc01 | CD8 | 4-1BB | YES |

*Activation assay of CAR-Tregs*

The activation assay was performed at day 9 of the culture. Briefly, 0.05 x $10^6$ Treg were seeded in 96 U bottom plate alone or in presence of anti CD28/antiCD3-coated beads (in a 2 to 1 Treg cells to beads ratio) or in presence of human IL-23R-coated beads (in a 1 to 1 Treg cell to beads ratio) in a 200 μl final volume. After 24h at 37°C, 5% CO2, cells were stained for CD4 and CD69 and then analyzed using flow cytometry. The monitoring of the CD69 spontaneous expression in CAR Treg cells as compared to untransduced Treg cells, allows to determine the ligand-independent tonic signaling of the CAR.

*Suppression assay of T cell proliferation*

The suppression assay was performed at day 9 of the culture. Briefly, Treg were recovered, counted and activated either through the TCR using anti CD28/antiCD3 coated beads (in a 1 to 1 Treg cell to beads ratio), or through the CAR using IL-23R- coated beads (in a 1 to 1 Treg cell to beads ratio) or culture without an activation step to evaluate their spontaneous suppressive activity.

In parallel, allogeneic Tconv cells (T conventional cells) were thawed, stained with Dye 450 and activated with anti CD28/anti CD3 coated beads (in a 3 to 1 Tconv cells to beads ratio). The day after, beads were removed from Tconv cells culture before their coculture with un-activated or activated transduced Treg cells.

At day 3, cells were harvested, and Tconv cells proliferation was assessed by flow cytometry through the monitoring of the dye 450 dilution. The percentage of inhibition of Tconv cells proliferation was calculated as followed:

$$100 - \frac{\text{\% of Tconv proliferation in presence of CAR-Treg} \times 100}{\text{\% of Tconv proliferation in absence of CAR-Treg}}$$

*Phenotype analysis of transduced Treg*

At day 9 of the cell culture, Treg cell phenotype was analyzed using flow cytometry, to ensure that CAR transduction did no impact the Treg status. Markers used for this analysis are listed in the **Table 2.**

**Table 2: Material and reagents**

| | Reagents | Manufacturer | Reference |
|---|---|---|---|
| Treg immuno-phenotyping | CD4 VioGreen | Miltenyi | 130-096-900 |
| | Helios eF450 (HamIgG) | eBioscience | 48-9883-42 |
| | CD25 PE | Miltenyi | 130-109-020 |
| | CD152 (CTLA-4) PECy7 (mIgG2a) | Biolegend | 369614 |
| | FoxP3 AF647 (mIg(G1; 2 μl) | BD | 560045 |
| | CD127-APC-Vio770 | Miltenyi | 130-109-438 |

**Results**

Treg cells were transduced with an IL23R-CAR according to the present invention (illustrated in **Figure 1**, construct 4-1BB TM/4-1BB). As shown in **Figure 2**, following the transduction, Treg cells retain their phenotype, with expression of CD4, CD25, FoxP3, Helios and CTLA4, and low expression of CD127.

Next, the ability of transduced Treg cells to be activated via the IL-23R CAR. was examined. For that, Treg cells were stimulated with IL-23R-coated beads and 24h after stimulation the status of activation was analyzed by monitoring CD69 expression using flow cytometry. As a positive control, cells were stimulated with anti-CD3/anti-CD28-coated beads (illustrating stimulation via their TCR) and as a negative control some cells were not stimulated.

Different CAR constructs were used (see **Table 1**).

The **Table 3** below shows the percentage of CD69-positive cells gated on the GFP+ cell population, and the cell viability measured in this experiment.

**Table 3**

| CAR construct | Stimulation | % of CD69 expression gated on GFP+ Treg cells | % of viability |
|---|---|---|---|
| CD28 TM/CD28 | No stimulation | 73.1 | 43.4 |
| | TCR stimulation | 86.3 | 35.7 |
| | CAR stimulation | 80.6 | 30.8 |
| 4-1BB TM/4-1BB | No stimulation | 19.7 | 81.8 |
| | TCR stimulation | 75.7 | 61.9 |
| | CAR stimulation | 35.9 | 47.8 |
| CD8 TM/4-1BB | No stimulation | 58.1 | 80.0 |
| | TCR stimulation | 81.8 | 60.1 |
| | CAR stimulation | 70.5 | 50.8 |

In the 4-1BBTM/4-1BB condition, a two-fold increase of CD69 expression is observed in presence of IL-23R-coated beads, demonstrating that this IL-23R CAR (comprising a 4-1BBTM/4-1BB) can be activated after interaction with its ligand (**Figure 3**).

In addition, these data demonstrate that, in absence of any ligand-specific activation, Treg cells transduced with a CAR comprising CD28TM/CD28 or CD8TM/4-1BB harbor a strong ligand-independent tonic signaling (73% and 58% respectively), and associated to a low viability in culture (<45%) for the CAR comprising CD28TM/CD28. Interestingly, Treg cells transduced with a CAR according to the present invention (4-1BB TM/ 4-1BB) present a low background of activation and a good viability in culture.

Next, CAR-Treg cells were evaluated for their capacity to suppress Tconv cells proliferation. As shown in **Figure 4**, the spontaneous suppressive activity of the IL23-R CAR comprising CD28TM/CD28 or CD8TM/4-1BB was too strong to highlight a specific CAR-mediated suppressive activity using IL23-R-coated beads (**Fig.4A** and **Fig.4C**). On the contrary, Treg cells expressing the CAR comprising 4-1BBTM/4-1BB harbored a low suppressive background, allowing the observation of a potent CAR-mediated suppressive activity after activation with IL23-R-coated beads (Fig.4, middle panel).

## Claims

**1.** A chimeric antigen receptor (CAR) specific for at least one IL-23 receptor (IL-23R), wherein said CAR comprises:

(i) an extracellular binding domain, wherein said binding domain binds to said IL-23R,
(ii) optionally an extracellular hinge domain,
(iii) a transmembrane domain derived from 4-1BB,
(iv) an intracellular signaling domain, and,
(v) optionally a tag and/or a leader sequence.

**2.** The CAR according to claim **1**, wherein the extracellular binding domain comprises a scFv fragment directed against said IL-23R.

**3.** The CAR according to claim **1** or claim **2**, wherein the extracellular binding domain comprises a scFv fragment directed against said IL-23R, wherein said scFv comprises
a heavy chain variable domain (VH) having the sequence of SEQ ID NO: 1 or a sequence having at least about 70% identity to SEQ ID NO: 1,
a light chain variable domain (VL) having a sequence selected from the group consisting of SEQ ID NOs: 2, 3 and 4 and sequences having at least about 70% identity to SEQ ID NO: 2, 3 or 4, and
optionally a linker between the VH and the VL,
preferably wherein said scFv has the sequence SEQ ID NO: 11 or a sequence having at least about 70% identity to SEQ ID NO: 11.

**4.** The CAR according to any one of claims **1** to **3**, wherein the hinge domain is a hinge region of human CD8, preferably having a sequence of SEQ ID NO: 20 or SEQ ID NO: 60, or a sequence having at least about 70% identity to SEQ ID NO: 20 or SEQ ID NO: 60.

**5.** The CAR according to any one of claims **1** to **4**, wherein the transmembrane domain is a transmembrane domain derived from the human 4-1BB, preferably having the sequence of SEQ ID NO: 28 or a sequence having at least about 70% identity to SEQ ID NO: 28.

**6.** The CAR according to any one of claims **1** to **5**, wherein the intracellular signaling domain comprises a costimulatory signaling domain of human 4-1BB, preferably having the sequence of SEQ ID NO: 36 or a sequence having at least about 70% identity to SEQ ID NO: 36 and a T cell primary signaling human CD3 zeta, preferably having the sequence of SEQ ID NO: 31 or a sequence having at least 70% identity to SEQ ID NO: 31.

**7.** The CAR according to any one of claims **1** to **6**, comprising a transmembrane domain derived from the human 4-1BB contiguous to a costimulatory signaling domain of human 4-1BB, the transmembrane and costimulatory signaling domains of 4-1BB having the sequence SEQ ID NO: 53.

**8.** The CAR according to any one of claims **1** to **7**, comprising

(i) an anti-IL-23R scFv, preferably comprising a VH having the sequence of SEQ ID NO: 1 and a VL having the sequence of SEQ ID NO: 2, linked by a $(G_4S)_3$ linker (SEQ ID NO: 9),
(ii) a hinge domain derived from CD8$\alpha$, preferably SEQ ID NO: 20 or SEQ ID NO: 60,
(iii) a human 4-1BB transmembrane domain, preferably SEQ ID NO: 28,
(iv) an intracellular signaling domain comprising a human 4-1BB signaling domain, preferably SEQ ID NO: 36 and a human CD3 zeta domain, preferably SEQ ID NO: 31, and
(v) optionally a tag and/or a leader sequence.

**9.** A nucleic acid sequence encoding a CAR according to any one of claims **1** to **8.**

**10.** A vector comprising the nucleic acid sequence according to claim **9.**

**11.** A T cell population, engineered to express on the cell surface a CAR according to any one of claims **1** to **8**.

**12.** The T cell population according to claim **11**, wherein said T cell population is a regulatory T cell population, preferably wherein said regulatory T cell population is selected from the group consisting of $CD4^+CD25^+Foxp3^+$ Treg, Tr1 cells, TGF-$\beta$ secreting Th3 cells, regulatory NKT cells, regulatory y$\delta$ T cells, regulatory $CD8^+$ T cells, and double negative regulatory T cells.

**13.** A composition comprising at least one T cell population engineered to express on the cell surface a CAR according to any one of claims **1** to **9**, wherein said composition is preferably a pharmaceutical composition further comprising at least one pharmaceutically acceptable excipient or carrier.

**14.** An *ex vivo* method for obtaining a T cell population engineered to express on the cell surface a CAR according to any one of claims **1** to **8**, wherein said *ex vivo* method comprises the genetic modification, preferably the transduction, of at least one T cell with a nucleic acid encoding an IL-23R-CAR, and optionally an expansion step of the transduced cells.

**15.** A T cell population according to claim **11** or **12**, or a composition according to claim **13** for use in treating an IL-23R expressing cell-mediated disease or disorder in a subject in need thereof, wherein said IL-23R expressing cell-mediated disease or disorder is preferably an autoimmune and/or inflammatory disease and/or disorder.

**16.** The T cell population or composition for use according to claim **15**, wherein said autoimmune and/or inflammatory disease and/or disorder is selected from the group consisting of inflammatory bowel diseases (such as, for example, Crohn’s disease and ulcerative colitis), systemic lupus erythematosus, rheumatoid arthritis, juvenile idiopathic arthritis, Sjögren syndrome, systemic sclerosis, ankylosing spondylitis, Type 1 diabetes, autoimmune thyroid disorders, multiple sclerosis, Myasthenia Gravis, psoriasis, psoriatic arthritis and uveitis, preferably wherein said autoimmune and/or inflammatory disease and/or disorder is Crohn’s disease.

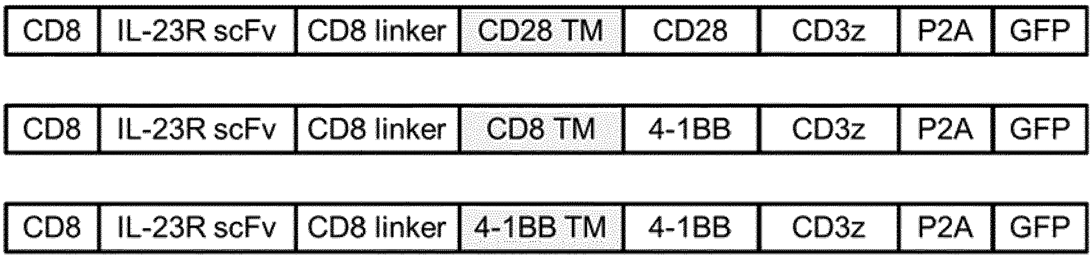
CD8
IL-23R scFv
CD8 linker
CD28 TM
CD28
CD3z
P2A
GFP
CD8
IL-23R scFv
CD8 linker
CD8 TM
4-1BB
CD3z
P2A
GFP
CD8
IL-23R scFv
CD8 linker
4-1BB TM
4-1BB
CD3z
P2A
GFP

FIG. 1

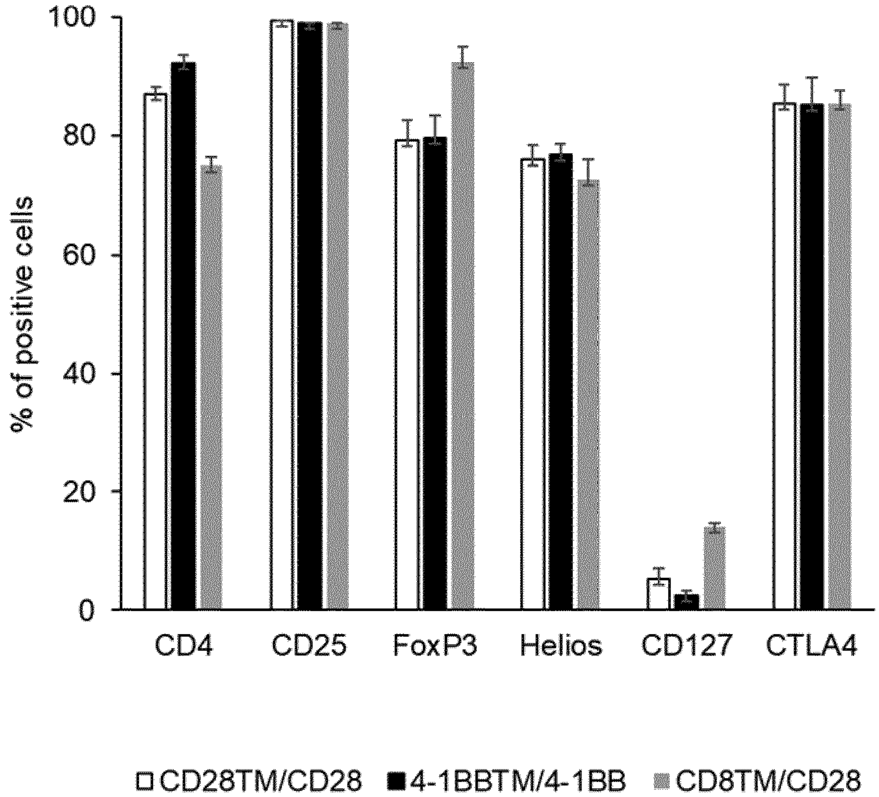
100
80
60
40
20
0
% of positive cells
CD4
CD25
FoxP3
Helios
CD127
CTLA4
CD28TM/CD28
4-1BBTM/4-1BB
CD8TM/CD28

FIG. 2

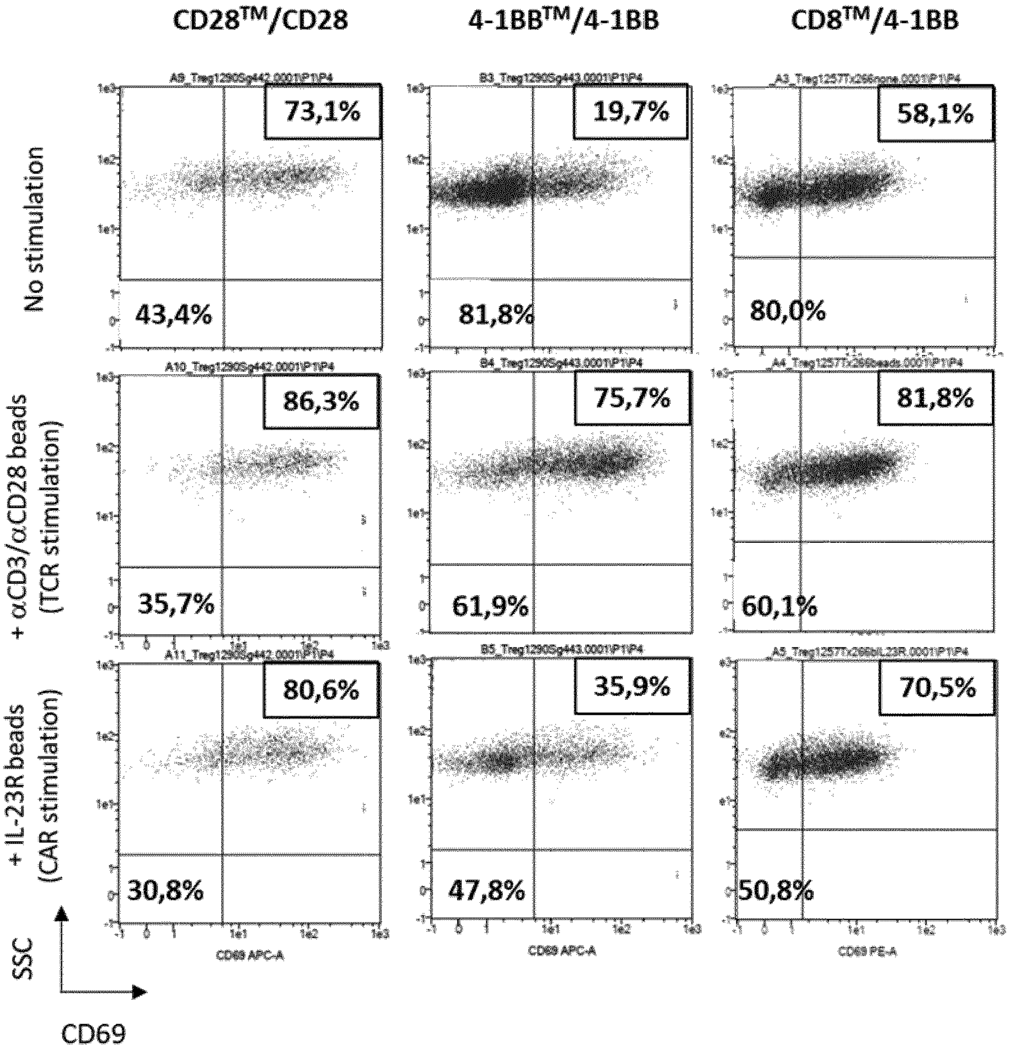
CD28TM/CD28
4-1BBTM/4-1BB
CD8TM/4-1BB
No stimulation
+ αCD3/αCD28 beads (TCR stimulation)
+ IL-23R beads (CAR stimulation)
73,1%
43,4%
19,7%
81,8%
58,1%
80,0%
86,3%
35,7%
75,7%
61,9%
81,8%
60,1%
80,6%
30,8%
35,9%
47,8%
70,5%
50,8%
SSC
CD69
CD69 APC-A
CD69 PE-A

FIG. 3

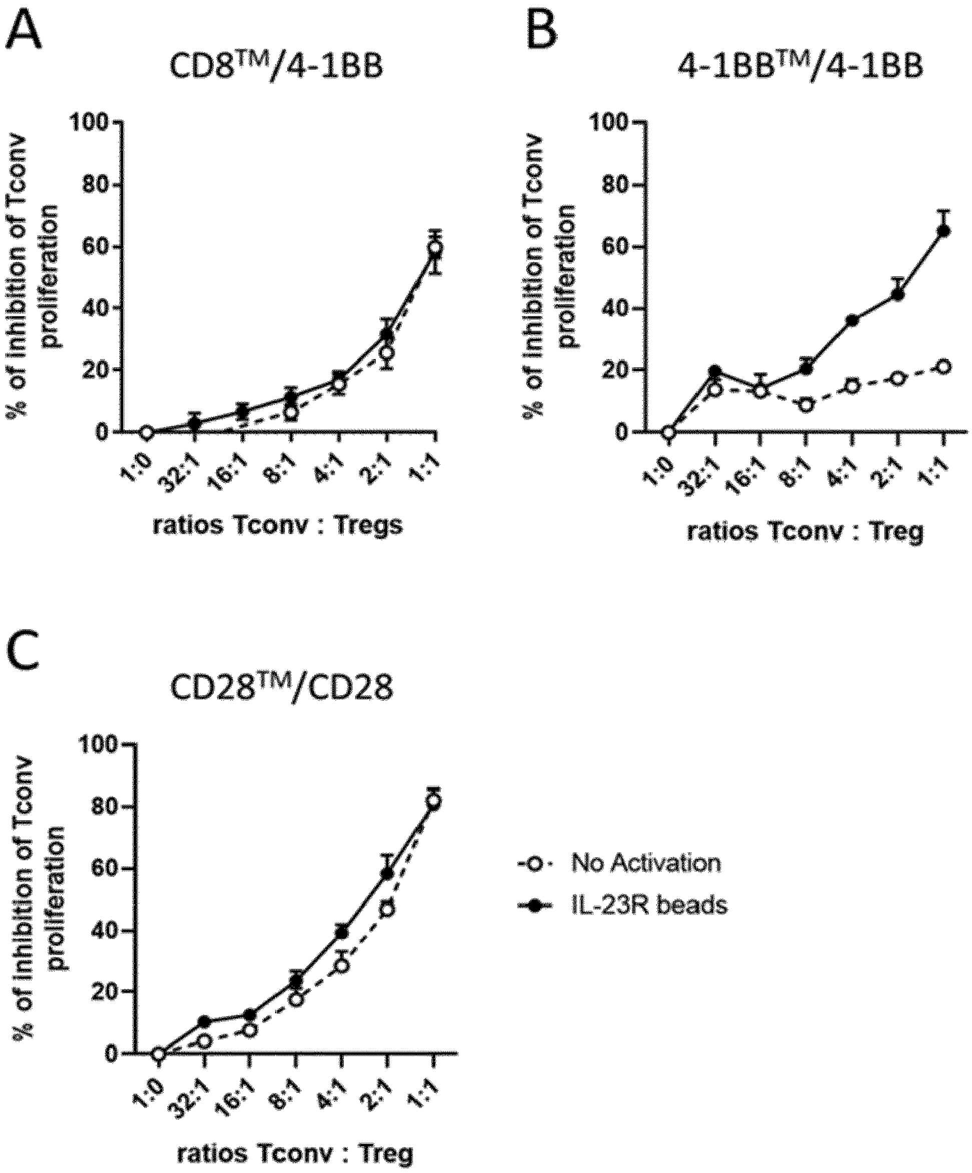
A
CD8TM/4-1BB
% of Inhibition of Tconv proliferation
100
80
60
40
20
0
1:0
32:1
16:1
8:1
4:1
2:1
1:1
ratios Tconv : Tregs
B
4-1BBTM/4-1BB
% of Inhibition of Tconv proliferation
ratios Tconv : Treg
C
CD28TM/CD28
% of Inhibition of Tconv proliferation
ratios Tconv : Treg
No Activation
IL-23R beads

FIG. 4

Europäisches Patentamt
European Patent Office
Office européen des brevets

# EUROPEAN SEARCH REPORT

Application Number
EP 19 30 6341

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2017/210749 A1 (ADELAIDE RES & INNOVATION PTY LTD [AU]) 14 December 2017 (2017-12-14)<br>* the whole document *<br>* paragraphs [[0004]], [[0036]], [[0514]]; claims *<br>* paragraphs [[0428]], [[0434]]; claim 58; example 9 *<br>----- | 1-16 | INV.<br>C07K14/725<br>C07K14/705<br>C07K16/28<br>A61P1/00<br>A61P17/06<br>A61P19/02<br>A61P37/02<br><br>ADD.<br>C12N5/0783 |
| Y | US 2018/348227 A1 (SADELAIN MICHEL [US] ET AL) 6 December 2018 (2018-12-06)<br>* the whole document *<br>----- | 1-16 | |
| Y | HAMID REZA MIRZAEI ET AL: "Construction and functional characterization of a fully human anti-CD19 chimeric antigen receptor (huCAR)-expressing primary human T cells", JOURNAL OF CELLULAR PHYSIOLOGY, vol. 234, no. 6, 26 October 2018 (2018-10-26), pages 9207-9215, XP055675897, US<br>ISSN: 0021-9541, DOI: 10.1002/jcp.27599<br>* the whole document *<br>* abstract *<br>* p. 9208, section 2.1; figure 1 *<br>----- | 1-16 | TECHNICAL FIELDS SEARCHED (IPC)<br>C07K<br>C12N<br>A61P |
| Y | WO 2018/224443 A1 (NUMAB INNOVATION AG [CH]) 13 December 2018 (2018-12-13)<br>* the whole document *<br>* page 41; table 1; sequence 1 *<br>----- | 1-16 | |
| L | WO 2019/197678 A1 (SANGAMO THERAPEUTICS FRANCE [FR]) 17 October 2019 (2019-10-17)<br>* the whole document *<br>* L: E-Y document; claims; sequence 55 *<br>-/-- | 1-16 | |

The present search report has been drawn up for all claims

2

| Place of search | Date of completion of the search | Examiner |
|---|---|---|
| Munich | 16 April 2020 | Madruga, Jaime |

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

Europäisches Patentamt
European Patent Office
Office européen des brevets

# EUROPEAN SEARCH REPORT

Application Number
EP 19 30 6341

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| L | -& DATABASE Geneseq [Online]<br><br>12 December 2019 (2019-12-12), "Anti IL-23R single chain variable fragment (scFv), SEQ 55.", XP002798286, retrieved from EBI accession no. GSP:BGW51241 Database accession no. BGW51241<br>* L: SEQUENCE INFORMATION; sequence *<br>----- | 1-16 | |
| L | WO 2020/030979 A2 (SANGAMO THERAPEUTICS FRANCE [FR]) 13 February 2020 (2020-02-13)<br>* the whole document *<br>* L: E-Y document; claim 27 *<br>----- | 1-16 | |

TECHNICAL FIELDS SEARCHED (IPC)

The present search report has been drawn up for all claims

2

| Place of search | Date of completion of the search | Examiner |
|---|---|---|
| Munich | 16 April 2020 | Madruga, Jaime |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 2 of 2

**ANNEX TO THE EUROPEAN SEARCH REPORT ON EUROPEAN PATENT APPLICATION NO.** EP 19 30 6341

This annex lists the patent family members relating to the patent documents cited in the above-mentioned European search report. The members are as contained in the European Patent Office EDP file on The European Patent Office is in no way liable for these particulars which are merely given for the purpose of information.

16-04-2020

| Patent document cited in search report | Publication date | Patent family member(s) | Publication date |
|---|---|---|---|
| WO 2017210749 A1 | 14-12-2017 | NONE | |
| US 2018348227 A1 | 06-12-2018 | AU 2017307610 A1 | 26-04-2018 |
| | | CA 3000514 A1 | 08-02-2018 |
| | | EP 3344263 A1 | 11-07-2018 |
| | | US 2018348227 A1 | 06-12-2018 |
| | | WO 2018027197 A1 | 08-02-2018 |
| WO 2018224443 A1 | 13-12-2018 | AU 2018280683 A1 | 17-10-2019 |
| | | CA 3065868 A1 | 13-12-2018 |
| | | CN 110719918 A | 21-01-2020 |
| | | EP 3635014 A1 | 15-04-2020 |
| | | KR 20200013230 A | 06-02-2020 |
| | | WO 2018224443 A1 | 13-12-2018 |
| WO 2019197678 A1 | 17-10-2019 | NONE | |
| WO 2020030979 A2 | 13-02-2020 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4816567 A
- US 6703199 B
- EP 0404097 A
- WO 9311161 A
- WO 2012138475 A
- WO 0196584 A
- WO 0129058 A
- US 6326193 B
- US 2006200869 A
- WO 2013153391 A
- WO 2016120216 A
- US 5350674 A
- US 5585362 A
- US 6352694 B
- US 6534055 B
- US 6905680 B
- US 6692964 B
- US 5858358 A
- US 6887466 B
- US 6905681 B
- US 7144575 B
- US 7067318 B
- US 7172869 B
- US 7232566 B
- US 7175843 B
- US 5883223 A
- US 6905874 B
- US 6797514 B
- US 6867041 B
- US 20060121005 A
- US 20040101519 A
- US 20060034810 A
- WO 2007110785 A
- US 2016024470 A
- US 20120321667 A
- US 20070036773 A
- US 20140068797 A
- US 8871445 B
- US 8865406 B
- US 8795965 B
- US 8771945 B
- US 8697359 B
- US 20110158957 A
- US 20120060230 A


### Non-patent literature cited in the description


- Basic and Clinical Immunology. Appleton & Lange, 1994, 71
- **KOHLER et al.** *Nature,* 1975, vol. 256, 495
- **CLACKSON et al.** *Nature,* 1991, vol. 352, 624-628
- **MARKS et al.** *J. Mol. Biol.,* 1991, vol. 222, 581-597
- **HOLLINGER ; HUDSON.** *Nature Biotechnology,* 2005, vol. 23, 1126-1136
- **HOLLIGER et al.** *Proc. Natl. Acad. Sci. USA,* 1993, vol. 90, 6444-6448
- Computational Molecular Biology. Oxford University Press, 1988
- Biocomputing: Informatics and Genome Projects. Academic Press, 1993
- Computer Analysis of Sequence Data. Humana Press, 1994
- **HEINJE, G.** Sequence Analysis in Molecular Biology. Academic Press, 1987
- Sequence Analysis Primer. M. Stockton Press, 1991
- **CARILLO et al.** *SIAM J. Applied Math.,* 1988, vol. 48, 1073
- **DEVEREUX et al.** Nucl. Acid. Res. Genetics Computer Group, University of Wisconsin, 1984, vol. 2, 387
- **ALTSCHUL et al.** *J. Mol. Biol.,* 1990, vol. 215, 403-410
- **ALTSCHUL et al.** BLAST Manual. NCB/NLM/NIH Bethesda
- **JONES et al.** *Nature,* 1986, vol. 321, 522-525
- **REICHMANN et al.** *Nature,* 1988, vol. 332, 323-329
- **PRESTA.** *Curr. Op. Struct. Biol.,* 1992, vol. 2, 593-596
- **MILONE et al.** *Mol. Ther.,* 2009, vol. 17 (8), 1453-1464
- **BATZER et al.** *Nucleic Acid Res.,* 1991, vol. 19, 5081
- **OHTSUKA et al.** *J. Biol. Chem.,* 1985, vol. 260, 2605-2608
- **ROSSOLINI et al.** *Mol. Cell. Probes,* 1994, vol. 8, 91-98
- **HARLOW et al.** Using Antibodies: A Laboratory Manual. Cold Spring Harbor Laboratory Press, 1999

- **HARLOW et al.** Antibodies: A Laboratory Manual. Cold Spring Harbor, 1989
- **HOUSTON et al.** *Proc. Natl. Acad. Sci. USA,* 1988, vol. 85, 5879-5883
- **BIRD et al.** *Science,* 1988, vol. 242, 423-426
- **KABAT et al.** Sequences of Proteins of Immunological Interest. Public Health Service, National Institutes of Health, 1991
- **AL-LAZIKANI et al.** *JMB,* 1997, vol. 273, 927-948
- **ROYBAL et al.** *Cell,* 2006
- **SAMBROOK et al.** Molecular Cloning: A Laboratory Manual. Cold Spring Harbor Laboratory, 2001
- **JONES et al.** *Frontiers in Pharmacology,* 2014, vol. 5, 254
- **UI-TEI et al.** *FEBS Letters,* 2000, vol. 479, 79-82
- **GHOSH et al.** *Glycobiology,* 1991, vol. 5, 505-10
- **SCHENBORN ; MIERENDORF.** *Nuc Acids Res.,* 1985, vol. 13, 6223-36
- **NACHEVA ; BERZAL-HERRANZ.** *Eur. J. Biochem.,* 2003, vol. 270, 1485-65
- **COUGOT et al.** *Trends in Biochem. Sci.,* 2001, vol. 29, 436-444
- **STEPINSKI et al.** *RNA,* 2001, vol. 7, 1468-95
- **ELANGO et al.** *Biochim. Biophys. Res. Commun.,* 2005, vol. 330, 958-966
- **NISHIKAWA et al.** *Hum Gene Ther.,* 2001, vol. 12 (8), 861-70
- **BERG et al.** *Transplant Proc.,* 1998, vol. 30 (8), 3975-3977
- **HAANEN et al.** *J. Exp. Med.,* 1999, vol. 190 (9), 13191328
- **GARLAND et al.** *J. Immunol Meth.,* 1999, vol. 227 (1-2), 53-63
- **GRISSA et al.** *BMC Bioinformatics,* 2007, vol. 8, 172
- **BARRANGOU et al.** *Science,* 2007, vol. 315, 1709-1712
- **MARRAGINI et al.** *Science,* 2008, vol. 322, 1843-1845
- **WIEDENHEFT et al.** *Nature,* 2012, vol. 482, 331-8
- **HORVATH et al.** *Science,* 2010, vol. 327, 167-170
- **MAKAROVA et al.** *Biology Direct,* 2006, vol. 1, 7
- **PENNISI.** *Science,* 2013, vol. 341, 833-836
- **CONG.** *Science,* 2013, vol. 339, 819-823
- **TSAI.** *Nature Biotechnol.,* 2014, vol. 32 (6), 569-576
- **BOCH.** *Nature Biotech.,* 2011, vol. 29, 135-6
- **BOCH et al.** *Science,* 2009, vol. 326, 1509-12
- **MOSCOU et al.** *Science,* 2009, vol. 326, 3501
- **CERMAK et al.** *Nucl. Acids Res.,* 2011, vol. 39, e82
- **MILLER et al.** *Nature Biotech.,* 2011, vol. 29, 143-8
- **HOCKEMEYER et al.** *Nature Biotech.,* 2011, vol. 29, 731-734
- **WOOD et al.** *Science,* 2011, vol. 333, 307
- **DOYON et al.** *Nature Methods,* 2010, vol. 8, 74-79
- **SZCZEPEK et al.** *Nature Biotech.,* 2007, vol. 25, 786-793
- **GUO et al.** *Mol. Biol.,* 2010, vol. 200, 96
- **ZHANG et al.** *Nature Biotech.,* 2011, vol. 29, 149-53
- **GEIBLER et al.** *PLoS ONE,* 2011, vol. 6, el9509
- **CARROLL et al.** *Genetics Society of America,* 2011, vol. 188, 773-782
- **KIM et al.** *Proc. Natl. Acad. Sci. USA,* 1996, vol. 93, 1156-1160
- **BITINAITE et al.** *Proc. Natl. Acad. Sci. USA,* 1998, vol. 95, 10570-5
- **PROVASI.** *Nature Med.,* 2011, vol. 18, 807-815
- **TORIKAI.** *Blood,* 2013, vol. 122, 1341-1349
- **CATHOMEN et al.** *Mol. Ther.,* 2008, vol. 16, 1200-7
- **QUO et al.** *Mol. Biol.,* 2010, vol. 400, 96
- **STODDARD.** *Structure,* 12 January 2011, vol. 19 (1), 7-15
- *Nucleic Acids Res.,* February 2014, vol. 42 (4), 2591-601
- **TAKEUCHI et al.** *Methods Mol Biol.,* 2015, vol. 1239, 105-32
- **CARL JUNE.** Adoptive T cell therapy for cancer in the clinic. *Journal of Clinical Investigation,* 2007, vol. 117, 1466-1476

SEQUENCE LISTING

<110> SANGAMO THERAPEUTICS FRANCE
ABEL Tobias
FENARD David
GERTNER-DARDENNE Julie

<120> NOVEL CHIMERIC ANTIGEN RECEPTOR SPECIFIC FOR INTERLEUKIN-23 RECEPTOR

<130> CV-1378/EP

<160> 86

<170> BiSSAP 1.3.6

<210> 1
<211> 121
<212> PRT
<213> Artificial Sequence

<220>
<223> VH

<400> 1
```
Glu Val Gln Leu Val Glu Ser Gly Gly Gly Leu Val Gln Pro Gly Gly
1               5                   10                  15
Ser Leu Arg Leu Ser Cys Ala Ala Ser Gly Ile Asp Phe Asn Ser Asn
            20                  25                  30
Tyr Tyr Met Cys Trp Val Arg Gln Ala Pro Gly Lys Gly Leu Glu Trp
        35                  40                  45
Ile Gly Cys Ile Tyr Val Gly Ser His Val Asn Thr Tyr Tyr Ala Asn
    50                  55                  60
Trp Ala Lys Gly Arg Phe Thr Ile Ser Arg Asp Asn Ser Lys Asn Thr
65                  70                  75                  80
Val Tyr Leu Gln Met Asn Ser Leu Arg Ala Glu Asp Thr Ala Val Tyr
                85                  90                  95
Tyr Cys Ala Thr Ser Gly Ser Ser Val Leu Tyr Phe Lys Phe Trp Gly
            100                 105                 110
Gln Gly Thr Leu Val Thr Val Ser Ser
        115                 120
```

<210> 2
<211> 113
<212> PRT
<213> Artificial Sequence

<220>
<223> VL

<400> 2
```
Asp Ile Gln Met Thr Gln Ser Pro Ser Ser Leu Ser Ala Ser Val Gly
1               5                   10                  15
Asp Arg Val Thr Ile Thr Cys Gln Ala Ser Glu Asn Ile Tyr Ser Phe
            20                  25                  30
Leu Ala Trp Tyr Gln Gln Lys Pro Gly Lys Ala Pro Lys Leu Leu Ile
        35                  40                  45
Tyr Ser Ala Ser Lys Leu Ala Ala Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Gln Gln Thr Asn Arg Tyr Ser Asn
```

```
                    85                  90                  95
Pro Asp Ile Tyr Asn Val Phe Gly Gln Gly Thr Lys Leu Thr Val Leu
            100                 105                 110
Gly
```

<210> 3
<211> 113
<212> PRT
<213> Artificial Sequence

<220>
<223> VL

<400> 3

```
Asp Ile Gln Met Thr Gln Ser Pro Ser Ser Leu Ser Ala Ser Val Gly
1               5                   10                  15
Asp Arg Val Thr Ile Thr Cys Gln Ala Ser Glu Asn Ile Tyr Ser Phe
            20                  25                  30
Leu Ala Trp Tyr Gln Gln Lys Pro Gly Lys Ala Pro Lys Leu Leu Ile
        35                  40                  45
Tyr Ser Ala Ser Lys Leu Ala Ala Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Gln Gln Thr Asn Arg Tyr Ser Asn
                85                  90                  95
Pro Asp Ile Tyr Asn Val Phe Gly Gly Gly Thr Lys Leu Thr Val Leu
            100                 105                 110
Gly
```

<210> 4
<211> 113
<212> PRT
<213> Artificial Sequence

<220>
<223> VL

<400> 4

```
Asp Ile Gln Met Thr Gln Ser Pro Ser Ser Leu Ser Ala Ser Val Gly
1               5                   10                  15
Asp Arg Val Thr Ile Thr Cys Gln Ala Ser Glu Asn Ile Tyr Ser Phe
            20                  25                  30
Leu Ala Trp Tyr Gln Gln Lys Pro Gly Lys Ala Pro Lys Leu Leu Ile
        35                  40                  45
Tyr Ser Ala Ser Lys Leu Ala Ala Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Gln Gln Thr Asn Arg Tyr Ser Asn
                85                  90                  95
Pro Asp Ile Tyr Asn Val Phe Gly Thr Gly Thr Lys Val Thr Val Leu
            100                 105                 110
Gly
```

<210> 5
<211> 4
<212> PRT

<213> Artificial Sequence

<220>
<223> linker

<400> 5
Gly Gly Gly Ser
1

<210> 6
<211> 16
<212> PRT
<213> Artificial Sequence

<220>
<223> linker

<400> 6
Gly Gly Gly Ser Gly Gly Gly Ser Gly Gly Gly Ser Gly Gly Gly Ser
1 5 10 15

<210> 7
<211> 5
<212> PRT
<213> Artificial Sequence

<220>
<223> linker

<400> 7
Gly Gly Gly Gly Ser
1 5

<210> 8
<211> 10
<212> PRT
<213> Artificial Sequence

<220>
<223> linker

<400> 8
Gly Gly Gly Gly Ser Gly Gly Gly Gly Ser
1 5 10

<210> 9
<211> 15
<212> PRT
<213> Artificial Sequence

<220>
<223> linker

<400> 9
Gly Gly Gly Gly Ser Gly Gly Gly Gly Ser Gly Gly Gly Gly Ser
1 5 10 15

<210> 10
<211> 20
<212> PRT
<213> Artificial Sequence

<220>
<223> linker

<400> 10
```
Gly Gly Gly Gly Ser Gly Gly Gly Gly Ser Gly Gly Gly Gly Ser Gly
1               5                   10                  15
Gly Gly Gly Ser
            20
```

<210> 11
<211> 249
<212> PRT
<213> Artificial Sequence

<220>
<223> scFv

<400> 11
```
Asp Ile Gln Met Thr Gln Ser Pro Ser Ser Leu Ser Ala Ser Val Gly
1               5                   10                  15
Asp Arg Val Thr Ile Thr Cys Gln Ala Ser Glu Asn Ile Tyr Ser Phe
            20                  25                  30
Leu Ala Trp Tyr Gln Gln Lys Pro Gly Lys Ala Pro Lys Leu Leu Ile
        35                  40                  45
Tyr Ser Ala Ser Lys Leu Ala Ala Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Gln Gln Thr Asn Arg Tyr Ser Asn
                85                  90                  95
Pro Asp Ile Tyr Asn Val Phe Gly Gln Gly Thr Lys Leu Thr Val Leu
            100                 105                 110
Gly Gly Gly Gly Gly Ser Gly Gly Gly Gly Ser Gly Gly Gly Gly Ser
        115                 120                 125
Glu Val Gln Leu Val Glu Ser Gly Gly Gly Leu Val Gln Pro Gly Gly
    130                 135                 140
Ser Leu Arg Leu Ser Cys Ala Ala Ser Gly Ile Asp Phe Asn Ser Asn
145                 150                 155                 160
Tyr Tyr Met Cys Trp Val Arg Gln Ala Pro Gly Lys Gly Leu Glu Trp
                165                 170                 175
Ile Gly Cys Ile Tyr Val Gly Ser His Val Asn Thr Tyr Tyr Ala Asn
            180                 185                 190
Trp Ala Lys Gly Arg Phe Thr Ile Ser Arg Asp Asn Ser Lys Asn Thr
        195                 200                 205
Val Tyr Leu Gln Met Asn Ser Leu Arg Ala Glu Asp Thr Ala Val Tyr
    210                 215                 220
Tyr Cys Ala Thr Ser Gly Ser Ser Val Leu Tyr Phe Lys Phe Trp Gly
225                 230                 235                 240
Gln Gly Thr Leu Val Thr Val Ser Ser
                245
```

<210> 12
<211> 249
<212> PRT
<213> Artificial Sequence

<220>
<223> scFv

<400> 12
Asp Ile Gln Met Thr Gln Ser Pro Ser Ser Leu Ser Ala Ser Val Gly
1               5                   10                  15
Asp Arg Val Thr Ile Thr Cys Gln Ala Ser Glu Asn Ile Tyr Ser Phe
            20                  25                  30
Leu Ala Trp Tyr Gln Gln Lys Pro Gly Lys Ala Pro Lys Leu Leu Ile
        35                  40                  45
Tyr Ser Ala Ser Lys Leu Ala Ala Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Gln Gln Thr Asn Arg Tyr Ser Asn
                85                  90                  95
Pro Asp Ile Tyr Asn Val Phe Gly Gly Gly Thr Lys Leu Thr Val Leu
            100                 105                 110
Gly Gly Gly Gly Gly Ser Gly Gly Gly Gly Ser Gly Gly Gly Gly Ser
        115                 120                 125
Glu Val Gln Leu Val Glu Ser Gly Gly Gly Leu Val Gln Pro Gly Gly
    130                 135                 140
Ser Leu Arg Leu Ser Cys Ala Ala Ser Gly Ile Asp Phe Asn Ser Asn
145                 150                 155                 160
Tyr Tyr Met Cys Trp Val Arg Gln Ala Pro Gly Lys Gly Leu Glu Trp
                165                 170                 175
Ile Gly Cys Ile Tyr Val Gly Ser His Val Asn Thr Tyr Tyr Ala Asn
            180                 185                 190
Trp Ala Lys Gly Arg Phe Thr Ile Ser Arg Asp Asn Ser Lys Asn Thr
        195                 200                 205
Val Tyr Leu Gln Met Asn Ser Leu Arg Ala Glu Asp Thr Ala Val Tyr
    210                 215                 220
Tyr Cys Ala Thr Ser Gly Ser Ser Val Leu Tyr Phe Lys Phe Trp Gly
225                 230                 235                 240
Gln Gly Thr Leu Val Thr Val Ser Ser
                245

<210> 13
<211> 249
<212> PRT
<213> Artificial Sequence

<220>
<223> scFv

<400> 13
Asp Ile Gln Met Thr Gln Ser Pro Ser Ser Leu Ser Ala Ser Val Gly
1               5                   10                  15
Asp Arg Val Thr Ile Thr Cys Gln Ala Ser Glu Asn Ile Tyr Ser Phe
            20                  25                  30
Leu Ala Trp Tyr Gln Gln Lys Pro Gly Lys Ala Pro Lys Leu Leu Ile
        35                  40                  45
Tyr Ser Ala Ser Lys Leu Ala Ala Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Gln Gln Thr Asn Arg Tyr Ser Asn
                85                  90                  95
Pro Asp Ile Tyr Asn Val Phe Gly Thr Gly Thr Lys Val Thr Val Leu
            100                 105                 110

Gly Gly Gly Gly Gly Ser Gly Gly Gly Gly Ser Gly Gly Gly Gly Ser
        115                 120                 125
Glu Val Gln Leu Val Glu Ser Gly Gly Gly Leu Val Gln Pro Gly Gly
    130                 135                 140
Ser Leu Arg Leu Ser Cys Ala Ala Ser Gly Ile Asp Phe Asn Ser Asn
145                 150                 155                 160
Tyr Tyr Met Cys Trp Val Arg Gln Ala Pro Gly Lys Gly Leu Glu Trp
                165                 170                 175
Ile Gly Cys Ile Tyr Val Gly Ser His Val Asn Thr Tyr Tyr Ala Asn
            180                 185                 190
Trp Ala Lys Gly Arg Phe Thr Ile Ser Arg Asp Asn Ser Lys Asn Thr
        195                 200                 205
Val Tyr Leu Gln Met Asn Ser Leu Arg Ala Glu Asp Thr Ala Val Tyr
    210                 215                 220
Tyr Cys Ala Thr Ser Gly Ser Ser Val Leu Tyr Phe Lys Phe Trp Gly
225                 230                 235                 240
Gln Gly Thr Leu Val Thr Val Ser Ser
                245

<210> 14
<211> 16
<212> PRT
<213> Artificial Sequence

<220>
<223> Hinge

<400> 14
Ala Gly Ser Ser Ser Ser Gly Gly Ser Thr Thr Gly Gly Ser Thr Thr
1               5                   10                  15

<210> 15
<211> 16
<212> PRT
<213> Artificial Sequence

<220>
<223> Hinge

<400> 15
Gly Thr Thr Ala Ala Ser Gly Ser Ser Gly Gly Ser Ser Ser Gly Ala
1               5                   10                  15

<210> 16
<211> 16
<212> PRT
<213> Artificial Sequence

<220>
<223> Hinge

<400> 16
Ser Ser Ala Thr Ala Thr Ala Gly Thr Gly Ser Ser Thr Gly Ser Thr
1               5                   10                  15

<210> 17
<211> 16
<212> PRT
<213> Artificial Sequence

<220>
<223> Hinge

<400> 17
Thr Ser Gly Ser Thr Gly Thr Ala Ala Ser Ser Thr Ser Thr Ser Thr
1               5                   10                  15

<210> 18
<211> 30
<212> DNA
<213> Artificial Sequence

<220>
<223> Hinge

<400> 18
ggtggcggag gttctggagg tggaggttcc

<210> 19
<211> 7
<212> PRT
<213> Artificial Sequence

<220>
<223> Hinge

<400> 19
Lys Ile Arg Arg Asp Ser Ser
1               5

<210> 20
<211> 45
<212> PRT
<213> Artificial Sequence

<220>
<223> CD8-derived hinge domain

<400> 20
Thr Thr Thr Pro Ala Pro Arg Pro Pro Thr Pro Ala Pro Thr Ile Ala
1               5                   10                  15
Ser Gln Pro Leu Ser Leu Arg Pro Glu Ala Cys Arg Pro Ala Ala Gly
            20                  25                  30
Gly Ala Val His Thr Arg Gly Leu Asp Phe Ala Cys Asp
        35                  40                  45

<210> 21
<211> 135
<212> DNA
<213> Artificial Sequence

```
<220>
<223> CD8-derived hinge domain

<400> 21
acaacaacac ctgctcctcg gcctcctaca ccagctccta caattgccag ccagccactg      60

tctctgaggc ccgaagcttg caggcctgct gctggcggag ccgtgcatac aagaggactg     120

gatttcgcct gcgac                                                      135


<210> 22
<211> 230
<212> PRT
<213> Artificial Sequence


<220>
<223> IgG4-derived hinge domain

<400> 22
Glu Ser Lys Tyr Gly Pro Pro Cys Pro Pro Cys Pro Ala Pro Glu Phe
1               5                   10                  15
Leu Gly Gly Pro Ser Val Phe Leu Phe Pro Pro Lys Pro Lys Asp Thr
            20                  25                  30
Leu Met Ile Ser Arg Thr Pro Glu Val Thr Cys Val Val Val Asp Val
        35                  40                  45
Ser Gln Glu Asp Pro Glu Val Gln Phe Asn Trp Tyr Val Asp Gly Val
    50                  55                  60
Glu Val His Asn Ala Lys Thr Lys Pro Arg Glu Glu Gln Phe Asn Ser
65                  70                  75                  80
Thr Tyr Arg Val Val Ser Val Leu Thr Val Leu His Gln Asp Trp Leu
                85                  90                  95
Asn Gly Lys Glu Tyr Lys Cys Lys Val Ser Asn Lys Gly Leu Pro Ser
            100                 105                 110
Ser Ile Glu Lys Thr Ile Ser Lys Ala Lys Gly Gln Pro Arg Glu Pro
        115                 120                 125
Gln Val Tyr Thr Leu Pro Pro Ser Gln Glu Glu Met Thr Lys Asn Gln
    130                 135                 140
Val Ser Leu Thr Cys Leu Val Lys Gly Phe Tyr Pro Ser Asp Ile Ala
145                 150                 155                 160
Val Glu Trp Glu Ser Asn Gly Gln Pro Glu Asn Asn Tyr Lys Thr Thr
                165                 170                 175
Pro Pro Val Leu Asp Ser Asp Gly Ser Phe Phe Leu Tyr Ser Arg Leu
            180                 185                 190
Thr Val Asp Lys Ser Arg Trp Gln Glu Gly Asn Val Phe Ser Cys Ser
        195                 200                 205
Val Met His Glu Ala Leu His Asn His Tyr Thr Gln Lys Ser Leu Ser
    210                 215                 220
Leu Ser Leu Gly Lys Met
225                 230

<210> 23
<211> 690
<212> DNA
<213> Artificial Sequence


<220>
<223> IgG4-derived hinge domain
```

<400> 23
gagagcaagt acggccctcc ctgccccccct tgccctgccc ccgagttcct gggcggaccc 60

agcgtgttcc tgttcccccc caagcccaag gacaccctga tgatcagccg gacccccgag 120

gtgacctgtg tggtggtgga cgtgtcccag gaggaccccg aggtccagtt caactggtac 180

gtggacggcg tggaggtgca caacgccaag accaagcccc gggaggagca gttcaatagc 240

acctaccggg tggtgtccgt gctgaccgtg ctgcaccagg actggctgaa cggcaaggaa 300

tacaagtgta aggtgtccaa caagggcctg cccagcagca tcgagaaaac catcagcaag 360

gccaagggcc agcctcggga gccccaggtg tacaccctgc cccctagcca agaggagatg 420

accaagaacc aggtgtccct gacctgcctg gtgaagggct tctaccccag cgacatcgcc 480

gtggagtggg agagcaacgg ccagcccgag aacaactaca agaccacccc ccctgtgctg 540

gacagcgacg gcagcttctt cctgtacagc cggctgaccg tggacaagag ccggtggcag 600

gagggcaacg tctttagctg ctccgtgatg cacgaggccc tgcacaacca ctacacccag 660

aagagcctga gcctgtccct gggcaagatg 690

<210> 24
<211> 282
<212> PRT
<213> Artificial Sequence

<220>
<223> IgD-derived hinge domain

<400> 24
Arg Trp Pro Glu Ser Pro Lys Ala Gln Ala Ser Ser Val Pro Thr Ala
1               5                   10                  15
Gln Pro Gln Ala Glu Gly Ser Leu Ala Lys Ala Thr Thr Ala Pro Ala
            20                  25                  30
Thr Thr Arg Asn Thr Gly Arg Gly Gly Glu Glu Lys Lys Lys Glu Lys
        35                  40                  45
Glu Lys Glu Glu Gln Glu Glu Arg Glu Thr Lys Thr Pro Glu Cys Pro
    50                  55                  60
Ser His Thr Gln Pro Leu Gly Val Tyr Leu Leu Thr Pro Ala Val Gln
65                  70                  75                  80
Asp Leu Trp Leu Arg Asp Lys Ala Thr Phe Thr Cys Phe Val Val Gly
                85                  90                  95
Ser Asp Leu Lys Asp Ala His Leu Thr Trp Glu Val Ala Gly Lys Val
            100                 105                 110
Pro Thr Gly Gly Val Glu Glu Gly Leu Leu Glu Arg His Ser Asn Gly
        115                 120                 125
Ser Gln Ser Gln His Ser Arg Leu Thr Leu Pro Arg Ser Leu Trp Asn
    130                 135                 140
Ala Gly Thr Ser Val Thr Cys Thr Leu Asn His Pro Ser Leu Pro Pro
145                 150                 155                 160
Gln Arg Leu Met Ala Leu Arg Glu Pro Ala Ala Gln Ala Pro Val Lys
                165                 170                 175
Leu Ser Leu Asn Leu Leu Ala Ser Ser Asp Pro Pro Glu Ala Ala Ser
            180                 185                 190
Trp Leu Leu Cys Glu Val Ser Gly Phe Ser Pro Pro Asn Ile Leu Leu
        195                 200                 205
Met Trp Leu Glu Asp Gln Arg Glu Val Asn Thr Ser Gly Phe Ala Pro

```
    210                 215                 220
Ala Arg Pro Pro Pro Gln Pro Gly Ser Thr Thr Phe Trp Ala Trp Ser
225                 230                 235                 240
Val Leu Arg Val Pro Ala Pro Pro Ser Pro Gln Pro Ala Thr Tyr Thr
                245                 250                 255
Cys Val Val Ser His Glu Asp Ser Arg Thr Leu Leu Asn Ala Ser Arg
            260                 265                 270
Ser Leu Glu Val Ser Tyr Val Thr Asp His
        275                 280
```

<210> 25
<211> 847
<212> DNA
<213> Artificial Sequence

<220>
<223> IgD-derived hinge domain

<400> 25

```
aggtggcccg aaagtcccaa ggcccaggca tctagtgttc ctactgcaca gccccaggca      60

gaaggcagcc tagccaaagc tactactgca cctgccacta cgcgcaatac tggccgtggc     120

ggggaggaga agaaaaagga gaaagagaaa gaagaacagg aagagaggga gaccaagacc     180

cctgaatgtc catcccatac ccagccgctg ggcgtctatc tcttgactcc cgcagtacag     240

gacttgtggc ttagagataa ggccaccttt acatgtttcg tcgtgggctc tgacctgaag     300

gatgcccatt tgacttggga ggttgccgga aaggtaccca caggggggt tgaggaaggg     360

ttgctggagc gccattccaa tggctctcag agccagcact caagactcac ccttccgaga     420

tccctgtgga acgccgggac ctctgtcaca tgtactctaa atcatcctag cctgccccca     480

cagcgtctga tggcccttag agagccagcc gcccaggcac cagttaagct tagcctgaat     540

ctgctcgcca gtagtgatcc cccagaggcc gccagctggc tcttatgcga agtgtccggc     600

tttagcccgc ccaacatctt gctcatgtgg ctggaggacc agcgagaagt gaacaccagc     660

ggcttcgctc cagcccggcc cccaccccag ccgggttcta ccacattctg ggcctggagt     720

gtcttaaggg tcccagcacc acctagcccc cagccagcca catacacctg tgttgtgtcc     780

catgaagata gcaggaccct gctaaatgct tctaggagtc tggaggtttc ctacgtgact     840

gaccatt                                                               847
```

<210> 26
<211> 39
<212> PRT
<213> Artificial Sequence

<220>
<223> CD28-derived hinge domain

<400> 26

```
Ile Glu Val Met Tyr Pro Pro Pro Tyr Leu Asp Asn Glu Lys Ser Asn
1               5                   10                  15
```

Gly Thr Ile Ile His Val Lys Gly Lys His Leu Cys Pro Ser Pro Leu
20 25 30
Phe Pro Gly Pro Ser Lys Pro
35

<210> 27
<211> 117
<212> DNA
<213> Artificial Sequence

<220>
<223> CD28-derived hinge domain

<400> 27
attgaagtta tgtatcctcc tccttaccta gacaatgaga agagcaatgg aaccattatc 60

catgtgaaag ggaaacacct ttgtccaagt cccctatttc ccggaccttc taagccc 117

<210> 28
<211> 27
<212> PRT
<213> Artificial Sequence

<220>
<223> 4-1BB-derived transmembrane domain

<400> 28
Ile Ile Ser Phe Phe Leu Ala Leu Thr Ser Thr Ala Leu Leu Phe Leu
1 5 10 15
Leu Phe Phe Leu Thr Leu Arg Phe Ser Val Val
20 25

<210> 29
<211> 81
<212> DNA
<213> Artificial Sequence

<220>
<223> 4-1BB-derived transmembrane domain

<400> 29
atcatctcct tctttcttgc gctgacctcg actgcgttgc tcttcctgct gttcttcctc 60

acgctccgtt tctctgttgt t 81

<210> 30
<211> 112
<212> PRT
<213> Artificial Sequence

<220>
<223> CD3zeta-derived intracellular domain

<400> 30
Arg Val Lys Phe Ser Arg Ser Ala Asp Ala Pro Ala Tyr Gln Gln Gly
1 5 10 15
Gln Asn Gln Leu Tyr Asn Glu Leu Asn Leu Gly Arg Arg Glu Glu Tyr

```
            20                  25                  30
Asp Val Leu Asp Lys Arg Arg Gly Arg Asp Pro Glu Met Gly Gly Lys
        35                  40                  45
Pro Arg Arg Lys Asn Pro Gln Glu Gly Leu Tyr Asn Glu Leu Gln Lys
    50                  55                  60
Asp Lys Met Ala Glu Ala Tyr Ser Glu Ile Gly Met Lys Gly Glu Arg
65                  70                  75                  80
Arg Arg Gly Lys Gly His Asp Gly Leu Tyr Gln Gly Leu Ser Thr Ala
                85                  90                  95
Thr Lys Asp Thr Tyr Asp Ala Leu His Met Gln Ala Leu Pro Pro Arg
            100                 105                 110
```

<210> 31
<211> 112
<212> PRT
<213> Artificial Sequence

<220>
<223> CD3zeta-derived intracellular domain

<400> 31

```
Arg Val Lys Phe Ser Arg Ser Ala Asp Ala Pro Ala Tyr Lys Gln Gly
1               5                   10                  15
Gln Asn Gln Leu Tyr Asn Glu Leu Asn Leu Gly Arg Arg Glu Glu Tyr
            20                  25                  30
Asp Val Leu Asp Lys Arg Arg Gly Arg Asp Pro Glu Met Gly Gly Lys
        35                  40                  45
Pro Arg Arg Lys Asn Pro Gln Glu Gly Leu Tyr Asn Glu Leu Gln Lys
    50                  55                  60
Asp Lys Met Ala Glu Ala Tyr Ser Glu Ile Gly Met Lys Gly Glu Arg
65                  70                  75                  80
Arg Arg Gly Lys Gly His Asp Gly Leu Tyr Gln Gly Leu Ser Thr Ala
                85                  90                  95
Thr Lys Asp Thr Tyr Asp Ala Leu His Met Gln Ala Leu Pro Pro Arg
            100                 105                 110
```

<210> 32
<211> 113
<212> PRT
<213> Artificial Sequence

<220>
<223> CD3zeta-derived intracellular domain

<400> 32

```
Arg Val Lys Phe Ser Arg Ser Ala Asp Ala Pro Ala Tyr Lys Gln Gly
1               5                   10                  15
Gln Asn Gln Leu Tyr Asn Glu Leu Asn Leu Gly Arg Arg Glu Glu Tyr
            20                  25                  30
Asp Val Leu Asp Lys Arg Arg Gly Arg Asp Pro Glu Met Gly Gly Lys
        35                  40                  45
Pro Gln Arg Arg Lys Asn Pro Gln Glu Gly Leu Tyr Asn Glu Leu Gln
    50                  55                  60
Lys Asp Lys Met Ala Glu Ala Tyr Ser Glu Ile Gly Met Lys Gly Glu
65                  70                  75                  80
Arg Arg Arg Gly Lys Gly His Asp Gly Leu Tyr Gln Gly Leu Ser Thr
                85                  90                  95
```

Ala Thr Lys Asp Thr Tyr Asp Ala Leu His Met Gln Ala Leu Pro Pro
100 105 110
Arg

<210> 33
<211> 113
<212> PRT
<213> Artificial Sequence

<220>
<223> CD3zeta-derived intracellular domain

<400> 33
Arg Val Lys Phe Ser Arg Ser Ala Asp Ala Pro Ala Tyr Gln Gln Gly
1 5 10 15
Gln Asn Gln Leu Tyr Asn Glu Leu Asn Leu Gly Arg Arg Glu Glu Tyr
20 25 30
Asp Val Leu Asp Lys Arg Arg Gly Arg Asp Pro Glu Met Gly Gly Lys
35 40 45
Pro Gln Arg Arg Lys Asn Pro Gln Glu Gly Leu Tyr Asn Glu Leu Gln
50 55 60
Lys Asp Lys Met Ala Glu Ala Tyr Ser Glu Ile Gly Met Lys Gly Glu
65 70 75 80
Arg Arg Arg Gly Lys Gly His Asp Gly Leu Tyr Gln Gly Leu Ser Thr
85 90 95
Ala Thr Lys Asp Thr Tyr Asp Ala Leu His Met Gln Ala Leu Pro Pro
100 105 110
Arg

<210> 34
<211> 336
<212> DNA
<213> Artificial Sequence

<220>
<223> CD3zeta-derived intracellular domain

<400> 34
agagtgaagt tcagcagatc cgccgacgct cctgcctatc agcagggcca aaaccagctg 60

tacaacgagc tgaacctggg gagaagagaa gagtacgacg tgctggacaa gcggagaggc 120

agagatcctg aaatgggcgg caagcccaga cggaagaatc ctcaagaggg cctgtataat 180

gagctgcaga aagacaagat ggccgaggcc tacagcgaga tcggaatgaa gggcgagcgc 240

agaagaggca agggacacga tggactgtac cagggcctga gcaccgccac caaggatacc 300

tatgatgccc tgcacatgca ggccctgcct ccaaga 336

<210> 35
<211> 336
<212> DNA
<213> Artificial Sequence

<220>
<223> CD3zeta-derived intracellular domain

<400> 35
agagtgaagt tcagcaggag cgcagacgcc cccgcgtaca agcagggcca gaaccagctc 60

tataacgagc tcaatctagg acgaagagag gagtacgatg ttttggacaa gagacgtggc 120

cgggaccctg agatgggggg aaagccgaga aggaagaacc ctcaggaagg cctgtacaat 180

gaactgcaga aagataagat ggcggaggcc tacagtgaga ttgggatgaa aggcgagcgc 240

cggaggggca aggggcacga tggcctttac cagggtctca gtacagccac caaggacacc 300

tacgacgccc ttcacatgca ggccctgccc cctcgc 336

<210> 36
<211> 42
<212> PRT
<213> Artificial Sequence

<220>
<223> 4-1BB-derived intracellular domain

<400> 36
Lys Arg Gly Arg Lys Lys Leu Leu Tyr Ile Phe Lys Gln Pro Phe Met
1 5 10 15
Arg Pro Val Gln Thr Thr Gln Glu Glu Asp Gly Cys Ser Cys Arg Phe
20 25 30
Pro Glu Glu Glu Glu Gly Gly Cys Glu Leu
35 40

<210> 37
<211> 48
<212> PRT
<213> Artificial Sequence

<220>
<223> CD27-derived intracellular domain

<400> 37
Gln Arg Arg Lys Tyr Arg Ser Asn Lys Gly Glu Ser Pro Val Glu Pro
1 5 10 15
Ala Glu Pro Cys Arg Tyr Ser Cys Pro Arg Glu Glu Glu Gly Ser Thr
20 25 30
Ile Pro Ile Gln Glu Asp Tyr Arg Lys Pro Glu Pro Ala Cys Ser Pro
35 40 45

<210> 38
<211> 41
<212> PRT
<213> Artificial Sequence

<220>
<223> CD28-derived intracellular domain

<400> 38
Arg Ser Lys Arg Ser Arg Leu Leu His Ser Asp Tyr Met Asn Met Thr
1 5 10 15

```
Pro Arg Arg Pro Gly Pro Thr Arg Lys His Tyr Gln Pro Tyr Ala Pro
            20                  25                  30
Pro Arg Asp Phe Ala Ala Tyr Arg Ser
        35                  40
```

<210> 39
<211> 126
<212> DNA
<213> Artificial Sequence

<220>
<223> 4-1BB-derived intracellular domain

<400> 39

```
aagcggggca gaaagaaact gctctacatc ttcaagcagc ccttcatgcg gcccgtgcag      60

accacacaag aggaagatgg ctgctcctgc agattccccg aggaagaaga aggcggctgc     120

gagctg                                                                126
```

<210> 40
<211> 144
<212> DNA
<213> Artificial Sequence

<220>
<223> CD27-derived intracellular domain

<400> 40

```
caacgaagga aatatagatc aaacaaagga gaaagtcctg tggagcctgc agagccttgt      60

cgttacagct gccccaggga ggaggagggc agcaccatcc ccatccagga ggattaccga     120

aaaccggagc ctgcctgctc cccc                                            144
```

<210> 41
<211> 123
<212> DNA
<213> Artificial Sequence

<220>
<223> CD28-derived intracellular domain

<400> 41

```
aggagtaaga ggagcaggct cctgcacagt gactacatga acatgactcc ccgccgcccc      60

gggcccaccc gcaagcatta ccagccctat gccccaccac gcgacttcgc agcctatcgc     120

tcc                                                                   123
```

<210> 42
<211> 22
<212> PRT
<213> Artificial Sequence

<220>

<223> CD8-derived Leader sequence

<400> 42
Met Ala Leu Pro Val Thr Ala Leu Leu Leu Pro Leu Ala Leu Leu Leu
1               5                   10                  15
His Ala Ala Arg Pro Ser
            20

<210> 43
<211> 9
<212> PRT
<213> Artificial Sequence

<220>
<223> Tag

<400> 43
Asn Trp Ser His Pro Gln Phe Glu Lys
1               5

<210> 44
<211> 10
<212> PRT
<213> Artificial Sequence

<220>
<223> Tag

<400> 44
Ser Ala Trp Ser His Pro Gln Phe Glu Lys
1               5                   10

<210> 45
<211> 22
<212> PRT
<213> Artificial Sequence

<220>
<223> P2A

<400> 45
Gly Ser Gly Ala Thr Asn Phe Ser Leu Leu Lys Gln Ala Gly Asp Val
1               5                   10                  15
Glu Glu Asn Pro Gly Pro
            20

<210> 46
<211> 239
<212> PRT
<213> Artificial Sequence

<220>
<223> GFP

<400> 46
Met Val Ser Lys Gly Glu Glu Leu Phe Thr Gly Val Val Pro Ile Leu
1               5                   10                  15
Val Glu Leu Asp Gly Asp Val Asn Gly His Lys Phe Ser Val Ser Gly
            20                  25                  30

```
Glu Gly Glu Gly Asp Ala Thr Tyr Gly Lys Leu Thr Leu Lys Phe Ile
        35                  40                  45
Cys Thr Thr Gly Lys Leu Pro Val Pro Trp Pro Thr Leu Val Thr Thr
    50                  55                  60
Leu Thr Tyr Gly Val Gln Cys Phe Ser Arg Tyr Pro Asp His Met Lys
65                  70                  75                  80
Gln His Asp Phe Phe Lys Ser Ala Met Pro Glu Gly Tyr Val Gln Glu
                85                  90                  95
Arg Thr Ile Phe Phe Lys Asp Asp Gly Asn Tyr Lys Thr Arg Ala Glu
            100                 105                 110
Val Lys Phe Glu Gly Asp Thr Leu Val Asn Arg Ile Glu Leu Lys Gly
        115                 120                 125
Ile Asp Phe Lys Glu Asp Gly Asn Ile Leu Gly His Lys Leu Glu Tyr
    130                 135                 140
Asn Tyr Asn Ser His Asn Val Tyr Ile Met Ala Asp Lys Gln Lys Asn
145                 150                 155                 160
Gly Ile Lys Val Asn Phe Lys Ile Arg His Asn Ile Glu Asp Gly Ser
                165                 170                 175
Val Gln Leu Ala Asp His Tyr Gln Gln Asn Thr Pro Ile Gly Asp Gly
            180                 185                 190
Pro Val Leu Leu Pro Asp Asn His Tyr Leu Ser Thr Gln Ser Ala Leu
        195                 200                 205
Ser Lys Asp Pro Asn Glu Lys Arg Asp His Met Val Leu Leu Glu Phe
    210                 215                 220
Val Thr Ala Ala Gly Ile Thr Leu Gly Met Asp Glu Leu Tyr Lys
225                 230                 235

<210> 47
<211> 228
<212> PRT
<213> Artificial Sequence


<220>
<223> CAR

<400> 47
Thr Thr Thr Pro Ala Pro Arg Pro Pro Thr Pro Ala Pro Thr Ile Ala
1               5                   10                  15
Ser Gln Pro Leu Ser Leu Arg Pro Glu Ala Cys Arg Pro Ala Ala Gly
            20                  25                  30
Gly Ala Val His Thr Arg Gly Leu Asp Phe Ala Cys Asp Ile Ile Ser
        35                  40                  45
Phe Phe Leu Ala Leu Thr Ser Thr Ala Leu Leu Phe Leu Leu Phe Phe
    50                  55                  60
Leu Thr Leu Arg Phe Ser Val Val Lys Arg Gly Arg Lys Lys Leu Leu
65                  70                  75                  80
Tyr Ile Phe Lys Gln Pro Phe Met Arg Pro Val Gln Thr Thr Gln Glu
                85                  90                  95
Glu Asp Gly Cys Ser Cys Arg Phe Pro Glu Glu Glu Glu Gly Gly Cys
            100                 105                 110
Glu Leu Thr Arg Arg Val Lys Phe Ser Arg Ser Ala Asp Ala Pro Ala
        115                 120                 125
Tyr Gln Gln Gly Gln Asn Gln Leu Tyr Asn Glu Leu Asn Leu Gly Arg
    130                 135                 140
Arg Glu Glu Tyr Asp Val Leu Asp Lys Arg Arg Gly Arg Asp Pro Glu
145                 150                 155                 160
Met Gly Gly Lys Pro Arg Arg Lys Asn Pro Gln Glu Gly Leu Tyr Asn
                165                 170                 175
Glu Leu Gln Lys Asp Lys Met Ala Glu Ala Tyr Ser Glu Ile Gly Met
            180                 185                 190
Lys Gly Glu Arg Arg Arg Gly Lys Gly His Asp Gly Leu Tyr Gln Gly
        195                 200                 205
```

Leu Ser Thr Ala Thr Lys Asp Thr Tyr Asp Ala Leu His Met Gln Ala
    210                 215                 220
Leu Pro Pro Arg
225

<210> 48
<211> 684
<212> DNA
<213> Artificial Sequence

<220>
<223> CAR

<400> 48
acaacaacac ctgctcctcg gcctcctaca ccagctccta caattgccag ccagccactg 60

tctctgaggc ccgaagcttg caggcctgct gctggcggag ccgtgcatac aagaggactg 120

gatttcgcct gcgacatcat ctccttcttt cttgcgctga cctcgactgc gttgctcttc 180

ctgctgttct tcctcacgct ccgtttctct gttgttaagc ggggcagaaa gaaactgctc 240

tacatcttca agcagccctt catgcggccc gtgcagacca cacaagagga agatggctgc 300

tcctgcagat tccccgagga agaagaaggc ggctgcgagc tgacgcgtag agtgaagttc 360

agcagatccg ccgacgctcc tgcctatcag caggggcaaa accagctgta caacgagctg 420

aacctgggga gaagagaaga gtacgacgtg ctggacaagc ggagaggcag agatcctgaa 480

atgggcggca agcccagacg gaagaatcct caagagggcc tgtataatga gctgcagaaa 540

gacaagatgg ccgaggccta cagcgagatc ggaatgaagg gcgagcgcag aagaggcaag 600

ggacacgatg gactgtacca gggcctgagc accgccacca aggataccta tgatgccctg 660

cacatgcagg ccctgcctcc aaga 684

<210> 49
<211> 1497
<212> DNA
<213> Artificial Sequence

<220>
<223> CAR

<400> 49
atggctctgc ctgtgacagc tctgctgctg cctctggctc tgcttcttca tgccgccaga 60

ccatctgagg tgcagctagt tgaatctggt ggaggcctgg ttcagcctgg tggctctctg 120

agactgtctt gtgccgccag cggcatcgac ttcaacagca actactacat gtgctgggtc 180

cgacaggccc ccggcaaagg acttgaatgg attggctgca tctacgtggg cagccatgtg 240

aacacctact acgcaaactg ggccaagggc agattcacca tcagccggga caacagcaag 300

aacaccgtgt acctgcagat gaacagcctg agagcagaag atactgccgt gtactactgt 360

gccacatctg gcagcagcgt gctgtacttc aagttctggg gccagggcac cctggtcaca 420

gtttcttcag gaggtggagg ctctggcggt ggaggaagtg gtgggggagg ctctgacatc 480

cagatgacac agagccccag cagcctgtct gcctctgtgg gagacagagt gaccatcact 540

tgccaggcca gcgagaacat ctacagcttc ctggcctggt accagcagaa gccaggcaag 600

gcccctaagc tgctgatcta cagcgcctct aaactagctg ccggggtgcc aagcagattt 660

tctggctctg gcagcggcac cgacttcacc ctgaccatat caagcctgca gcctgaggac 720

ttcgccacct actactgcca gcagaccaac aggtacagca accccgacat ctacaacgtg 780

tttggacagg gcaccaagct gacagtgctt ggaacaacaa cacctgctcc tcggcctcct 840

acaccagctc ctacaattgc cagccagcca ctgtctctga ggcccgaagc ttgcaggcct 900

gctgctggcg gagccgtgca tacaagagga ctggatttcg cctgcgacat catctccttc 960

tttcttgcgc tgacctcgac tgcgttgctc ttcctgctgt tcttcctcac gctccgtttc 1020

tctgttgtta agcggggcag aaagaaactg ctctacatct tcaagcagcc cttcatgcgg 1080

cccgtgcaga ccacacaaga ggaagatggc tgctcctgca gattccccga ggaagaagaa 1140

ggcggctgcg agctgacgcg tagagtgaag ttcagcagat ccgccgacgc tcctgcctat 1200

cagcagggcc aaaaccagct gtacaacgag ctgaacctgg ggagaagaga agagtacgac 1260

gtgctggaca agcggagagg cagagatcct gaaatgggcg gcaagcccag acggaagaat 1320

cctcaagagg gcctgtataa tgagctgcag aaagacaaga tggccgaggc ctacagcgag 1380

atcggaatga agggcgagcg cagaagaggc aagggacacg atggactgta ccagggcctg 1440

agcaccgcca ccaaggatac ctatgatgcc ctgcacatgc aggccctgcc tccaaga 1497

<210> 50
<211> 499
<212> PRT
<213> Artificial Sequence

<220>
<223> CAR

<400> 50
Met Ala Leu Pro Val Thr Ala Leu Leu Leu Pro Leu Ala Leu Leu Leu
1 5 10 15
His Ala Ala Arg Pro Ser Glu Val Gln Leu Val Glu Ser Gly Gly Gly
20 25 30
Leu Val Gln Pro Gly Gly Ser Leu Arg Leu Ser Cys Ala Ala Ser Gly
35 40 45
Ile Asp Phe Asn Ser Asn Tyr Tyr Met Cys Trp Val Arg Gln Ala Pro
50 55 60
Gly Lys Gly Leu Glu Trp Ile Gly Cys Ile Tyr Val Gly Ser His Val
65 70 75 80
Asn Thr Tyr Tyr Ala Asn Trp Ala Lys Gly Arg Phe Thr Ile Ser Arg
85 90 95
Asp Asn Ser Lys Asn Thr Val Tyr Leu Gln Met Asn Ser Leu Arg Ala
100 105 110
Glu Asp Thr Ala Val Tyr Tyr Cys Ala Thr Ser Gly Ser Ser Val Leu

```
        115                 120                 125
Tyr Phe Lys Phe Trp Gly Gln Gly Thr Leu Val Thr Val Ser Ser Gly
    130                 135                 140
Gly Gly Gly Ser Gly Gly Gly Gly Ser Gly Gly Gly Gly Ser Asp Ile
145                 150                 155                 160
Gln Met Thr Gln Ser Pro Ser Ser Leu Ser Ala Ser Val Gly Asp Arg
                165                 170                 175
Val Thr Ile Thr Cys Gln Ala Ser Glu Asn Ile Tyr Ser Phe Leu Ala
            180                 185                 190
Trp Tyr Gln Gln Lys Pro Gly Lys Ala Pro Lys Leu Leu Ile Tyr Ser
        195                 200                 205
Ala Ser Lys Leu Ala Ala Gly Val Pro Ser Arg Phe Ser Gly Ser Gly
    210                 215                 220
Ser Gly Thr Asp Phe Thr Leu Thr Ile Ser Ser Leu Gln Pro Glu Asp
225                 230                 235                 240
Phe Ala Thr Tyr Tyr Cys Gln Gln Thr Asn Arg Tyr Ser Asn Pro Asp
                245                 250                 255
Ile Tyr Asn Val Phe Gly Gln Gly Thr Lys Leu Thr Val Leu Gly Thr
            260                 265                 270
Thr Thr Pro Ala Pro Arg Pro Pro Thr Pro Ala Pro Thr Ile Ala Ser
        275                 280                 285
Gln Pro Leu Ser Leu Arg Pro Glu Ala Cys Arg Pro Ala Ala Gly Gly
    290                 295                 300
Ala Val His Thr Arg Gly Leu Asp Phe Ala Cys Asp Ile Ile Ser Phe
305                 310                 315                 320
Phe Leu Ala Leu Thr Ser Thr Ala Leu Leu Phe Leu Leu Phe Phe Leu
                325                 330                 335
Thr Leu Arg Phe Ser Val Val Lys Arg Gly Arg Lys Lys Leu Leu Tyr
            340                 345                 350
Ile Phe Lys Gln Pro Phe Met Arg Pro Val Gln Thr Thr Gln Glu Glu
        355                 360                 365
Asp Gly Cys Ser Cys Arg Phe Pro Glu Glu Glu Glu Gly Gly Cys Glu
    370                 375                 380
Leu Thr Arg Arg Val Lys Phe Ser Arg Ser Ala Asp Ala Pro Ala Tyr
385                 390                 395                 400
Gln Gln Gly Gln Asn Gln Leu Tyr Asn Glu Leu Asn Leu Gly Arg Arg
                405                 410                 415
Glu Glu Tyr Asp Val Leu Asp Lys Arg Arg Gly Arg Asp Pro Glu Met
            420                 425                 430
Gly Gly Lys Pro Arg Arg Lys Asn Pro Gln Glu Gly Leu Tyr Asn Glu
        435                 440                 445
Leu Gln Lys Asp Lys Met Ala Glu Ala Tyr Ser Glu Ile Gly Met Lys
    450                 455                 460
Gly Glu Arg Arg Arg Gly Lys Gly His Asp Gly Leu Tyr Gln Gly Leu
465                 470                 475                 480
Ser Thr Ala Thr Lys Asp Thr Tyr Asp Ala Leu His Met Gln Ala Leu
                485                 490                 495
Pro Pro Arg
```

<210> 51
<211> 135
<212> PRT
<213> Artificial Sequence

<220>
<223> Peptide

<400> 51

```
Cys Pro Tyr Ser Asn Pro Ser Leu Cys Ser Gly Gly Gly Gly Ser Glu
1               5                   10                  15
Leu Pro Thr Gln Gly Thr Phe Ser Asn Val Ser Thr Asn Val Ser Pro
```

```
            20                  25                  30
Ala Lys Pro Thr Thr Thr Ala Cys Pro Tyr Ser Asn Pro Ser Leu Cys
        35                  40                  45
Ser Gly Gly Gly Gly Ser Pro Ala Pro Arg Pro Pro Thr Pro Ala Pro
    50                  55                  60
Thr Ile Ala Ser Gln Pro Leu Ser Leu Arg Pro Glu Ala Cys Arg Pro
65                  70                  75                  80
Ala Ala Gly Gly Ala Val His Thr Arg Gly Leu Asp Phe Ala Cys Asp
                85                  90                  95
Ile Tyr Ile Trp Ala Pro Leu Ala Gly Thr Cys Gly Val Leu Leu Leu
            100                 105                 110
Ser Leu Val Ile Thr Leu Tyr Cys Asn His Arg Asn Arg Arg Arg Val
        115                 120                 125
Cys Lys Cys Pro Arg Pro Trp
    130                 135
```

<210> 52
<211> 9
<212> PRT
<213> Artificial Sequence

<220>
<223> LAGLIDADG motif

<400> 52

```
Leu Ala Gly Leu Ile Asp Ala Asp Gly
1               5
```

<210> 53
<211> 69
<212> PRT
<213> Artificial Sequence

<220>
<223> 4-1BB-derived transmembrane and intracellular domains

<400> 53

```
Ile Ile Ser Phe Phe Leu Ala Leu Thr Ser Thr Ala Leu Leu Phe Leu
1               5                   10                  15
Leu Phe Phe Leu Thr Leu Arg Phe Ser Val Val Lys Arg Gly Arg Lys
            20                  25                  30
Lys Leu Leu Tyr Ile Phe Lys Gln Pro Phe Met Arg Pro Val Gln Thr
        35                  40                  45
Thr Gln Glu Glu Asp Gly Cys Ser Cys Arg Phe Pro Glu Glu Glu Glu
    50                  55                  60
Gly Gly Cys Glu Leu
65
```

<210> 54
<211> 66
<212> DNA
<213> Artificial Sequence

<220>
<223> CD8-derived Leader sequence

<400> 54

```
atggctctgc ctgtgacagc tctgctgctg cctctggctc tgcttcttca tgccgccaga      60

ccatct                                                                 66
```

<210> 55
<211> 45
<212> DNA
<213> Artificial Sequence

<220>
<223> linker

<400> 55
ggaggtggag gctctggcgg tggaggaagt ggtgggggag gctct 45

<210> 56
<211> 363
<212> DNA
<213> Artificial Sequence

<220>
<223> VH

<400> 56
gaggtgcagc tagttgaatc tggtggaggc ctggttcagc ctggtggctc tctgagactg 60

tcttgtgccg ccagcggcat cgacttcaac agcaactact acatgtgctg ggtccgacag 120

gcccccggca aaggacttga atggattggc tgcatctacg tgggcagcca tgtgaacacc 180

tactacgcaa actgggccaa gggcagattc accatcagcc gggacaacag caagaacacc 240

gtgtacctgc agatgaacag cctgagagca gaagatactg ccgtgtacta ctgtgccaca 300

tctggcagca gcgtgctgta cttcaagttc tggggccagg gcaccctggt cacagtttct 360

tca 363

<210> 57
<211> 339
<212> DNA
<213> Artificial Sequence

<220>
<223> VL

<400> 57
gacatccaga tgacacagag ccccagcagc ctgtctgcct ctgtgggaga cagagtgacc 60

atcacttgcc aggccagcga gaacatctac agcttcctgg cctggtacca gcagaagcca 120

ggcaaggccc ctaagctgct gatctacagc gcctctaaac tagctgccgg ggtgccaagc 180

agattttctg gctctggcag cggcaccgac ttcaccctga ccatatcaag cctgcagcct 240

gaggacttcg ccacctacta ctgccagcag accaacaggt acagcaaccc cgacatctac 300

aacgtgtttg gacagggcac caagctgaca gtgcttgga 339

<210> 58
<211> 66
<212> DNA
<213> Artificial Sequence

<220>
<223> P2A

<400> 58
ggaagcggag ctactaactt cagcctgctg aagcaggctg gagacgtgga ggagaaccct 60

ggacct 66

<210> 59
<211> 720
<212> DNA
<213> Artificial Sequence

<220>
<223> GFP

<400> 59
atggtgagca agggcgagga gctgttcacc ggggtggtgc ccatcctggt cgagctggac 60

ggcgacgtaa acggccacaa gttcagcgtg tccggcgagg gcgagggcga tgccacctac 120

ggcaagctga ccctgaagtt catctgcacc accggcaagc tgcccgtgcc ctggcccacc 180

ctcgtgacca ccctgaccta cggcgtgcag tgcttcagcc gctaccccga ccacatgaag 240

cagcacgact tcttcaagtc cgccatgccc gaaggctacg tccaggagcg caccatcttc 300

ttcaaggacg acggcaacta caagacccgc gccgaggtga agttcgaggg cgacaccctg 360

gtgaaccgca tcgagctgaa gggcatcgac ttcaaggagg acggcaacat cctggggcac 420

aagctggagt acaactacaa cagccacaac gtctatatca tggccgacaa gcagaagaac 480

ggcatcaagg tgaacttcaa gatccgccac aacatcgagg acggcagcgt gcagctcgcc 540

gaccactacc agcagaacac ccccatcggc gacggccccg tgctgctgcc cgacaaccac 600

tacctgagca cccagtccgc cctgagcaaa gaccccaacg agaagcgcga tcacatggtc 660

ctgctggagt tcgtgaccgc cgccgggatc actctcggca tggacgagct gtacaagtaa 720

<210> 60
<211> 67
<212> PRT
<213> Artificial Sequence

<220>
<223> CD8-derived hinge domain

<400> 60
Ser Ala Leu Ser Asn Ser Ile Met Tyr Phe Ser His Phe Val Pro Val
1               5                   10                  15
Phe Leu Pro Ala Lys Pro Thr Thr Thr Pro Ala Pro Arg Pro Pro Thr

```
                20                  25                  30
Pro Ala Pro Thr Ile Ala Ser Gln Pro Leu Ser Leu Arg Pro Glu Ala
        35                  40                  45
Cys Arg Pro Ala Ala Gly Gly Ala Val His Thr Arg Gly Leu Asp Phe
    50                  55                  60
Ala Cys Asp
65
```

<210> 61
<211> 201
<212> DNA
<213> Artificial Sequence

<220>
<223> CD8-derived hinge domain

<400> 61

```
agcgccctga gcaacagcat catgtacttc agccacttcg tgcccgtgtt tctgcccgcc      60

aaacctacaa caacacctgc tcctcggcct cctacaccag ctcctacaat tgccagccag     120

ccactgtctc tgaggcccga agcttgcagg cctgctgctg gcggagccgt gcatacaaga     180

ggactggatt tcgcctgcga c                                               201
```

<210> 62
<211> 2289
<212> DNA
<213> Artificial Sequence

<220>
<223> CAR

<400> 62

```
atggctctgc ctgtgacagc tctgctgctg cctctggctc tgcttcttca tgccgccaga      60

ccatctgagg tgcagctagt tgaatctggt ggaggcctgg ttcagcctgg tggctctctg     120

agactgtctt gtgccgccag cggcatcgac ttcaacagca actactacat gtgctgggtc     180

cgacaggccc ccggcaaagg acttgaatgg attggctgca tctacgtggg cagccatgtg     240

aacacctact acgcaaactg ggccaagggc agattcacca tcagccggga caacagcaag     300

aacaccgtgt acctgcagat gaacagcctg agagcagaag atactgccgt gtactactgt     360

gccacatctg gcagcagcgt gctgtacttc aagttctggg gccagggcac cctggtcaca     420

gtttcttcag gaggtggagg ctctggcggt ggaggaagtg gtgggggagg ctctgacatc     480

cagatgacac agagccccag cagcctgtct gcctctgtgg gagacagagt gaccatcact     540

tgccaggcca gcgagaacat ctacagcttc ctggcctggt accagcagaa gccaggcaag     600

gcccctaagc tgctgatcta cagcgcctct aaactagctg ccggggtgcc aagcagattt     660

tctggctctg gcagcggcac cgacttcacc ctgaccatat caagcctgca gcctgaggac     720

ttcgccacct actactgcca gcagaccaac aggtacagca accccgacat ctacaacgtg     780
```

tttggacagg gcaccaagct gacagtgctt ggaacaacaa cacctgctcc tcggcctcct 840

acaccagctc ctacaattgc cagccagcca ctgtctctga ggcccgaagc ttgcaggcct 900

gctgctggcg gagccgtgca tacaagagga ctggatttcg cctgcgacat catctccttc 960

tttcttgcgc tgacctcgac tgcgttgctc ttcctgctgt tcttcctcac gctccgtttc 1020

tctgttgtta agcggggcag aaagaaactg ctctacatct tcaagcagcc cttcatgcgg 1080

cccgtgcaga ccacacaaga ggaagatggc tgctcctgca gattccccga ggaagaagaa 1140

ggcggctgcg agctgacgcg tagagtgaag ttcagcagat ccgccgacgc tcctgcctat 1200

cagcagggcc aaaaccagct gtacaacgag ctgaacctgg ggagaagaga agagtacgac 1260

gtgctggaca agcggagagg cagagatcct gaaatgggcg gcaagcccag acggaagaat 1320

cctcaagagg gcctgtataa tgagctgcag aaagacaaga tggccgaggc ctacagcgag 1380

atcggaatga agggcgagcg cagaagaggc aagggacacg atggactgta ccagggcctg 1440

agcaccgcca ccaaggatac ctatgatgcc ctgcacatgc aggccctgcc tccaagagct 1500

agcggaagcg gagctactaa cttcagcctg ctgaagcagg ctggagacgt ggaggagaac 1560

cctggaccta tggtgagcaa gggcgaggag ctgttcaccg gggtggtgcc catcctggtc 1620

gagctggacg gcgacgtaaa cggccacaag ttcagcgtgt ccggcgaggg cgagggcgat 1680

gccacctacg gcaagctgac cctgaagttc atctgcacca ccggcaagct gcccgtgccc 1740

tggcccaccc tcgtgaccac cctgacctac ggcgtgcagt gcttcagccg ctaccccgac 1800

cacatgaagc agcacgactt cttcaagtcc gccatgcccg aaggctacgt ccaggagcgc 1860

accatcttct tcaaggacga cggcaactac aagacccgcg ccgaggtgaa gttcgagggc 1920

gacaccctgg tgaaccgcat cgagctgaag ggcatcgact tcaaggagga cggcaacatc 1980

ctggggcaca agctggagta caactacaac agccacaacg tctatatcat ggccgacaag 2040

cagaagaacg gcatcaaggt gaacttcaag atccgccaca acatcgagga cggcagcgtg 2100

cagctcgccg accactacca gcagaacacc cccatcggcg acggccccgt gctgctgccc 2160

gacaaccact acctgagcac ccagtccgcc ctgagcaaag accccaacga gaagcgcgat 2220

cacatggtcc tgctggagtt cgtgaccgcc gccgggatca ctctcggcat ggacgagctg 2280

tacaagtaa 2289

<210> 63
<211> 762
<212> PRT
<213> Artificial Sequence

<220>
<223> CAR

<400> 63

Met Ala Leu Pro Val Thr Ala Leu Leu Leu Pro Leu Ala Leu Leu Leu
1               5                   10                  15
His Ala Ala Arg Pro Ser Glu Val Gln Leu Val Glu Ser Gly Gly Gly
            20                  25                  30
Leu Val Gln Pro Gly Gly Ser Leu Arg Leu Ser Cys Ala Ala Ser Gly
        35                  40                  45
Ile Asp Phe Asn Ser Asn Tyr Tyr Met Cys Trp Val Arg Gln Ala Pro
    50                  55                  60
Gly Lys Gly Leu Glu Trp Ile Gly Cys Ile Tyr Val Gly Ser His Val
65                  70                  75                  80
Asn Thr Tyr Tyr Ala Asn Trp Ala Lys Gly Arg Phe Thr Ile Ser Arg
                85                  90                  95
Asp Asn Ser Lys Asn Thr Val Tyr Leu Gln Met Asn Ser Leu Arg Ala
            100                 105                 110
Glu Asp Thr Ala Val Tyr Tyr Cys Ala Thr Ser Gly Ser Ser Val Leu
        115                 120                 125
Tyr Phe Lys Phe Trp Gly Gln Gly Thr Leu Val Thr Val Ser Ser Gly
    130                 135                 140
Gly Gly Gly Ser Gly Gly Gly Gly Ser Gly Gly Gly Gly Ser Asp Ile
145                 150                 155                 160
Gln Met Thr Gln Ser Pro Ser Ser Leu Ser Ala Ser Val Gly Asp Arg
                165                 170                 175
Val Thr Ile Thr Cys Gln Ala Ser Glu Asn Ile Tyr Ser Phe Leu Ala
            180                 185                 190
Trp Tyr Gln Gln Lys Pro Gly Lys Ala Pro Lys Leu Leu Ile Tyr Ser
        195                 200                 205
Ala Ser Lys Leu Ala Ala Gly Val Pro Ser Arg Phe Ser Gly Ser Gly
    210                 215                 220
Ser Gly Thr Asp Phe Thr Leu Thr Ile Ser Ser Leu Gln Pro Glu Asp
225                 230                 235                 240
Phe Ala Thr Tyr Tyr Cys Gln Gln Thr Asn Arg Tyr Ser Asn Pro Asp
                245                 250                 255
Ile Tyr Asn Val Phe Gly Gln Gly Thr Lys Leu Thr Val Leu Gly Thr
            260                 265                 270
Thr Thr Pro Ala Pro Arg Pro Pro Thr Pro Ala Pro Thr Ile Ala Ser
        275                 280                 285
Gln Pro Leu Ser Leu Arg Pro Glu Ala Cys Arg Pro Ala Ala Gly Gly
    290                 295                 300
Ala Val His Thr Arg Gly Leu Asp Phe Ala Cys Asp Ile Ile Ser Phe
305                 310                 315                 320
Phe Leu Ala Leu Thr Ser Thr Ala Leu Leu Phe Leu Leu Phe Phe Leu
                325                 330                 335
Thr Leu Arg Phe Ser Val Val Lys Arg Gly Arg Lys Lys Leu Leu Tyr
            340                 345                 350
Ile Phe Lys Gln Pro Phe Met Arg Pro Val Gln Thr Thr Gln Glu Glu
        355                 360                 365
Asp Gly Cys Ser Cys Arg Phe Pro Glu Glu Glu Glu Gly Gly Cys Glu
    370                 375                 380
Leu Thr Arg Arg Val Lys Phe Ser Arg Ser Ala Asp Ala Pro Ala Tyr
385                 390                 395                 400
Gln Gln Gly Gln Asn Gln Leu Tyr Asn Glu Leu Asn Leu Gly Arg Arg
                405                 410                 415
Glu Glu Tyr Asp Val Leu Asp Lys Arg Arg Gly Arg Asp Pro Glu Met
            420                 425                 430
Gly Gly Lys Pro Arg Arg Lys Asn Pro Gln Glu Gly Leu Tyr Asn Glu
        435                 440                 445
Leu Gln Lys Asp Lys Met Ala Glu Ala Tyr Ser Glu Ile Gly Met Lys
    450                 455                 460
Gly Glu Arg Arg Arg Gly Lys Gly His Asp Gly Leu Tyr Gln Gly Leu
465                 470                 475                 480
Ser Thr Ala Thr Lys Asp Thr Tyr Asp Ala Leu His Met Gln Ala Leu
                485                 490                 495
Pro Pro Arg Ala Ser Gly Ser Gly Ala Thr Asn Phe Ser Leu Leu Lys

```
            500                 505                 510
Gln Ala Gly Asp Val Glu Glu Asn Pro Gly Pro Met Val Ser Lys Gly
        515                 520                 525
Glu Glu Leu Phe Thr Gly Val Val Pro Ile Leu Val Glu Leu Asp Gly
    530                 535                 540
Asp Val Asn Gly His Lys Phe Ser Val Ser Gly Glu Gly Glu Gly Asp
545                 550                 555                 560
Ala Thr Tyr Gly Lys Leu Thr Leu Lys Phe Ile Cys Thr Thr Gly Lys
                565                 570                 575
Leu Pro Val Pro Trp Pro Thr Leu Val Thr Thr Leu Thr Tyr Gly Val
            580                 585                 590
Gln Cys Phe Ser Arg Tyr Pro Asp His Met Lys Gln His Asp Phe Phe
        595                 600                 605
Lys Ser Ala Met Pro Glu Gly Tyr Val Gln Glu Arg Thr Ile Phe Phe
    610                 615                 620
Lys Asp Asp Gly Asn Tyr Lys Thr Arg Ala Glu Val Lys Phe Glu Gly
625                 630                 635                 640
Asp Thr Leu Val Asn Arg Ile Glu Leu Lys Gly Ile Asp Phe Lys Glu
                645                 650                 655
Asp Gly Asn Ile Leu Gly His Lys Leu Glu Tyr Asn Tyr Asn Ser His
            660                 665                 670
Asn Val Tyr Ile Met Ala Asp Lys Gln Lys Asn Gly Ile Lys Val Asn
        675                 680                 685
Phe Lys Ile Arg His Asn Ile Glu Asp Gly Ser Val Gln Leu Ala Asp
    690                 695                 700
His Tyr Gln Gln Asn Thr Pro Ile Gly Asp Gly Pro Val Leu Leu Pro
705                 710                 715                 720
Asp Asn His Tyr Leu Ser Thr Gln Ser Ala Leu Ser Lys Asp Pro Asn
                725                 730                 735
Glu Lys Arg Asp His Met Val Leu Leu Glu Phe Val Thr Ala Ala Gly
            740                 745                 750
Ile Thr Leu Gly Met Asp Glu Leu Tyr Lys
        755                 760
```

<210> 64
<211> 1494
<212> DNA
<213> Artificial Sequence

<220>
<223> CAR

<400> 64

```
atggctctgc ctgtgacagc tctgctgctg cctctggctc tgcttcttca tgccgccaga      60

ccatctgagg tgcagctagt tgaatctggt ggaggcctgg ttcagcctgg tggctctctg     120

agactgtctt gtgccgccag cggcatcgac ttcaacagca actactacat gtgctgggtc     180

cgacaggccc ccggcaaagg acttgaatgg attggctgca tctacgtggg cagccatgtg     240

aacacctact acgcaaactg ggccaagggc agattcacca tcagccggga caacagcaag     300

aacaccgtgt acctgcagat gaacagcctg agagcagaag atactgccgt gtactactgt     360

gccacatctg gcagcagcgt gctgtacttc aagttctggg gccagggcac cctggtcaca     420

gtttcttcag gaggtggagg ctctggcggt ggaggaagtg gtgggggagg ctctgacatc     480

cagatgacac agagccccag cagcctgtct gcctctgtgg gagacagagt gaccatcact     540

tgccaggcca gcgagaacat ctacagcttc ctggcctggt accagcagaa gccaggcaag     600
```

gcccctaagc tgctgatcta cagcgcctct aaactagctg ccggggtgcc aagcagattt 660

tctggctctg gcagcggcac cgacttcacc ctgaccatat caagcctgca gcctgaggac 720

ttcgccacct actactgcca gcagaccaac aggtacagca accccgacat ctacaacgtg 780

tttggacagg gcaccaagct gacagtgctt ggaacaacaa cacctgctcc tcggcctcct 840

acaccagctc ctacaattgc cagccagcca ctgtctctga ggcccgaagc ttgcaggcct 900

gctgctggcg gagccgtgca tacaagagga ctggatttcg cctgcgacat catctccttc 960

tttcttgcgc tgacctcgac tgcgttgctc ttcctgctgt tcttcctcac gctccgtttc 1020

tctgttgtta agcggggcag aaagaaactg ctctacatct tcaagcagcc cttcatgcgg 1080

cccgtgcaga ccacacaaga ggaagatggc tgctcctgca gattccccga ggaagaagaa 1140

ggcggctgcg agctgagagt gaagttcagc agatccgccg acgctcctgc ctatcagcag 1200

ggccaaaacc agctctacaa cgagctgaac ctggggagaa gagaagagta cgacgtgctg 1260

gacaagcgga gaggcagaga tcctgaaatg ggcggcaagc ccagacggaa gaatcctcaa 1320

gagggcctgt ataatgagct acagaaagac aagatggcag aggcctacag cgagatcgga 1380

atgaagggcg agcgcagaag aggcaaggga cacgatggac tgtaccaggg cctgagcacc 1440

gccaccaagg atacctatga tgccctgcac atgcaggccc tgcctccaag atag 1494

<210> 65
<211> 497
<212> PRT
<213> Artificial Sequence

<220>
<223> CAR

<400> 65
Met Ala Leu Pro Val Thr Ala Leu Leu Leu Pro Leu Ala Leu Leu Leu
1 5 10 15
His Ala Ala Arg Pro Ser Glu Val Gln Leu Val Glu Ser Gly Gly Gly
20 25 30
Leu Val Gln Pro Gly Gly Ser Leu Arg Leu Ser Cys Ala Ala Ser Gly
35 40 45
Ile Asp Phe Asn Ser Asn Tyr Tyr Met Cys Trp Val Arg Gln Ala Pro
50 55 60
Gly Lys Gly Leu Glu Trp Ile Gly Cys Ile Tyr Val Gly Ser His Val
65 70 75 80
Asn Thr Tyr Tyr Ala Asn Trp Ala Lys Gly Arg Phe Thr Ile Ser Arg
85 90 95
Asp Asn Ser Lys Asn Thr Val Tyr Leu Gln Met Asn Ser Leu Arg Ala
100 105 110
Glu Asp Thr Ala Val Tyr Tyr Cys Ala Thr Ser Gly Ser Ser Val Leu
115 120 125
Tyr Phe Lys Phe Trp Gly Gln Gly Thr Leu Val Thr Val Ser Ser Gly
130 135 140
Gly Gly Gly Ser Gly Gly Gly Gly Ser Gly Gly Gly Gly Ser Asp Ile
145 150 155 160
Gln Met Thr Gln Ser Pro Ser Ser Leu Ser Ala Ser Val Gly Asp Arg

165 170 175
Val Thr Ile Thr Cys Gln Ala Ser Glu Asn Ile Tyr Ser Phe Leu Ala
180 185 190
Trp Tyr Gln Gln Lys Pro Gly Lys Ala Pro Lys Leu Leu Ile Tyr Ser
195 200 205
Ala Ser Lys Leu Ala Ala Gly Val Pro Ser Arg Phe Ser Gly Ser Gly
210 215 220
Ser Gly Thr Asp Phe Thr Leu Thr Ile Ser Ser Leu Gln Pro Glu Asp
225 230 235 240
Phe Ala Thr Tyr Tyr Cys Gln Gln Thr Asn Arg Tyr Ser Asn Pro Asp
245 250 255
Ile Tyr Asn Val Phe Gly Gln Gly Thr Lys Leu Thr Val Leu Gly Thr
260 265 270
Thr Thr Pro Ala Pro Arg Pro Pro Thr Pro Ala Pro Thr Ile Ala Ser
275 280 285
Gln Pro Leu Ser Leu Arg Pro Glu Ala Cys Arg Pro Ala Ala Gly Gly
290 295 300
Ala Val His Thr Arg Gly Leu Asp Phe Ala Cys Asp Ile Ile Ser Phe
305 310 315 320
Phe Leu Ala Leu Thr Ser Thr Ala Leu Leu Phe Leu Leu Phe Phe Leu
325 330 335
Thr Leu Arg Phe Ser Val Val Lys Arg Gly Arg Lys Lys Leu Leu Tyr
340 345 350
Ile Phe Lys Gln Pro Phe Met Arg Pro Val Gln Thr Thr Gln Glu Glu
355 360 365
Asp Gly Cys Ser Cys Arg Phe Pro Glu Glu Glu Glu Gly Gly Cys Glu
370 375 380
Leu Arg Val Lys Phe Ser Arg Ser Ala Asp Ala Pro Ala Tyr Gln Gln
385 390 395 400
Gly Gln Asn Gln Leu Tyr Asn Glu Leu Asn Leu Gly Arg Arg Glu Glu
405 410 415
Tyr Asp Val Leu Asp Lys Arg Arg Gly Arg Asp Pro Glu Met Gly Gly
420 425 430
Lys Pro Arg Arg Lys Asn Pro Gln Glu Gly Leu Tyr Asn Glu Leu Gln
435 440 445
Lys Asp Lys Met Ala Glu Ala Tyr Ser Glu Ile Gly Met Lys Gly Glu
450 455 460
Arg Arg Arg Gly Lys Gly His Asp Gly Leu Tyr Gln Gly Leu Ser Thr
465 470 475 480
Ala Thr Lys Asp Thr Tyr Asp Ala Leu His Met Gln Ala Leu Pro Pro
485 490 495
Arg

<210> 66
<211> 226
<212> PRT
<213> Artificial Sequence

<220>
<223> CAR

<400> 66
Thr Thr Thr Pro Ala Pro Arg Pro Pro Thr Pro Ala Pro Thr Ile Ala
1 5 10 15
Ser Gln Pro Leu Ser Leu Arg Pro Glu Ala Cys Arg Pro Ala Ala Gly
20 25 30
Gly Ala Val His Thr Arg Gly Leu Asp Phe Ala Cys Asp Ile Ile Ser
35 40 45
Phe Phe Leu Ala Leu Thr Ser Thr Ala Leu Leu Phe Leu Leu Phe Phe
50 55 60
Leu Thr Leu Arg Phe Ser Val Val Lys Arg Gly Arg Lys Lys Leu Leu

```
65                  70                  75                  80
Tyr Ile Phe Lys Gln Pro Phe Met Arg Pro Val Gln Thr Thr Gln Glu
                85                  90                  95
Glu Asp Gly Cys Ser Cys Arg Phe Pro Glu Glu Glu Glu Gly Gly Cys
            100                 105                 110
Glu Leu Arg Val Lys Phe Ser Arg Ser Ala Asp Ala Pro Ala Tyr Gln
        115                 120                 125
Gln Gly Gln Asn Gln Leu Tyr Asn Glu Leu Asn Leu Gly Arg Arg Glu
    130                 135                 140
Glu Tyr Asp Val Leu Asp Lys Arg Arg Gly Arg Asp Pro Glu Met Gly
145                 150                 155                 160
Gly Lys Pro Arg Arg Lys Asn Pro Gln Glu Gly Leu Tyr Asn Glu Leu
                165                 170                 175
Gln Lys Asp Lys Met Ala Glu Ala Tyr Ser Glu Ile Gly Met Lys Gly
            180                 185                 190
Glu Arg Arg Arg Gly Lys Gly His Asp Gly Leu Tyr Gln Gly Leu Ser
        195                 200                 205
Thr Ala Thr Lys Asp Thr Tyr Asp Ala Leu His Met Gln Ala Leu Pro
    210                 215                 220
Pro Arg
225
```

<210> 67
<211> 681
<212> DNA
<213> Artificial Sequence

<220>
<223> CAR

<400> 67

```
acaacaacac ctgctcctcg gcctcctaca ccagctccta caattgccag ccagccactg      60

tctctgaggc ccgaagcttg caggcctgct gctggcggag ccgtgcatac aagaggactg     120

gatttcgcct gcgacatcat ctccttcttt cttgcgctga cctcgactgc gttgctcttc     180

ctgctgttct tcctcacgct ccgtttctct gttgttaagc ggggcagaaa gaaactgctc     240

tacatcttca agcagccctt catgcggccc gtgcagacca cacaagagga agatggctgc     300

tcctgcagat tccccgagga agaagaaggc ggctgcgagc tgagagtgaa gttcagcaga     360

tccgccgacg ctcctgccta tcagcagggc caaaaccagc tctacaacga gctgaacctg     420

gggagaagag aagagtacga cgtgctggac aagcggagag gcagagatcc tgaaatgggc     480

ggcaagccca gacggaagaa tcctcaagag ggcctgtata atgagctaca gaaagacaag     540

atggcagagg cctacagcga gatcggaatg aagggcgagc gcagaagagg caagggacac     600

gatggactgt accagggcct gagcaccgcc accaaggata cctatgatgc cctgcacatg     660

caggccctgc ctccaagata g                                               681
```

<210> 68
<211> 1563
<212> DNA
<213> Artificial Sequence

<220>
<223> CAR

<400> 68
atggctctgc ctgtgacagc tctgctgctg cctctggctc tgcttcttca tgccgccaga 60

ccatctgagg tgcagctagt tgaatctggt ggaggcctgg ttcagcctgg tggctctctg 120

agactgtctt gtgccgccag cggcatcgac ttcaacagca actactacat gtgctgggtc 180

cgacaggccc ccggcaaagg acttgaatgg attggctgca tctacgtggg cagccatgtg 240

aacacctact acgcaaactg ggccaagggc agattcacca tcagccggga caacagcaag 300

aacaccgtgt acctgcagat gaacagcctg agagcagaag atactgccgt gtactactgt 360

gccacatctg gcagcagcgt gctgtacttc aagttctggg gccagggcac cctggtcaca 420

gtttcttcag gaggtggagg ctctggcggt ggaggaagtg gtgggggagg ctctgacatc 480

cagatgacac agagccccag cagcctgtct gcctctgtgg gagacagagt gaccatcact 540

tgccaggcca gcgagaacat ctacagcttc ctggcctggt accagcagaa gccaggcaag 600

gcccctaagc tgctgatcta cagcgcctct aaactagctg ccggggtgcc aagcagattt 660

tctggctctg gcagcggcac cgacttcacc ctgaccatat caagcctgca gcctgaggac 720

ttcgccacct actactgcca gcagaccaac aggtacagca accccgacat ctacaacgtg 780

tttggacagg gcaccaagct gacagtgctt ggaagcgccc tgagcaacag catcatgtac 840

ttcagccact tcgtgcccgt gtttctgccc gccaaaccta caacaacacc tgctcctcgg 900

cctcctacac cagctcctac aattgccagc cagccactgt ctctgaggcc cgaagcttgc 960

aggcctgctg ctggcggagc cgtgcataca agaggactgg atttcgcctg cgacatcatc 1020

tccttctttc ttgcgctgac ctcgactgcg ttgctcttcc tgctgttctt cctcacgctc 1080

cgtttctctg ttgttaagcg gggcagaaag aaactgctct acatcttcaa gcagcccttc 1140

atgcggcccg tgcagaccac acaagaggaa gatggctgct cctgcagatt ccccgaggaa 1200

gaagaaggcg gctgcgagct gacgcgtaga gtgaagttca gcagatccgc cgacgctcct 1260

gcctatcagc agggccaaaa ccagctgtac aacgagctga acctggggag aagagaagag 1320

tacgacgtgc tggacaagcg gagaggcaga gatcctgaaa tgggcggcaa gcccagacgg 1380

aagaatcctc aagagggcct gtataatgag ctgcagaaag acaagatggc cgaggcctac 1440

agcgagatcg gaatgaaggg cgagcgcaga agaggcaagg gacacgatgg actgtaccag 1500

ggcctgagca ccgccaccaa ggatacctat gatgccctgc acatgcaggc cctgcctcca 1560

aga 1563

<210> 69
<211> 521
<212> PRT
<213> Artificial Sequence

<220>
<223> CAR

<400> 69
Met Ala Leu Pro Val Thr Ala Leu Leu Leu Pro Leu Ala Leu Leu Leu
1               5                   10                  15
His Ala Ala Arg Pro Ser Glu Val Gln Leu Val Glu Ser Gly Gly Gly
            20                  25                  30
Leu Val Gln Pro Gly Gly Ser Leu Arg Leu Ser Cys Ala Ala Ser Gly
        35                  40                  45
Ile Asp Phe Asn Ser Asn Tyr Tyr Met Cys Trp Val Arg Gln Ala Pro
    50                  55                  60
Gly Lys Gly Leu Glu Trp Ile Gly Cys Ile Tyr Val Gly Ser His Val
65                  70                  75                  80
Asn Thr Tyr Tyr Ala Asn Trp Ala Lys Gly Arg Phe Thr Ile Ser Arg
                85                  90                  95
Asp Asn Ser Lys Asn Thr Val Tyr Leu Gln Met Asn Ser Leu Arg Ala
            100                 105                 110
Glu Asp Thr Ala Val Tyr Tyr Cys Ala Thr Ser Gly Ser Ser Val Leu
        115                 120                 125
Tyr Phe Lys Phe Trp Gly Gln Gly Thr Leu Val Thr Val Ser Ser Gly
    130                 135                 140
Gly Gly Gly Ser Gly Gly Gly Gly Ser Gly Gly Gly Gly Ser Asp Ile
145                 150                 155                 160
Gln Met Thr Gln Ser Pro Ser Ser Leu Ser Ala Ser Val Gly Asp Arg
                165                 170                 175
Val Thr Ile Thr Cys Gln Ala Ser Glu Asn Ile Tyr Ser Phe Leu Ala
            180                 185                 190
Trp Tyr Gln Gln Lys Pro Gly Lys Ala Pro Lys Leu Leu Ile Tyr Ser
        195                 200                 205
Ala Ser Lys Leu Ala Ala Gly Val Pro Ser Arg Phe Ser Gly Ser Gly
    210                 215                 220
Ser Gly Thr Asp Phe Thr Leu Thr Ile Ser Ser Leu Gln Pro Glu Asp
225                 230                 235                 240
Phe Ala Thr Tyr Tyr Cys Gln Gln Thr Asn Arg Tyr Ser Asn Pro Asp
                245                 250                 255
Ile Tyr Asn Val Phe Gly Gln Gly Thr Lys Leu Thr Val Leu Gly Ser
                260                 265                 270
Ala Leu Ser Asn Ser Ile Met Tyr Phe Ser His Phe Val Pro Val Phe
        275                 280                 285
Leu Pro Ala Lys Pro Thr Thr Thr Pro Ala Pro Arg Pro Pro Thr Pro
    290                 295                 300
Ala Pro Thr Ile Ala Ser Gln Pro Leu Ser Leu Arg Pro Glu Ala Cys
305                 310                 315                 320
Arg Pro Ala Ala Gly Gly Ala Val His Thr Arg Gly Leu Asp Phe Ala
                325                 330                 335
Cys Asp Ile Ile Ser Phe Phe Leu Ala Leu Thr Ser Thr Ala Leu Leu
            340                 345                 350
Phe Leu Leu Phe Phe Leu Thr Leu Arg Phe Ser Val Val Lys Arg Gly
        355                 360                 365
Arg Lys Lys Leu Leu Tyr Ile Phe Lys Gln Pro Phe Met Arg Pro Val
    370                 375                 380
Gln Thr Thr Gln Glu Glu Asp Gly Cys Ser Cys Arg Phe Pro Glu Glu
385                 390                 395                 400
Glu Glu Gly Gly Cys Glu Leu Thr Arg Arg Val Lys Phe Ser Arg Ser
                405                 410                 415
Ala Asp Ala Pro Ala Tyr Gln Gln Gly Gln Asn Gln Leu Tyr Asn Glu
            420                 425                 430
Leu Asn Leu Gly Arg Arg Glu Glu Tyr Asp Val Leu Asp Lys Arg Arg
        435                 440                 445
Gly Arg Asp Pro Glu Met Gly Gly Lys Pro Arg Arg Lys Asn Pro Gln

```
        450                 455                 460
Glu Gly Leu Tyr Asn Glu Leu Gln Lys Asp Lys Met Ala Glu Ala Tyr
465                 470                 475                 480
Ser Glu Ile Gly Met Lys Gly Glu Arg Arg Arg Gly Lys Gly His Asp
                485                 490                 495
Gly Leu Tyr Gln Gly Leu Ser Thr Ala Thr Lys Asp Thr Tyr Asp Ala
            500                 505                 510
Leu His Met Gln Ala Leu Pro Pro Arg
        515                 520
```

<210> 70
<211> 2355
<212> DNA
<213> Artificial Sequence

<220>
<223> CAR

<400> 70

```
atggctctgc ctgtgacagc tctgctgctg cctctggctc tgcttcttca tgccgccaga      60

ccatctgagg tgcagctagt tgaatctggt ggaggcctgg ttcagcctgg tggctctctg     120

agactgtctt gtgccgccag cggcatcgac ttcaacagca actactacat gtgctgggtc     180

cgacaggccc ccggcaaagg acttgaatgg attggctgca tctacgtggg cagccatgtg     240

aacacctact acgcaaactg ggccaagggc agattcacca tcagccggga caacagcaag     300

aacaccgtgt acctgcagat gaacagcctg agagcagaag atactgccgt gtactactgt     360

gccacatctg gcagcagcgt gctgtacttc aagttctggg gccagggcac cctggtcaca     420

gtttcttcag gaggtggagg ctctggcggt ggaggaagtg gtgggggagg ctctgacatc     480

cagatgacac agagccccag cagcctgtct gcctctgtgg gagacagagt gaccatcact     540

tgccaggcca gcgagaacat ctacagcttc ctggcctggt accagcagaa gccaggcaag     600

gcccctaagc tgctgatcta cagcgcctct aaactagctg ccggggtgcc aagcagattt     660

tctggctctg gcagcggcac cgacttcacc ctgaccatat caagcctgca gcctgaggac     720

ttcgccacct actactgcca gcagaccaac aggtacagca accccgacat ctacaacgtg     780

tttggacagg gcaccaagct gacagtgctt ggaagcgccc tgagcaacag catcatgtac     840

ttcagccact tcgtgcccgt gtttctgccc gccaaaccta caacaacacc tgctcctcgg     900

cctcctacac cagctcctac aattgccagc cagccactgt ctctgaggcc cgaagcttgc     960

aggcctgctg ctggcggagc cgtgcataca agaggactgg atttcgcctg cgacatcatc    1020

tccttctttc ttgcgctgac ctcgactgcg ttgctcttcc tgctgttctt cctcacgctc    1080

cgtttctctg ttgttaagcg gggcagaaag aaactgctct acatcttcaa gcagcccttc    1140

atgcggcccg tgcagaccac acaagaggaa gatggctgct cctgcagatt ccccgaggaa    1200

gaagaaggcg gctgcgagct gacgcgtaga gtgaagttca gcagatccgc cgacgctcct    1260

gcctatcagc agggccaaaa ccagctgtac aacgagctga acctggggag aagagaagag    1320
```

```
tacgacgtgc tggacaagcg gagaggcaga gatcctgaaa tgggcggcaa gcccagacgg    1380
aagaatcctc aagagggcct gtataatgag ctgcagaaag acaagatggc cgaggcctac    1440
agcgagatcg gaatgaaggg cgagcgcaga agaggcaagg gacacgatgg actgtaccag    1500
ggcctgagca ccgccaccaa ggatacctat gatgccctgc acatgcaggc cctgcctcca    1560
agagctagcg gaagcggagc tactaacttc agcctgctga agcaggctgg agacgtggag    1620
gagaaccctg gacctatggt gagcaagggc gaggagctgt tcaccggggt ggtgcccatc    1680
ctggtcgagc tggacggcga cgtaaacggc cacaagttca gcgtgtccgg cgagggcgag    1740
ggcgatgcca cctacggcaa gctgaccctg aagttcatct gcaccaccgg caagctgccc    1800
gtgccctggc ccaccctcgt gaccaccctg acctacggcg tgcagtgctt cagccgctac    1860
cccgaccaca tgaagcagca cgacttcttc aagtccgcca tgcccgaagg ctacgtccag    1920
gagcgcacca tcttcttcaa ggacgacggc aactacaaga cccgcgccga ggtgaagttc    1980
gagggcgaca ccctggtgaa ccgcatcgag ctgaagggca tcgacttcaa ggaggacggc    2040
aacatcctgg ggcacaagct ggagtacaac tacaacagcc acaacgtcta tatcatggcc    2100
gacaagcaga agaacggcat caaggtgaac ttcaagatcc gccacaacat cgaggacggc    2160
agcgtgcagc tcgccgacca ctaccagcag aacaccccca tcggcgacgg ccccgtgctg    2220
ctgcccgaca accactacct gagcacccag tccgccctga gcaaagaccc caacgagaag    2280
cgcgatcaca tggtcctgct ggagttcgtg accgccgccg ggatcactct cggcatggac    2340
gagctgtaca agtaa                                                     2355
```

<210> 71
<211> 784
<212> PRT
<213> Artificial Sequence

<220>
<223> CAR

<400> 71

```
Met Ala Leu Pro Val Thr Ala Leu Leu Leu Pro Leu Ala Leu Leu Leu
1               5                   10                  15
His Ala Ala Arg Pro Ser Glu Val Gln Leu Val Glu Ser Gly Gly Gly
            20                  25                  30
Leu Val Gln Pro Gly Gly Ser Leu Arg Leu Ser Cys Ala Ala Ser Gly
        35                  40                  45
Ile Asp Phe Asn Ser Asn Tyr Tyr Met Cys Trp Val Arg Gln Ala Pro
    50                  55                  60
Gly Lys Gly Leu Glu Trp Ile Gly Cys Ile Tyr Val Gly Ser His Val
65                  70                  75                  80
Asn Thr Tyr Tyr Ala Asn Trp Ala Lys Gly Arg Phe Thr Ile Ser Arg
                85                  90                  95
Asp Asn Ser Lys Asn Thr Val Tyr Leu Gln Met Asn Ser Leu Arg Ala
            100                 105                 110
Glu Asp Thr Ala Val Tyr Tyr Cys Ala Thr Ser Gly Ser Ser Val Leu
```

```
        115                 120                 125
Tyr Phe Lys Phe Trp Gly Gln Gly Thr Leu Val Thr Val Ser Ser Gly
    130                 135                 140
Gly Gly Gly Ser Gly Gly Gly Gly Ser Gly Gly Gly Gly Ser Asp Ile
145                 150                 155                 160
Gln Met Thr Gln Ser Pro Ser Ser Leu Ser Ala Ser Val Gly Asp Arg
                165                 170                 175
Val Thr Ile Thr Cys Gln Ala Ser Glu Asn Ile Tyr Ser Phe Leu Ala
            180                 185                 190
Trp Tyr Gln Gln Lys Pro Gly Lys Ala Pro Lys Leu Leu Ile Tyr Ser
        195                 200                 205
Ala Ser Lys Leu Ala Ala Gly Val Pro Ser Arg Phe Ser Gly Ser Gly
    210                 215                 220
Ser Gly Thr Asp Phe Thr Leu Thr Ile Ser Ser Leu Gln Pro Glu Asp
225                 230                 235                 240
Phe Ala Thr Tyr Tyr Cys Gln Gln Thr Asn Arg Tyr Ser Asn Pro Asp
                245                 250                 255
Ile Tyr Asn Val Phe Gly Gln Gly Thr Lys Leu Thr Val Leu Gly Ser
            260                 265                 270
Ala Leu Ser Asn Ser Ile Met Tyr Phe Ser His Phe Val Pro Val Phe
        275                 280                 285
Leu Pro Ala Lys Pro Thr Thr Thr Pro Ala Pro Arg Pro Pro Thr Pro
    290                 295                 300
Ala Pro Thr Ile Ala Ser Gln Pro Leu Ser Leu Arg Pro Glu Ala Cys
305                 310                 315                 320
Arg Pro Ala Ala Gly Gly Ala Val His Thr Arg Gly Leu Asp Phe Ala
                325                 330                 335
Cys Asp Ile Ile Ser Phe Phe Leu Ala Leu Thr Ser Thr Ala Leu Leu
            340                 345                 350
Phe Leu Leu Phe Phe Leu Thr Leu Arg Phe Ser Val Val Lys Arg Gly
        355                 360                 365
Arg Lys Lys Leu Leu Tyr Ile Phe Lys Gln Pro Phe Met Arg Pro Val
    370                 375                 380
Gln Thr Thr Gln Glu Glu Asp Gly Cys Ser Cys Arg Phe Pro Glu Glu
385                 390                 395                 400
Glu Glu Gly Gly Cys Glu Leu Thr Arg Arg Val Lys Phe Ser Arg Ser
                405                 410                 415
Ala Asp Ala Pro Ala Tyr Gln Gln Gly Gln Asn Gln Leu Tyr Asn Glu
            420                 425                 430
Leu Asn Leu Gly Arg Arg Glu Glu Tyr Asp Val Leu Asp Lys Arg Arg
        435                 440                 445
Gly Arg Asp Pro Glu Met Gly Gly Lys Pro Arg Arg Lys Asn Pro Gln
    450                 455                 460
Glu Gly Leu Tyr Asn Glu Leu Gln Lys Asp Lys Met Ala Glu Ala Tyr
465                 470                 475                 480
Ser Glu Ile Gly Met Lys Gly Glu Arg Arg Arg Gly Lys Gly His Asp
                485                 490                 495
Gly Leu Tyr Gln Gly Leu Ser Thr Ala Thr Lys Asp Thr Tyr Asp Ala
            500                 505                 510
Leu His Met Gln Ala Leu Pro Pro Arg Ala Ser Gly Ser Gly Ala Thr
        515                 520                 525
Asn Phe Ser Leu Leu Lys Gln Ala Gly Asp Val Glu Glu Asn Pro Gly
    530                 535                 540
Pro Met Val Ser Lys Gly Glu Glu Leu Phe Thr Gly Val Val Pro Ile
545                 550                 555                 560
Leu Val Glu Leu Asp Gly Asp Val Asn Gly His Lys Phe Ser Val Ser
                565                 570                 575
Gly Glu Gly Glu Gly Asp Ala Thr Tyr Gly Lys Leu Thr Leu Lys Phe
            580                 585                 590
Ile Cys Thr Thr Gly Lys Leu Pro Val Pro Trp Pro Thr Leu Val Thr
        595                 600                 605
Thr Leu Thr Tyr Gly Val Gln Cys Phe Ser Arg Tyr Pro Asp His Met
    610                 615                 620
```

```
Lys Gln His Asp Phe Phe Lys Ser Ala Met Pro Glu Gly Tyr Val Gln
625                 630                 635                 640
Glu Arg Thr Ile Phe Phe Lys Asp Asp Gly Asn Tyr Lys Thr Arg Ala
                645                 650                 655
Glu Val Lys Phe Glu Gly Asp Thr Leu Val Asn Arg Ile Glu Leu Lys
            660                 665                 670
Gly Ile Asp Phe Lys Glu Asp Gly Asn Ile Leu Gly His Lys Leu Glu
        675                 680                 685
Tyr Asn Tyr Asn Ser His Asn Val Tyr Ile Met Ala Asp Lys Gln Lys
    690                 695                 700
Asn Gly Ile Lys Val Asn Phe Lys Ile Arg His Asn Ile Glu Asp Gly
705                 710                 715                 720
Ser Val Gln Leu Ala Asp His Tyr Gln Gln Asn Thr Pro Ile Gly Asp
                725                 730                 735
Gly Pro Val Leu Leu Pro Asp Asn His Tyr Leu Ser Thr Gln Ser Ala
            740                 745                 750
Leu Ser Lys Asp Pro Asn Glu Lys Arg Asp His Met Val Leu Leu Glu
        755                 760                 765
Phe Val Thr Ala Ala Gly Ile Thr Leu Gly Met Asp Glu Leu Tyr Lys
    770                 775                 780
```

<210> 72
<211> 1560
<212> DNA
<213> Artificial Sequence

<220>
<223> CAR

<400> 72

```
atggctctgc ctgtgacagc tctgctgctg cctctggctc tgcttcttca tgccgccaga      60
ccatctgagg tgcagctagt tgaatctggt ggaggcctgg ttcagcctgg tggctctctg     120
agactgtctt gtgccgccag cggcatcgac ttcaacagca actactacat gtgctgggtc     180
cgacaggccc ccggcaaagg acttgaatgg attggctgca tctacgtggg cagccatgtg     240
aacacctact acgcaaactg ggccaagggc agattcacca tcagccggga caacagcaag     300
aacaccgtgt acctgcagat gaacagcctg agagcagaag atactgccgt gtactactgt     360
gccacatctg gcagcagcgt gctgtacttc aagttctggg gccagggcac cctggtcaca     420
gtttcttcag gaggtggagg ctctggcggt ggaggaagtg gtgggggagg ctctgacatc     480
cagatgacac agagccccag cagcctgtct gcctctgtgg gagacagagt gaccatcact     540
tgccaggcca gcgagaacat ctacagcttc ctggcctggt accagcagaa gccaggcaag     600
gcccctaagc tgctgatcta cagcgcctct aaactagctg ccggggtgcc aagcagattt     660
tctggctctg gcagcggcac cgacttcacc ctgaccatat caagcctgca gcctgaggac     720
ttcgccacct actactgcca gcagaccaac aggtacagca accccgacat ctacaacgtg     780
tttggacagg gcaccaagct gacagtgctt ggaagcgccc tgagcaacag catcatgtac     840
ttcagccact tcgtgcccgt gtttctgccc gccaaaccta caacaacacc tgctcctcgg     900
```

```
cctcctacac cagctcctac aattgccagc cagccactgt ctctgaggcc cgaagcttgc     960

aggcctgctg ctggcggagc cgtgcataca agaggactgg atttcgcctg cgacatcatc    1020

tccttctttc ttgcgctgac ctcgactgcg ttgctcttcc tgctgttctt cctcacgctc    1080

cgtttctctg ttgttaagcg gggcagaaag aaactgctct acatcttcaa gcagcccttc    1140

atgcggcccg tgcagaccac acaagaggaa gatggctgct cctgcagatt ccccgaggaa    1200

gaagaaggcg gctgcgagct gagagtgaag ttcagcagat ccgccgacgc tcctgcctat    1260

cagcagggcc aaaaccagct ctacaacgag ctgaacctgg ggagaagaga agagtacgac    1320

gtgctggaca agcggagagg cagagatcct gaaatgggcg gcaagcccag acggaagaat    1380

cctcaagagg gcctgtataa tgagctacag aaagacaaga tggcagaggc ctacagcgag    1440

atcggaatga agggcgagcg cagaagaggc aagggacacg atggactgta ccagggcctg    1500

agcaccgcca ccaaggatac ctatgatgcc ctgcacatgc aggccctgcc tccaagatag    1560
```

<210> 73
<211> 519
<212> PRT
<213> Artificial Sequence

<220>
<223> CAR

<400> 73
```
Met Ala Leu Pro Val Thr Ala Leu Leu Leu Pro Leu Ala Leu Leu Leu
1               5                   10                  15
His Ala Ala Arg Pro Ser Glu Val Gln Leu Val Glu Ser Gly Gly Gly
            20                  25                  30
Leu Val Gln Pro Gly Gly Ser Leu Arg Leu Ser Cys Ala Ala Ser Gly
        35                  40                  45
Ile Asp Phe Asn Ser Asn Tyr Tyr Met Cys Trp Val Arg Gln Ala Pro
    50                  55                  60
Gly Lys Gly Leu Glu Trp Ile Gly Cys Ile Tyr Val Gly Ser His Val
65                  70                  75                  80
Asn Thr Tyr Tyr Ala Asn Trp Ala Lys Gly Arg Phe Thr Ile Ser Arg
                85                  90                  95
Asp Asn Ser Lys Asn Thr Val Tyr Leu Gln Met Asn Ser Leu Arg Ala
            100                 105                 110
Glu Asp Thr Ala Val Tyr Tyr Cys Ala Thr Ser Gly Ser Ser Val Leu
        115                 120                 125
Tyr Phe Lys Phe Trp Gly Gln Gly Thr Leu Val Thr Val Ser Ser Gly
    130                 135                 140
Gly Gly Gly Ser Gly Gly Gly Gly Ser Gly Gly Gly Gly Ser Asp Ile
145                 150                 155                 160
Gln Met Thr Gln Ser Pro Ser Ser Leu Ser Ala Ser Val Gly Asp Arg
                165                 170                 175
Val Thr Ile Thr Cys Gln Ala Ser Glu Asn Ile Tyr Ser Phe Leu Ala
            180                 185                 190
Trp Tyr Gln Gln Lys Pro Gly Lys Ala Pro Lys Leu Leu Ile Tyr Ser
        195                 200                 205
Ala Ser Lys Leu Ala Ala Gly Val Pro Ser Arg Phe Ser Gly Ser Gly
    210                 215                 220
Ser Gly Thr Asp Phe Thr Leu Thr Ile Ser Ser Leu Gln Pro Glu Asp
225                 230                 235                 240
```

Phe Ala Thr Tyr Tyr Cys Gln Gln Thr Asn Arg Tyr Ser Asn Pro Asp
245 250 255
Ile Tyr Asn Val Phe Gly Gln Gly Thr Lys Leu Thr Val Leu Gly Ser
260 265 270
Ala Leu Ser Asn Ser Ile Met Tyr Phe Ser His Phe Val Pro Val Phe
275 280 285
Leu Pro Ala Lys Pro Thr Thr Thr Pro Ala Pro Arg Pro Pro Thr Pro
290 295 300
Ala Pro Thr Ile Ala Ser Gln Pro Leu Ser Leu Arg Pro Glu Ala Cys
305 310 315 320
Arg Pro Ala Ala Gly Gly Ala Val His Thr Arg Gly Leu Asp Phe Ala
325 330 335
Cys Asp Ile Ile Ser Phe Phe Leu Ala Leu Thr Ser Thr Ala Leu Leu
340 345 350
Phe Leu Leu Phe Phe Leu Thr Leu Arg Phe Ser Val Val Lys Arg Gly
355 360 365
Arg Lys Lys Leu Leu Tyr Ile Phe Lys Gln Pro Phe Met Arg Pro Val
370 375 380
Gln Thr Thr Gln Glu Glu Asp Gly Cys Ser Cys Arg Phe Pro Glu Glu
385 390 395 400
Glu Glu Gly Gly Cys Glu Leu Arg Val Lys Phe Ser Arg Ser Ala Asp
405 410 415
Ala Pro Ala Tyr Gln Gln Gly Gln Asn Gln Leu Tyr Asn Glu Leu Asn
420 425 430
Leu Gly Arg Arg Glu Glu Tyr Asp Val Leu Asp Lys Arg Arg Gly Arg
435 440 445
Asp Pro Glu Met Gly Gly Lys Pro Arg Arg Lys Asn Pro Gln Glu Gly
450 455 460
Leu Tyr Asn Glu Leu Gln Lys Asp Lys Met Ala Glu Ala Tyr Ser Glu
465 470 475 480
Ile Gly Met Lys Gly Glu Arg Arg Arg Gly Lys Gly His Asp Gly Leu
485 490 495
Tyr Gln Gly Leu Ser Thr Ala Thr Lys Asp Thr Tyr Asp Ala Leu His
500 505 510
Met Gln Ala Leu Pro Pro Arg
515

<210> 74
<211> 250
<212> PRT
<213> Artificial Sequence

<220>
<223> CAR

<400> 74
Ser Ala Leu Ser Asn Ser Ile Met Tyr Phe Ser His Phe Val Pro Val
1 5 10 15
Phe Leu Pro Ala Lys Pro Thr Thr Thr Pro Ala Pro Arg Pro Pro Thr
20 25 30
Pro Ala Pro Thr Ile Ala Ser Gln Pro Leu Ser Leu Arg Pro Glu Ala
35 40 45
Cys Arg Pro Ala Ala Gly Gly Ala Val His Thr Arg Gly Leu Asp Phe
50 55 60
Ala Cys Asp Ile Ile Ser Phe Phe Leu Ala Leu Thr Ser Thr Ala Leu
65 70 75 80
Leu Phe Leu Leu Phe Phe Leu Thr Leu Arg Phe Ser Val Val Lys Arg
85 90 95
Gly Arg Lys Lys Leu Leu Tyr Ile Phe Lys Gln Pro Phe Met Arg Pro
100 105 110
Val Gln Thr Thr Gln Glu Glu Asp Gly Cys Ser Cys Arg Phe Pro Glu
115 120 125

```
Glu Glu Glu Gly Gly Cys Glu Leu Thr Arg Arg Val Lys Phe Ser Arg
    130                 135                 140
Ser Ala Asp Ala Pro Ala Tyr Gln Gln Gly Gln Asn Gln Leu Tyr Asn
145                 150                 155                 160
Glu Leu Asn Leu Gly Arg Arg Glu Glu Tyr Asp Val Leu Asp Lys Arg
                165                 170                 175
Arg Gly Arg Asp Pro Glu Met Gly Gly Lys Pro Arg Arg Lys Asn Pro
            180                 185                 190
Gln Glu Gly Leu Tyr Asn Glu Leu Gln Lys Asp Lys Met Ala Glu Ala
        195                 200                 205
Tyr Ser Glu Ile Gly Met Lys Gly Glu Arg Arg Arg Gly Lys Gly His
    210                 215                 220
Asp Gly Leu Tyr Gln Gly Leu Ser Thr Ala Thr Lys Asp Thr Tyr Asp
225                 230                 235                 240
Ala Leu His Met Gln Ala Leu Pro Pro Arg
                245                 250
```

<210> 75
<211> 750
<212> DNA
<213> Artificial Sequence

<220>
<223> CAR

<400> 75
```
agcgccctga gcaacagcat catgtacttc agccacttcg tgcccgtgtt tctgcccgcc      60

aaacctacaa caacacctgc tcctcggcct cctacaccag ctcctacaat tgccagccag     120

ccactgtctc tgaggcccga agcttgcagg cctgctgctg gcggagccgt gcatacaaga     180

ggactggatt tcgcctgcga catcatctcc ttctttcttg cgctgacctc gactgcgttg     240

ctcttcctgc tgttcttcct cacgctccgt ttctctgttg ttaagcgggg cagaaagaaa     300

ctgctctaca tcttcaagca gcccttcatg cggcccgtgc agaccacaca agaggaagat     360

ggctgctcct gcagattccc cgaggaagaa gaaggcggct gcgagctgac gcgtagagtg     420

aagttcagca gatccgccga cgctcctgcc tatcagcagg gccaaaacca gctgtacaac     480

gagctgaacc tggggagaag agaagagtac gacgtgctgg acaagcggag aggcagagat     540

cctgaaatgg gcggcaagcc cagacggaag aatcctcaag agggcctgta taatgagctg     600

cagaaagaca agatggccga ggcctacagc gagatcggaa tgaagggcga gcgcagaaga     660

ggcaagggac acgatggact gtaccagggc ctgagcaccg ccaccaagga tacctatgat     720

gccctgcaca tgcaggccct gcctccaaga                                      750
```

<210> 76
<211> 248
<212> PRT
<213> Artificial Sequence

<220>
<223> CAR

<400> 76
Ser Ala Leu Ser Asn Ser Ile Met Tyr Phe Ser His Phe Val Pro Val
1               5                   10                  15
Phe Leu Pro Ala Lys Pro Thr Thr Thr Pro Ala Pro Arg Pro Pro Thr
            20                  25                  30
Pro Ala Pro Thr Ile Ala Ser Gln Pro Leu Ser Leu Arg Pro Glu Ala
        35                  40                  45
Cys Arg Pro Ala Ala Gly Gly Ala Val His Thr Arg Gly Leu Asp Phe
    50                  55                  60
Ala Cys Asp Ile Ile Ser Phe Phe Leu Ala Leu Thr Ser Thr Ala Leu
65                  70                  75                  80
Leu Phe Leu Leu Phe Phe Leu Thr Leu Arg Phe Ser Val Val Lys Arg
                85                  90                  95
Gly Arg Lys Lys Leu Leu Tyr Ile Phe Lys Gln Pro Phe Met Arg Pro
            100                 105                 110
Val Gln Thr Thr Gln Glu Glu Asp Gly Cys Ser Cys Arg Phe Pro Glu
        115                 120                 125
Glu Glu Glu Gly Gly Cys Glu Leu Arg Val Lys Phe Ser Arg Ser Ala
    130                 135                 140
Asp Ala Pro Ala Tyr Gln Gln Gly Gln Asn Gln Leu Tyr Asn Glu Leu
145                 150                 155                 160
Asn Leu Gly Arg Arg Glu Glu Tyr Asp Val Leu Asp Lys Arg Arg Gly
                165                 170                 175
Arg Asp Pro Glu Met Gly Gly Lys Pro Arg Arg Lys Asn Pro Gln Glu
            180                 185                 190
Gly Leu Tyr Asn Glu Leu Gln Lys Asp Lys Met Ala Glu Ala Tyr Ser
        195                 200                 205
Glu Ile Gly Met Lys Gly Glu Arg Arg Arg Gly Lys Gly His Asp Gly
    210                 215                 220
Leu Tyr Gln Gly Leu Ser Thr Ala Thr Lys Asp Thr Tyr Asp Ala Leu
225                 230                 235                 240
His Met Gln Ala Leu Pro Pro Arg
                245

<210> 77
<211> 747
<212> DNA
<213> Artificial Sequence

<220>
<223> CAR

<400> 77
agcgccctga gcaacagcat catgtacttc agccacttcg tgcccgtgtt tctgcccgcc 60

aaacctacaa caacacctgc tcctcggcct cctacaccag ctcctacaat tgccagccag 120

ccactgtctc tgaggcccga agcttgcagg cctgctgctg gcggagccgt gcatacaaga 180

ggactggatt tcgcctgcga catcatctcc ttctttcttg cgctgacctc gactgcgttg 240

ctcttcctgc tgttcttcct cacgctccgt ttctctgttg ttaagcgggg cagaaagaaa 300

ctgctctaca tcttcaagca gcccttcatg cggcccgtgc agaccacaca agaggaagat 360

ggctgctcct gcagattccc cgaggaagaa gaaggcggct gcgagctgag agtgaagttc 420

agcagatccg ccgacgctcc tgcctatcag cagggccaaa accagctcta caacgagctg 480

aacctgggga gaagagaaga gtacgacgtg ctggacaagc ggagaggcag agatcctgaa 540

atgggcggca agcccagacg gaagaatcct caagagggcc tgtataatga gctacagaaa 600

gacaagatgg cagaggccta cagcgagatc ggaatgaagg gcgagcgcag aagaggcaag 660

ggacacgatg gactgtacca gggcctgagc accgccacca aggataccta tgatgccctg 720

cacatgcagg ccctgcctcc aagatag 747

<210> 78
<211> 336
<212> DNA
<213> Artificial Sequence

<220>
<223> CD3zeta-derived intracellular domain

<400> 78
agagtgaagt tcagcaggag cgcagacgcc cccgcgtacc agcagggcca gaaccagctc 60

tataacgagc tcaatctagg acgaagagag gagtacgatg ttttggacaa gagacgtggc 120

cgggaccctg agatgggggg aaagccgaga aggaagaacc ctcaggaagg cctgtacaat 180

gaactgcaga aagataagat ggcggaggcc tacagtgaga ttgggatgaa aggcgagcgc 240

cggaggggca aggggcacga tggcctttac cagggtctca gtacagccac caaggacacc 300

tacgacgccc ttcacatgca ggccctgccc cctcgc 336

<210> 79
<211> 126
<212> DNA
<213> Artificial Sequence

<220>
<223> 4-1BB-derived intracellular domain

<400> 79
aaacggggca gaaagaaact cctgtatata ttcaaacaac catttatgag accagtacaa 60

actactcaag aggaagatgg ctgtagctgc cgatttccag aagaagaaga aggaggatgt 120

gaactg 126

<210> 80
<211> 21
<212> PRT
<213> Artificial Sequence

<220>
<223> CD8-derived Leader sequence

<400> 80
Met Ala Leu Pro Val Thr Ala Leu Leu Leu Pro Leu Ala Leu Leu Leu
1 5 10 15
His Ala Ala Arg Pro
20

<210> 81
<211> 135
<212> DNA
<213> Artificial Sequence

<220>
<223> CD8-derived hinge domain

<400> 81
accacgacgc cagcgccgcg accaccaaca ccggcgccca ccatcgcgtc gcagcccctg 60

tccctgcgcc cagaggcgtg ccggccagcg gcggggggcg cagtgcacac gagggggctg 120

gacttcgcct gtgat 135

<210> 82
<211> 4
<212> PRT
<213> Artificial Sequence

<220>
<223> Cys2His2

<400> 82
Cys Cys His His
1

<210> 83
<211> 135
<212> DNA
<213> Artificial Sequence

<220>
<223> CD8-derived hinge domain

<400> 83
acaacaaccc ctgctcctcg gcctcctaca ccagctccta caattgccag ccagccactg 60

tctctgaggc ccgaagcttg tagacctgct gctggcggag ccgtgcatac aagaggactg 120

gatttcgcct gcgac 135

<210> 84
<211> 123
<212> DNA
<213> Artificial Sequence

<220>
<223> CD28-derived intracellular domain

<400> 84
cggagcaaga gaagcagact gctgcacagc gactacatga acatgacccc tagacggccc 60

ggacctacca gaaagcacta ccagccttac gctcctccta gagacttcgc cgcctacaga 120

tcc 123

<210> 85
<211> 336
<212> DNA
<213> Artificial Sequence

<220>
<223> CD3zeta-derived intracellular domain

<400> 85
agagtgaagt tcagcagatc cgccgacgct cctgcctatc agcagggcca aaaccagctc 60

tacaacgagc tgaacctggg gagaagagaa gagtacgacg tgctggacaa gcggagaggc 120

agagatcctg aaatgggcgg caagcccaga cggaagaatc ctcaagaggg cctgtataat 180

gagctacaga aagacaagat ggcagaggcc tacagcgaga tcggaatgaa gggcgagcgc 240

agaagaggca agggacacga tggactgtac cagggcctga gcaccgccac caaggatacc 300

tatgatgccc tgcacatgca ggccctgcct ccaaga 336

<210> 86
<211> 339
<212> DNA
<213> Artificial Sequence

<220>
<223> CD3zeta-derived intracellular domain

<400> 86
agagtgaagt tcagcagatc cgccgacgct cctgcctatc agcagggcca aaaccagctc 60

tacaacgagc tgaacctggg gagaagagaa gagtacgacg tgctggacaa gcggagaggc 120

agagatcctg aaatgggcgg caagcccaga cggaagaatc ctcaagaggg cctgtataat 180

gagctacaga aagacaagat ggcagaggcc tacagcgaga tcggaatgaa gggcgagcgc 240

agaagaggca agggacacga tggactgtac cagggcctga gcaccgccac caaggatacc 300

tatgatgccc tgcacatgca ggccctgcct ccaagatag 339